# EUROPEAN PATENT APPLICATION

(11) **EP 2 484 762 A1**
(43) Date of publication of application: **08.08.2012**
(21) Application number: 10820647.5
(22) Date of filing: 30.09.2010
(51) Int. Cl.: C12N 15/09, C12M 1/00, C12Q 1/68

(54) **METHOD FOR PREDICTING THERAPEUTIC EFFECT OF IMMUNOTHERAPY ON CANCER PATIENT AND/OR PROGNOSIS AFTER IMMUNOTHERAPY, AND GENE SET AND KIT TO BE USED THEREIN**

(30) Priority: 02.10.2009 JP 2009230279
(71) Applicant: Kurume University, Kurume-shi Fukuoka 830-0011 (JP)
(72) Inventor: ITOH, Kyogo, Kurume-shi Fukuoka 830-0011 (JP); NOGUCHI, Masanori, Kurume-shi Fukuoka 830-0011 (JP); YAMADA, Akira, Kurume-shi Fukuoka 830-0011 (JP); SHICHIJO, Shigeki, Kurume-shi Fukuoka 830-0011 (JP); KOMATSU, Nobukazu, Kurume-shi Fukuoka 830-0011 (JP)
(74) Representative: Vossius & Partner
(86) International application number: PCT/JP2010/067088
(87) International publication number: WO 2011/040532

(57) **Abstract**

The present invention provides a method for predicting effect of immunotherapy on a cancer patient and/or prognosis of the cancer patient after the immunotherapy, and a gene set and a kit for use in the method.

## Description

### Technical Field

The present invention provides a method for predicting effect of immunotherapy on a cancer patient and/or prognosis of the cancer patient after the immunotherapy, and a gene set and a kit for use in the method. The present application claims the benefit of Japanese patent application number 2009-230279 and the subject matter of which is hereby incorporated herein by reference.

### Background Art

Cancer immunotherapy is still not an optimal therapeutic option for all patients although it has been affective for some patients. One of the reasons is that immunotherapy relys on immunity which considerably varies among different individuals to suppress proliferation of cancer cells. At present, it is not possible to predict effect of cancer immunotherapy and thus effectiveness of the therapy cannot be determined unless the therapy has done. It has been know that effect of chemotherapy on a breast cancer patient is predicted by determination of gene expression level. However, this prediction method is complicated because it involves not only gene expression level but also other factors. In addition, this method only directs to chemotherapy on breast cancer (patent literature 1). So far, methods for predicting effect of cancer immunotherapy or prognosis of a patient after immunotherapy have not been known.
Patent literature: JP-A-2008-536094

### [Disclosure of Invention]

### [Problem to be Solved by the Invention]

An object of the present invention is to provide a method for predicting effect of immunotherapy on a cancer patient with accuracy.

### [Means of Solving the Problem]

Based on the results of peptide vaccine therapy on prostate cancer patients over the years, the present inventors tried to predict effect of cancer immunotherapy. First, gene expression profiles of prostate cancer patients before peptide vaccine therapy were analyzed with DNA micorarray. Then, the patients were classified into good prognosis group and poor prognosis group according to their survival time after the therapy, and correlation between expression level of each gene before the therapy and survival time after the therapy was analyzed. As a result, some genes were found to have correlation between the two. Finally, it was confirmed that survival time of a patient after immunotherapy could be predicted from expression level of those genes and the present invention has been accomplished.

Thus, the present invention provides a method for predicting effect of immunotherapy on a cancer patient and/or prognosis of the cancer patient after the immunotherapy, which comprises the steps of:
(1) determining expression level of each gene in a gene set which consists of at least 1 gene selected from the group of genes shown in any of Tables 1-5 and
(2) performing statistical processing of the expression level to calculate predicted survival time.

The present invention further provides a gene set for predicting effect of immunotherapy on a cancer patient and/or prognosis of the cancer patient after the immunotherapy, which consists of at least 1 gene selected from the group of genes shown in any of Tables 1-5.

The present invention further provides a kit for predicting effect of immunotherapy on a cancer patient and/or prognosis of the cancer patient after the immunotherapy, which comprises a probe or primer for each gene in the aforementioned gene set.

### Effect of the invention

According to the present invention, it has become possible to predict effect of immunotherapy on a cancer patient and/or prognosis of the cancer patient after the immunotherapy by determining gene expression profile of the cancer patient before initiation of the immunotherapy. The present invention can offer useful information in selecting therapeutic options for cancer patients.

### Brief Description of Drawings

[Fig. 1] Distribution of predicted survival time of 40 prostate cancer patients.
[Fig. 2] Pre-processing of microarray data.
[Fig. 3] Distribution of top 300 genes selected by Pearson product-moment correlation coefficient.
[Fig. 4] Accuracy of survival time prediction by different gene selection methods.
[Fig. 5] Accuracy of survival time prediction with 30 gene sets each consisting of genes selected in descending order from the top (the first gene) of top 300 genes selected by Pearson product-moment correlation coefficient (Table 1). The vertical axis represents number of patients correctly predicted.
[Fig. 6] Comparison between predicted survival time (calculated with the gene set consisting of top 50 genes selected by Pearson product-moment correlation coefficient, latent variable 3) and actual survival time of each patient.
[Fig. 7] Kaplan-Meier curve prepared according to the results of survival time prediction.
[Fig. 8] Accuracy of survival time prediction with 30 gene sets each consisting of genes selected in ascending order from the bottom (the 300th gene) of top 300 genes selected by Pearson product-moment correlation coefficient (Table 1). The vertical axis represents number of patients correctly predicted.
[Fig. 9] Accuracy of survival time prediction with 30 gene sets each consisting of genes randomly selected from top 300 genes selected by Pearson product-moment correlation coefficient (Table 1). The vertical axis represents number of patients correctly predicted.
[Fig. 10] Results of survival time prediction with good prognosis group (survival time was 300 days or more) and poor prognosis group (survival time was less than 300 days).
[Fig. 11-1] Results of survival time prediction with one gene randomly selected from top 300 genes selected by Pearson product-moment correlation coefficient (Table 1) (1) (the rank in Table 1, is shown in the left of each graph).
[Fig. 11-2] Results of survival time prediction with one gene randomly selected from top 300 genes selected by Pearson product-moment correlation coefficient (Table 1) (2) (the rank in Table 1 is shown in the left of each graph).
[Fig. 11-3] Results of survival time prediction with one gene randomly selected from top 300 genes selected by Pearson product-moment correlation coefficient (Table 1) (3) (the rank in Table 1 is shown in the left of each graph).

### Description of Embodiments

The prediction method of the present invention uses a gene or genes of which expression levels closely correlate survival time of a cancer patient after immunotherapy. The gene set of the present invention consists of at least 1 gene selected from the group of genes shown in any of Tables 1-5. The genes may be selected from any of Tables 1-5 in descending order from the top (the first gene), in ascending order from the bottom (the 300th gene), or randomly. In view of prediction accuracy, the genes are preferred to be selected in descending order from the top of any of the tables.

Number of genes in a gene set is not specifically limited and may be 1 or more. For example, for a gene set, 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 genes may be selected.

The cancer patient to be treated by the present invention may be, but not limited to, a cancer patient of prostate cancer, pancreatic cancer, breast cancer, or liver cancer. Particularly, the present invention is suitable for a prostate cancer patient.

In the present invention, immunotherapy refers to a therapeutic method for treating cancer by stimulating immune response in a cancer patient against a tumor antigen protein. The immunotherapy of the present invention may include peptide vaccine therapy which uses a tumor antigen peptide, adoptive immunotherapy which uses lymphocytes such as cytotoxic T cells or natural killer cells, DNA vaccine therapy wherein a virus vector expressing a tumor antigen protein or peptide is introduced into a living body, and dendritic cell vaccine therapy wherein dendritic cells presenting a tumor antigen peptide are administered. Particularly, the present invention is suitable for peptide vaccine therapy.

In the present invention, expression level of a gene may be determined by any conventional method. Methods for determining expression level of a gene may include DNA microarray analysis, DNA chip analysis, PCR analysis, and Northern blot analysis, and particularly, DNA microarray analysis is suitable for the present invention.

DNA microarray analysis uses a microarray containing a probe for a gene to be determined. The microarray may be HumanWG-6 v3.0 Expression BeadChip (llumina). Alternatively, it is possible to prepare any desired microarray by synthesizing a probe for a gene to be determined and immobilizing the probe on a suitable substrate such as a slide glass. Methods for preparing a microarray are well known in the art. Analysis of microarray data is also well known in the art and may be performed according to MICROARRAYS FOR AN INTEGRATIVE GENOMICS, translated by Yujin Hoshida, Springer-Verlag Tokyo, for example.

A patient-derived sample used for determination of expression level of a gene may be, but not limited to, peripheral blood collected from a patient.

Expression level of a gene may be determined by DNA microarray analysis as follows. Briefly, total RNA is extracted and purified from peripheral blood of a patient. Then, biotinated cRNA is synthesized by using, for example, Illumina TotalPrep RNA Amplification Kit (Ambion). The biotinated cRNA is hybridized to a microarray and reacted with Cy3-labeled streptavidin. The resulting microarray is scanned by a dedicated scanner and Cy3 fluorescence of each spot is quantified by a dedicated software such as BeadStudio. Accordingly, expression level of the gene may be determined.

Predicated survival time of a patient may be calculated, for example, by applying the expression level of each gene of the gene set of the present invention in the patient into the regression equation of PLS regression analysis as shown in the following examples. Alternatively, it may be calculated by determining expression level of each gene of any gene set of the present invention in the patient group of the examples or other patient groups and performing statistical processing by SVM (Support Vector Machine), Regularized Least Squares, or Principal Component Analysis.

The gene set of the present invention is used for predicting effect of immunotherapy on a cancer patient and/or prognosis of the cancer patient after the immunotherapy and may be used for preparing a probe for DNA microarray or a primer for PCR for use in the prediction method of the present invention.

The kit of the present invention comprises a probe or primer which is capably of determining expression level of each gene of the gene set of the present invention. The probe or primer for each gene may be synthesized by a conventional method based on the sequence of the gene. The kit may comprise other reagents required for determination of expression level. The kit of the present invention may be used for DNA microarray analysis, DNA chip analysis, PCR analysis, and Northern blot analysis. A kit for DNA microarray analysis may comprise a microarray containing the probe as described above immobilized on a suitable substrate.

### Examples

### Example 1

### 1. Analysis of gene expression profile before peptide vaccine therapy using DNA microarray

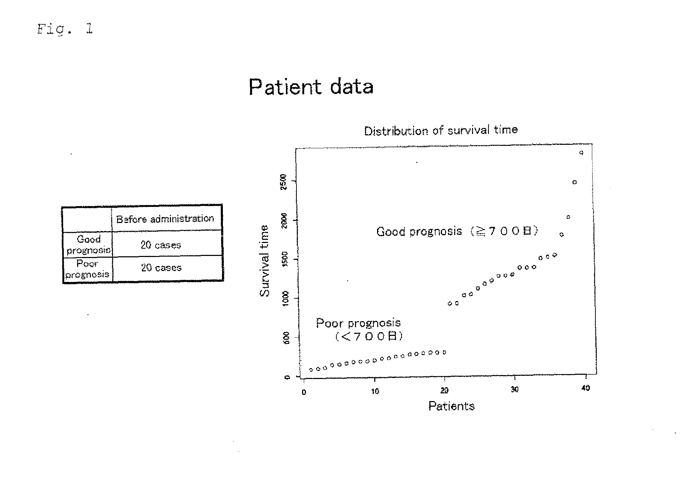

Patient-derived samples were peripheral blood which had been collected from prostate cancer patients who gave informed consent under the protocol approved by Kurume University ethical committee when the patients were diagnosed as recurrent prostate cancer in the past clinical trials. Gene expression profiles of 40 prostate cancer patients before peptide vaccine therapy were analyzed by using DNA microarray (HumanWG-6 v3.0 Expression BeadChip (Ilumina)). The prostate cancer patients included 20 good prognosis patients (whose survival time after peptide vaccine therapy was 700 days or more) and 20 poor prognosis patients (whose survival time after peptide vaccine therapy was less than 700 days) (Fig. 1).

### (I) RNA extraction and purification from peripheral blood of patients

1. To peripheral blood sample of a patient, TRIzol LS (Invitrogen) was added at a ratio 1: 3 and mixed such that the mixture became turbid.
2. To 750 µl TRIzol LS solution, 200 µl chloroform was added and mixed such that the mixture became turbid, and the resulting solution was centrifuged.
3. The supernatant obtained was removed to a fresh tube and added with ethanol 0.55 times as much as the supernatant.
4. The sample obtained in item 3 was mounted on a column of SV Total RNA Isolation System (Promega) and passed through the filter.
5. The filter was washed with 500 µl washing buffer.
6. Total RNA was eluted with 80 µl nuclease-free water.
7. Concentration of RNA was determined by using a spectrophotometer, and quality of RNA was checked by electrophoresis by using Experion system (Biorad).

### (II) cRNA synthesis for microarray using Illumine TotalPrep RNA Amplification Kit (Ambion)

(1) Synthesis of single-stranded cDNA by reverse transcription
   1. To 500 µg total RNA each, nuclease-free water was added up to 11 µl.
   2. The solution obtained item 1 was added with 9 µl Reverse Transcription Master Mix and incubated for 2 hours at 42°C.

### (2) Synthesis of double-stranded DNA

1. To the tube of item (1)-2, 80 µl Second Strand Master Mix was added.
2. The tube was incubated for 2 hours at 16°C.

### (3) Purification of cDNA

1. To the tube, 2.50 µl cDNA Binding Buffer was added.
2. The solution obtained in item 1 was mounted on cDNA Filter Cartridge and passed through the filter by centrifugation.
3. The filter was washed with 500 µl washing buffer.
4. cDNA was eluted with 19 µl nuclease-free water which was pre-heated to 50-55°C,

### (4) Synthesis of cRNA by reverse transcription reaction in vitro

1. To the cDNA sample obtained in item (3)-4, 7.5 µl IVT Master Mix was added.
2. The tube obtained in item 1 was incubated for 14 hours at 37°C.
3. To the tube obtained in item 2, 75 µl nuclease-free water was added.

### (5) Purification of cRNA

1. To the tube, 350 µl cRNA Binding Buffer was added.
2. To the tube, 250 µl of 100 % ethanol was added and mixed such that the fixture became turbid.
3. The sample obtained in item 2 was mounted on cRNA Filter Cartridge and passed through the filter by centrifugation.
4. The filter was washed with 650 µl washing buffer.
5. cRNA was eluted with 100 µl nuclease-free water which was pre-heated to 50-55°C.
6. Concentration of cRNA of the resulting solution was determined and the solution was used as a hybridization sample.

### (III) Microarray hybridization

(1) Preparation of cRNA for hybridization
1. To 500 µg total RNA, nuclease-free water was added up to 10 µl.
2. The solution obtained in item 1 was added with 20 µl GEX-HYB and incubated for 5 minutes at 65°C.

### (2) Hybridization

1. cRNA sample thus prepared was applied to HumanWG-6 v3.0 Expression BeadChip placed in a dedicated chamber.
2. The dedicated chamber was covered and incubated for 18 hours at 55°C.

### (IV) Washing and staining of microarrays

### (1) Washing of microarrays

1. The covers of the micorarrays were removed in Wash E1BC solution.
2. The arrays were immediately set in a slide rack and washed with 1 × High-Temp Wash buffer which was pre-heated to 55°C.
3. The arrays were washed in Wash E1BC solution for 5 minutes.
4. The arrays were washed in ethanol for 5 minutes.
5. The arrays were washed in Wash E1BC solution for 5 minutes.
6. To each of straining trays 4 ml Block E1 buffer was added, and then the arrays were set in the staining trays one by one to perform blocking for 10 minutes at room temperature.
7. To each of straining trays 2 µl streptoavidin-Cy3 per 2 ml Block E1 buffer was added, and then the arrays were set in the staining trays one by one to perform staining for 10 minutes at room temperature.
8. The arrays were washed in Wash E1BC solution for 5 minutes and dried by centrifugation.

### (V) Scanning and Quantification

1. The array were set in a dedicated scanner (Illumina) and scanned in a standard mode.
2. After scanning, each spot on the microarrays was quantified by using a dedicated software BeadStudio.

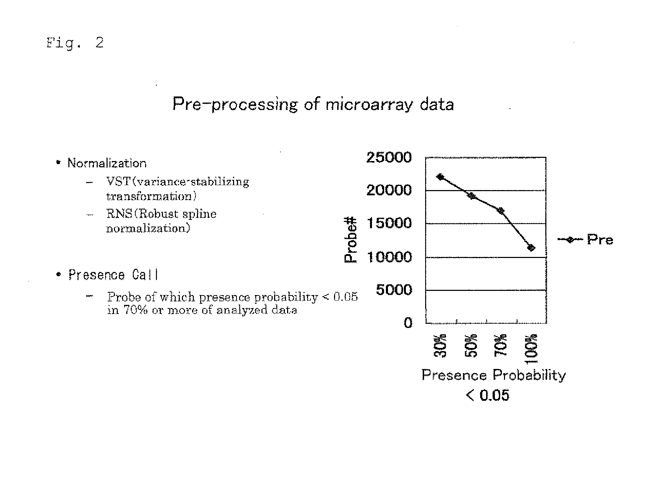

The microarray data obtained was normalized with VST (Variance Stabilizing Transformation) and RSN (robust spline normalization). Expression level of a gene was determined to be significant when the gene showed Presence Probability &#38;#60 0.05 compared to a negative control (expression level measured with a probe for a gene which was not present in the microarray) (Fig. 2). The following analysis was performed using the genes showing Presence Probability !#38;#60 0.05 in 70% or more of the 40 patients.

### 2: Selection of a gene set for building PLS regression model

The prostate cancer patients of Example 1 were classified into 2 groups, good prognosis group (survival time after peptide vaccine therapy was 700 days or more) and poor prognosis group (survival time after peptide vaccine therapy was less than 700 days) and genes were selected based on correlation between expression level of each gene and survival time, and expression variation between the two groups. Expression variation was represented as increase or decrease of mean expression level of poor prognosis group compared to that of good prognosis group as a control.

For analysis, the hollowing five statistical methods were used (A: method for analysis of correlation between expression level of a gene and survival time, B: method for analysis of expression variation between good prognosis group and poor prognosis group).
Pearson product-moment correlation coefficient (A)
Limma^{b} (B)
SAM^{a} (B)
Rank Prod^{c} (B)
Spearman's rank correlation coefficient (A)

### References

^{a} Tusher et al. Significance analysis of microarrays applied to the ionizing radiation response. Proc Natal Acad Sci USA (2001) vol. 98 (9) pp. 5116-21.
^{b} Smyth. Linear models and empirical bayes methods for assessing differential expression in microarray experiments. Statistical applications in genetics and molecular biology (2004) vol. 3 pp. Article.
^{c} Breitling et al. Rank products: a simply, yet powerful, new method to detect differentially regulated genes in replicated microarray experiments. FEBS Letters (2004) vol. 573 (1-3) pp. 83-92,
   Those references are herein incorporated by reference.

### 3: Analysis of PLS regression model

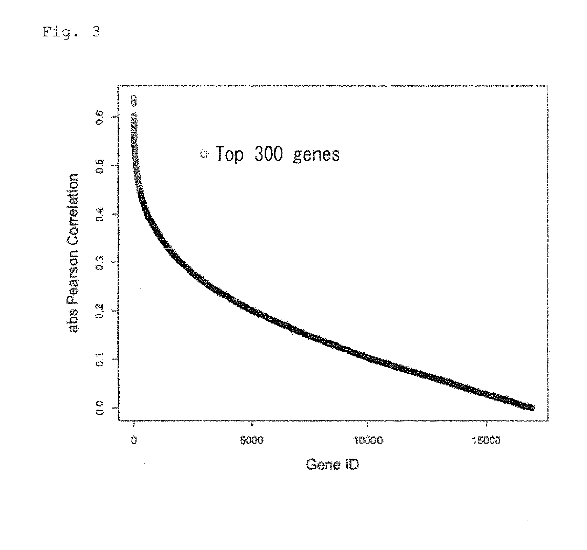

PLS (Partial Least Square) regression model was built for prediction of survival time of each patient after peptide vaccine therapy. Specifically, from each of the five gene sets consisting of top 300 genes selected by one of the above five methods, respectively (Tables 1-5, Fig. 3), 30 gene sets each comprising genes in multiples of 10 (i. e., 10, 20, ... or 300 genes) from the top were extracted. Then, PLS regression model was built with latent variable 1 to 10.

Analysis of the regression model was performed according to Leave One Out Cross Validation (LOOCV) method. Tamely, for 40 prostate cancer patients in total, regression model was built using results of 39 patients, and the regression model thus built was used to predict survival time of the remaining 1 patient.

For example, calculation of survival time with a gene set consisting of top 50 genes is as follows. Predicated survival time was calculated according to the following regression equation.
Predicated survival time = a1g1 + a2g2 + a3g3 + ... + a50g50 + E (formula 1),
whererin each of a1, a2, a3, ... and a50 is a coefficient for expression level of a gene used to build the regression model; each of g1, g2, g3, g4, ..., g50 is expression level of a gene in a patient to be analyzed; and E is a constant term of the regression equation).

Table 6 shows coefficients used for the prediction of survival time of patients with the gene set consisting of top 50 genes selected by Pearson product-moment correlation coefficient, latent variable 3.

### References

[1] Bjrn-Helge Mevik and Ron Wehrens Journal of Statistical Software: Vol 18, Issue 2 (2007), &#38;#34; The pls Package: Principal Componentand Partial Least Squares Repression in R&#38;#34;
[2] Geladi, P. and B. Kowalski (1986) Martial least-squares regression: Atutorial. Analytica Chimica Acta 185:1-17. Those references are herein incorporated by preference.

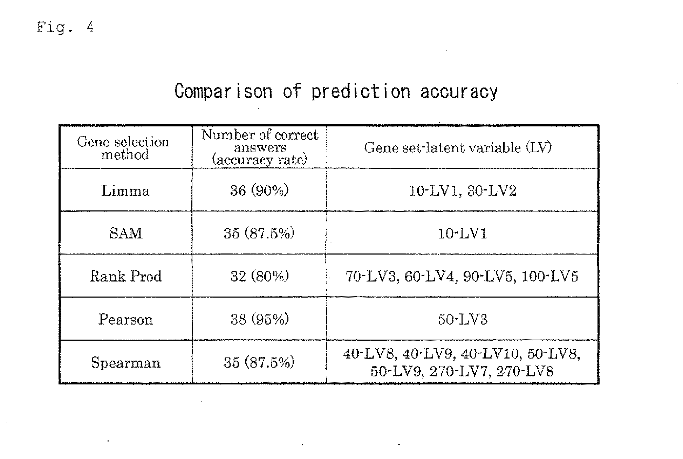

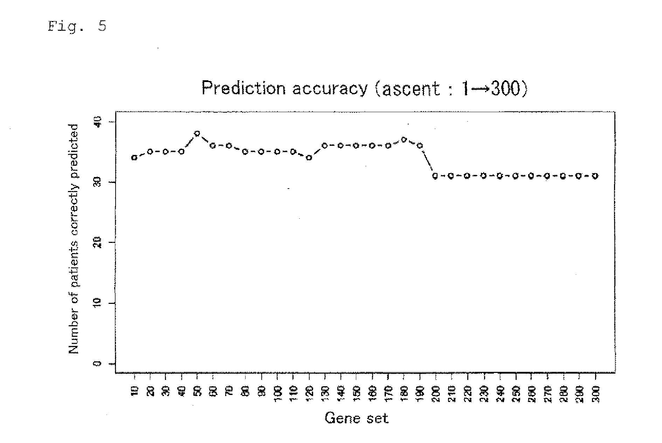

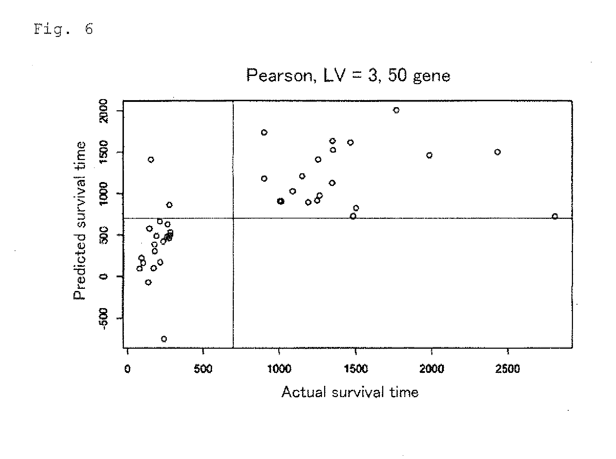

The answer was determined to be correct when the predicted survival time was 700 days or more for a good prognosis patient or less than 700 days for a poor prognosis patient, and accuracy rate (prediction accuracy) of the regression model was calculated. When evaluated, all the regression models showed the best prediction accuracy more than 80 % (Fig. 4). Particularly, the gene set consisting of top 50 genes selected by Pearson product-moment correlation coefficient, latent variable 3 showed the highest prediction accuracy, which was 95%. Prediction accuracy of survival time prediction with this gene set and comparison between the predicted survivals time and the actual survival time are shown in Figs. 5 and 6. For the patients in the right-upper and left-lower sections in Fig. 6, predicted survival time reflected actual survival time. For the patients in the left-upper section, prognosis after the immunotherapy was predicted as good, but actual survival time was shorter than the prediction. On the other hand, there was no patient whose prognosis was predicted as poor but actual survival time was longer than the prediction (Fig. 6, the right-lower section). Those results indicate that the method of the present invention can predict a patient who would not be efficiently treated by immunotherapy before initiation of the immunotherapy.

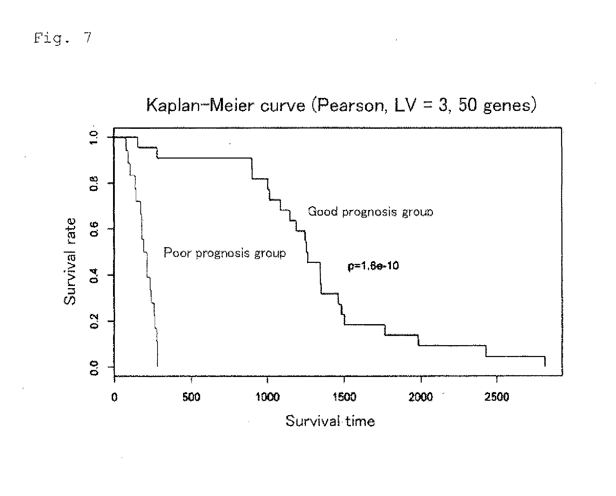

In addition, Kaplan-Meier curve was prepared using actual survival time of good and poor prognosis groups classified with the gene set consisting of top 50 genes selected by Pearson product-moment correlation coefficient, latent variable 3 and then log rank test was performer. As a result, it was revealed that the present method could classify the patients into good and poor prognosis groups with probability p = 1.6e-10 (Fig. 7).

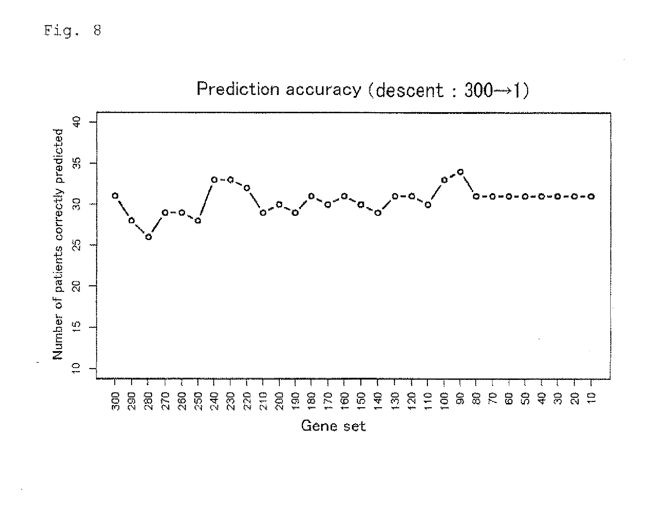

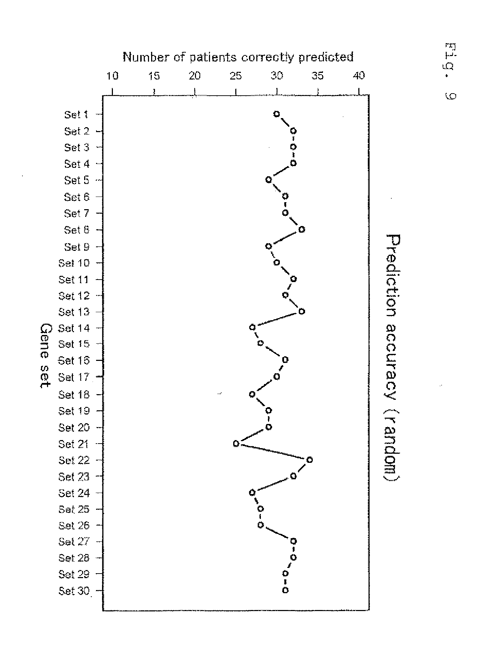

Then, from the gene set of Table 1, 30 gene sets each consisting of genes in multiples of 10 (i. e., 10, 20, ... or 300 genes) in ascending order from the bottom were extracted and PLS regression model was built with latent variable 3 (Fig. 8). In this case, prediction accuracy was 65-85%. Also, from the same gene set, 30 gene sets were extracted randomly (Table 7) and PLS regression model was built with latent variable 3 (Fig. 9). In this case, prediction accuracy was 62.5-85%. Those results demonstrate that survival time after immunotherapy can be predicted by any gene selection method.

### Example 2

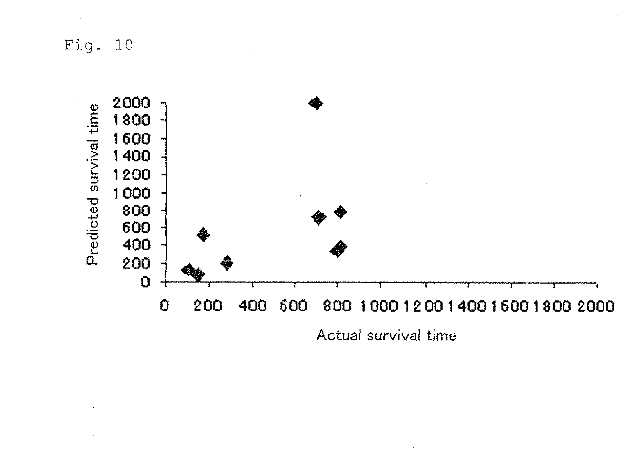

Gene Expression data of 9 prostate cancer patients were further included in addition to those of 40 prostate cancer patients of Example 1, and the patients were classified into good prognosis group (survival time was 300 days or more) and poor prognosis group (survival time was less than 300 days). Then, PLS regression model was built with a gene set consisting of top 50 genes selected by Pearson product-moment correlation coefficient, latent variable 3 and survival time of the 9 prostate cancer patients newly included were predicted (Fig. 10). In this example, prediction was correct in 8 of 9 patients.

### Example 3

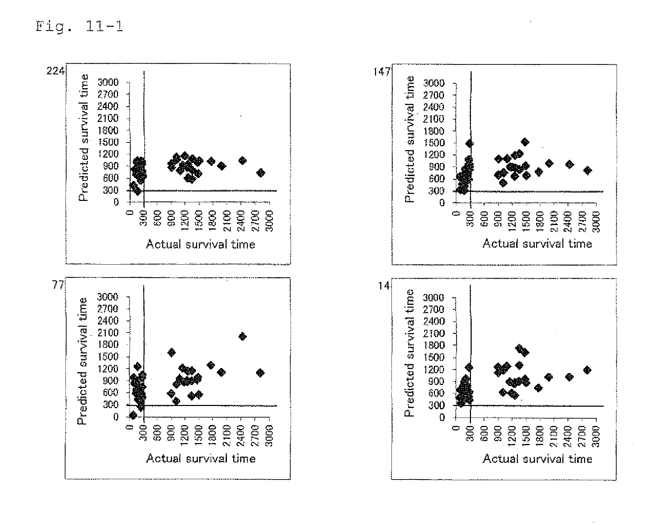

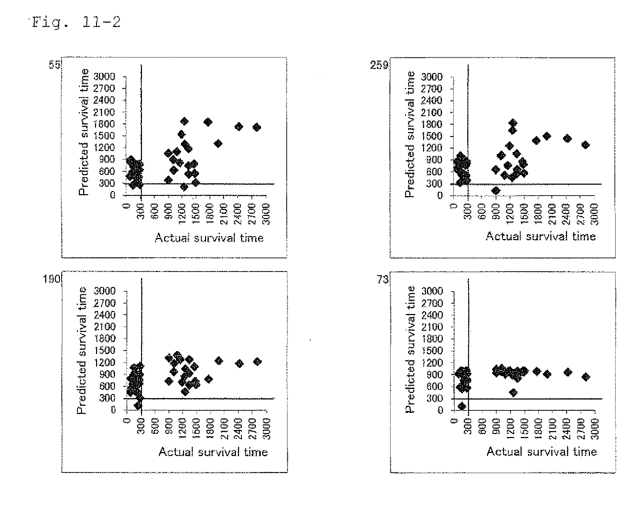

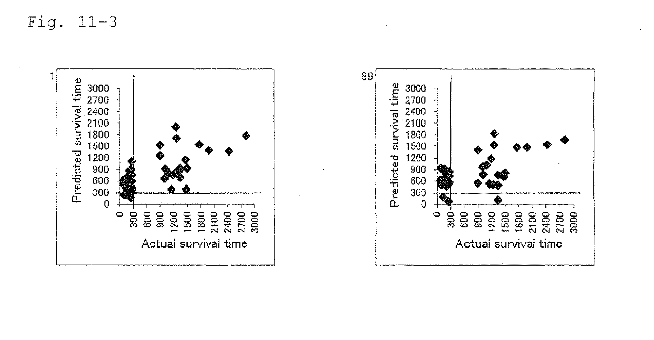

For 40 prostate cancer patients of Example 1, a primacy linear regression equation was prepared according to their survival time and expression level of one of top 300 genes selected by Pearson product-moment correlation coefficient (Table 1) . Then, predicted survival time of a patient was calculated with the equation thus prepared and expression level in the patient, and the calculated predicted survival time (y axis) and actual survival time (x axis) were shown in a graph (Fig. 11). The graphs show results of 10 genes randomly selected from Table 1 by generating a random number between 1 and 300 and regarding the number as a rank in the table. As a result, it was demonstrated that survival time could be predicted based on expression level of only one gene.

Table 1: List of top 300 genes selected by Pearson product-moment correlation coefficient.

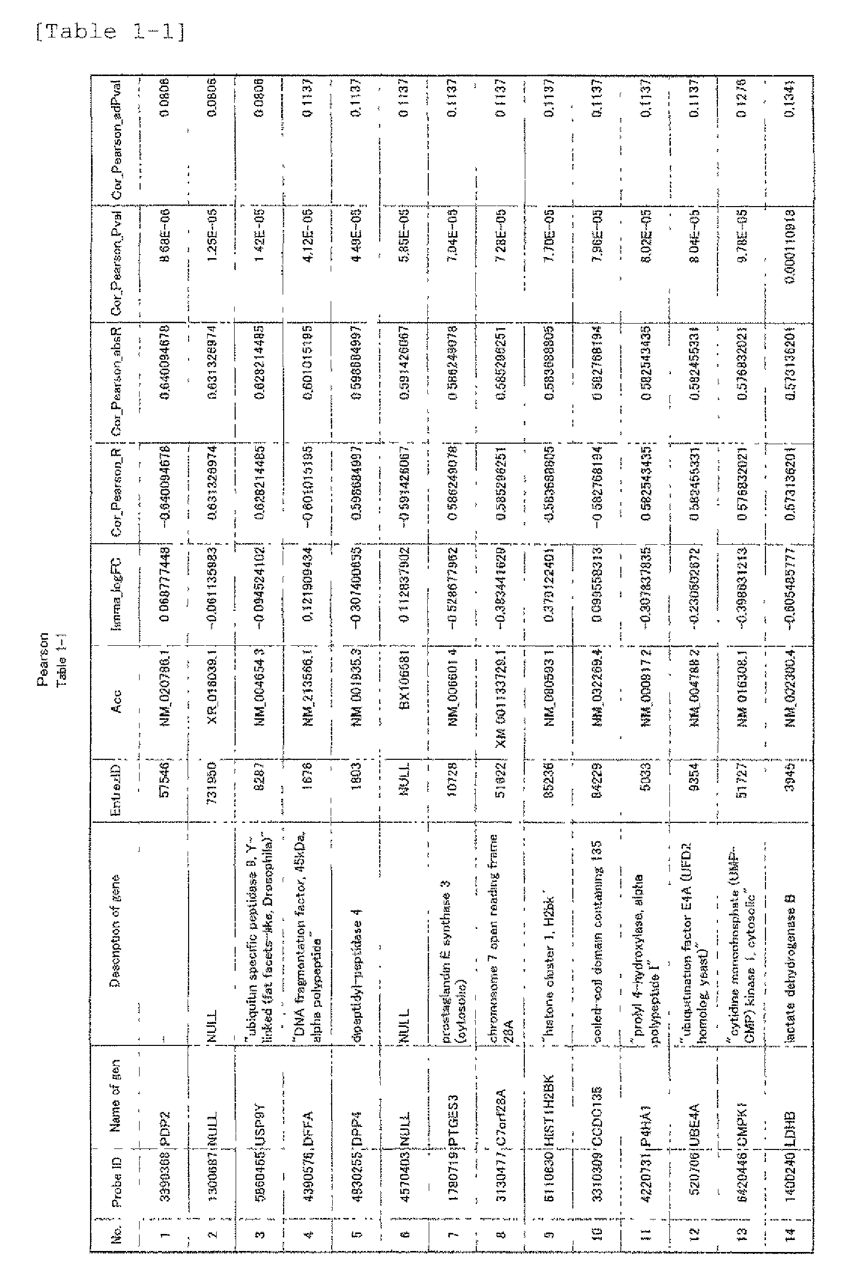

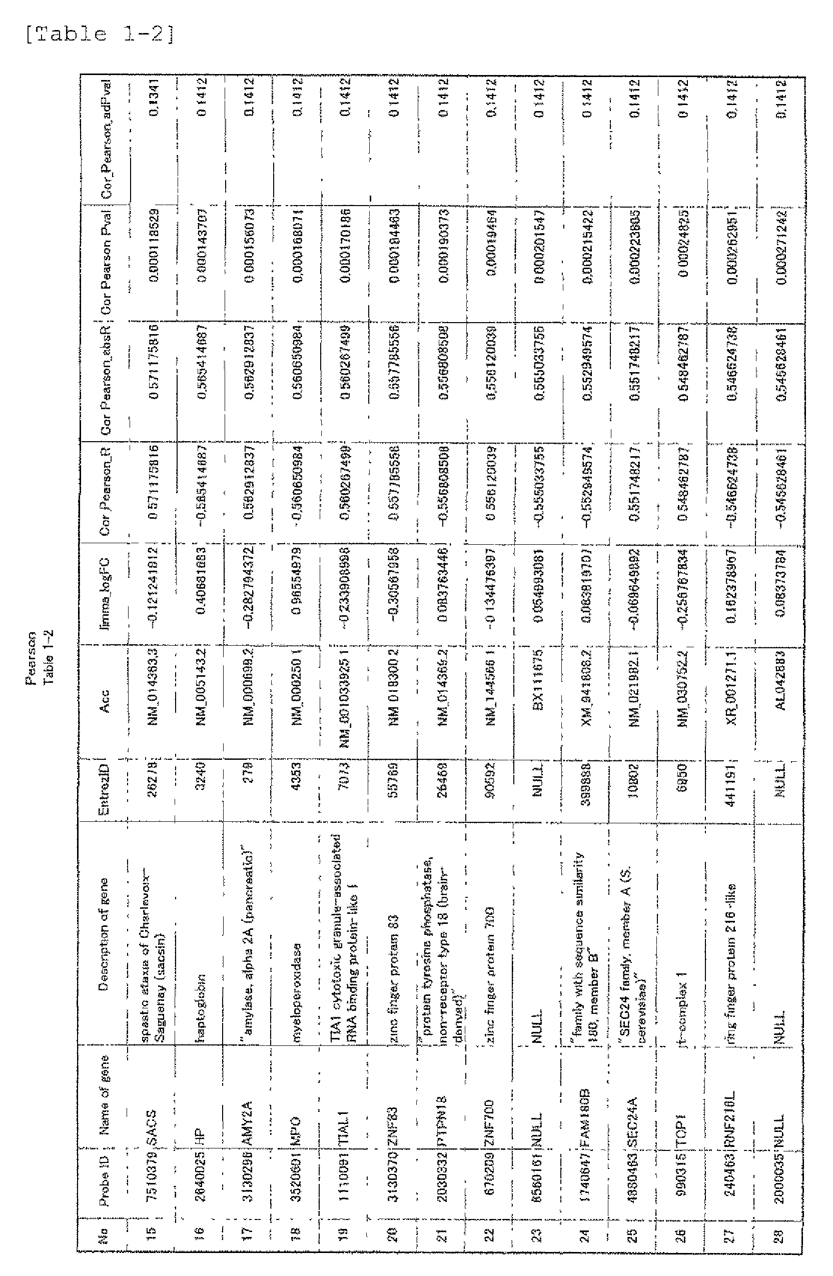

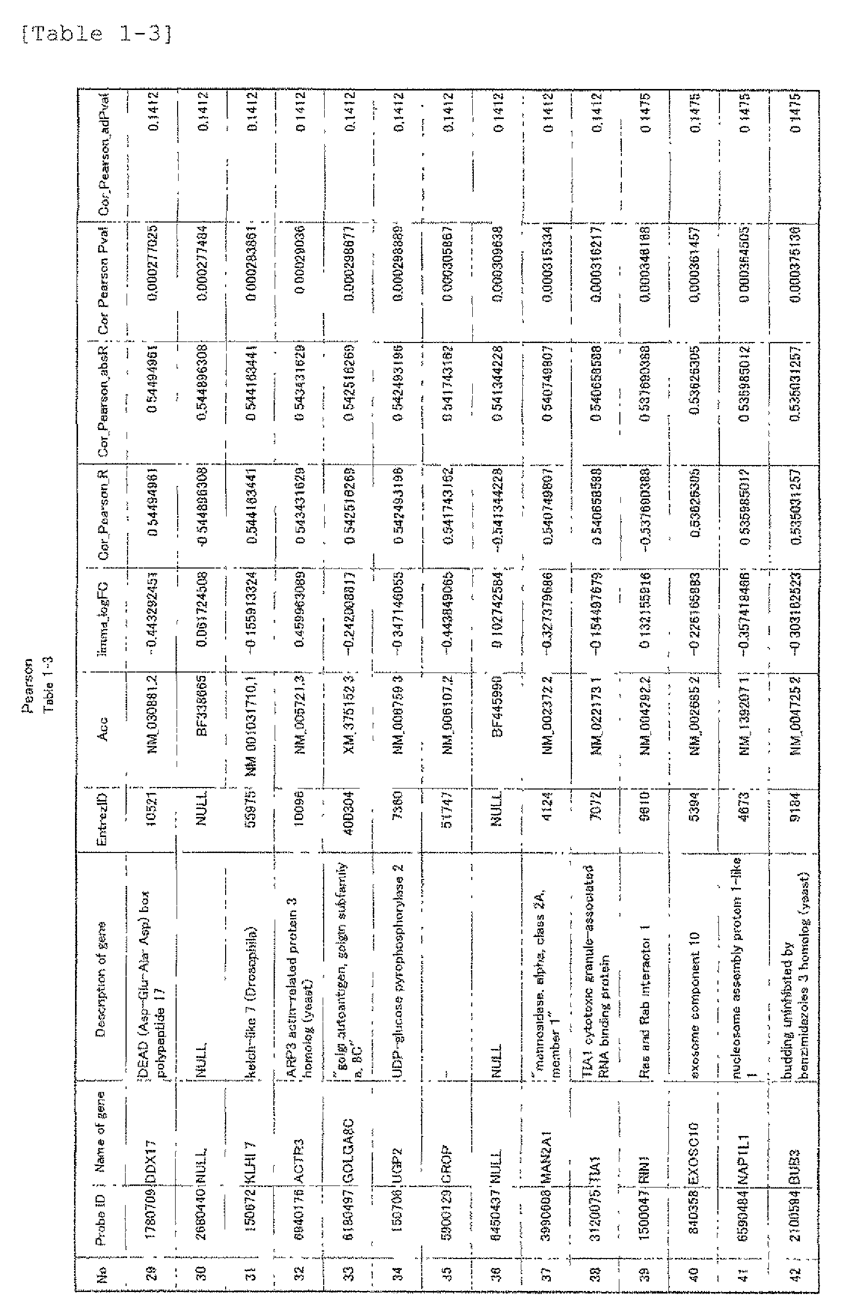

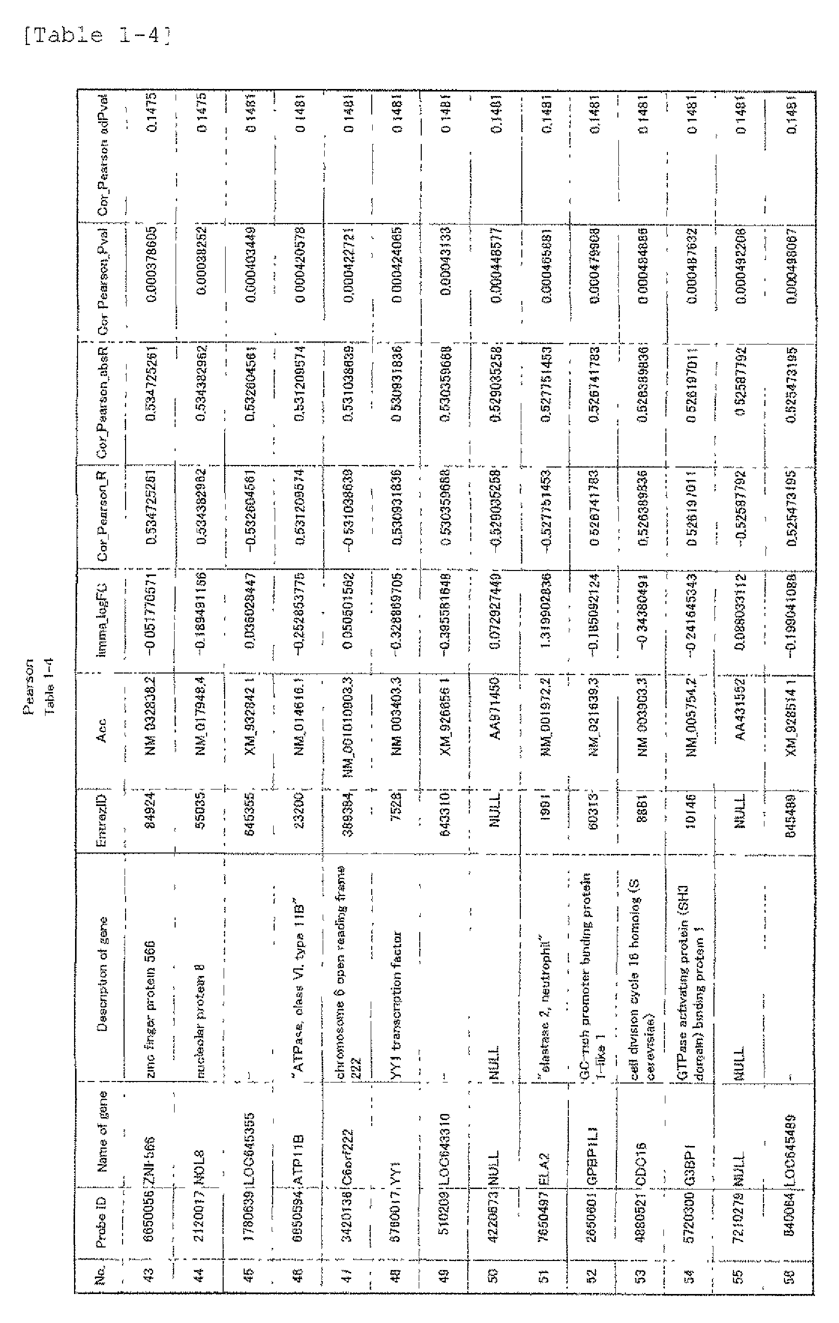

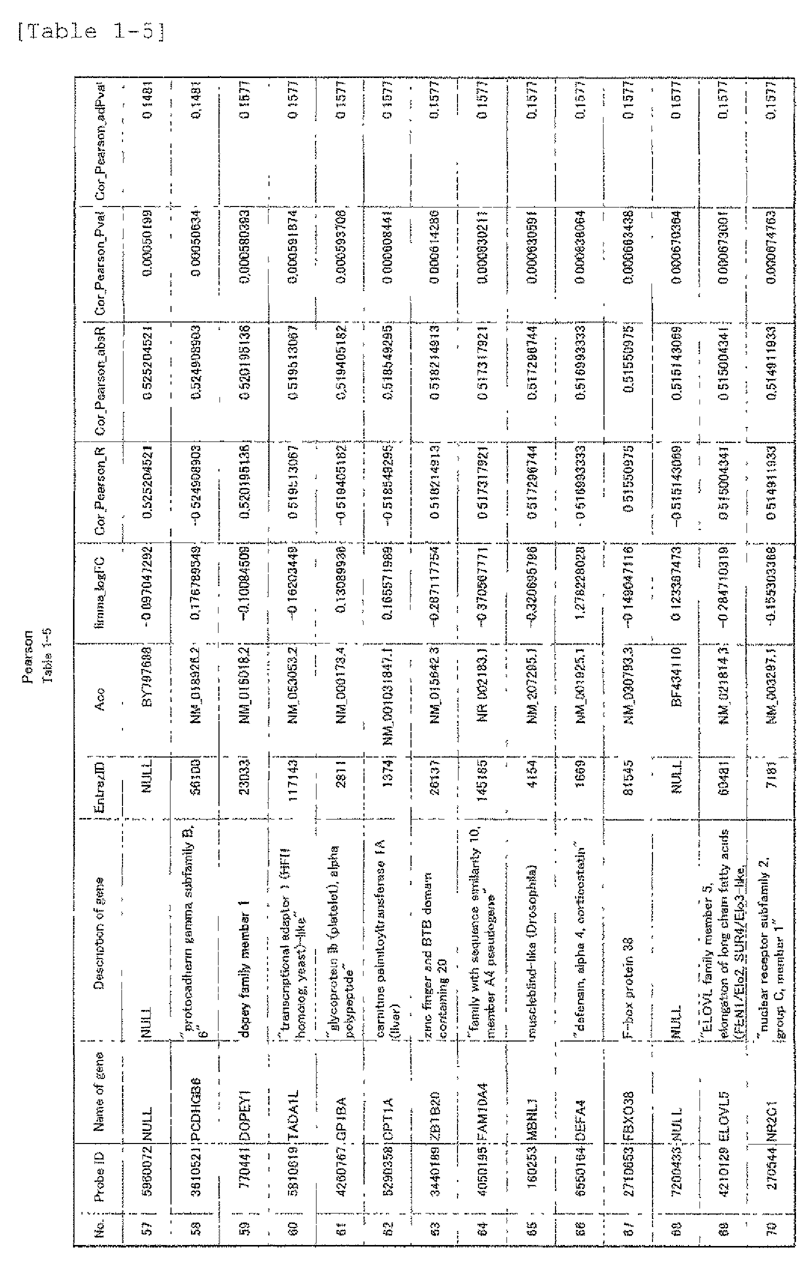

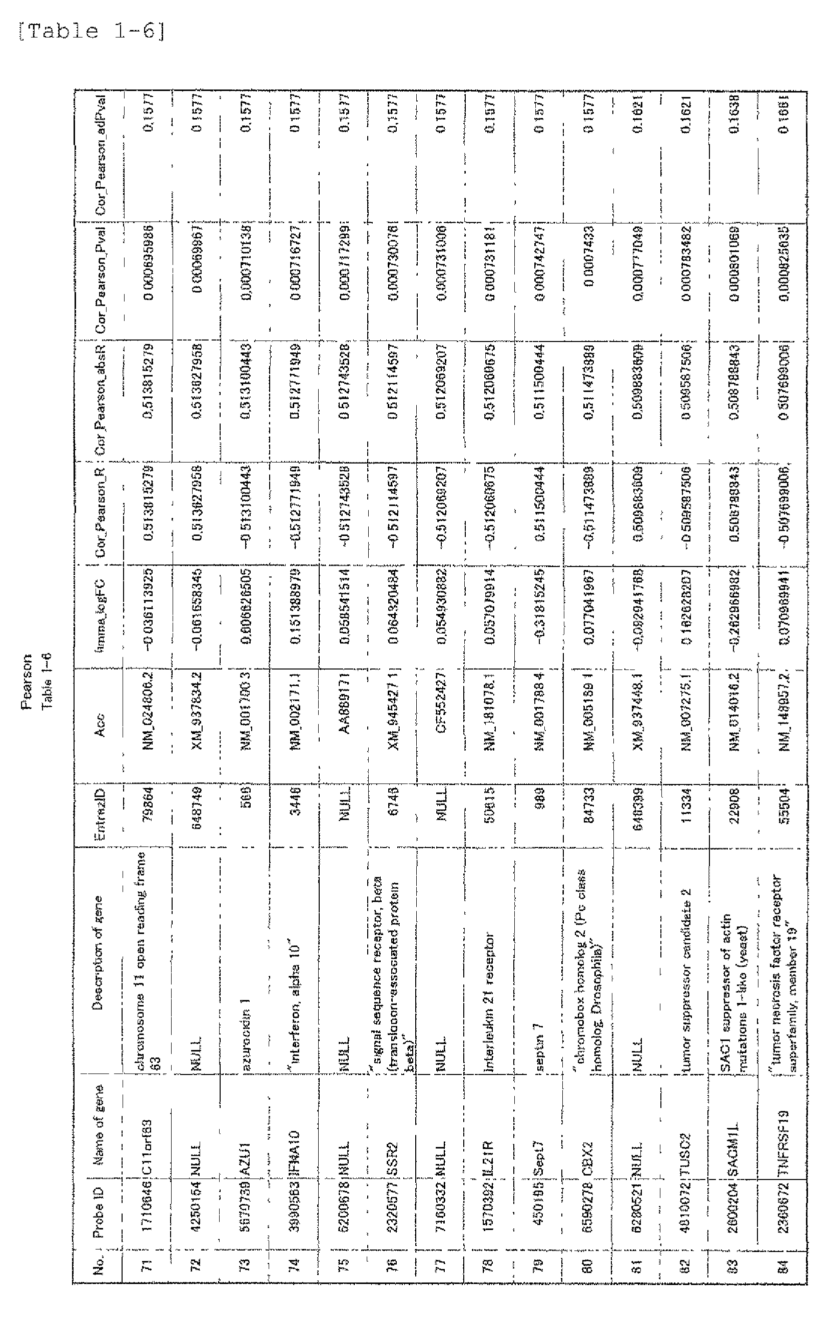

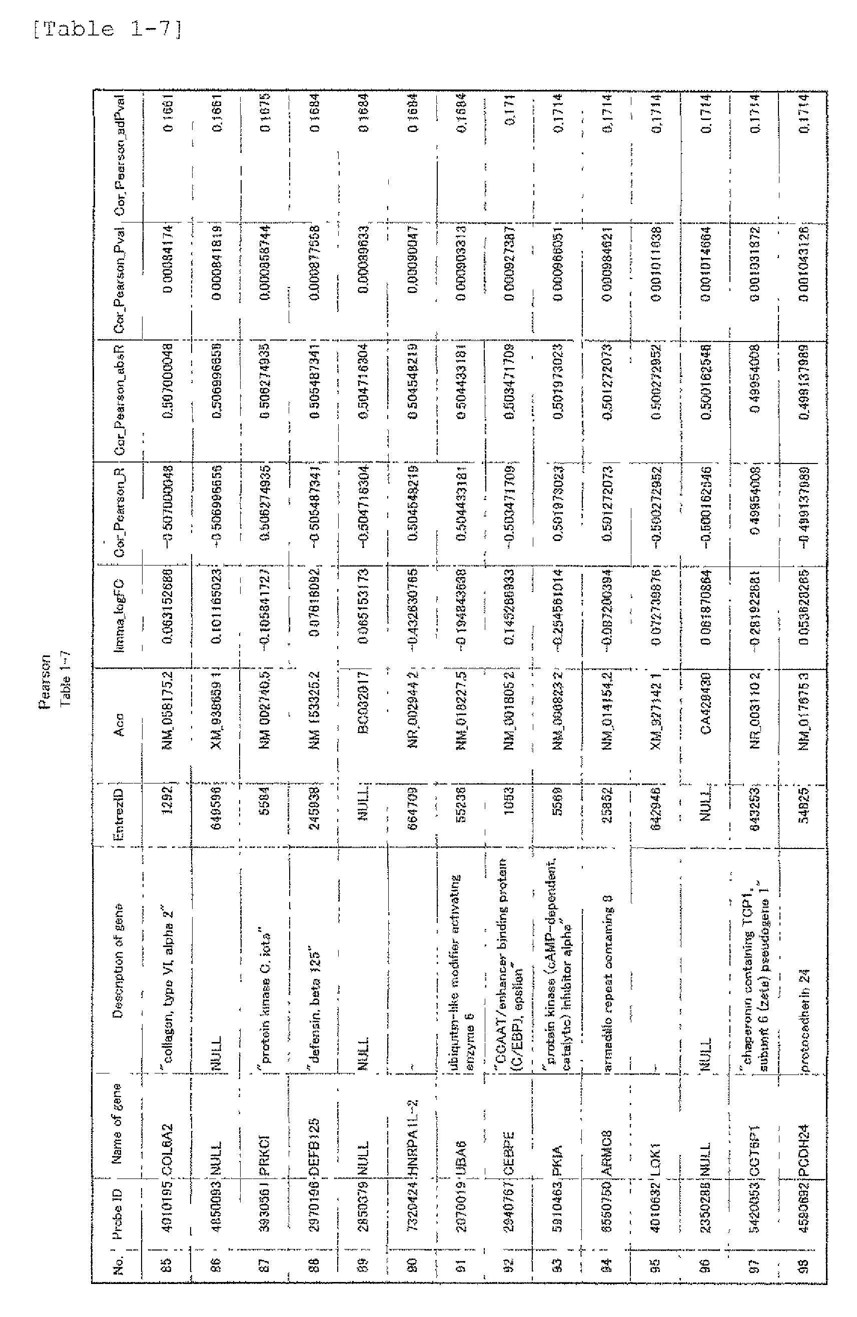

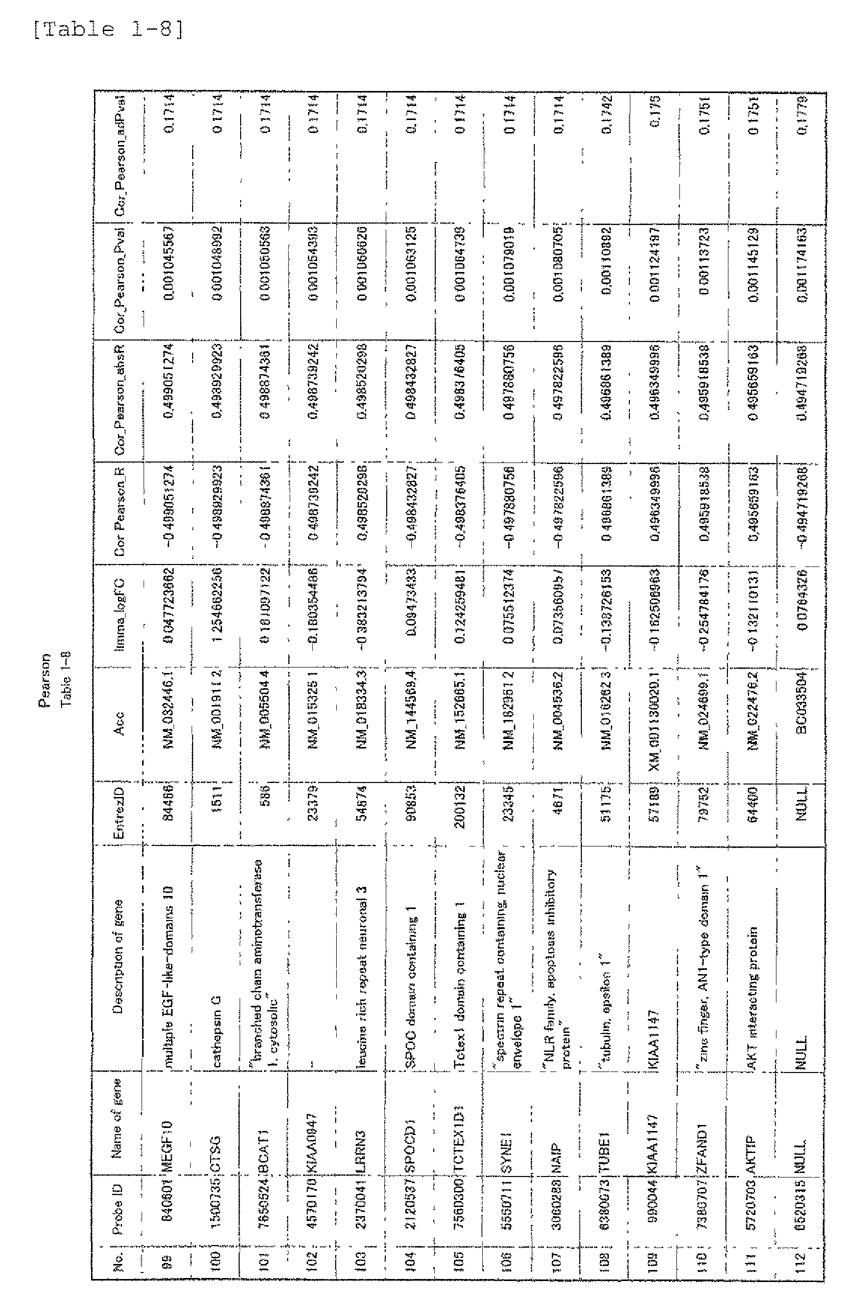

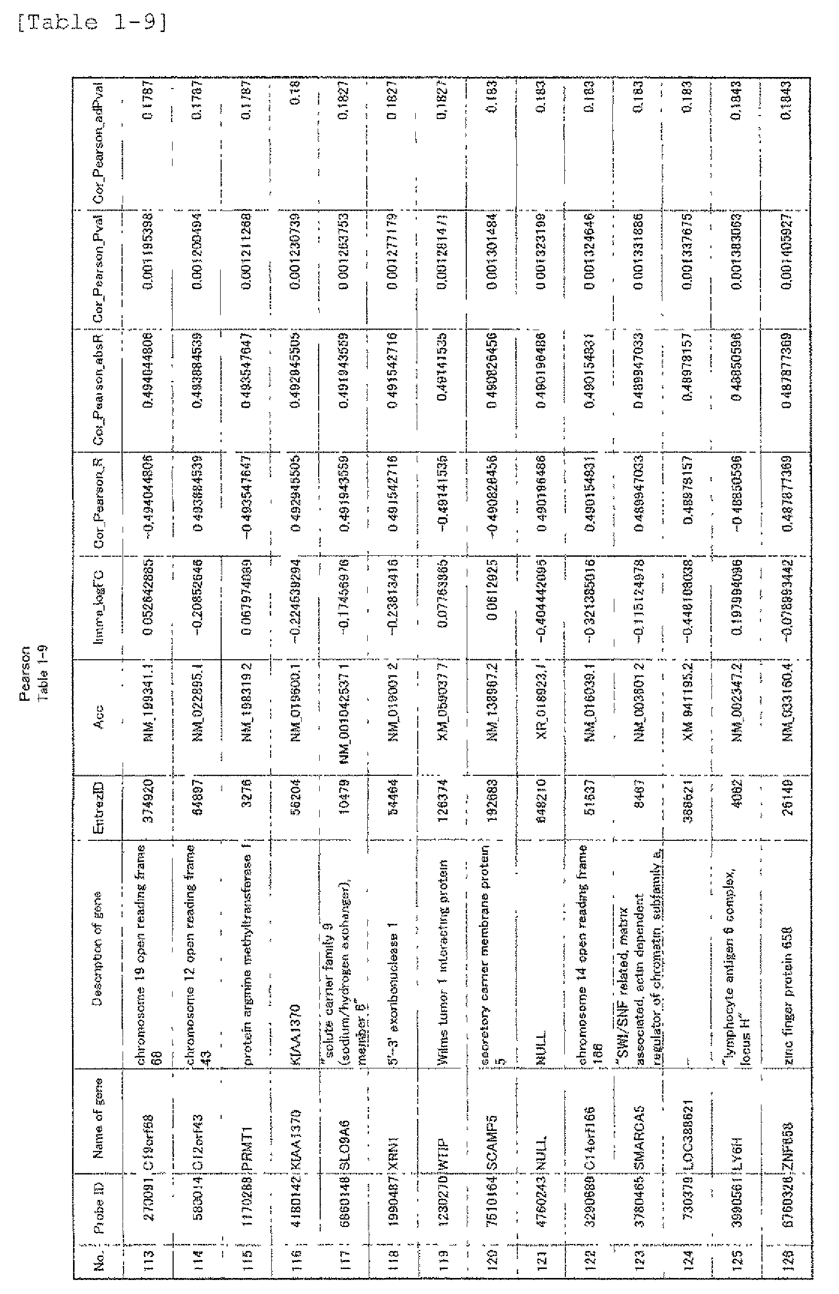

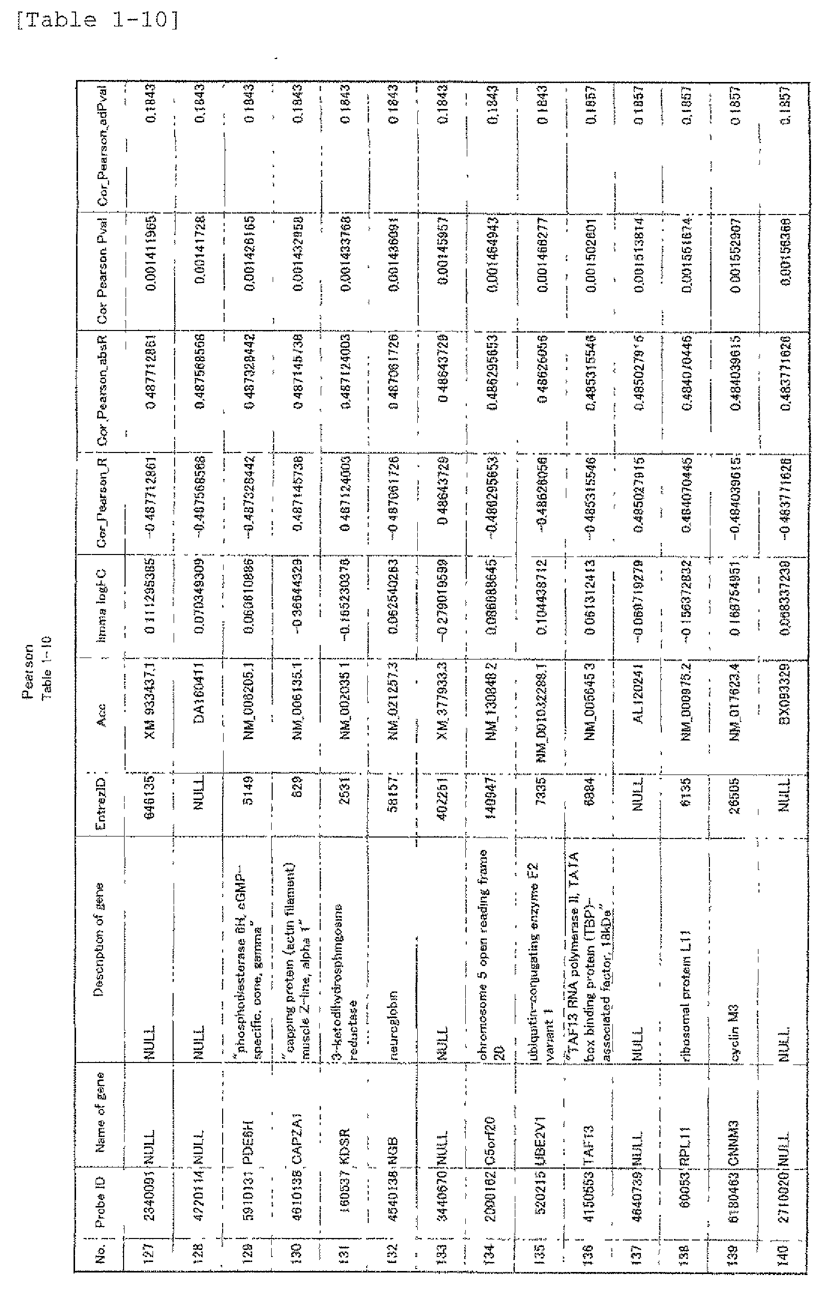

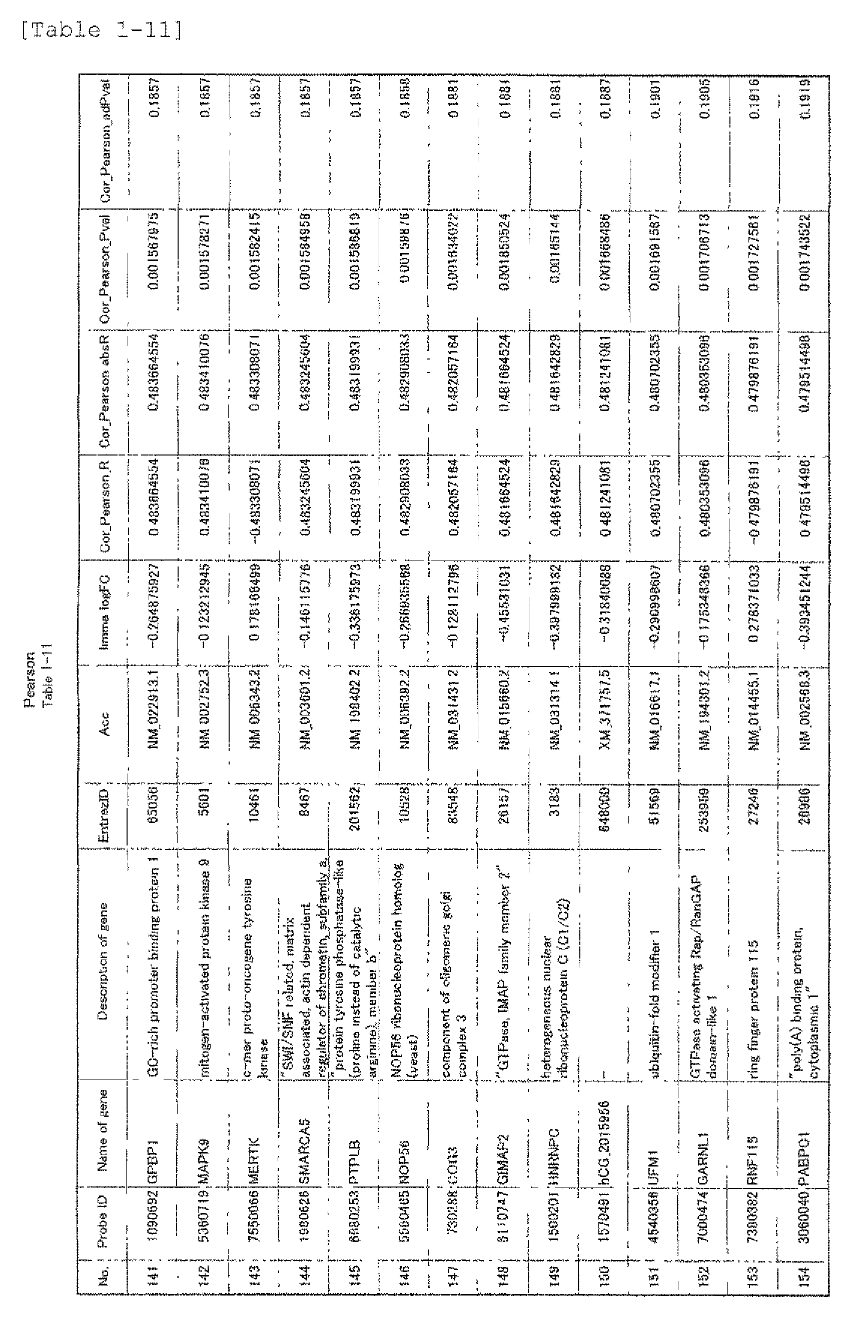

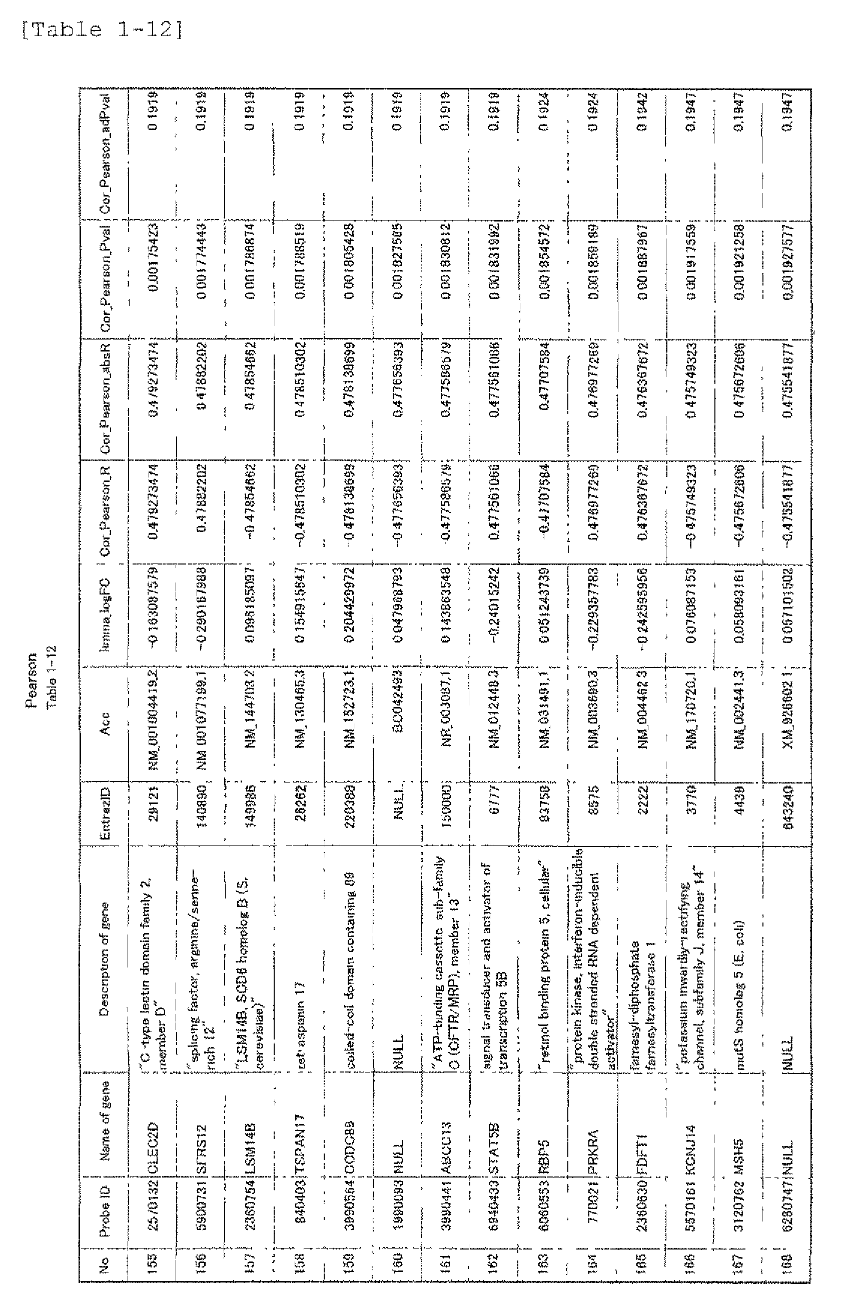

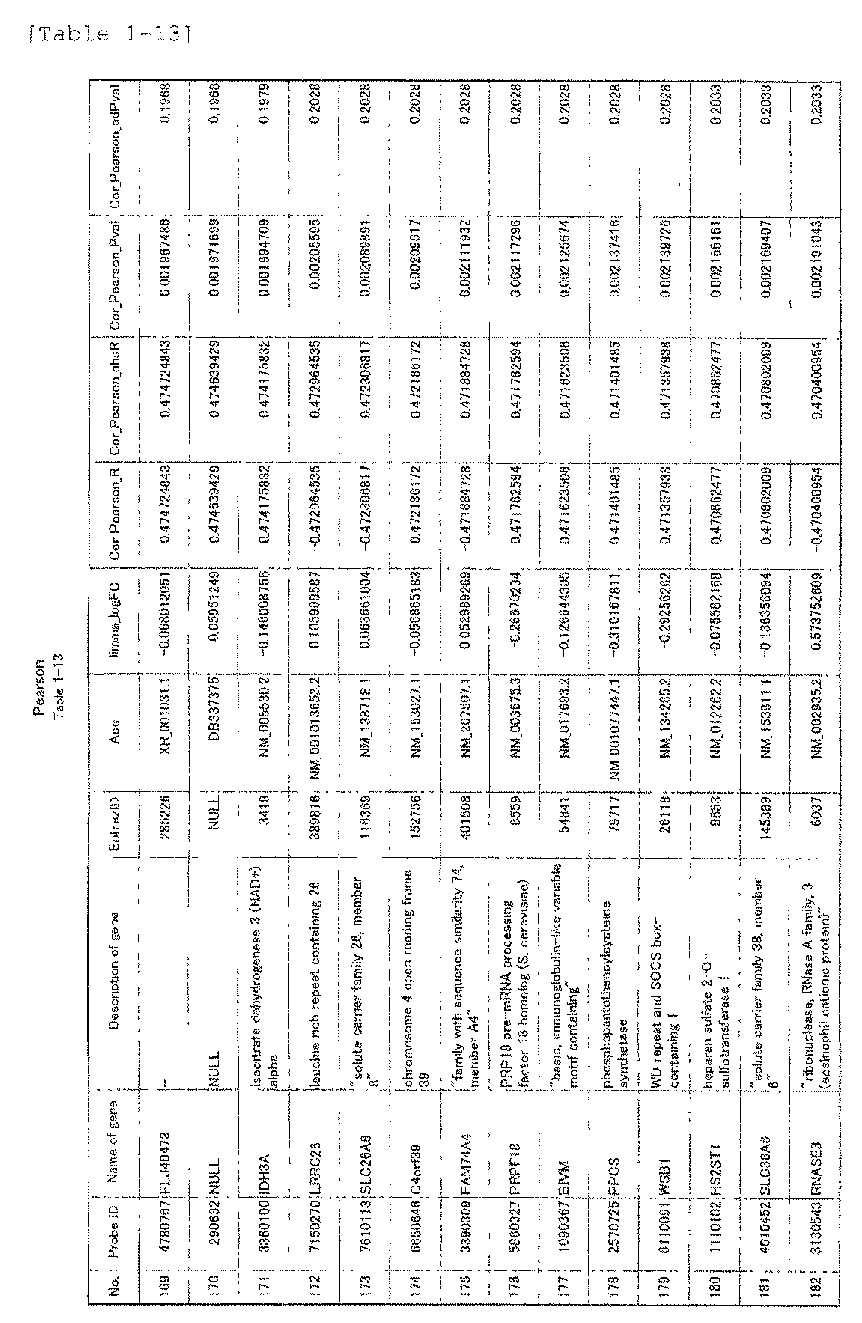

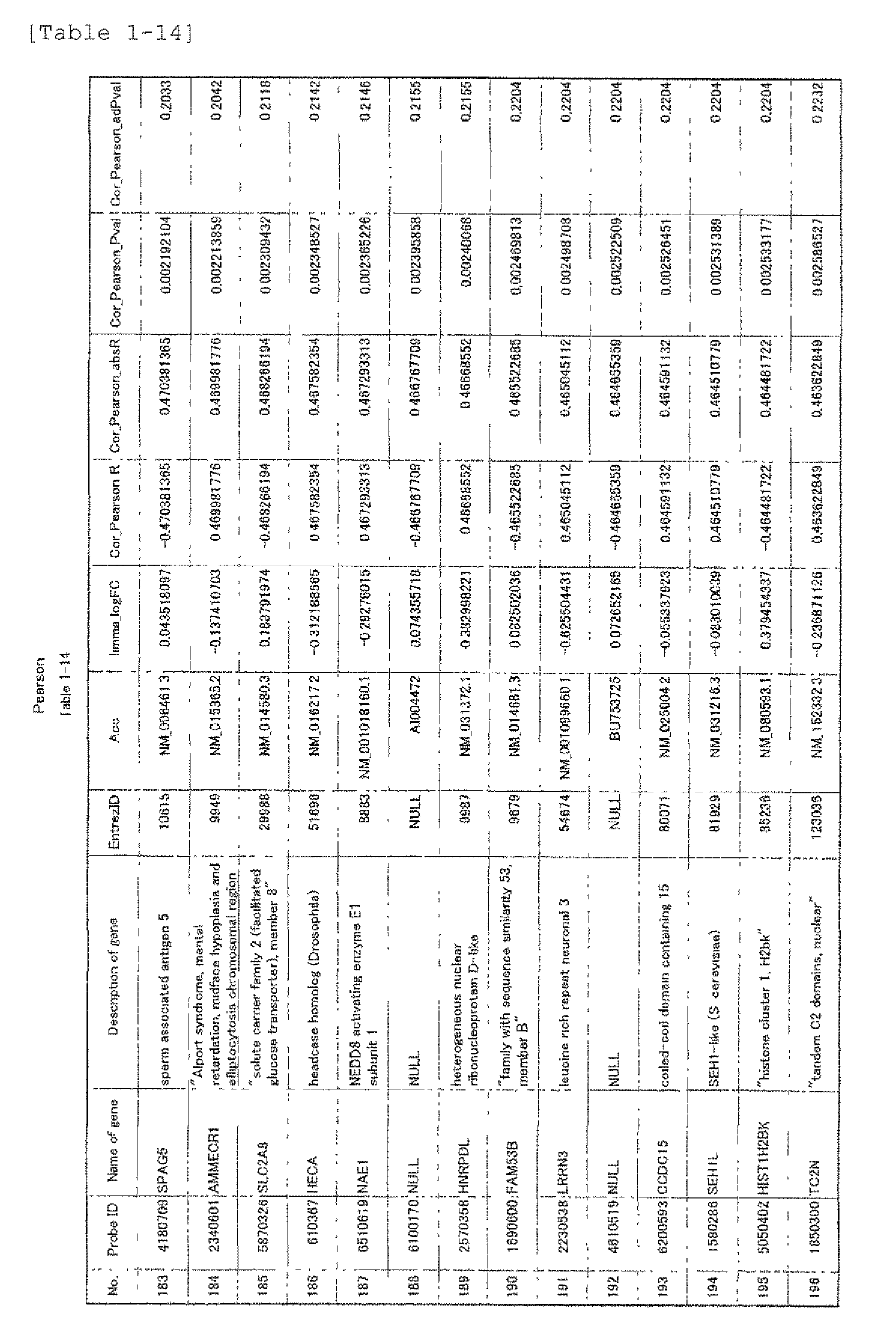

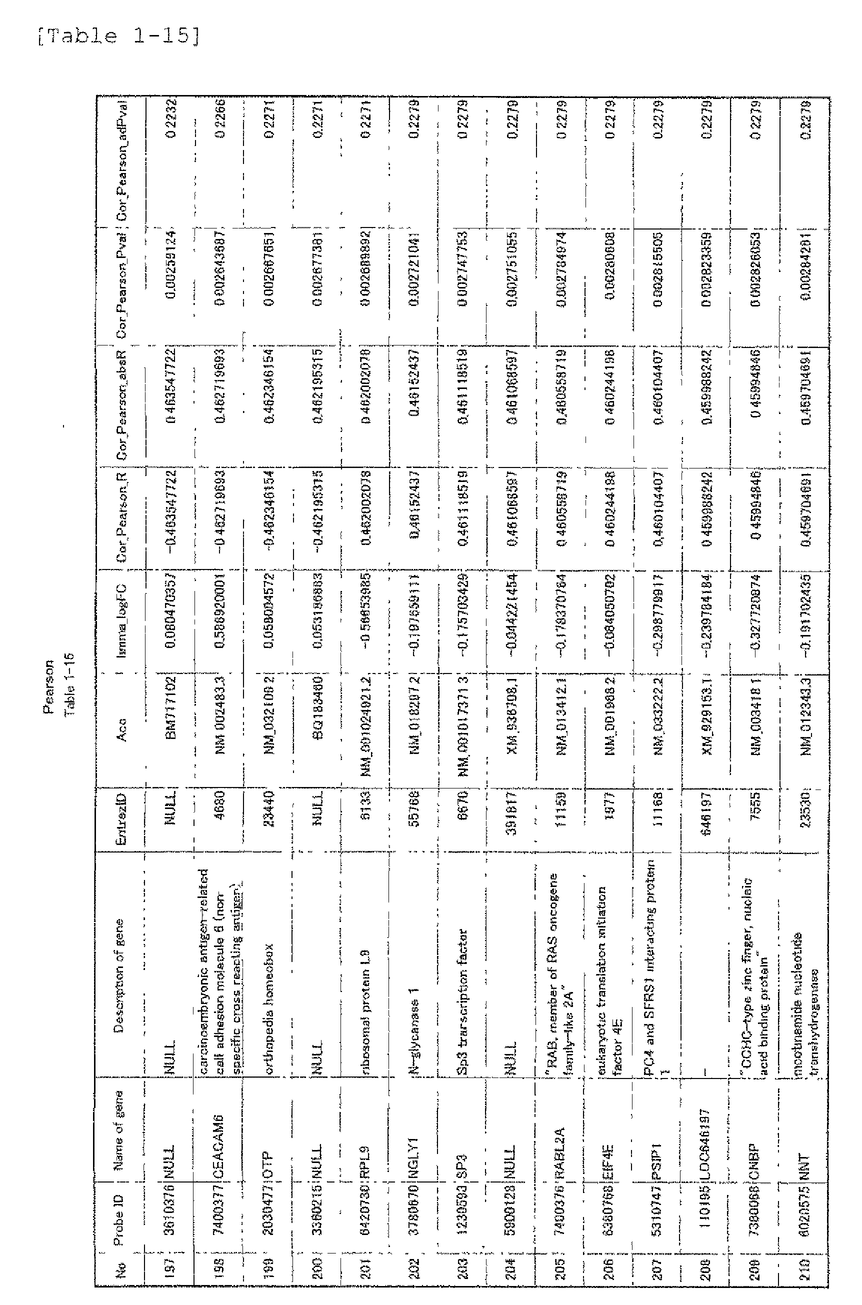

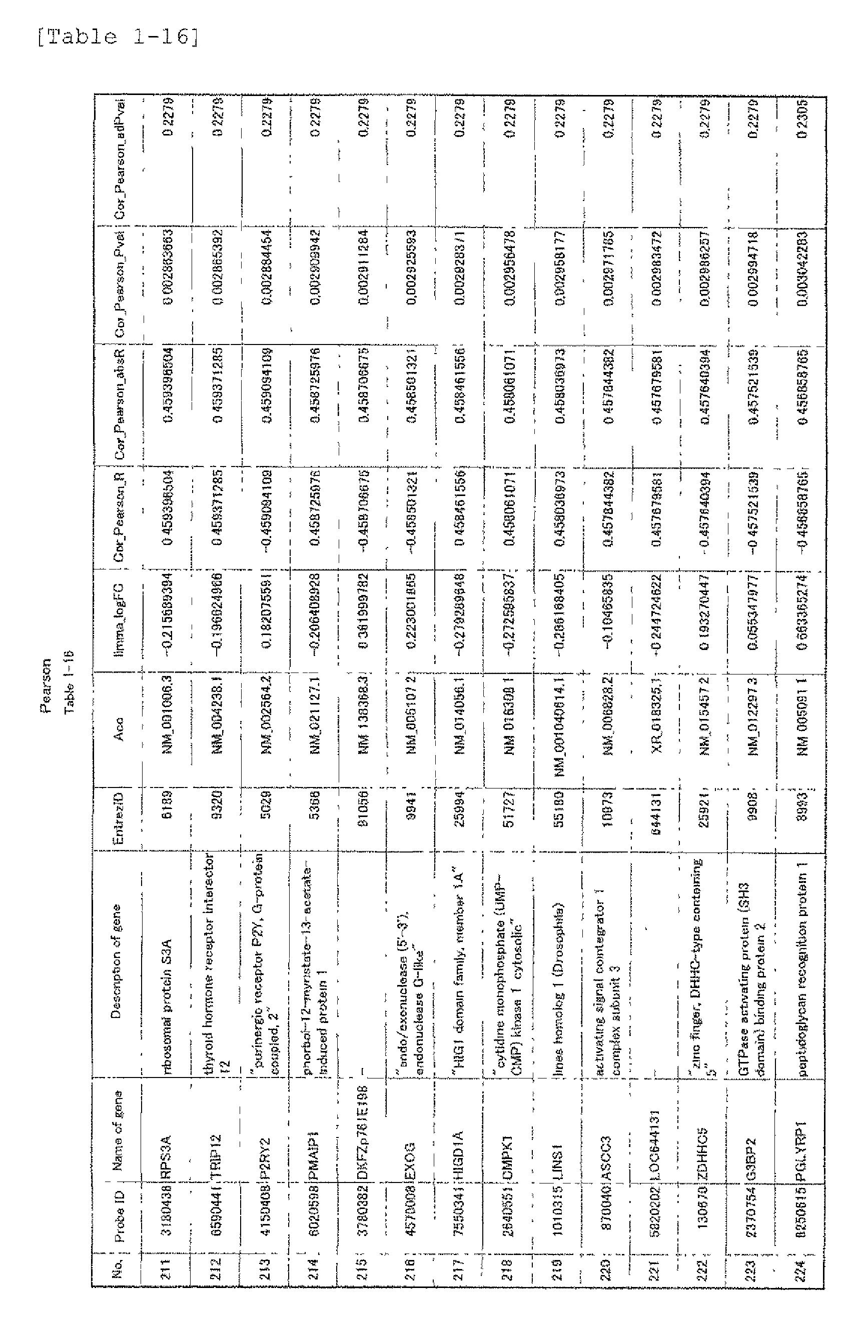

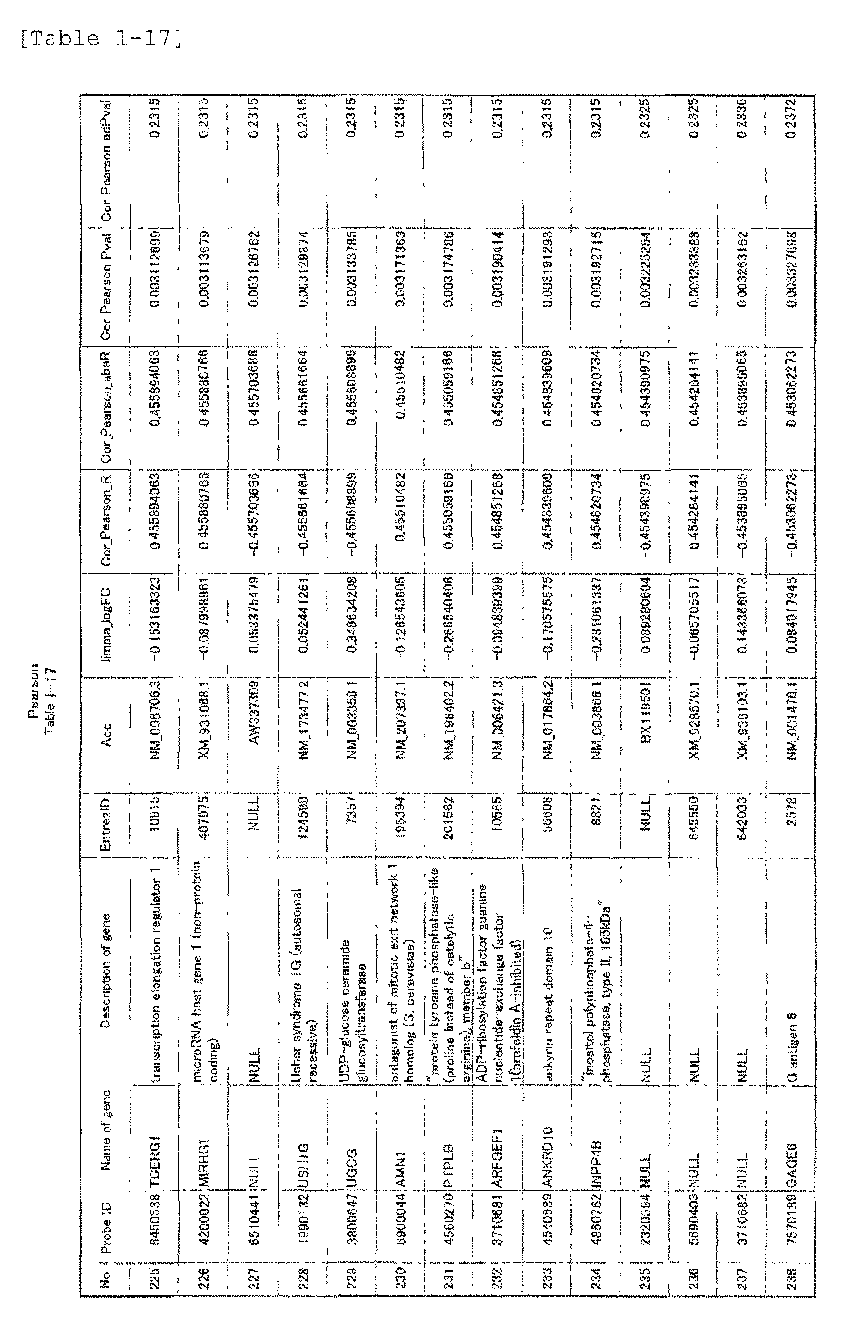

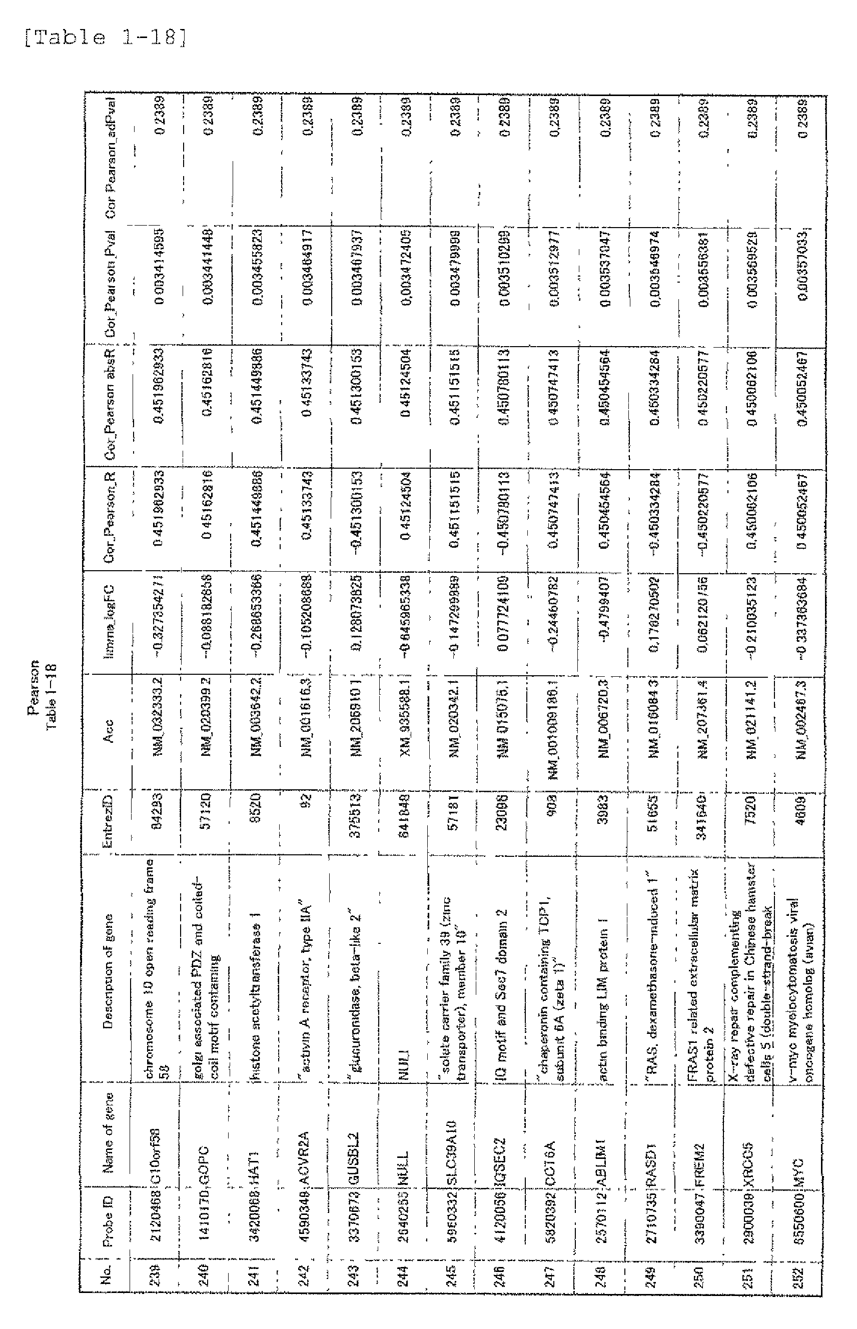

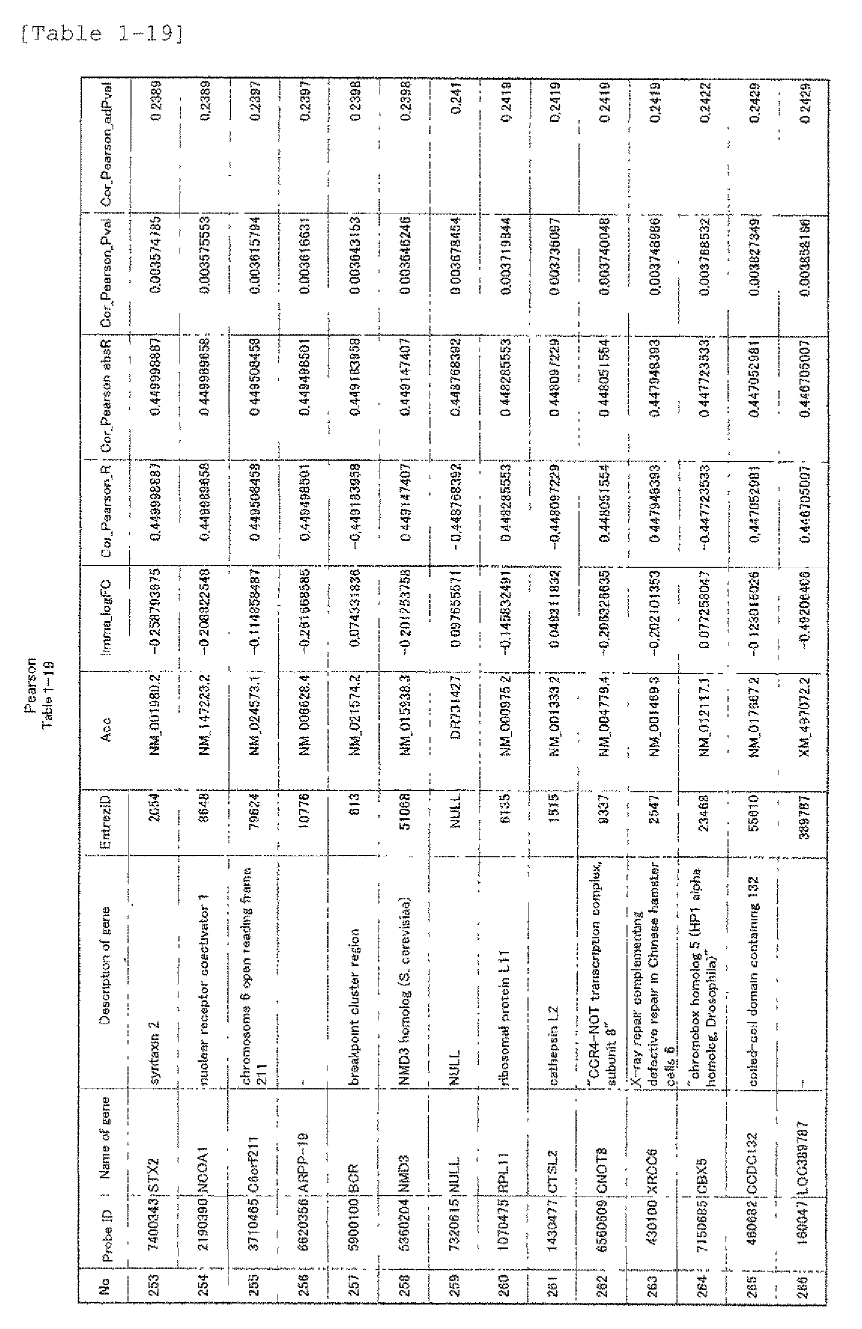

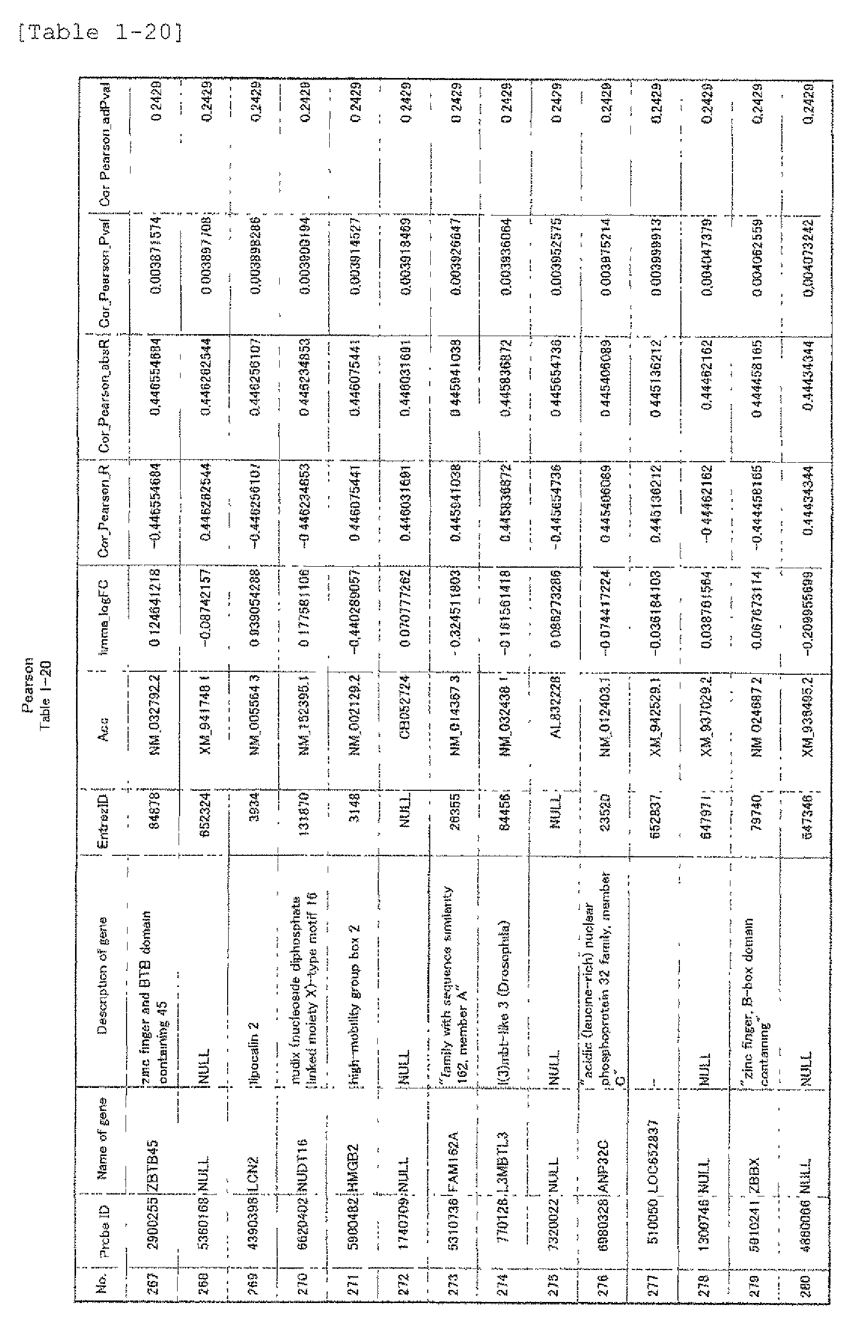

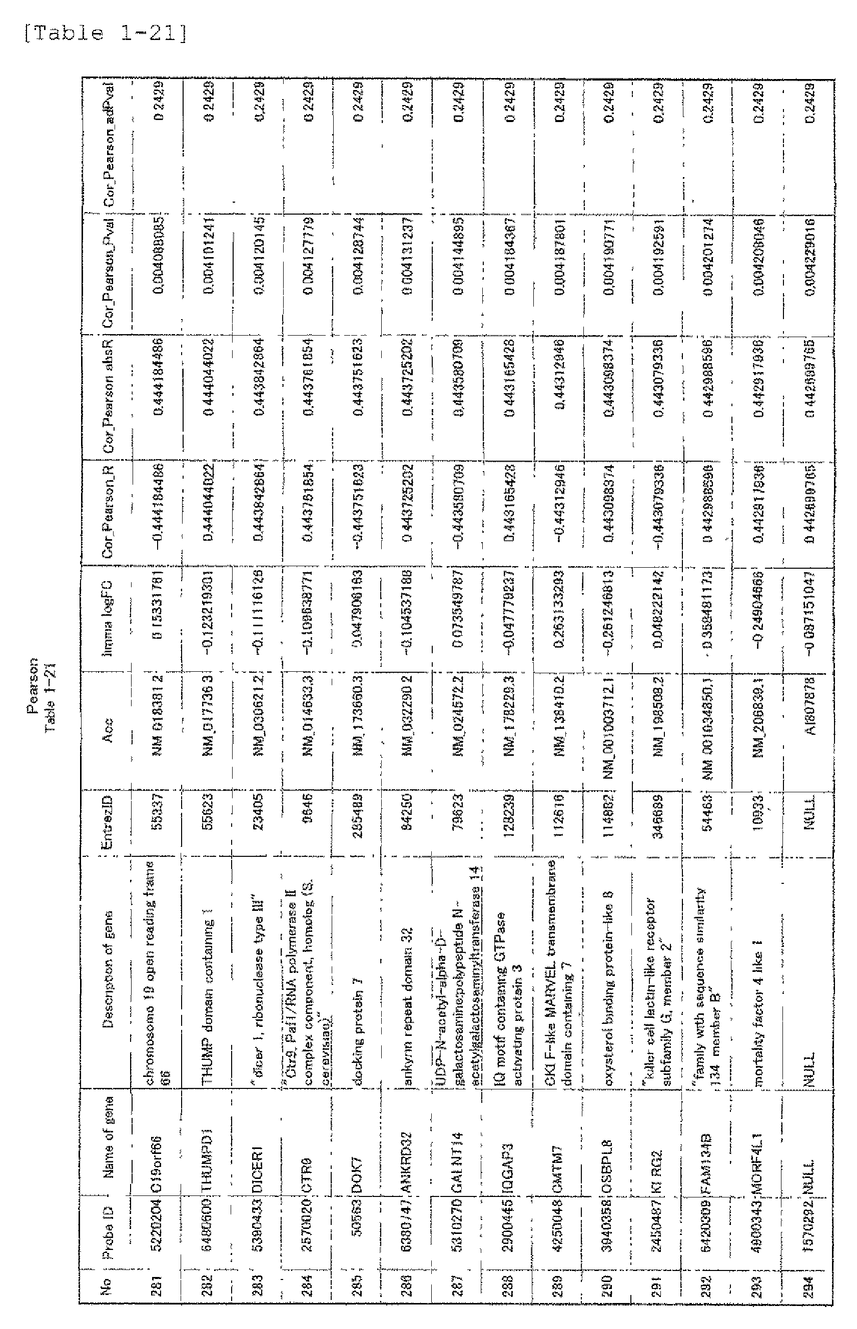

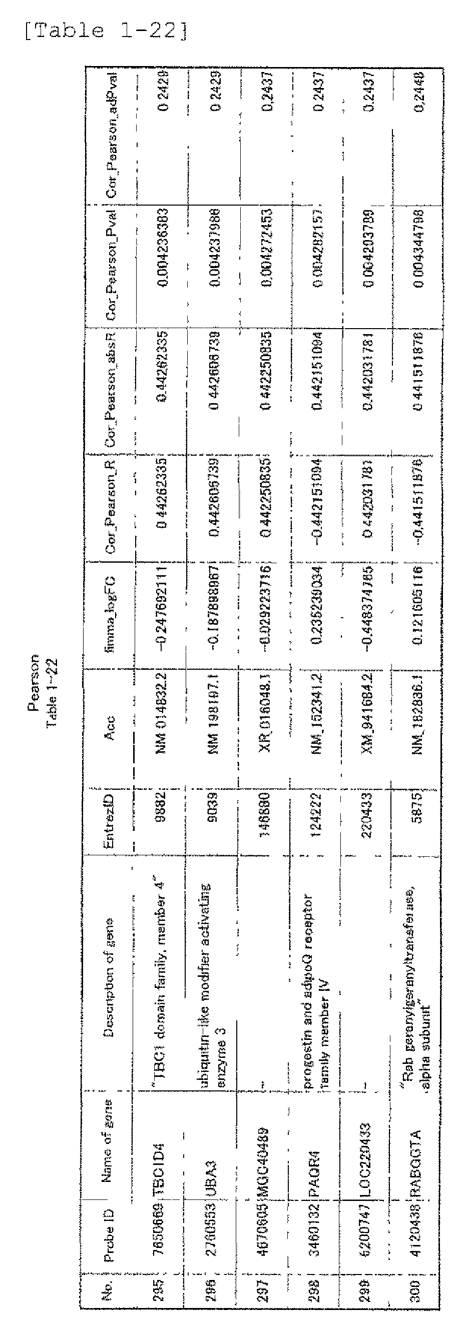

Table 2: List of top 300 genes selected by Limma.

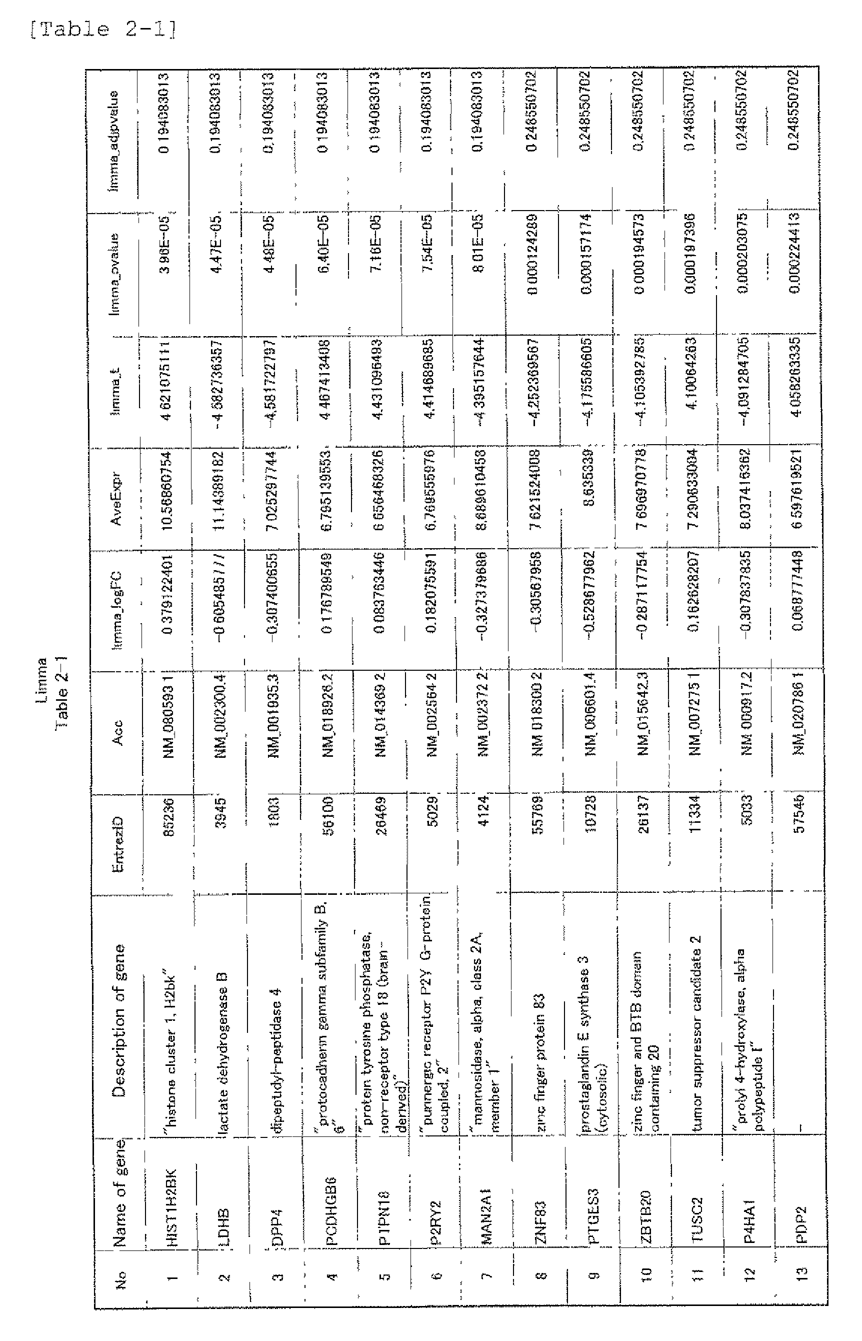

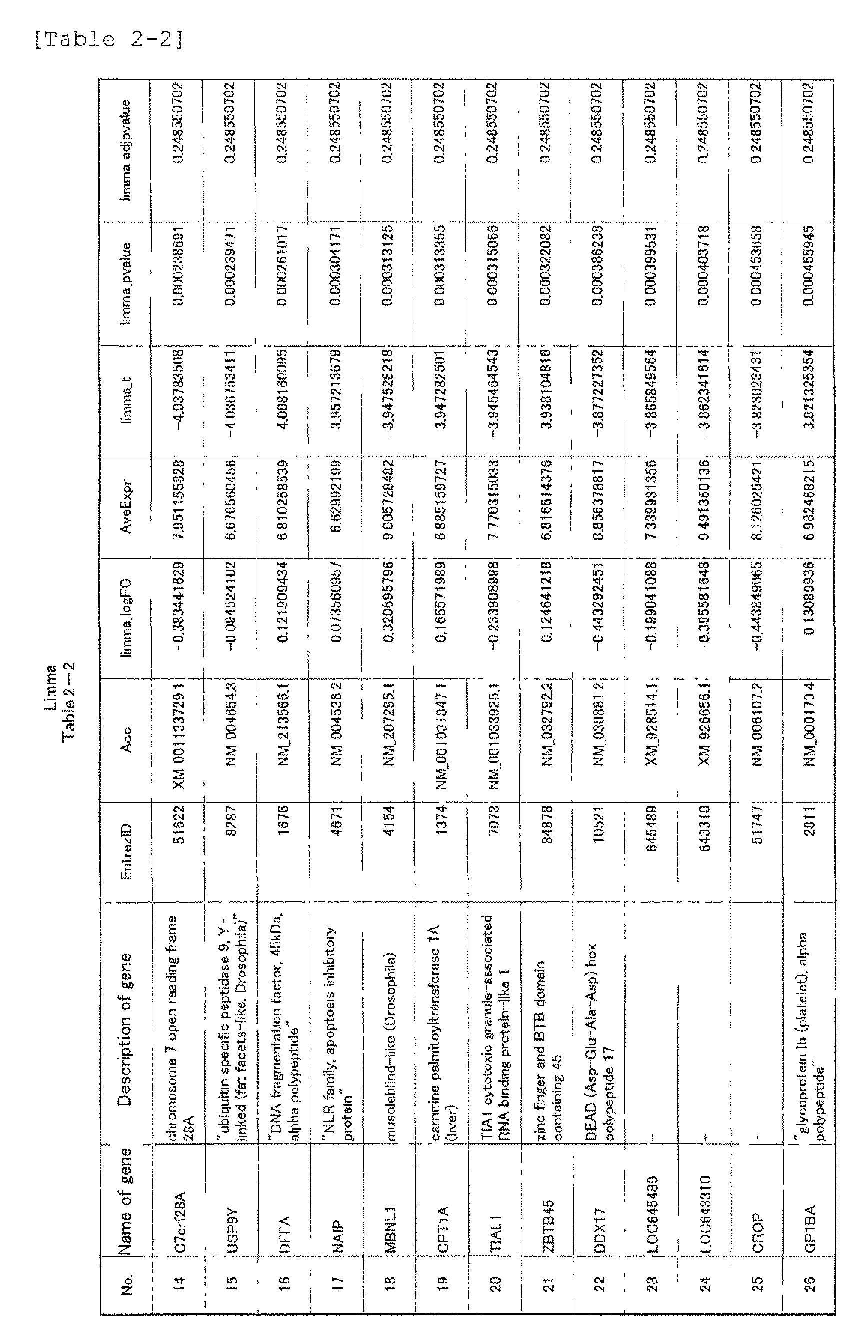

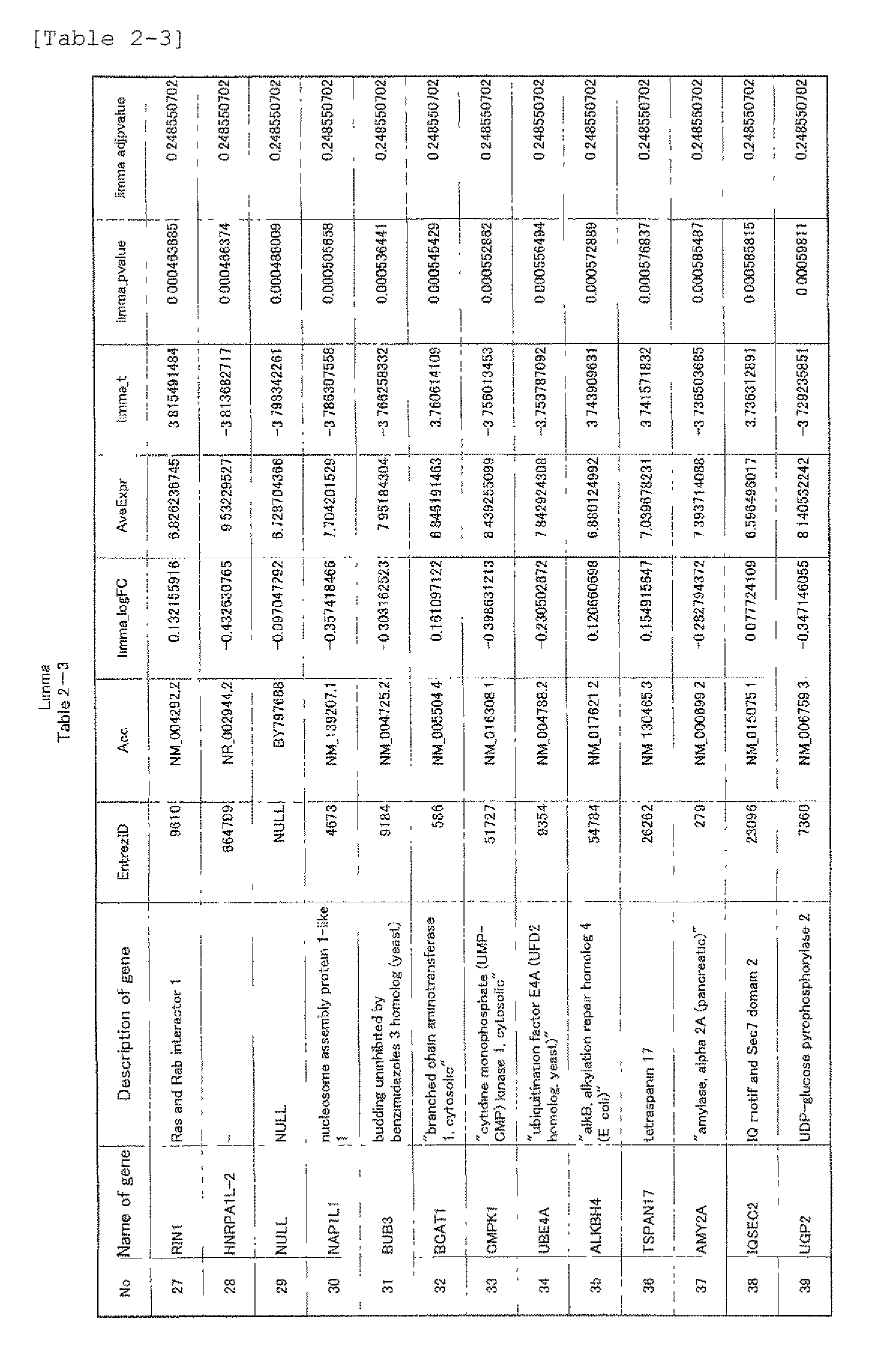

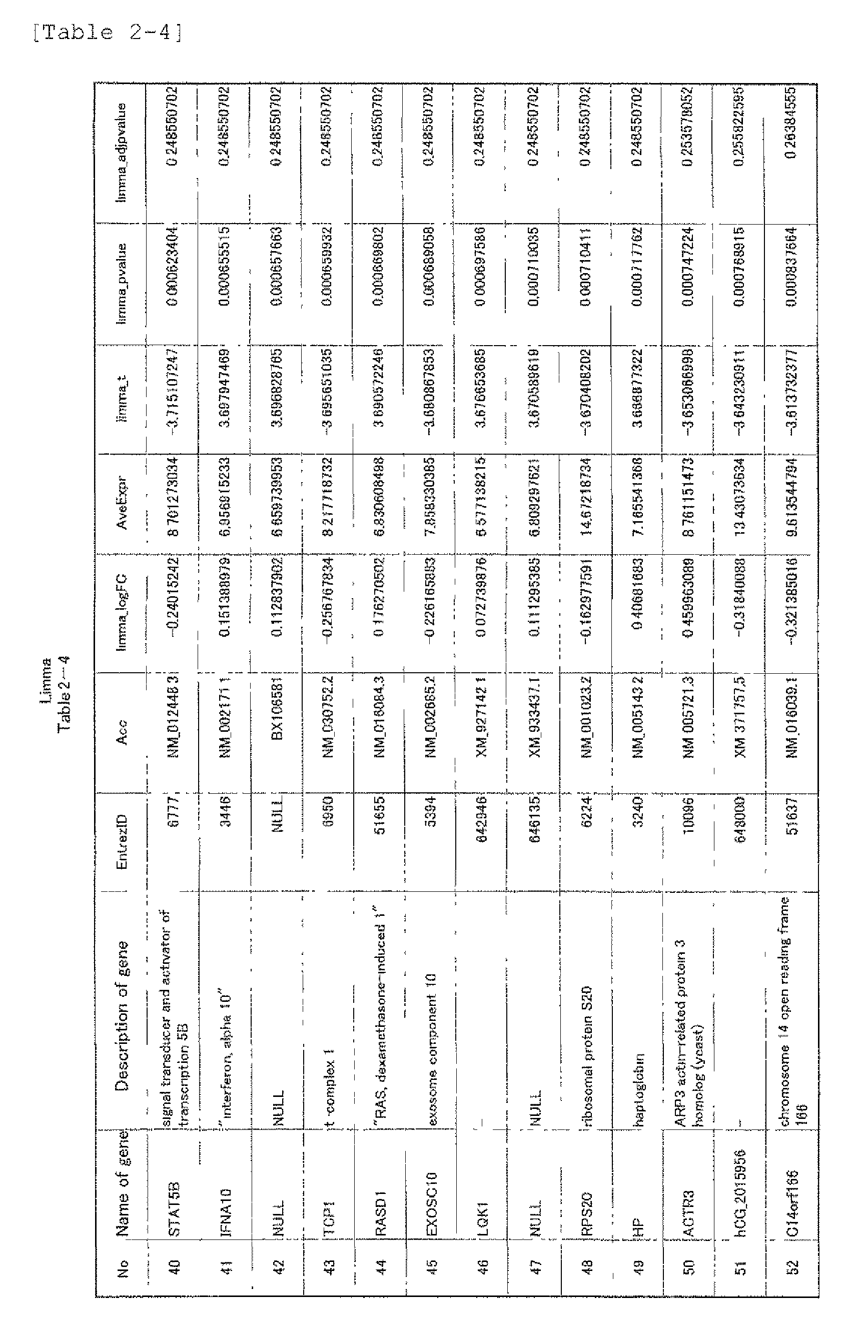

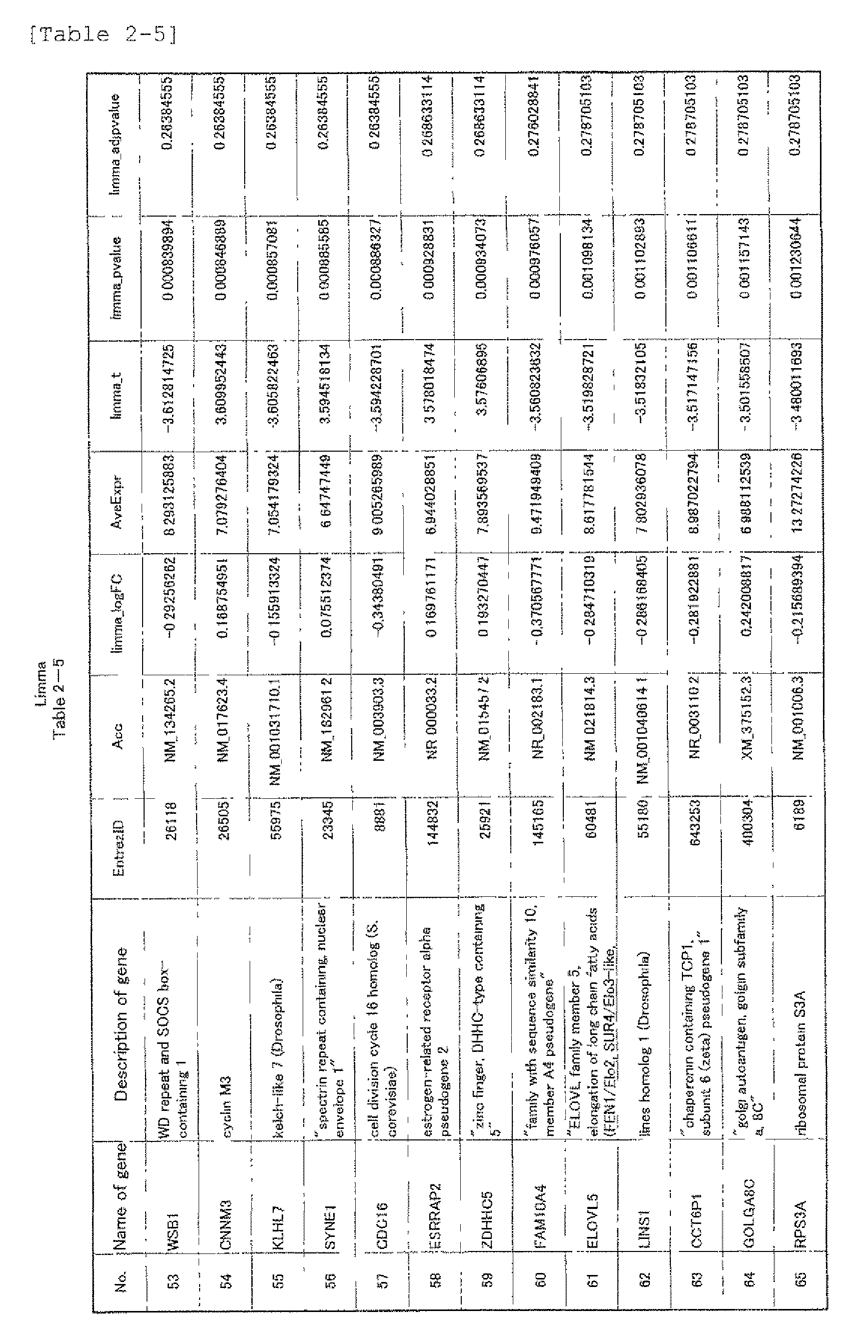

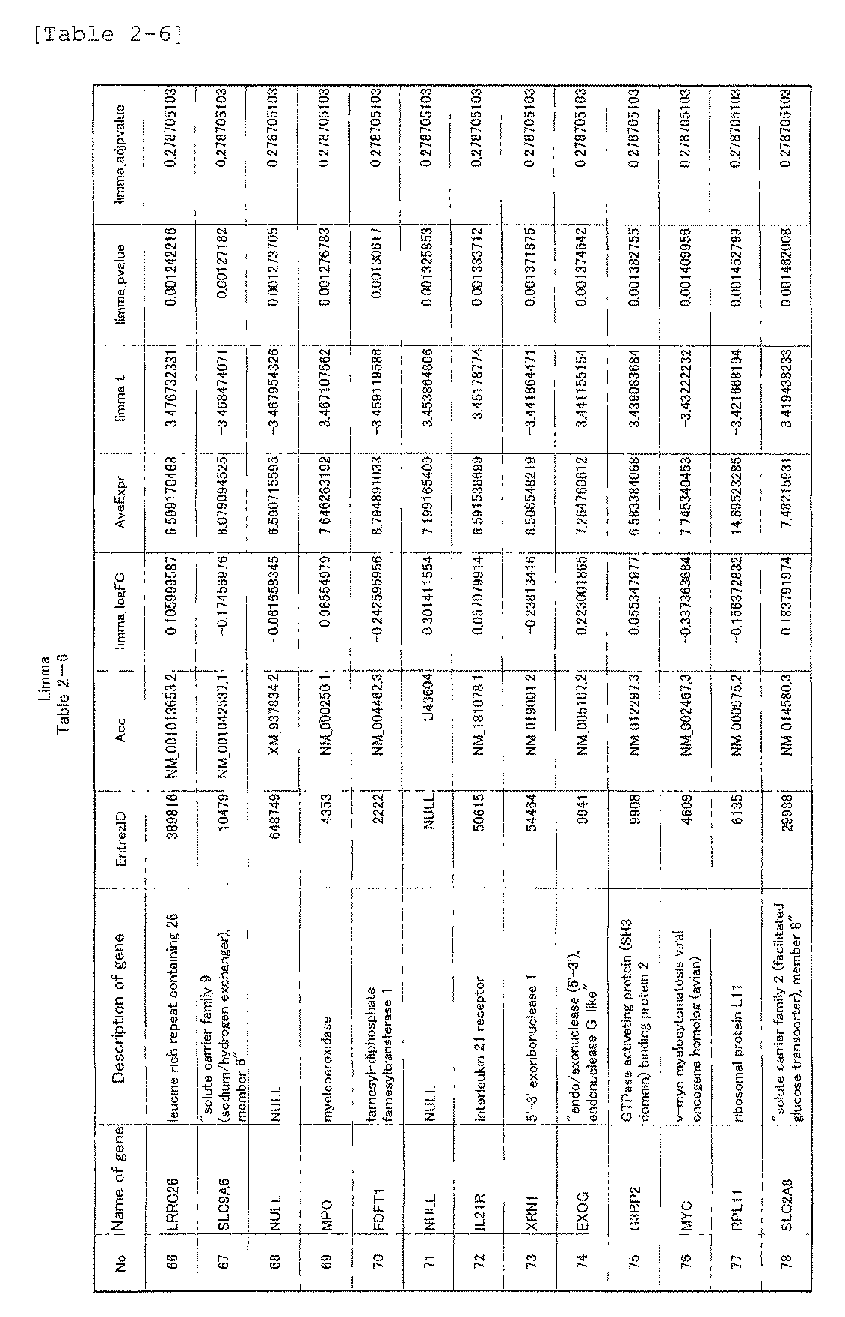

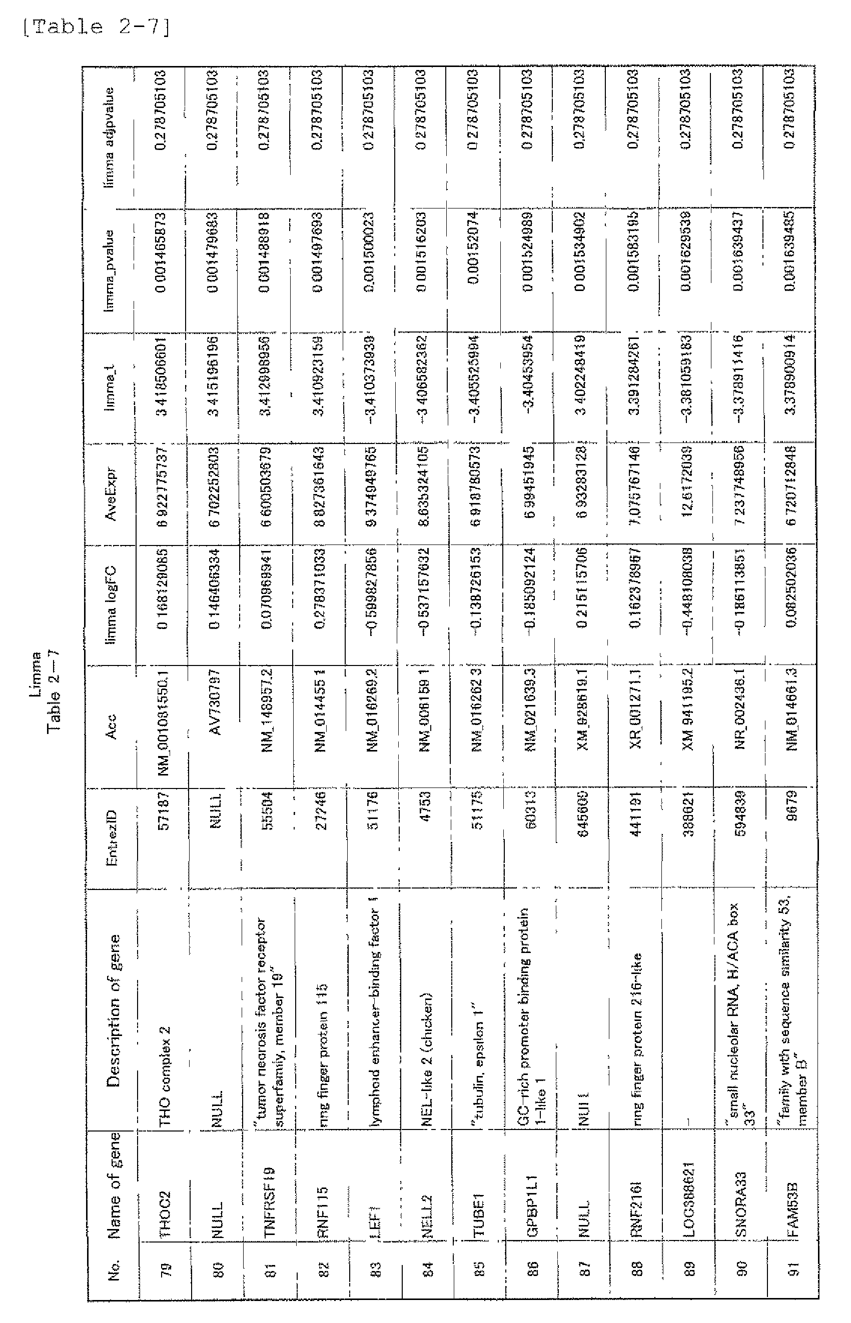

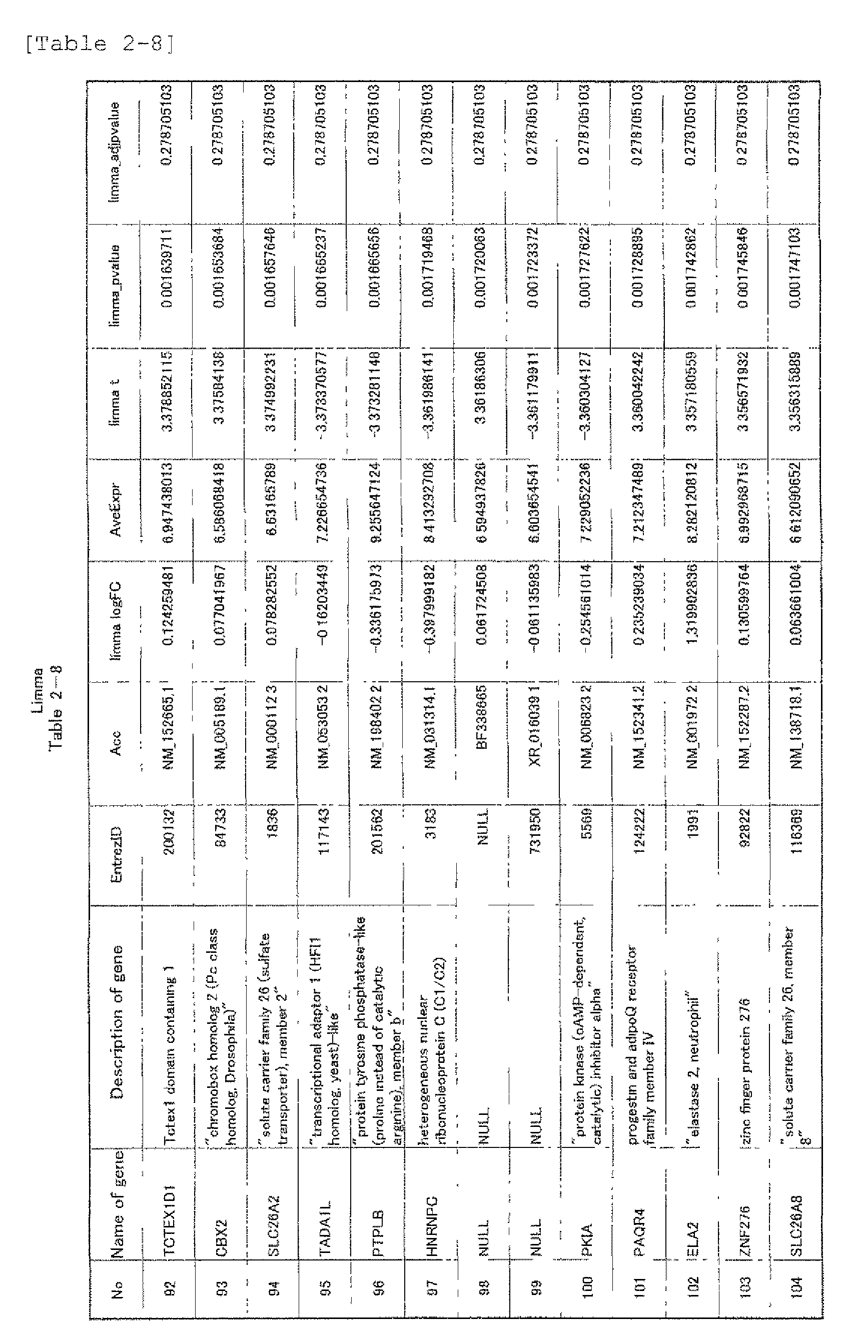

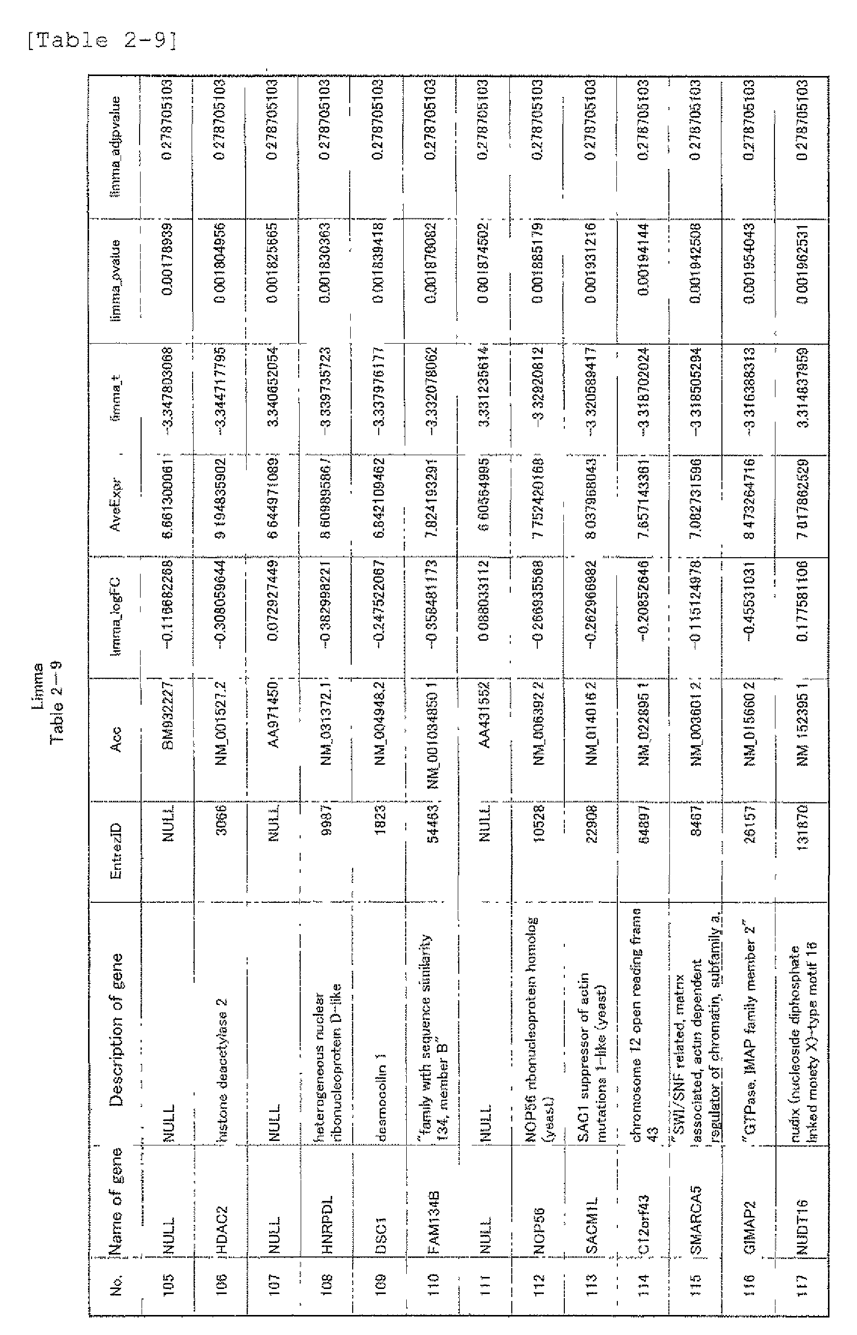

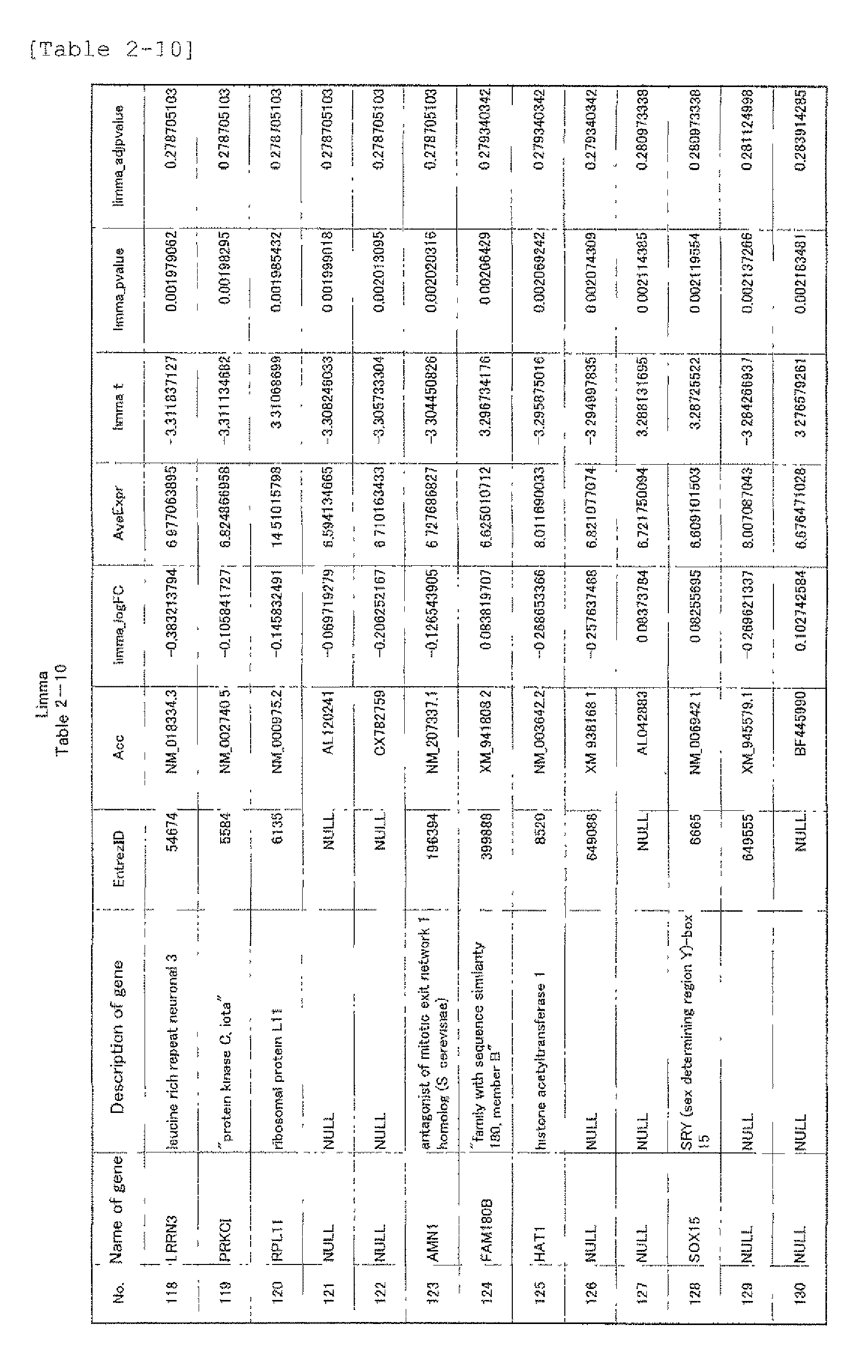

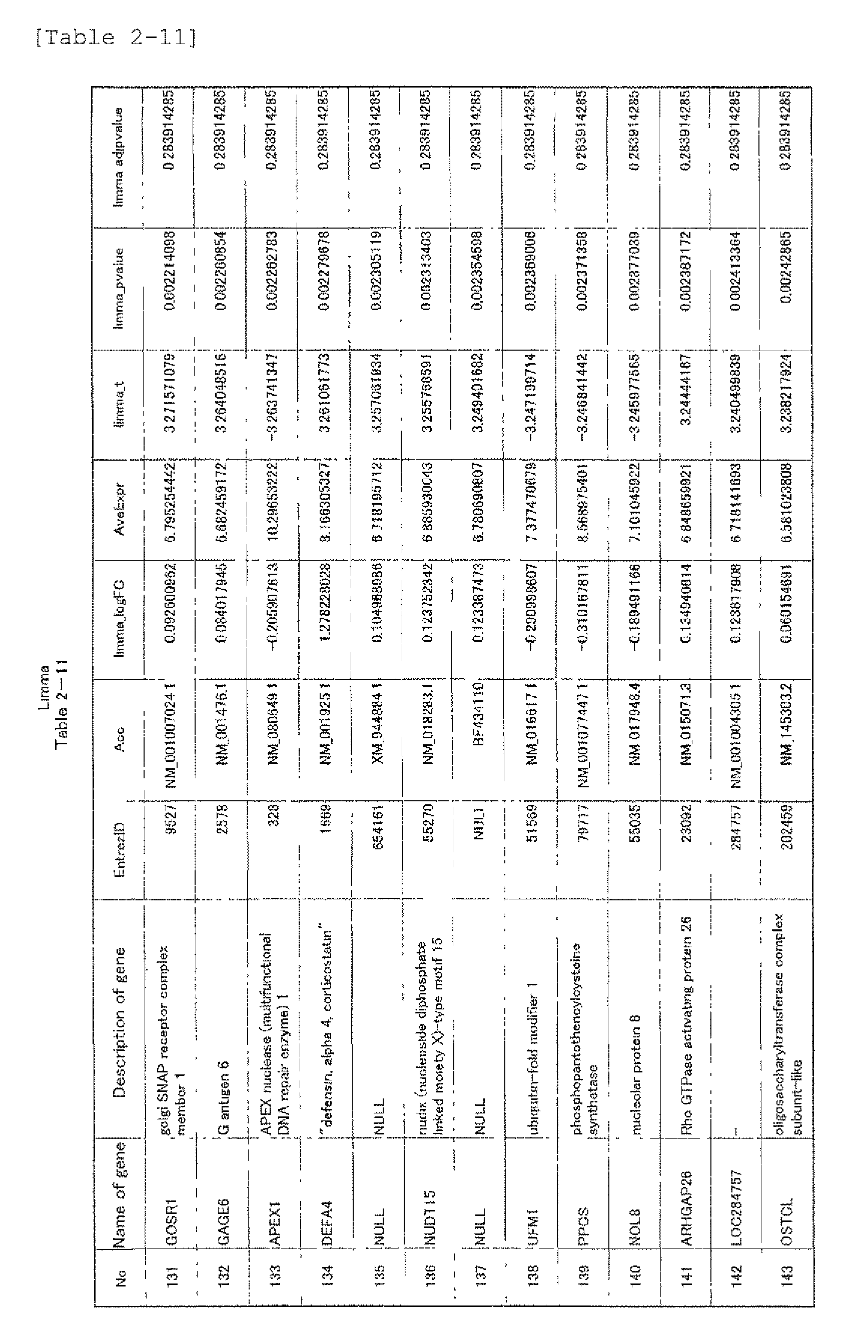

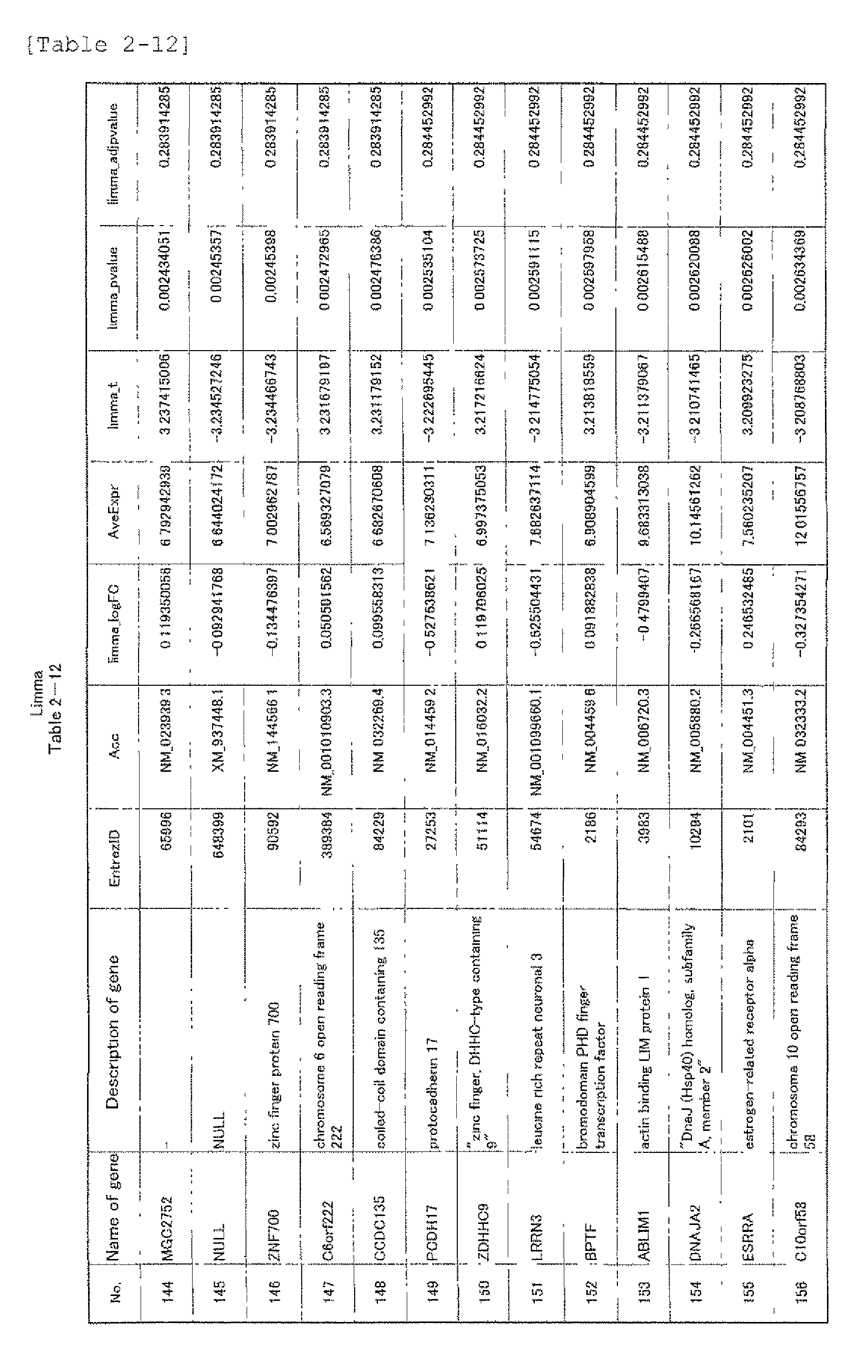

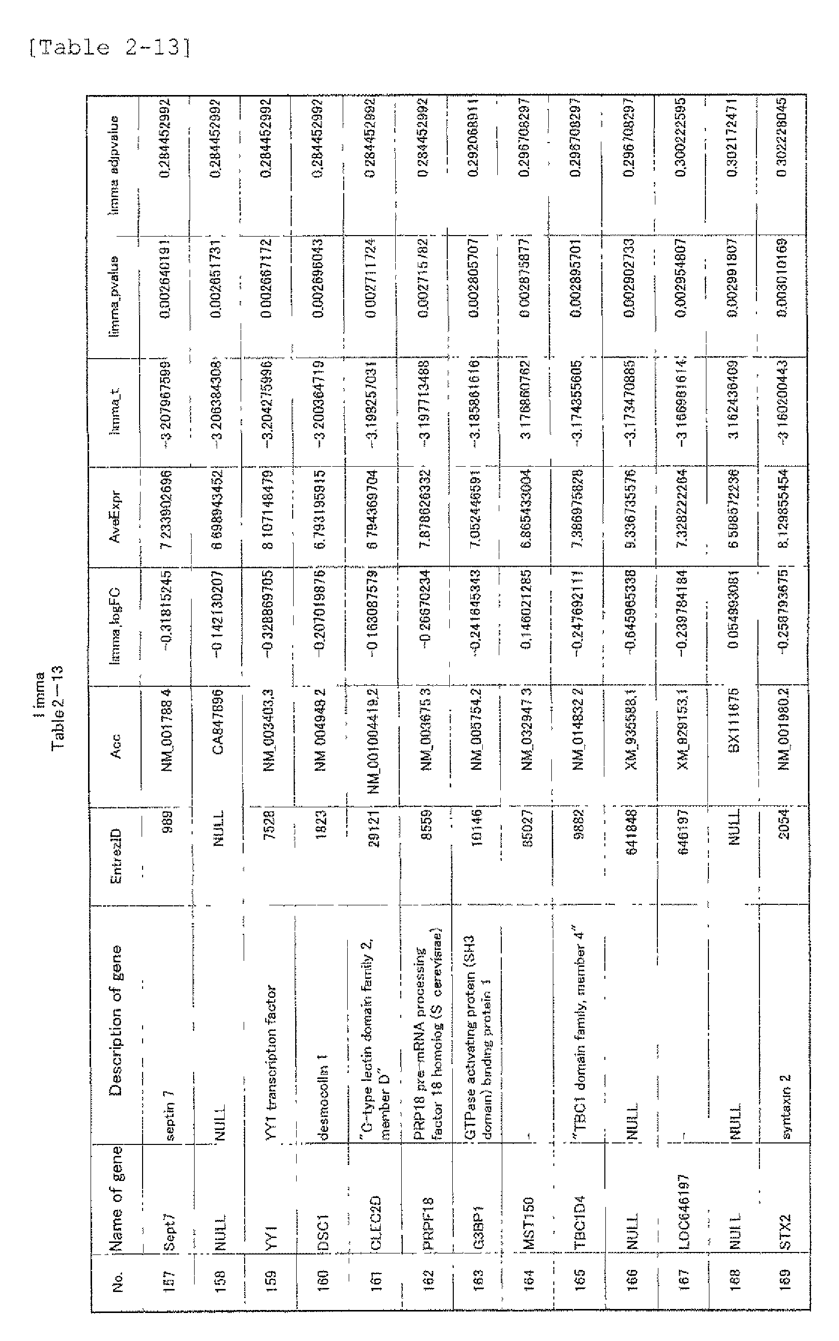

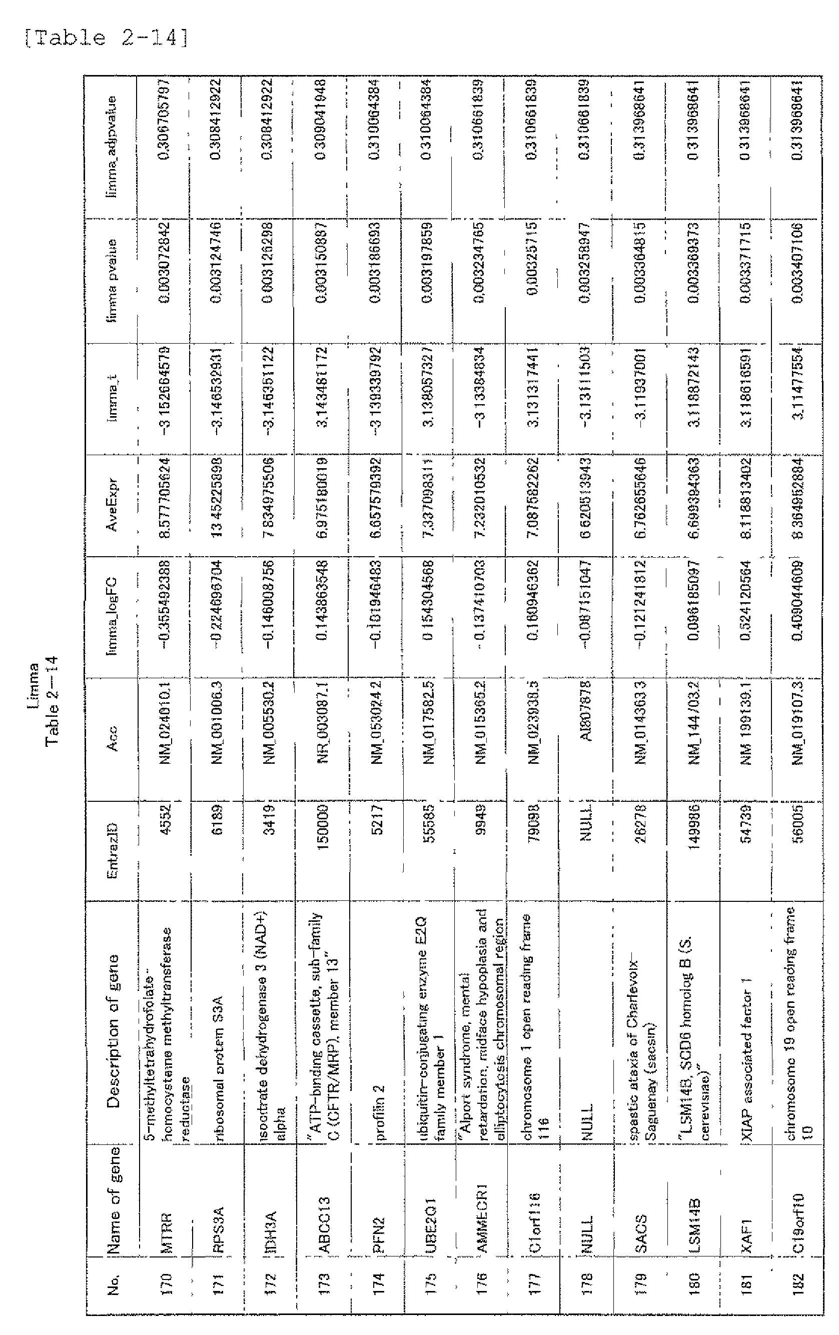

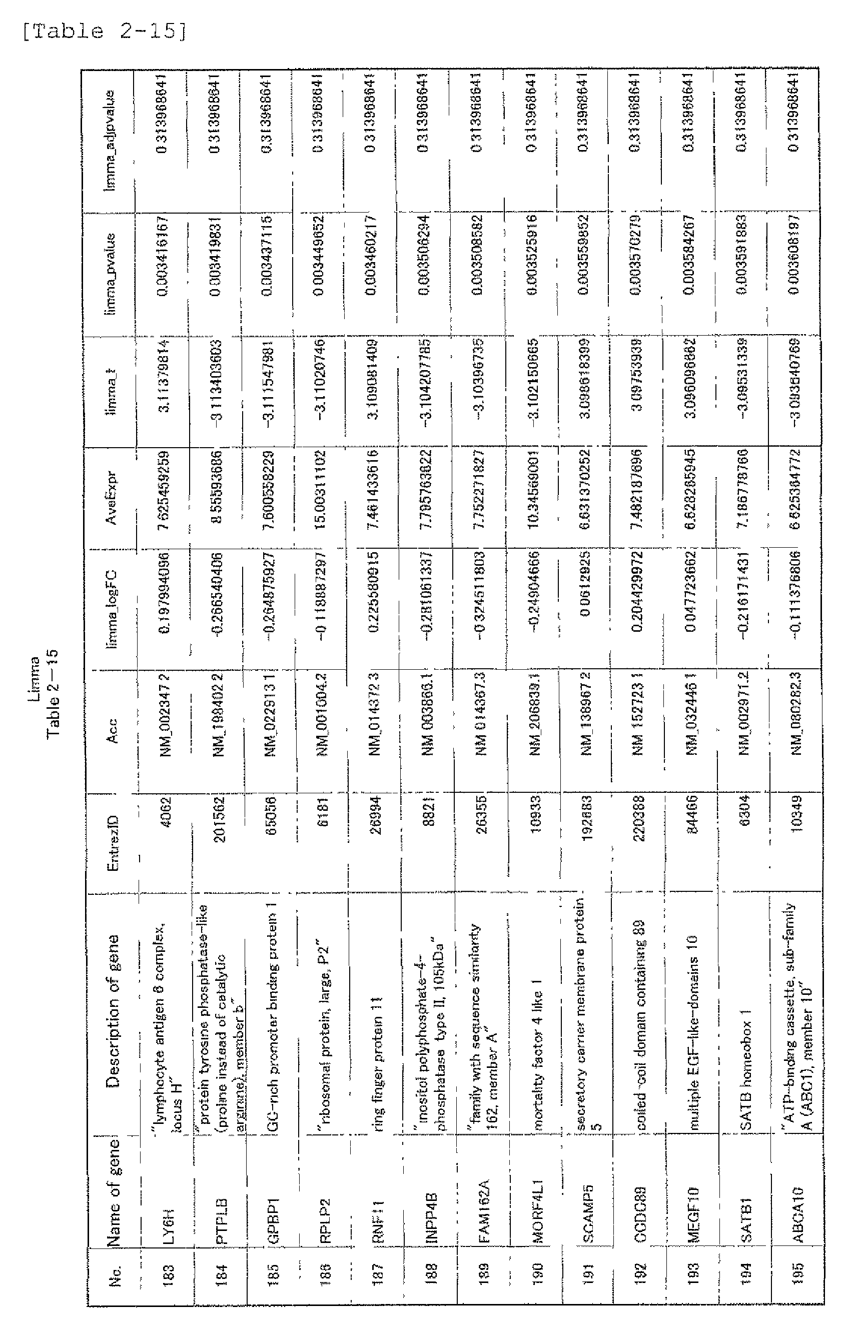

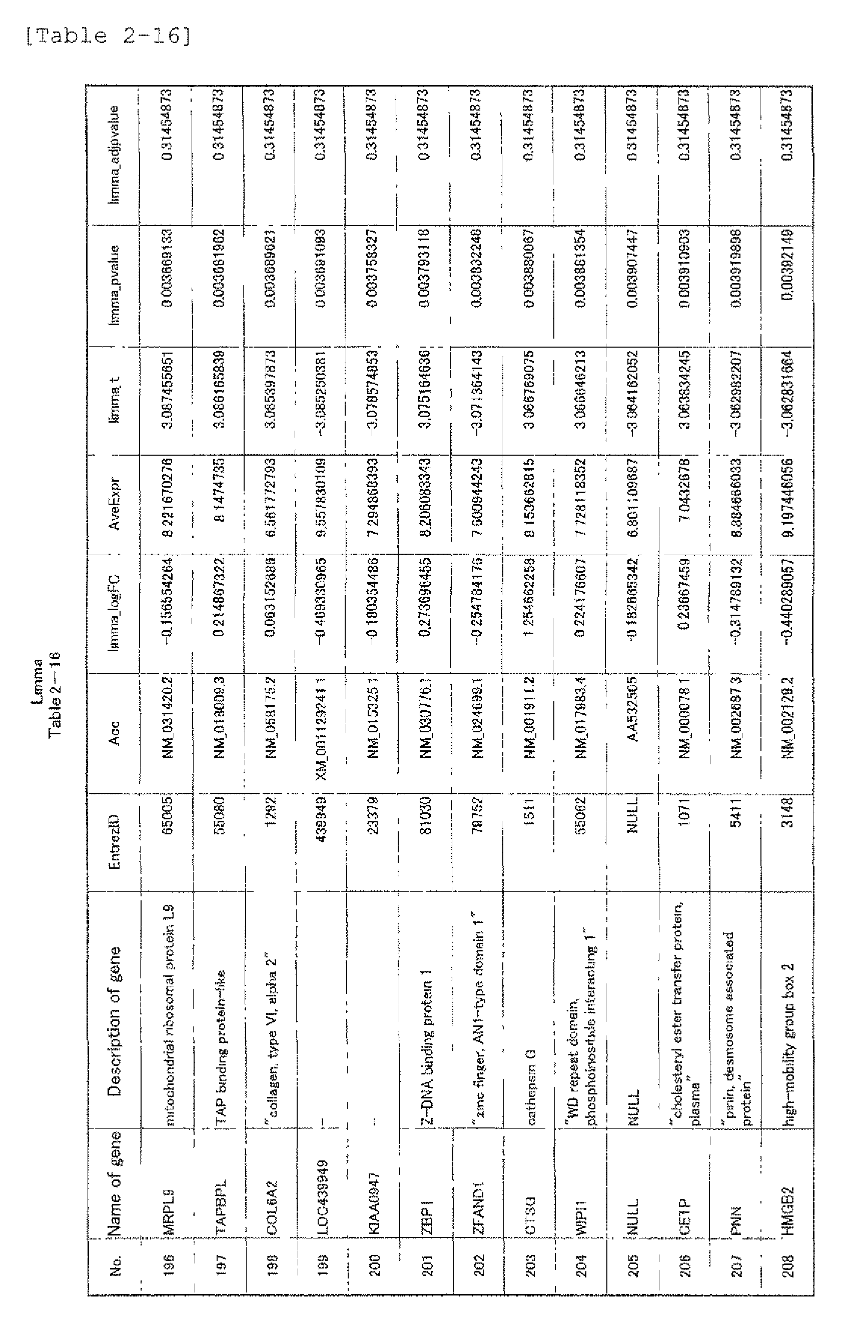

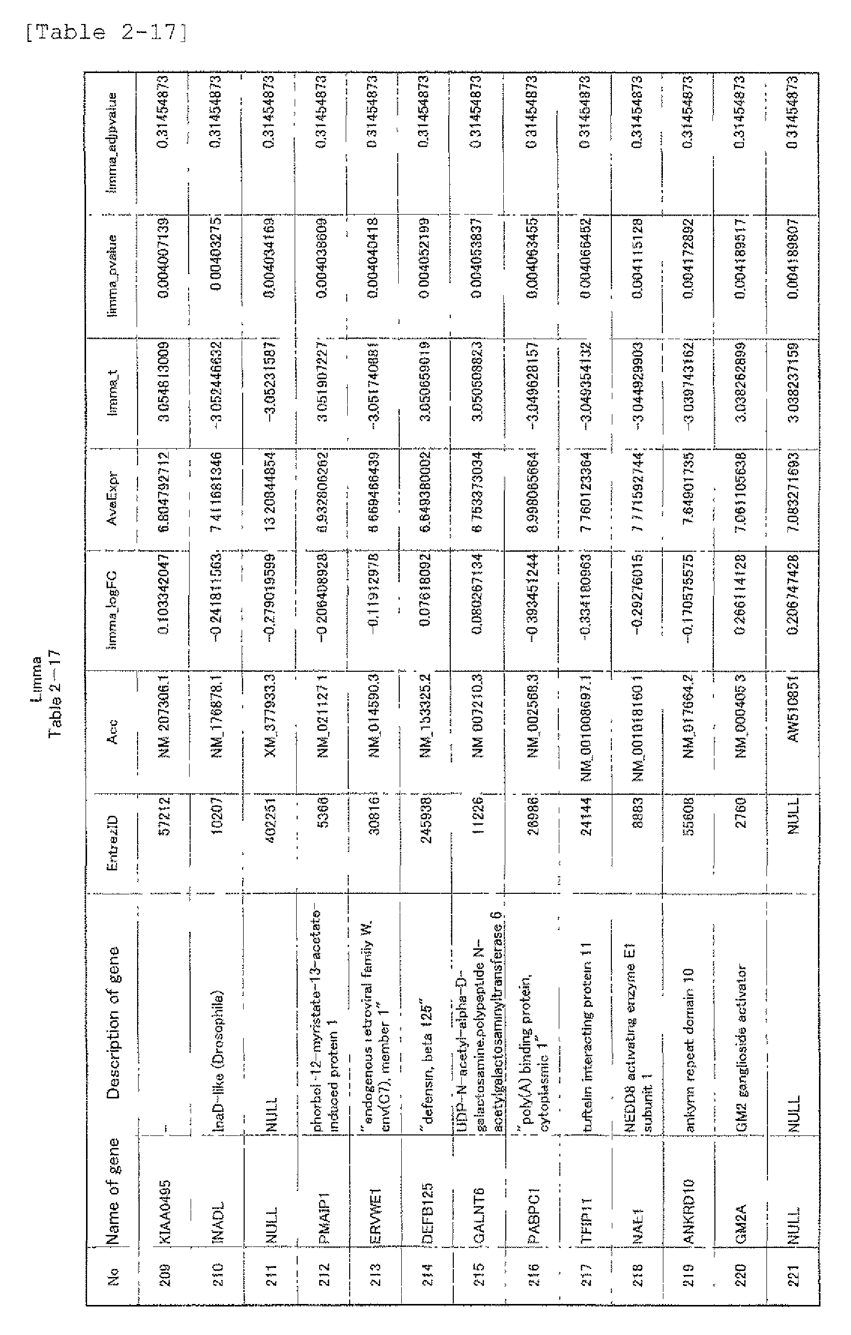

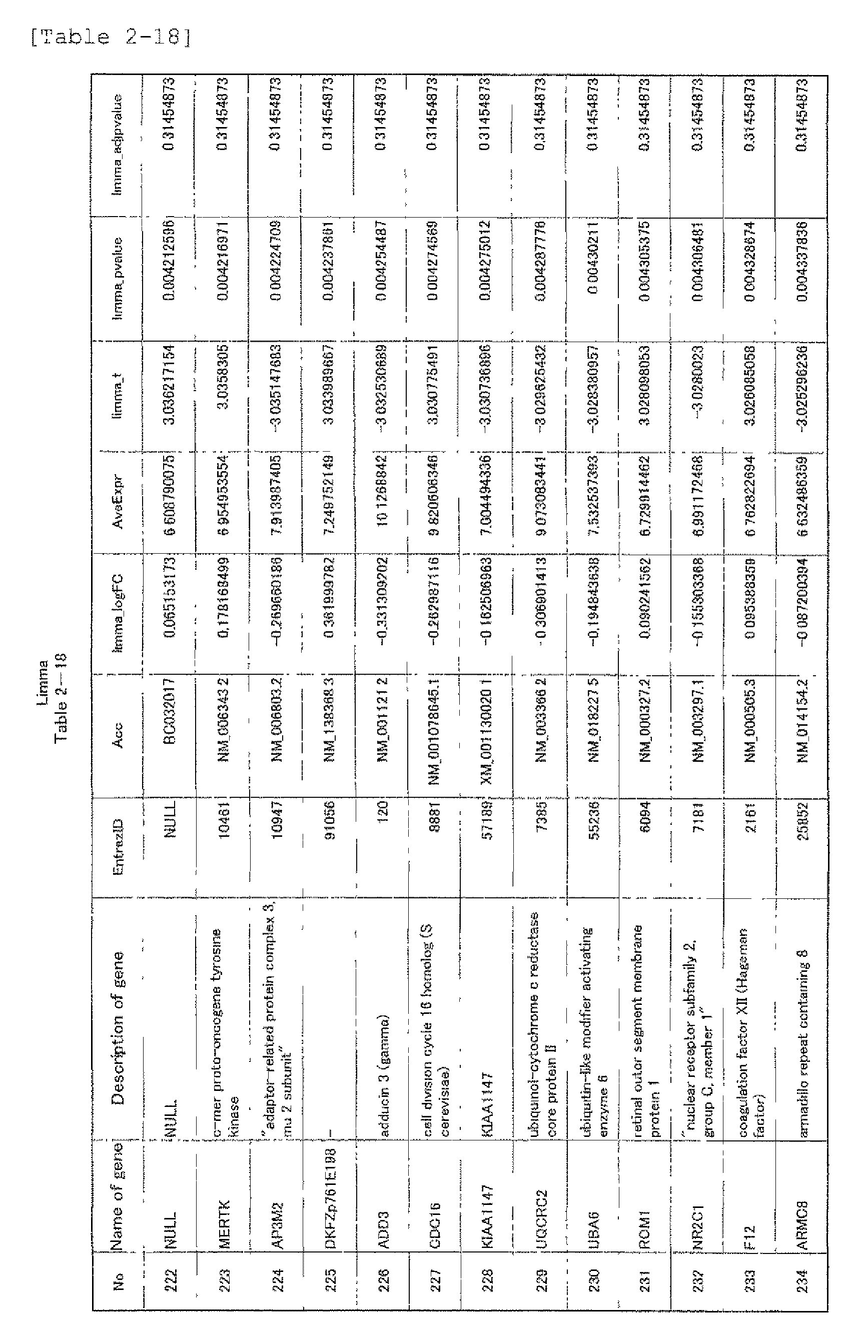

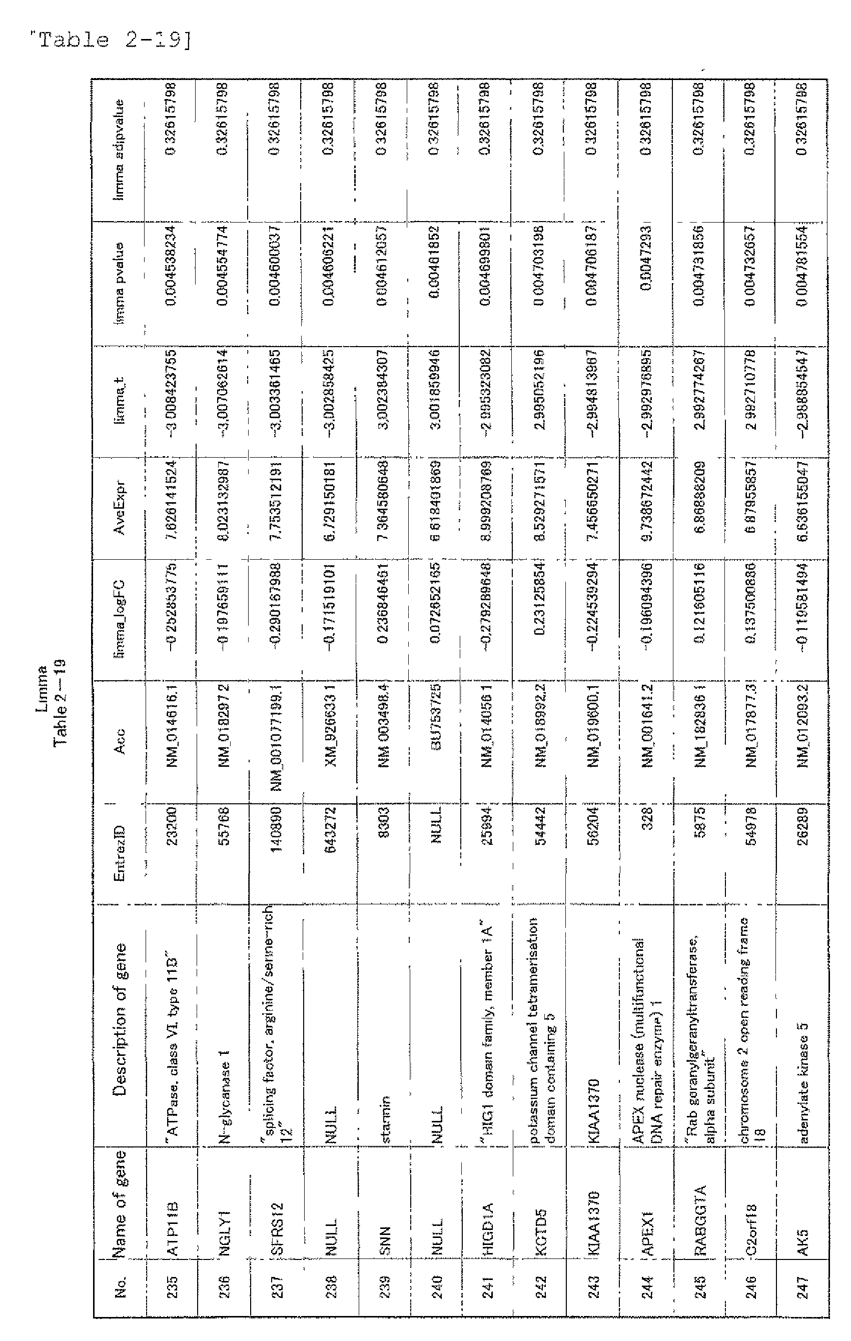

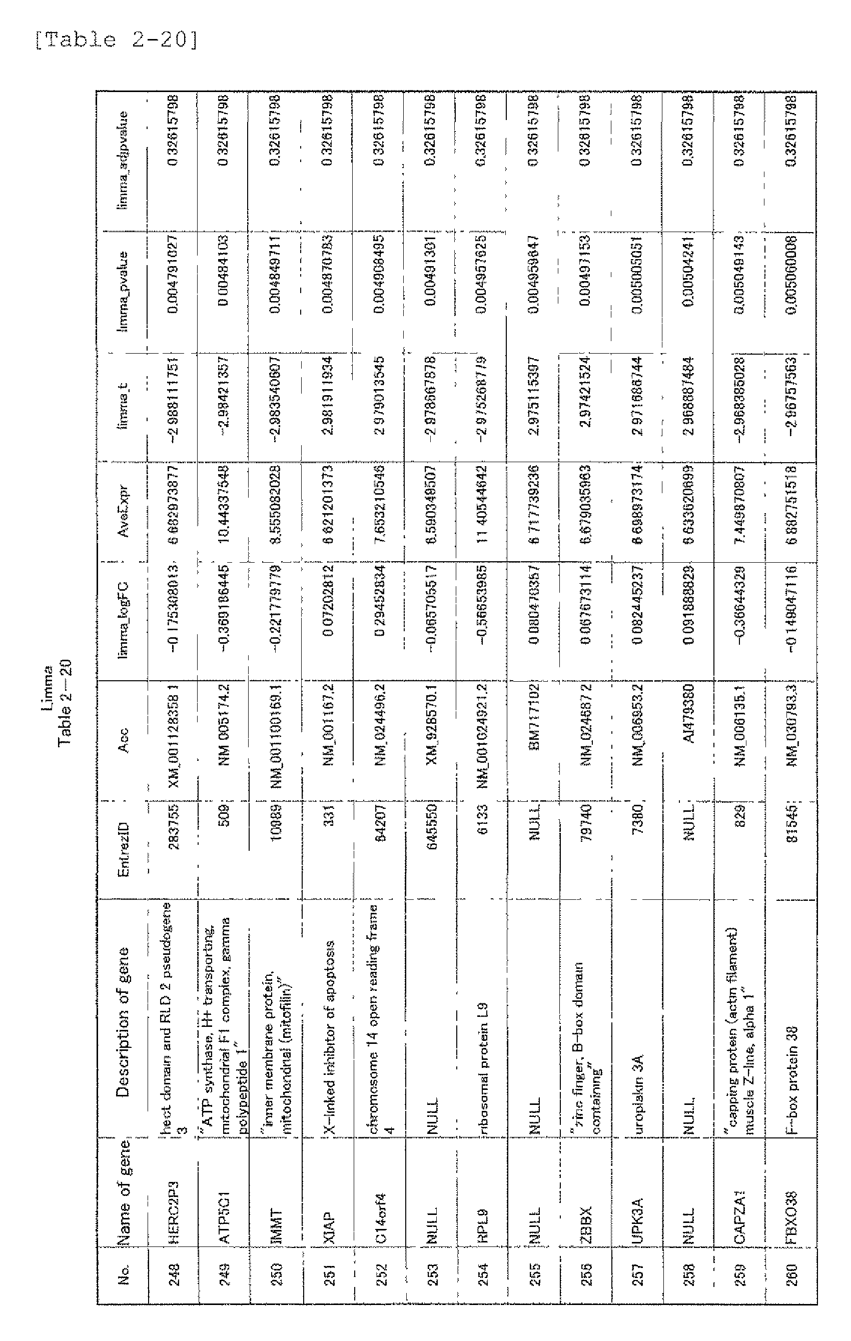

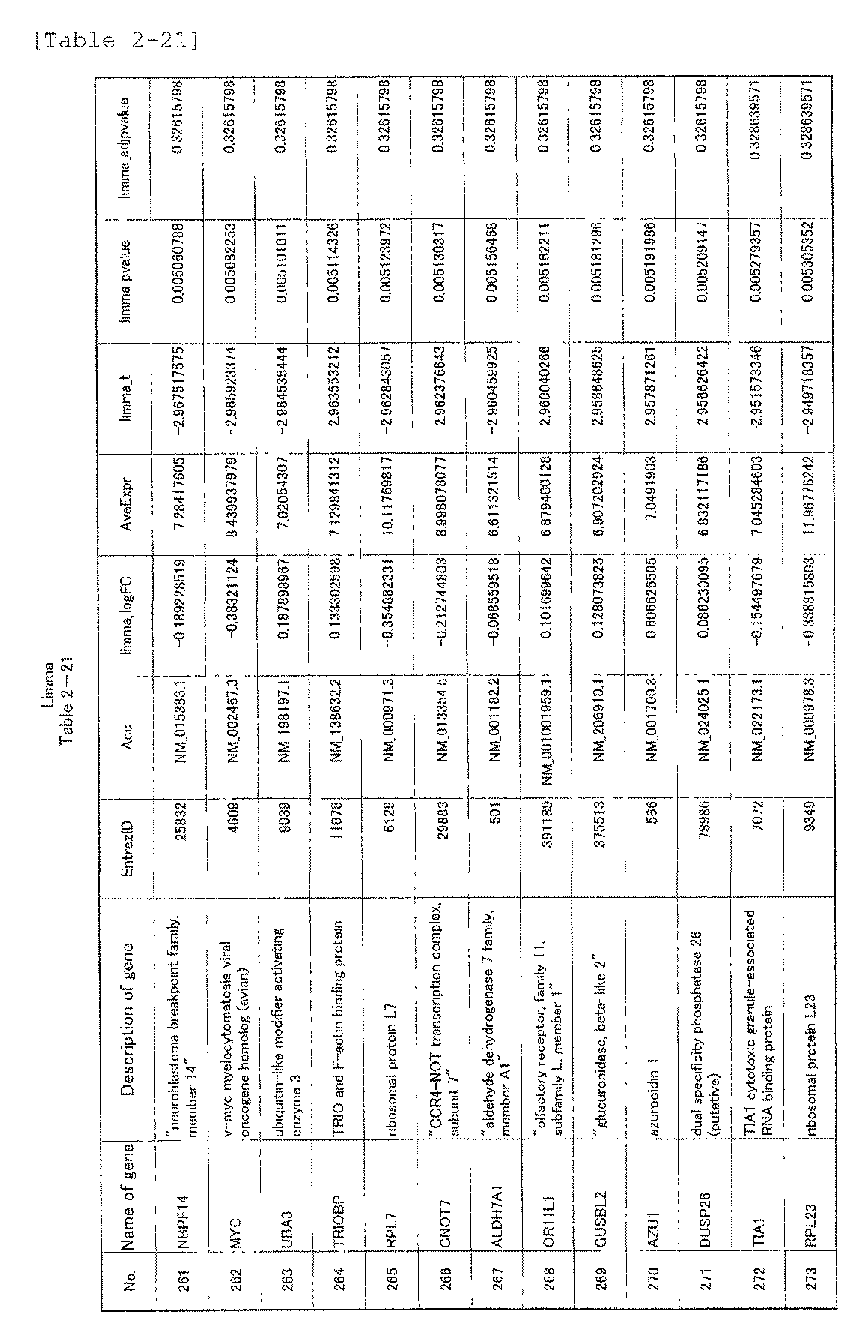

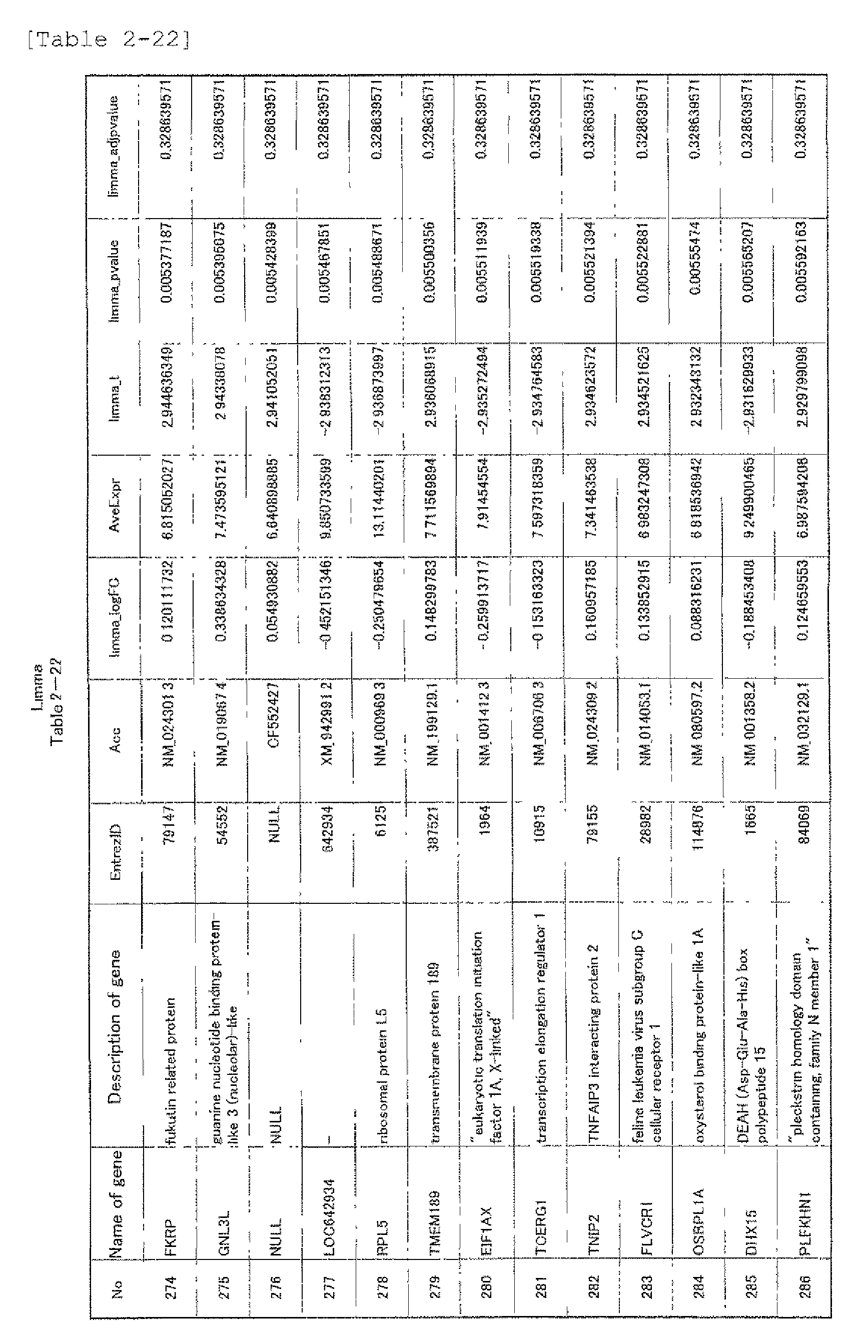

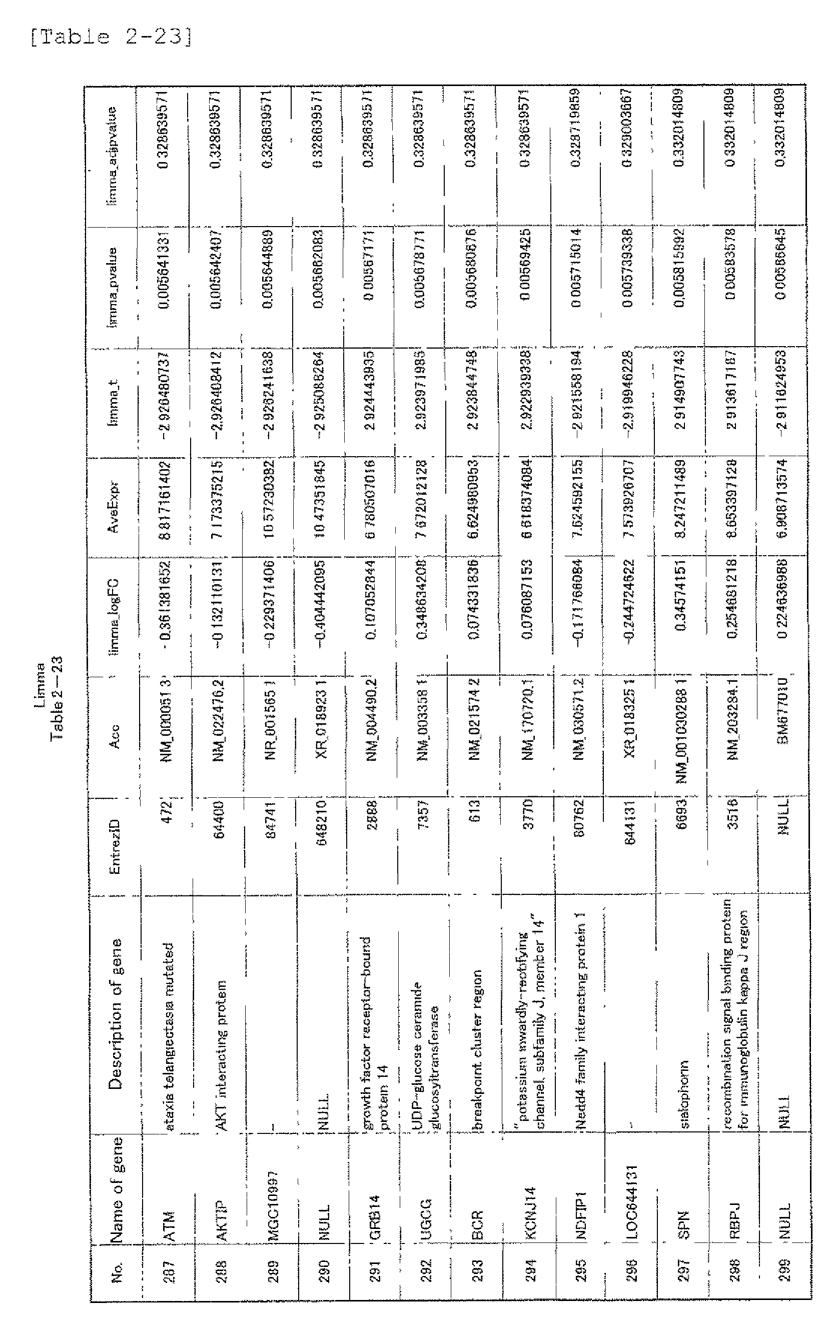

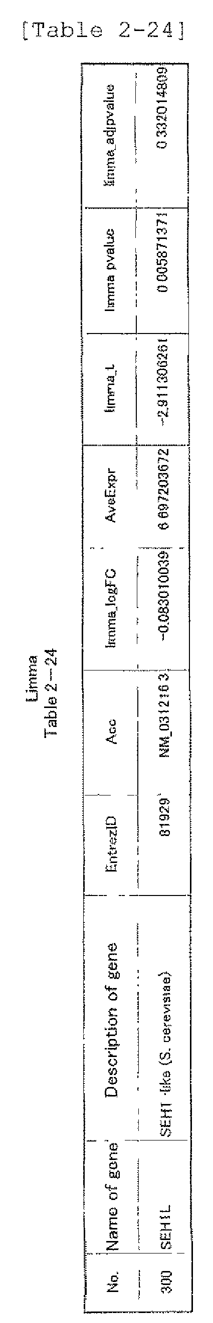

Table 3: List of top 300 genes selected by SAM.

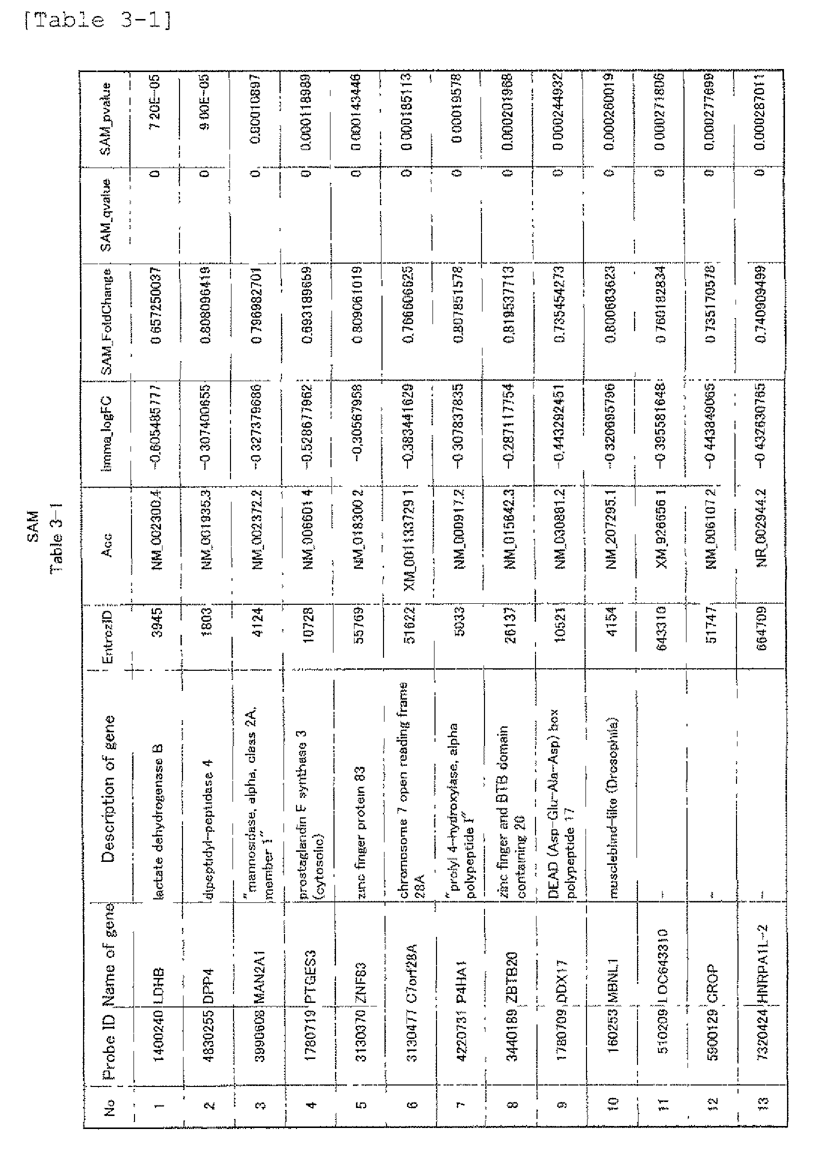

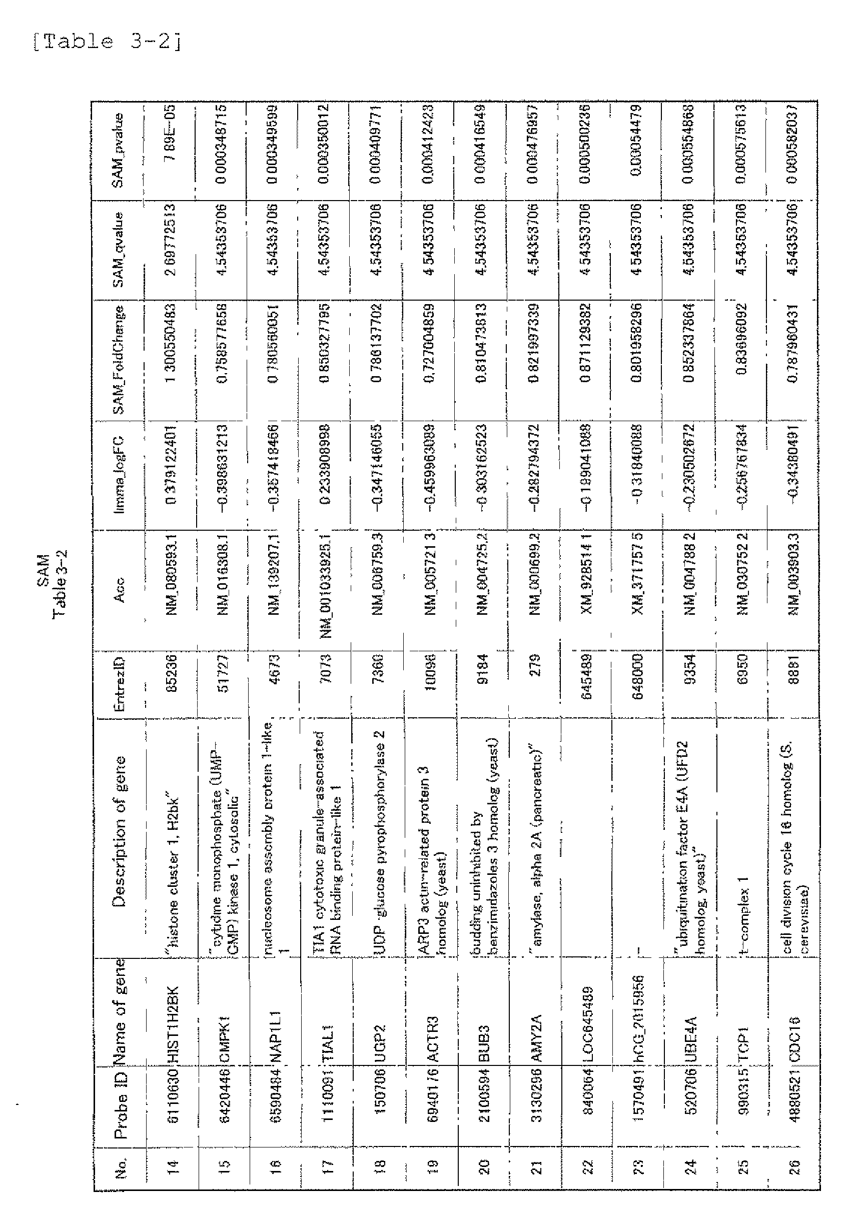

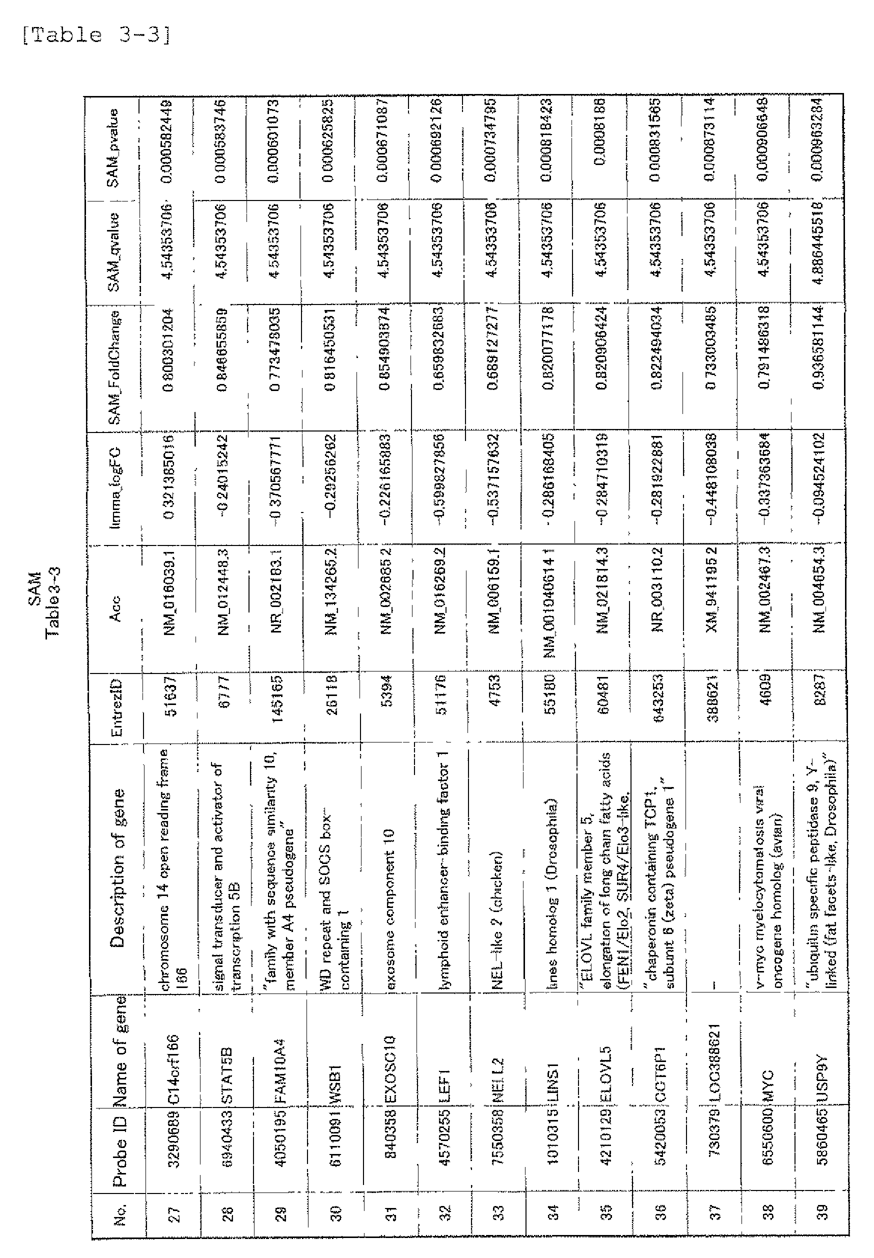

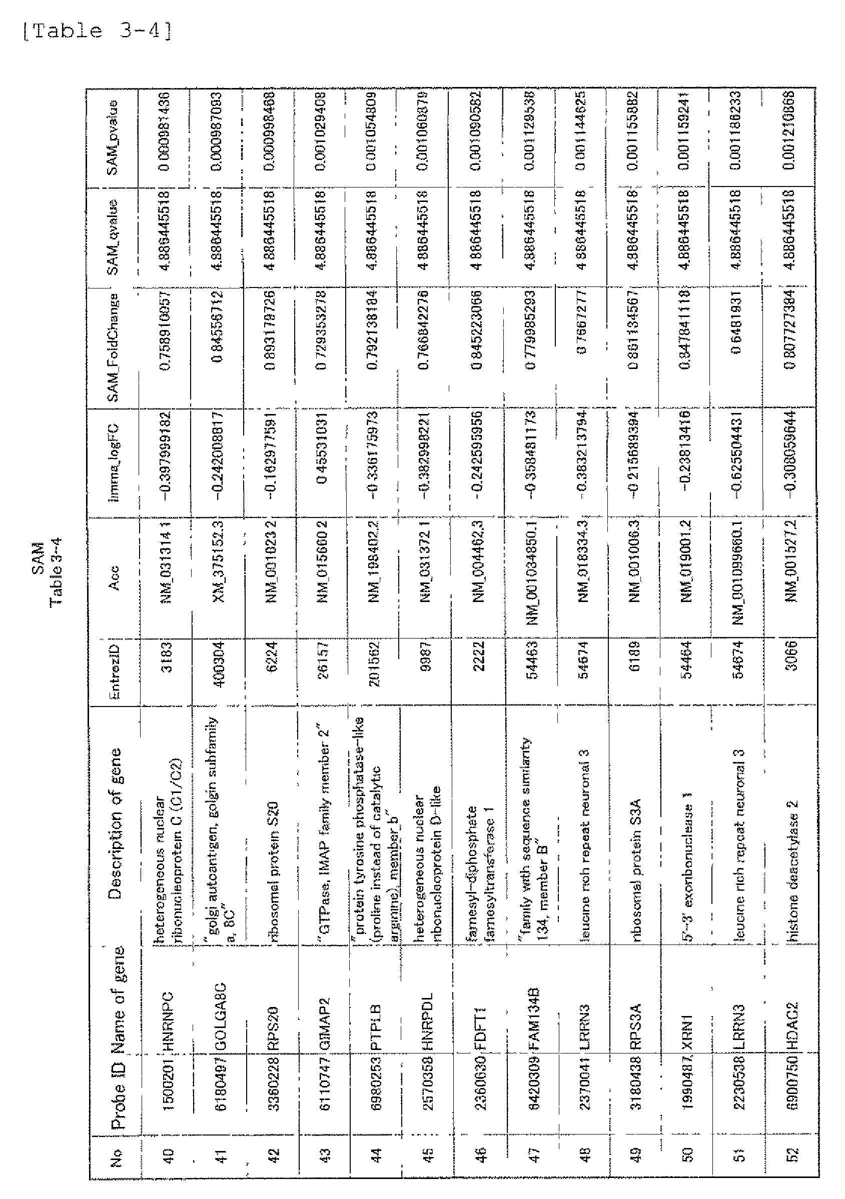

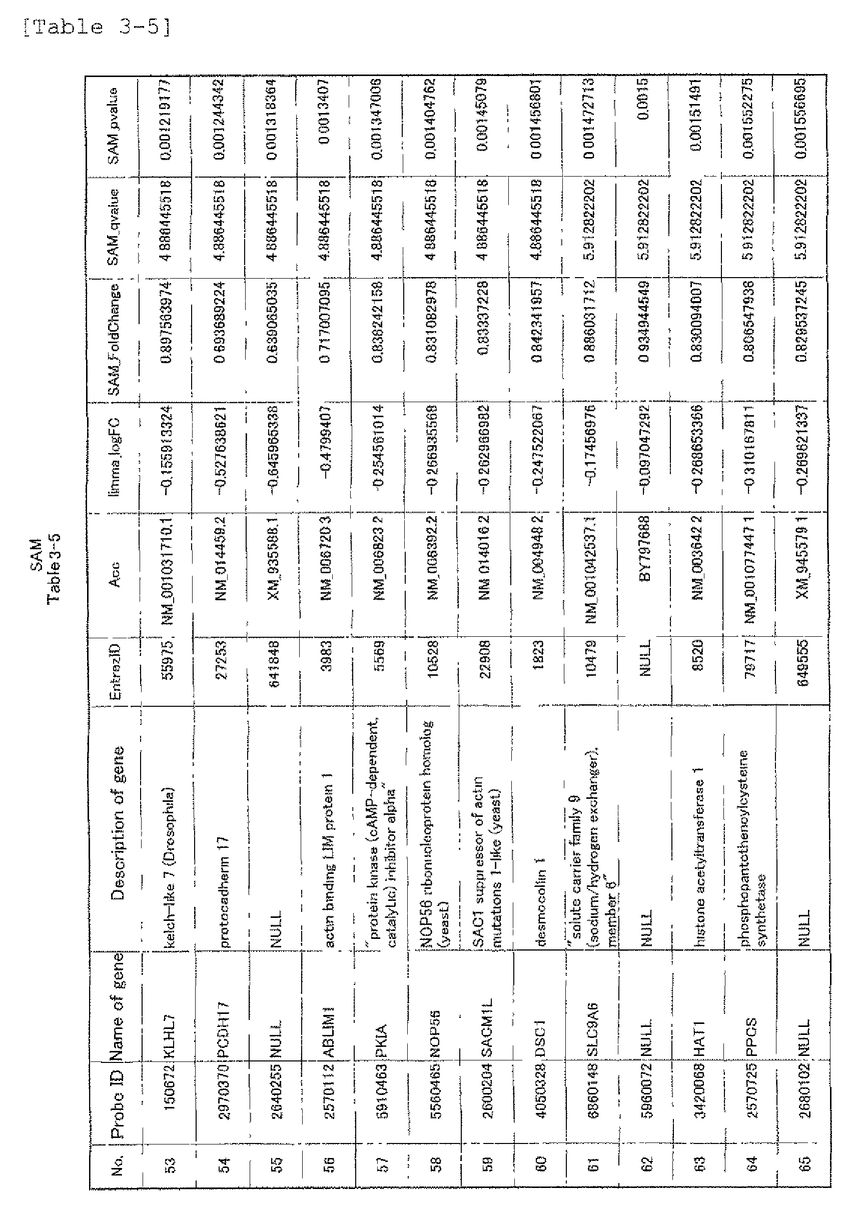

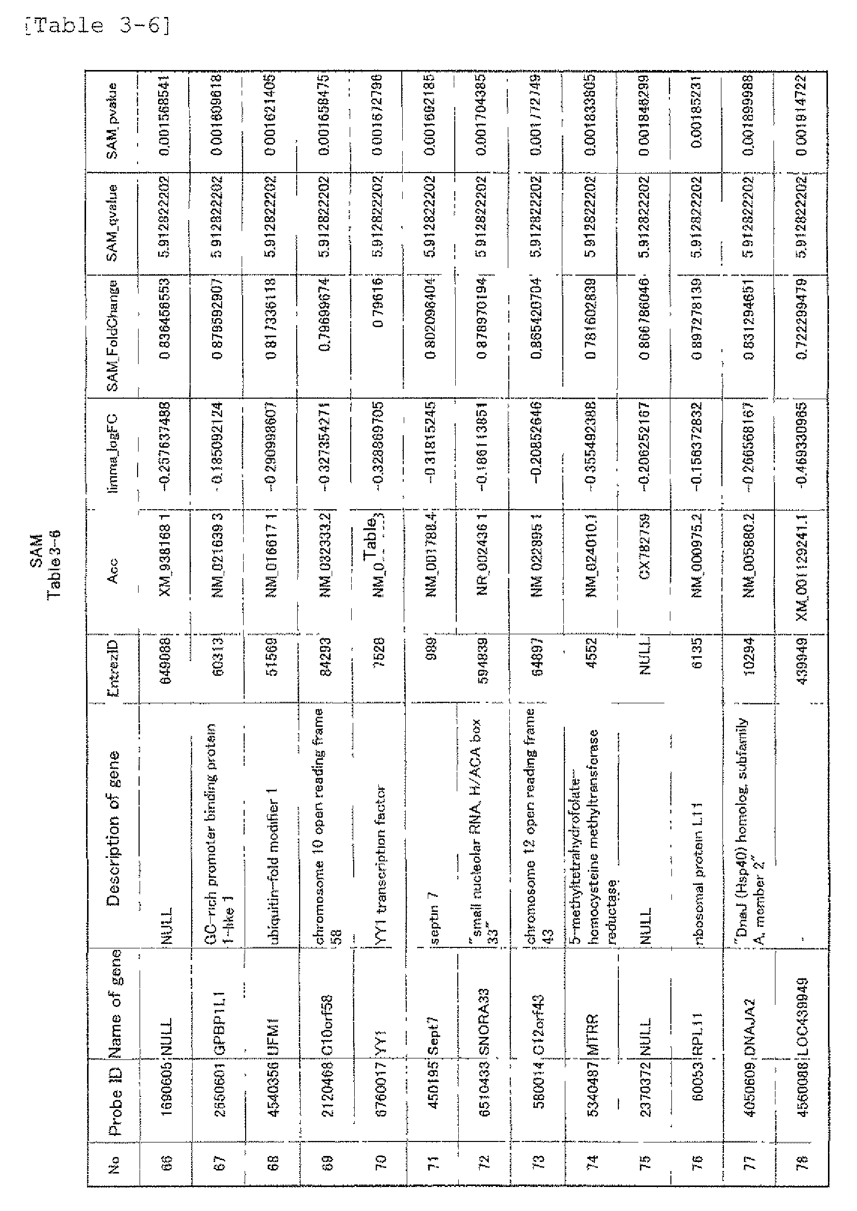

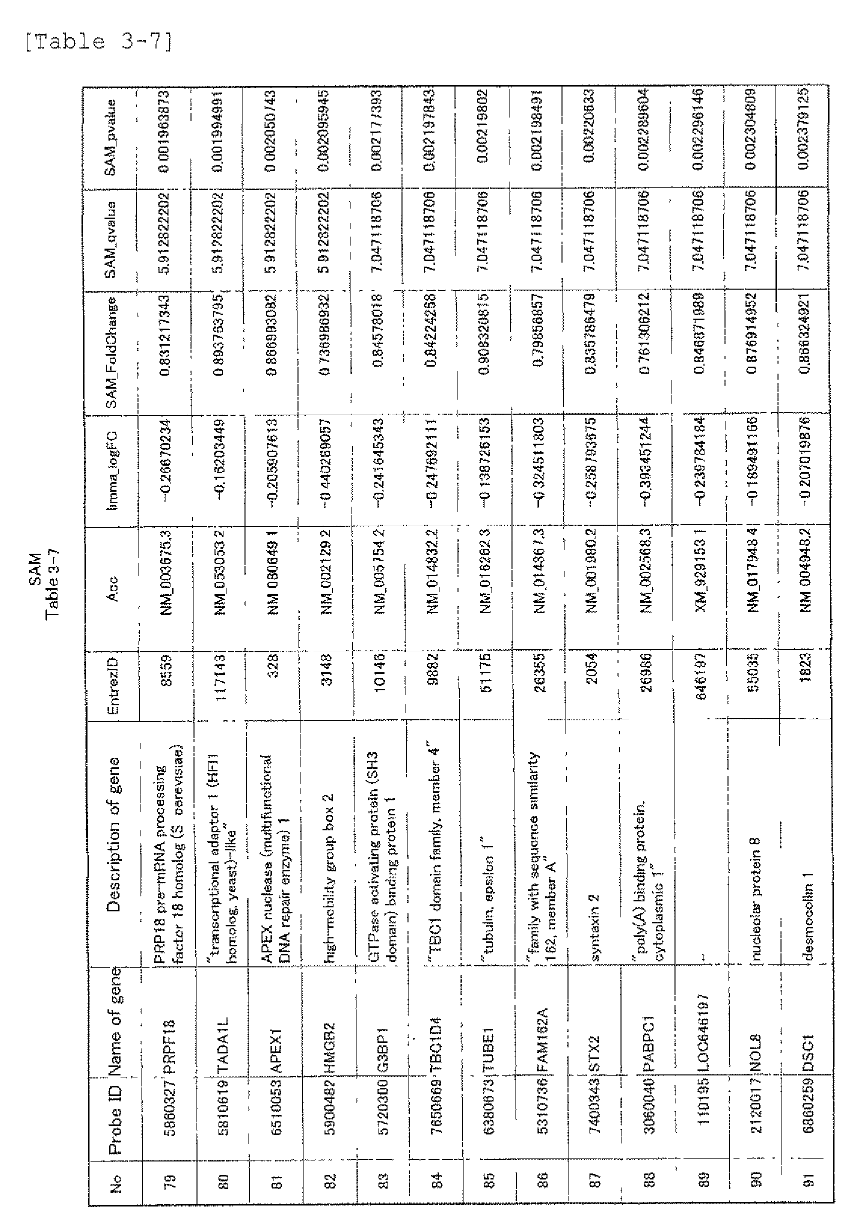

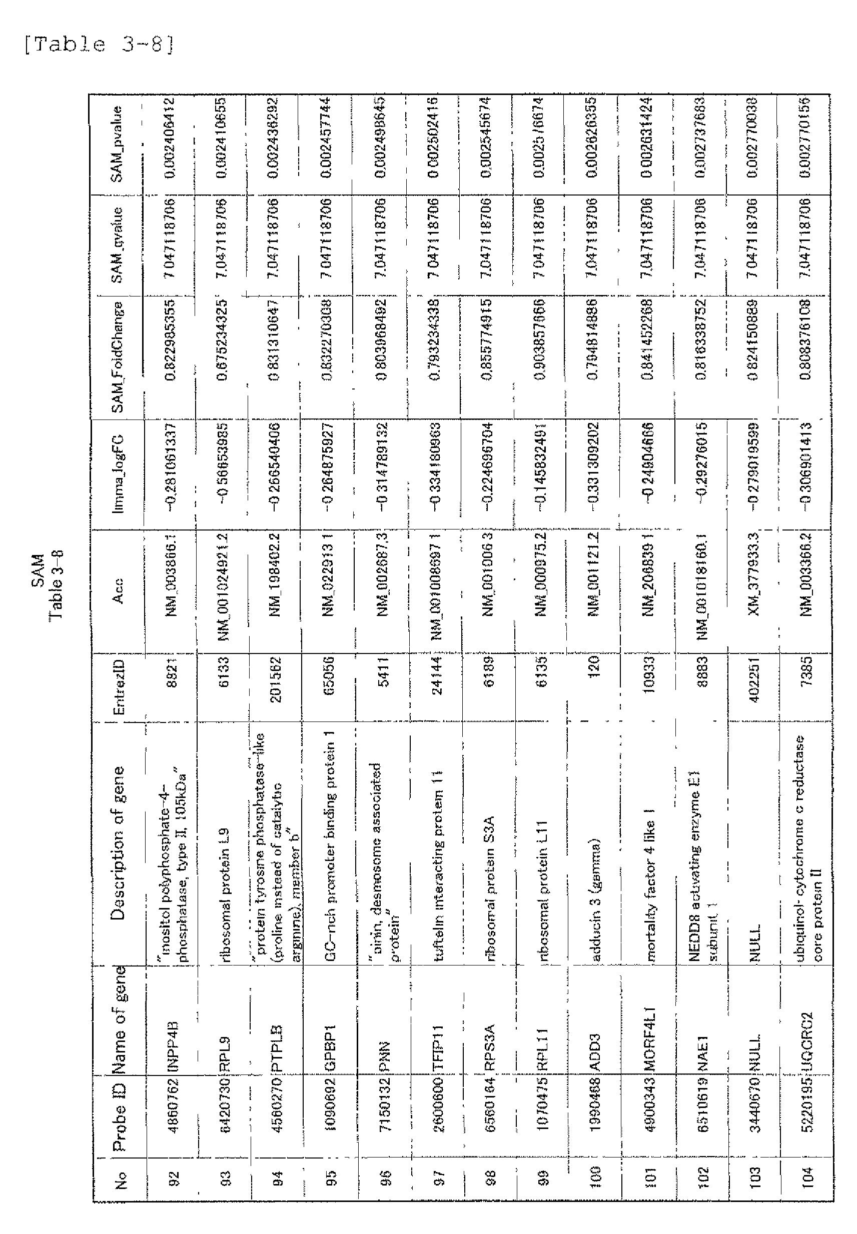

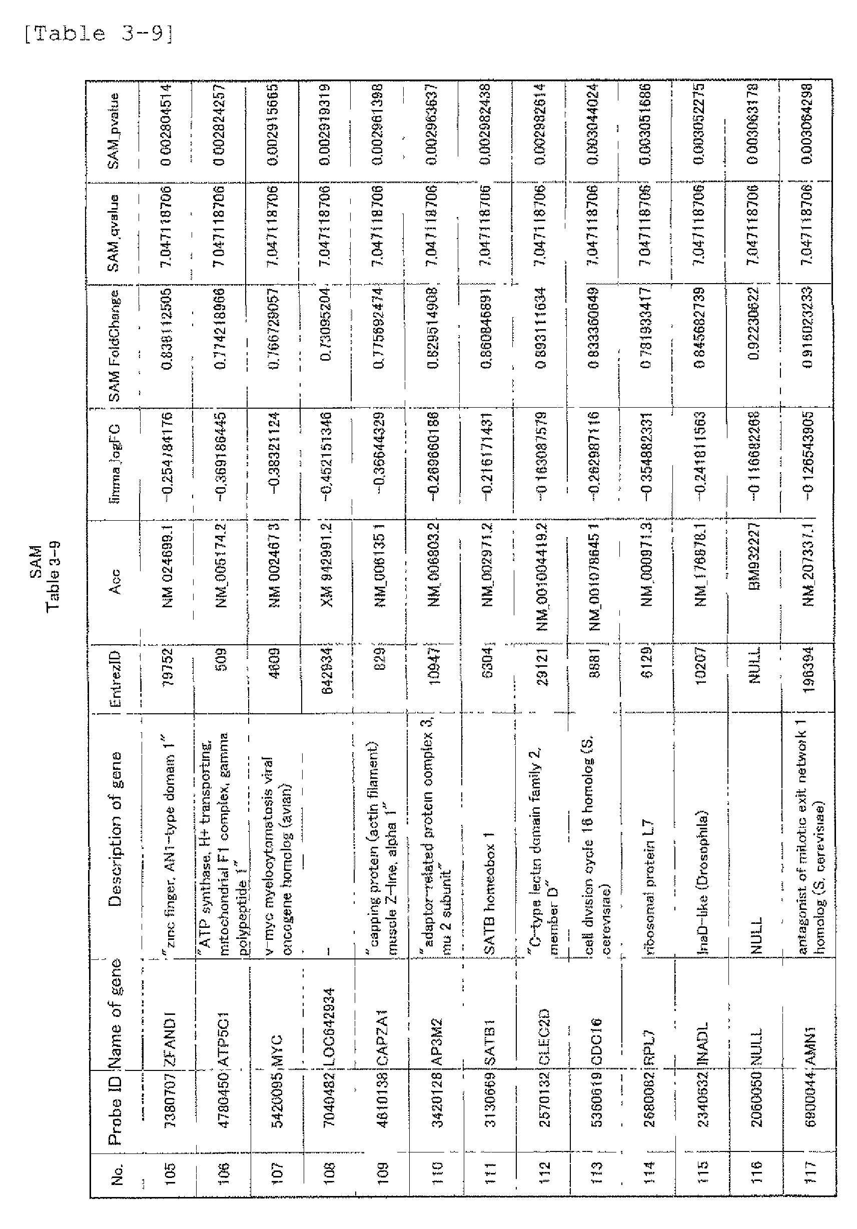

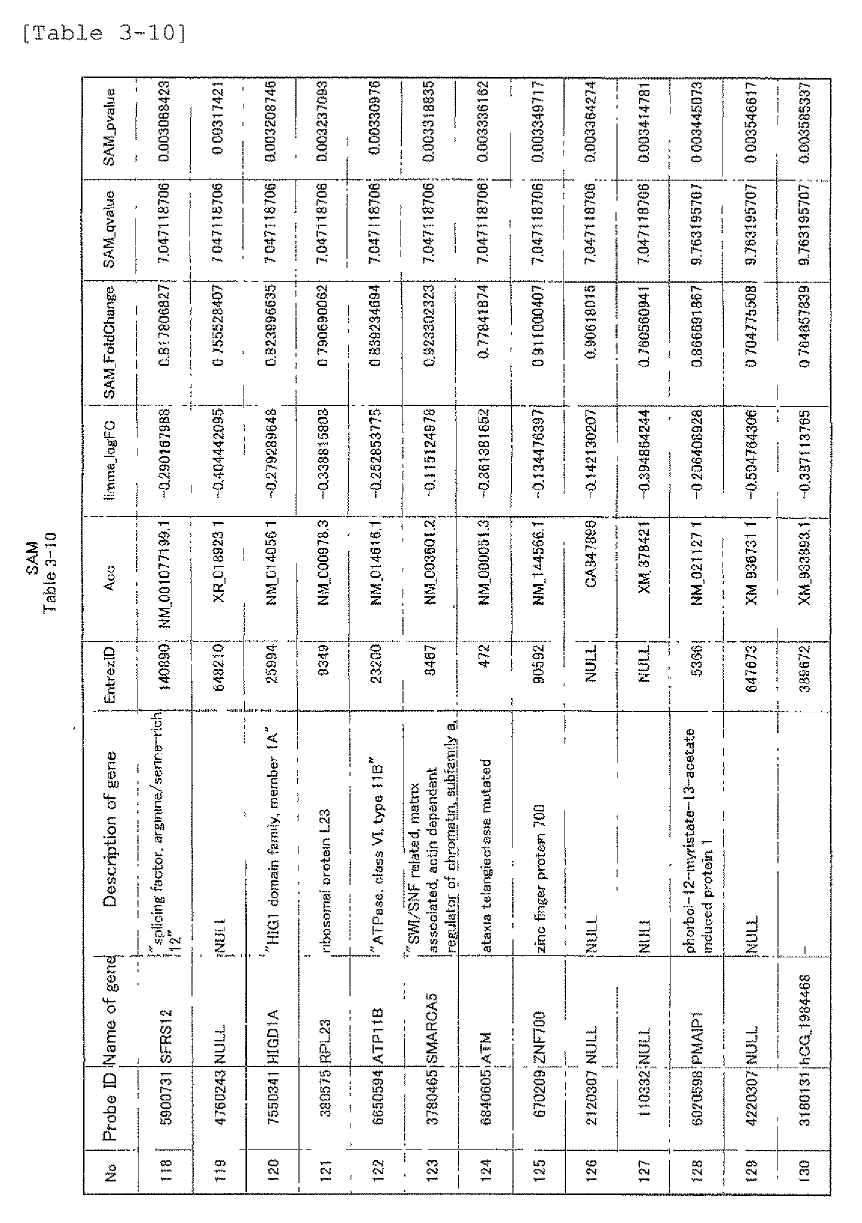

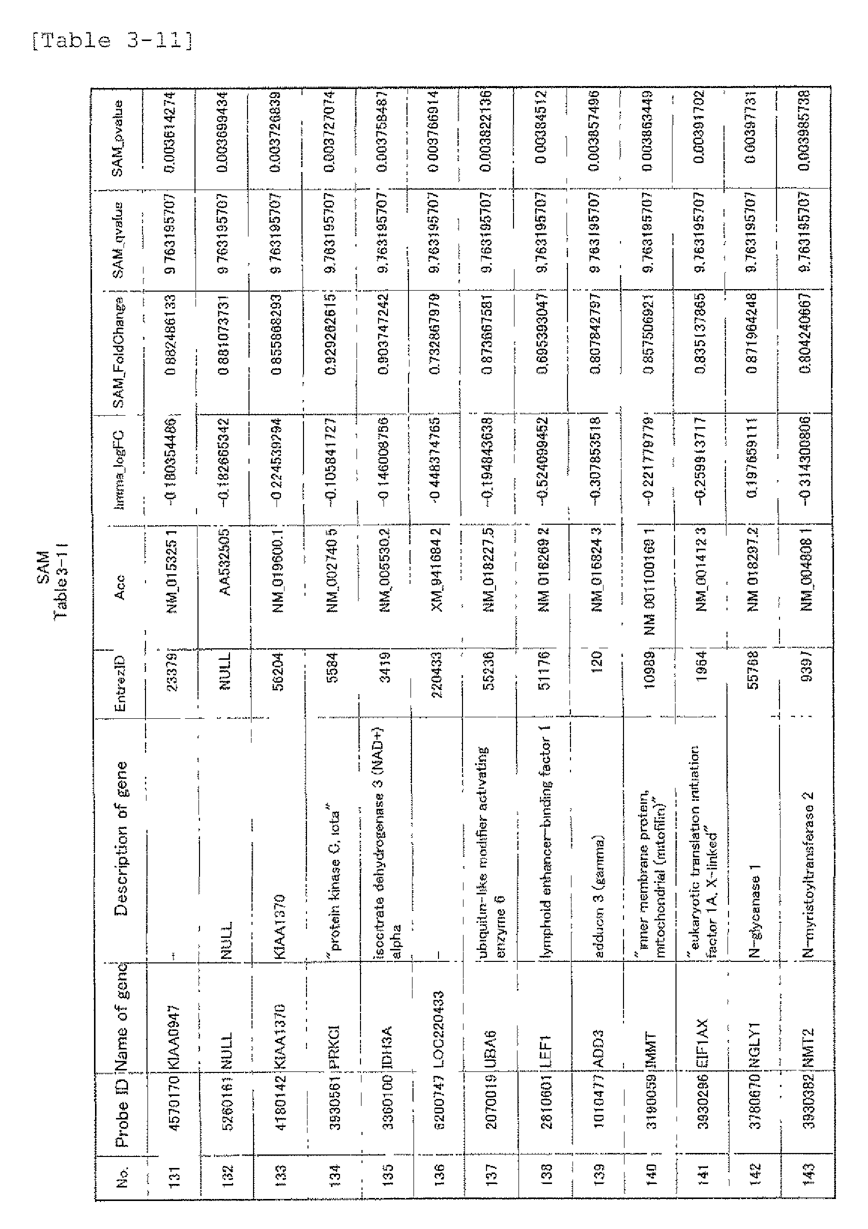

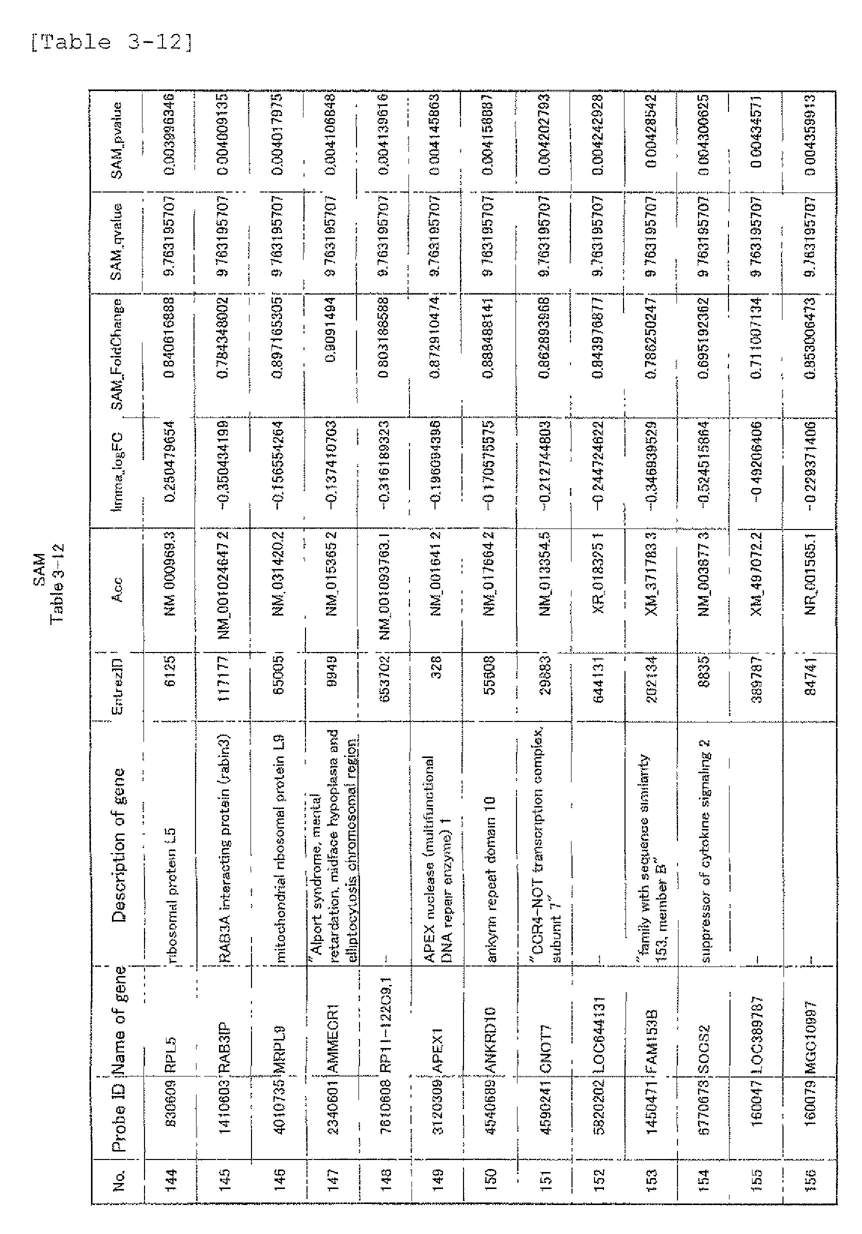

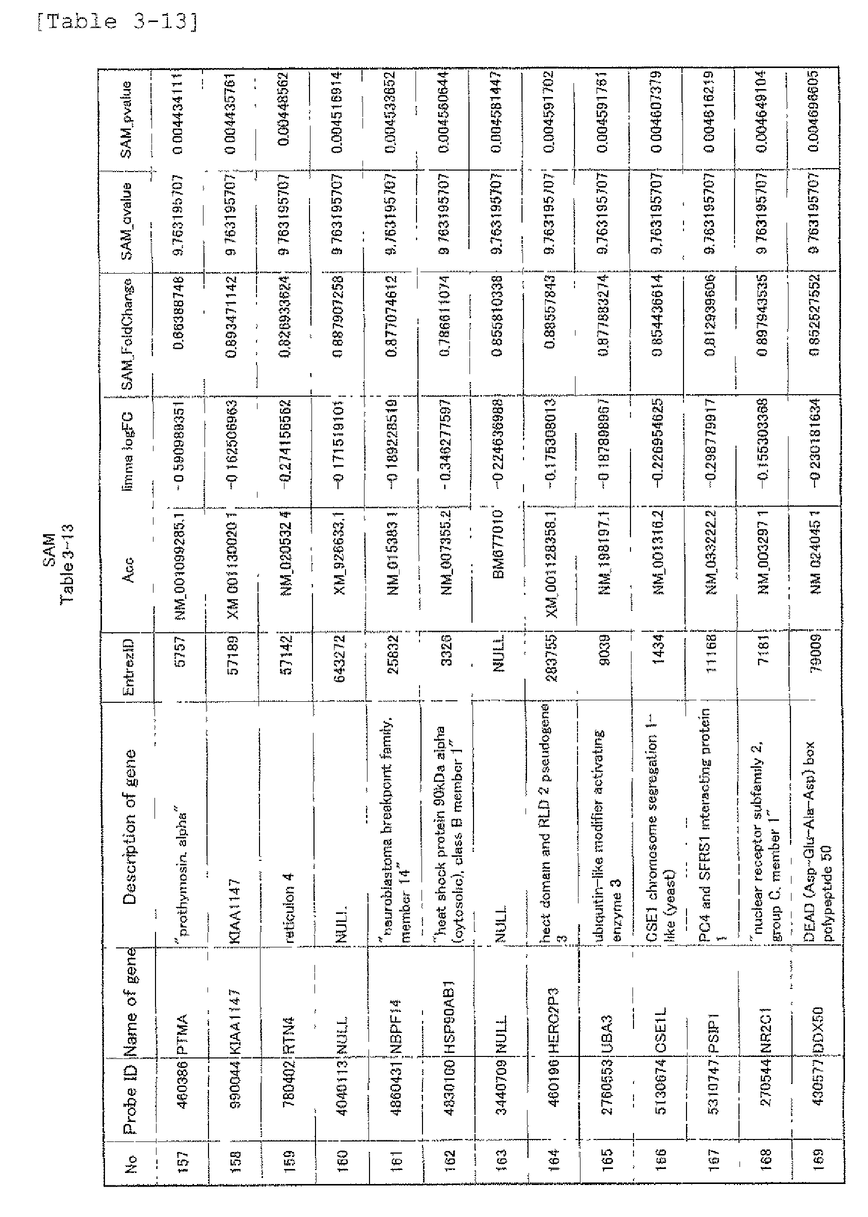

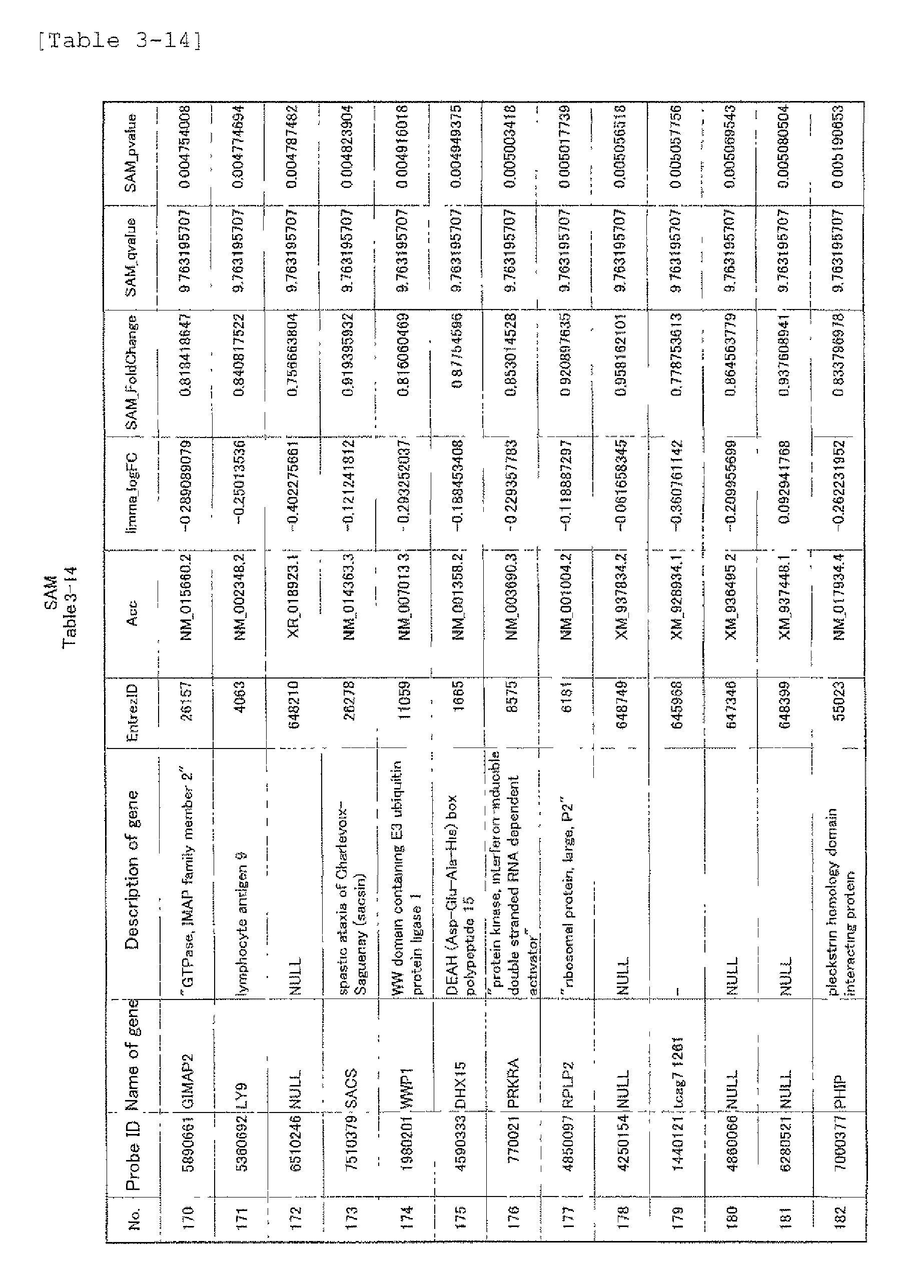

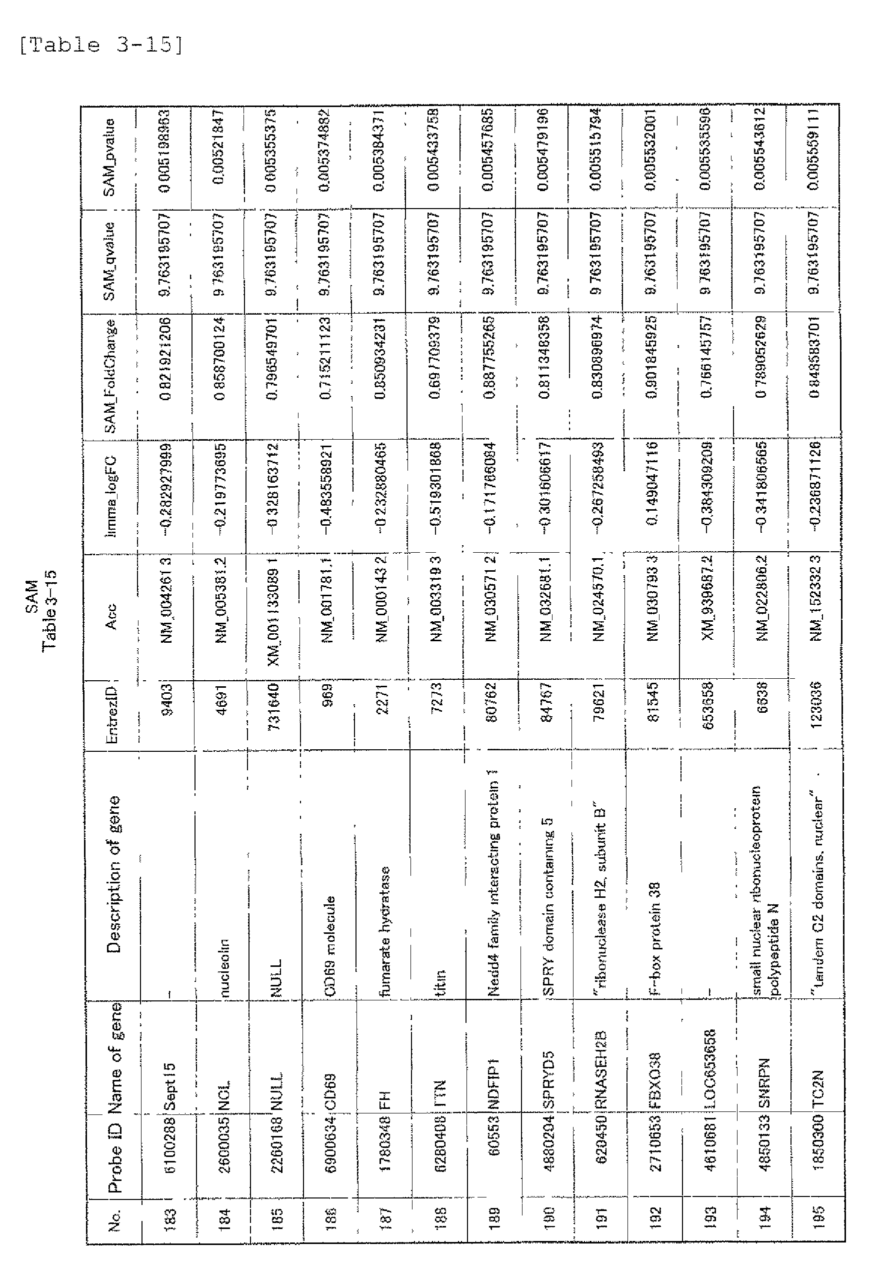

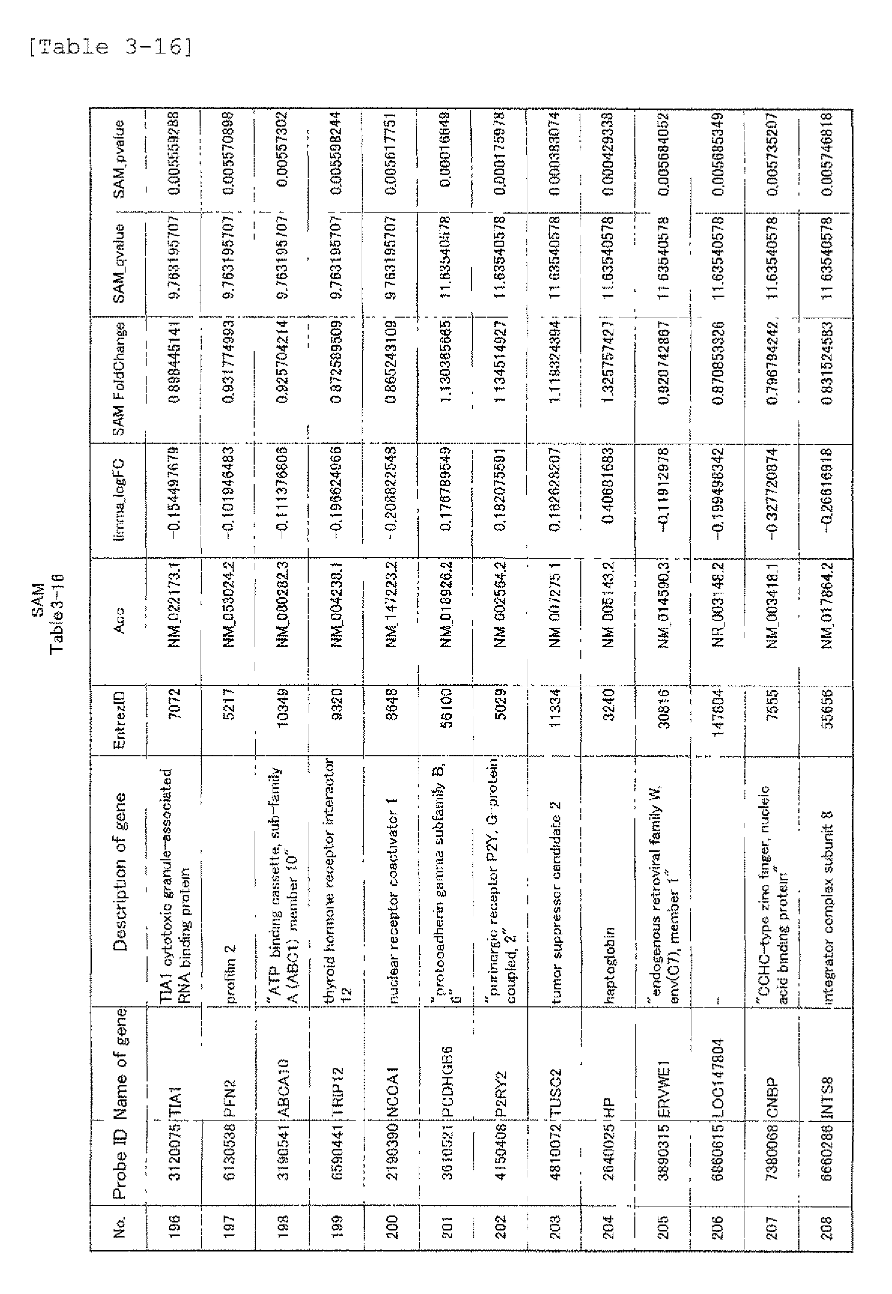

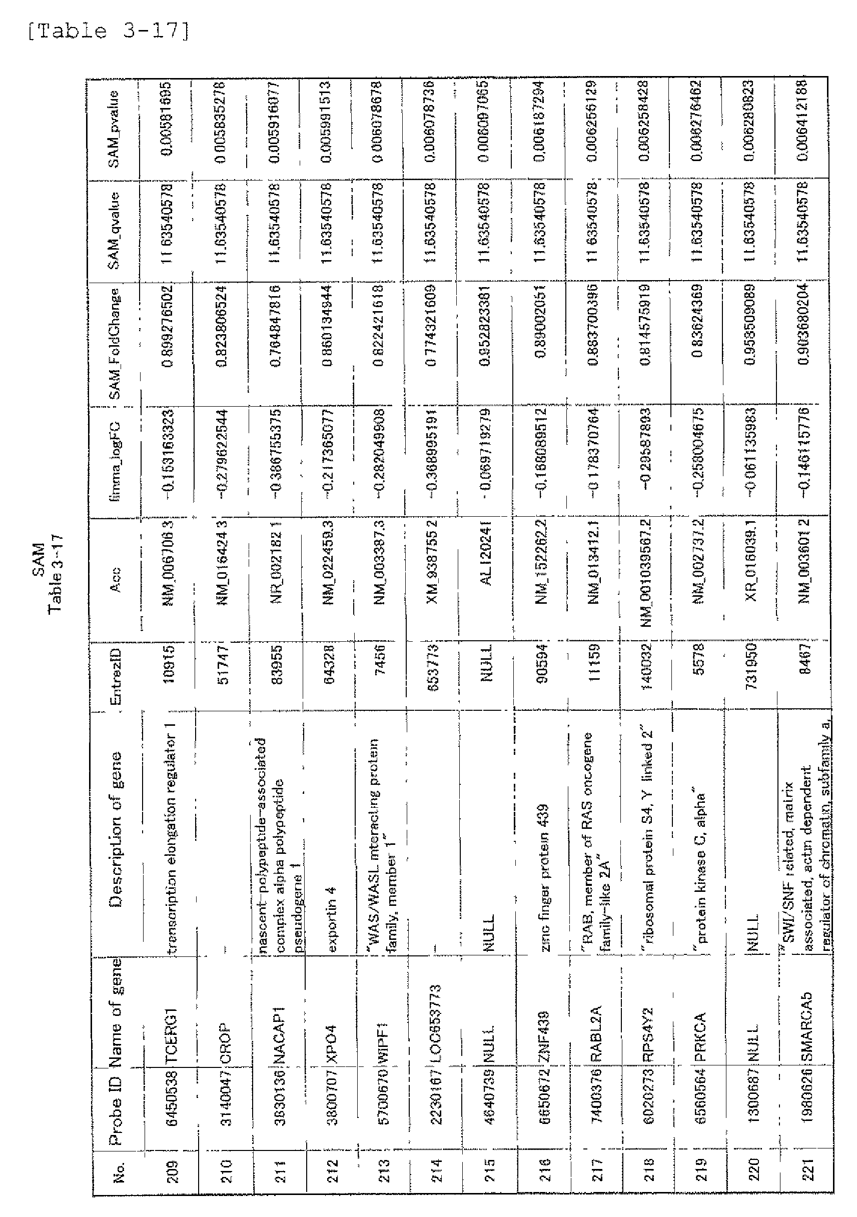

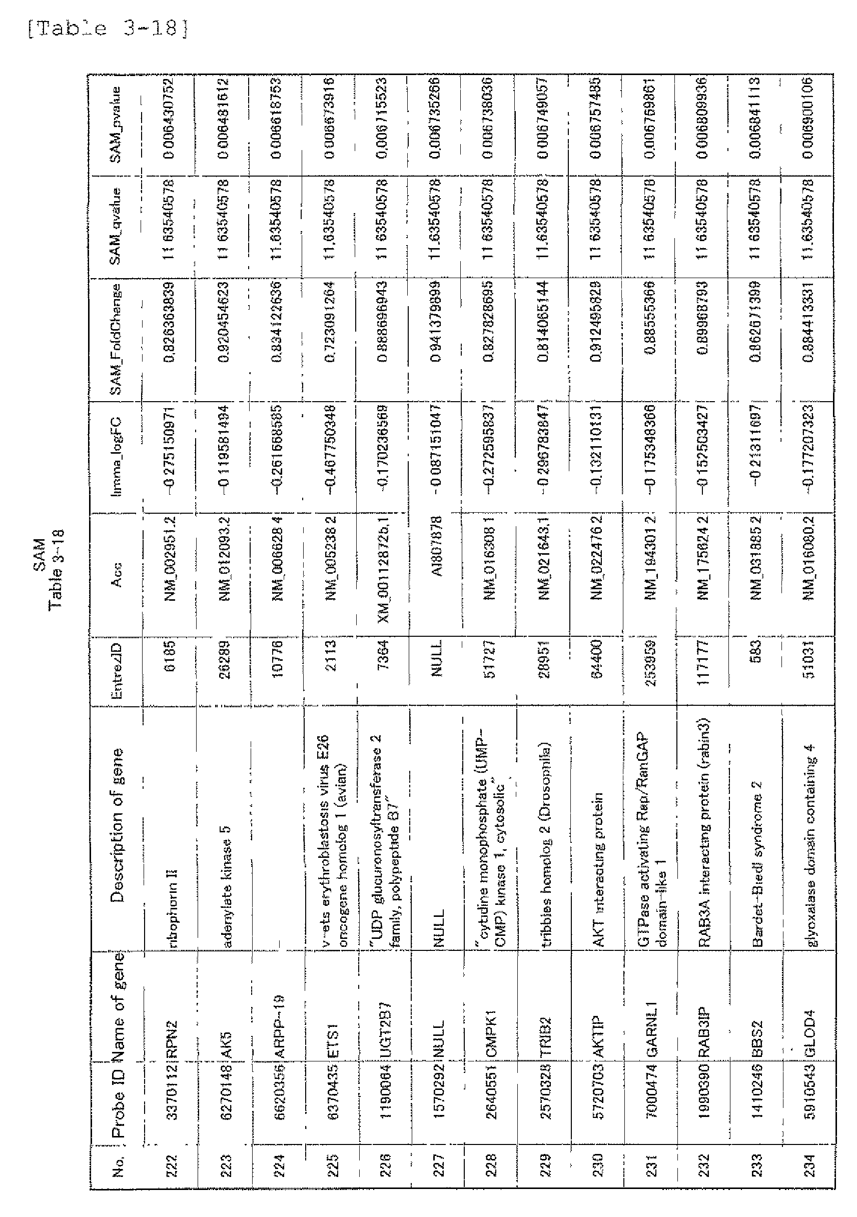

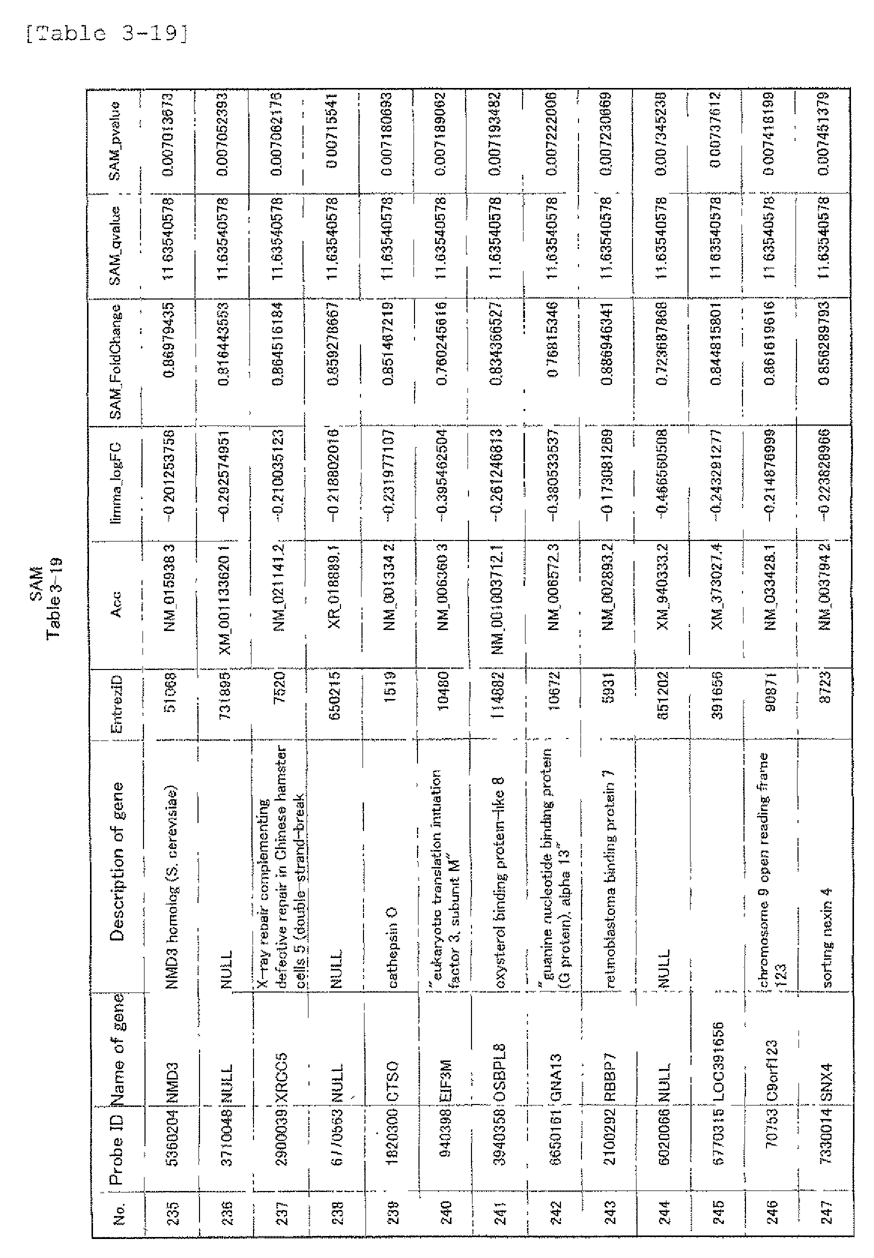

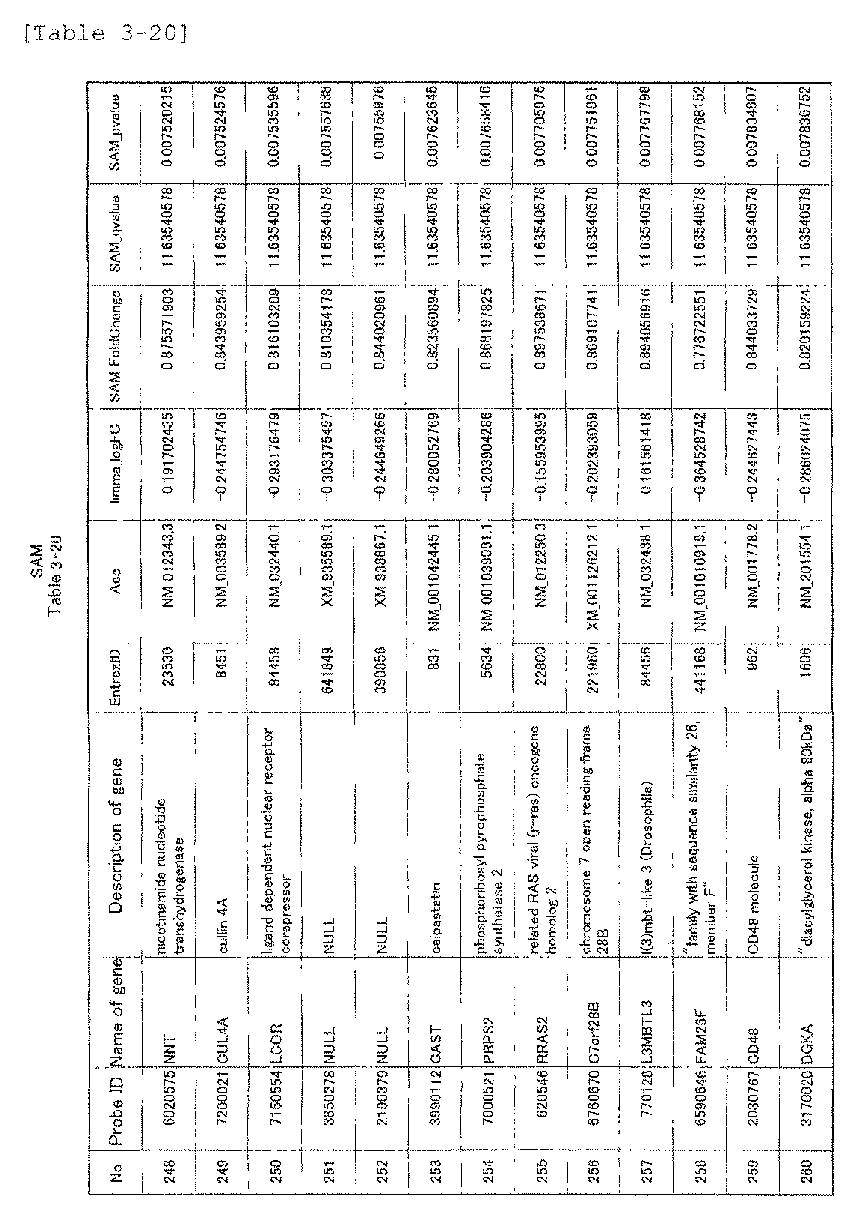

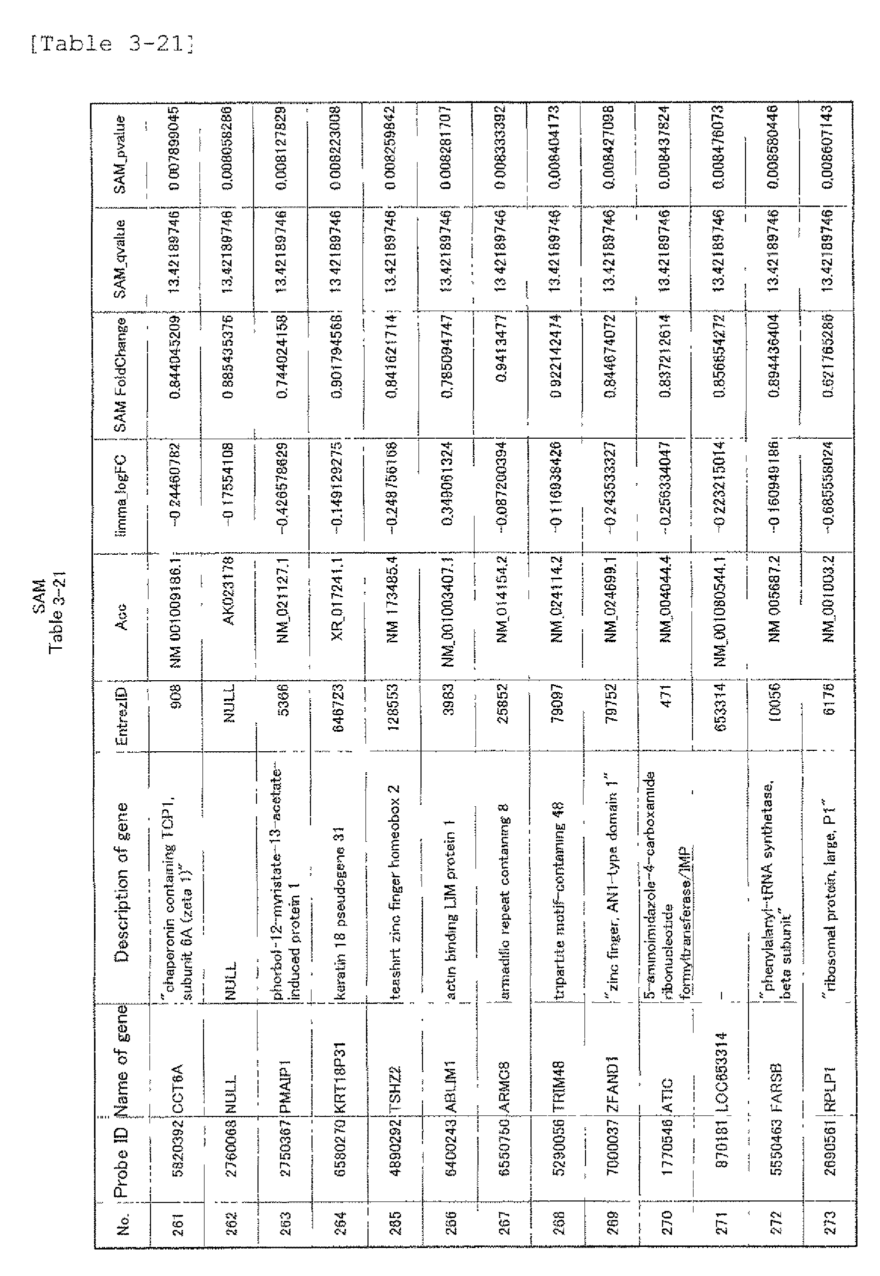

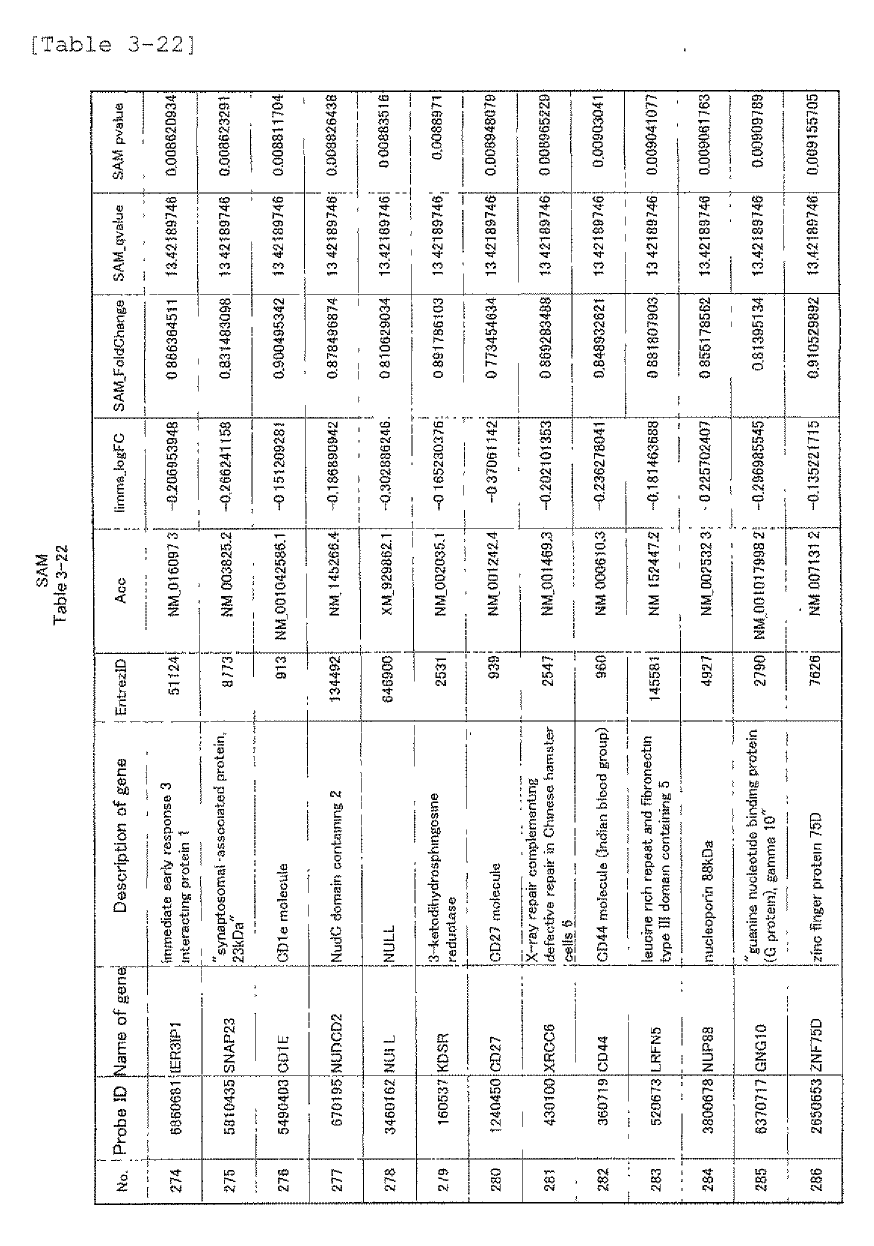

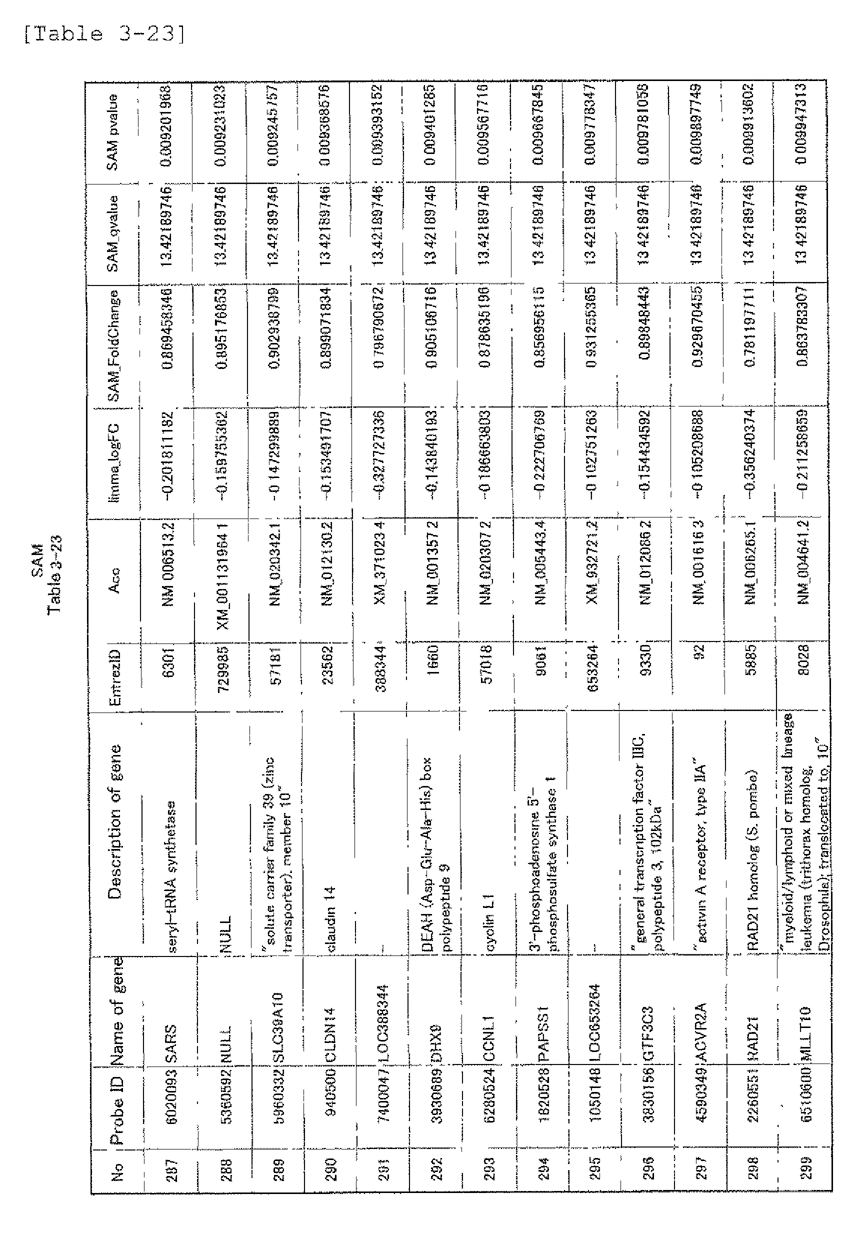

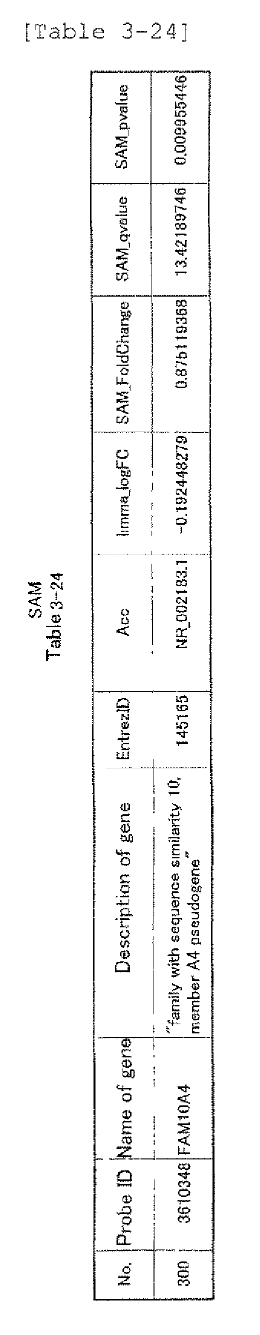

Table 4: List of top 300 genes selected by Rank Prod.

**[Table 4-1]**

| RankProd | | | | | | | | | |
|---|---|---|---|---|---|---|---|---|---|
| Table 4-1 | | | | | | | | | |
| No. | Probe ID | Name of gene | Description of gene | EntrezID | Acc | RP_Rsum | RP_FC | RP_Pfp | Rip_pval |
| 1 | 2480600 | LOC728358 | - | 728358 | NM 001042500.1 | 206.9319 | 0.3679 | 0 | 0 |
| 2 | 7150170 | LOC728358 | - | 728358 | NM_001042500.1 | 217.1554 | 0.3807 | 0 | 0 |
| 3 | 2970747 | DEFA3 | defensin, alpha 3, neutrophil- | 1668 specific | NM_005217.2 | 221.4639. | 0.3779 | 0 | 0 |
| 4 | 4540239 | DEFA1 | defensin, alpha 1 | 1667 | NM_004084.2 | 226.007 | 0.387 | 0 | 0 |
| 5 | 5080692 | HLA-A29.1 | - | 649853 | NM_001080840.1 | 232.3536 | 0.3703 | 0 | 0 |
| 6 | 870477 | LOC728358 | - | 728358 | NM_001042500.1 | 249.17 | 0.3939. | 0 | 0 |
| 7 | 7650497 | ELA2 | elastase 2, neutrophil | 1991' | NM_001972.2 | 273.8273 | 0.4006 | 0 | 0 |
| 8 | 6550164 | DEFA4 | defensin, alpha 4, corticostatin | 1669 | NM_001925.1 | 309.0369 | 0.4123 | 0 | 0 |
| 9 | 770400 | NULL | NULL | 653600 | XM_928349.1 | 324.4866 | 0.4475 | 0 | 0 |
| 10 | 1500735 | CTSG | cathepsin G | 1511 | NM 001911.2 | 324.7636 | 0.4191 | 0 | 0 |
| 11 | 3890349 | HIST1H4C | histone cluster 1, H4_{G} | 8364 | NM_003542.3 | 342.3622 | 0.5327 | 0 | 0 |

**[Table 4-2]**

| RankProd | | | | | | | | | |
|---|---|---|---|---|---|---|---|---|---|
| Table 4-2 | | | | | | | | | |
| No. | Probe ID | Name of gene | Description of gene | EntrezlD | Acc | RP_Rsum | RP_FC | RP_pfp | RP_pval |
| 12 | 7330398 | HLA-DRB4 | major historompatibility complex, class II, DR beta 4 | 3126 | NM_021983.4 | 395.251 | 1.5402 | 0 | 0 |
| 13 | 5860075 | CAMP | cathelicidin antimicrobial peptide | 820 | NM_004345.3 | 426.4961 | 0.4888 | 0 | 0 |
| 14 | 7050021 | PRKARIA | protein kinase, cAMP-dependent, regulatory, type 1, alpha (tissue specific extinguisher 1) | 5573 | NM_002734.3 | 440.111 | 1.6574 | 0 | 0 |
| 15 | 4390398 | LCN2 | lipocalin 2 | 3934 | NM_005564.3 | 4686986 | 0.5216 | 0 | 0 |
| 16 | 4180768 | ALAS2 | aminolevulinate, delta-, synthase 2 | 212 | NM_001037968.1 | 494.2717 | 0.6935 | 0 | 0 |
| 17 | 2690561 | RPLP1 | ribosomal protein, large, P1 | 6176 | NM_001003.2 | 510.521 | 1.6083 | 0 | 0 |
| 18 | 4050717 | MYOM2 | myomesin (M-protein) 2, 165kDa | 9172 | NM_003970.1 | 529.0537 | 0.6829 | 0 | 0 |
| 19 | 3520601 | MPO | /myeloperoxidase | 4353 | NM_000250.1 | 552.0527 | 0.5121 | 0 | 0 |
| 20 | 1450358 | HBD | hemoglobin, delta | 3045 | NM_000519.3 | 555.1003 | 0.636 | 0 | 0 |
| 21 | 620544 | HLA--DRB6 | major histocompatibility complex, class II, DR beta 6 (pseudogene) | 3128 | NR_0012981 | 5995065 | 1.2864 | 0 | 0 |
| 22 | 6660288 | CCL3L1 | chemokine (C-C motif) ligand 3-like | 6349 | NM_021006.4 | 608.9901 | 1.3171 | 0 | 0 |

**[Table 4-3]**

| RankProd Table 4-3 | | | | | | | | | |
|---|---|---|---|---|---|---|---|---|---|
| No. | Probe lD | Name of gene | Description of gene | EntrezID | Acc | RP_Rsum | RP_FC | RiP_pfp | RP_pval |
| 23 | 2350274 | ERAF | erythroid associated factor | 51327 | NM_016633.2 | 674.2449 | 0.6242 | 0 | 0 |
| 24 | 6560376 | RPS26L1 | ribosomal protein S26-like 1 | 441502 | NR_002309.1 | 627.4099 | 0.6128 | 0 | 0 |
| 25 | 6960195 | NULL | NULL | 650646 | XM_942527.2 | 659.4889 | 0,6133 | 0 | 0 |
| 26 | 2230538 | LRRN3 | leucine rich repeat neuronal 3 | 54674 | NM_001099660.1 | 663.6339 | 1.5428 | 0 | 0 |
| 27 | 6180743 | LAIR2 | leukocyte-associated 2 immunoglobulin-like receptor 2 | 3904 | NM_021270.2 | 674.7421 | 0.6789 | 0 | 0 |
| 28 | 2230601 | UTS2 | urotensin 2 | 10911 | NM_021995.1 | 682.1642 | 1.3557 | 0 | 0 |
| 29 | 6590377 | RPS26 | ribosomal protein S26 | 6231 | NM_001029.3 | 684.9927 | 0.6457 | 0 | 0 |
| 30 | 2640255 | NULL | NULL | 641848 | XM_935588.1 | 694.3737 | 1.5648 | 0 | 0 |
| 31 | 5890730 | RPS26L | - | 400156 | XR_017804.1 | 998 1257 | 0.6497 | 0 | 0 |
| 32 | 3520102 | CCL4L1 | chemokine (C-G motif) ligand 4-like | 9560 | NM_001001435.2 | 700.5086 | 1.4296 | 0 | 0 |
| 33 | 6860048 | UTS2 | urotensin 2 | 1091f | NM_021995.1 | 717.5432 | 1.4062 | 0 | 0 |

**[Table 4-4]**

| RankProd | | | | | | | | | |
|---|---|---|---|---|---|---|---|---|---|
| Table 4-4 | | | | | | | | | |
| No. | Probe ID | Name of gene | Description of gene | EntrezID | Acc | RP_Rsum | RP_FC | RP_pfp | RP_pval |
| 34 | 5260484 | HLA-DRBI | major histocompatibility complex, class II, DR beta 1 | 3123 | NM_002124.1 | 718.7641 | 0.7652 | 0 | 0 |
| 35 | 50278 | LAIR2 | leukocyte-associated immunoglobulin-like receptor 2 | 3904 | NM_002288.3 | 739.419 | 0.6969 | 0 | 0 |
| 36 | 4570255 | LEF1 | lymphoid enhancer-binding factor 1 | 51176 | NM_016269.2 | 762.7748 | 1-5155 | 0 | 0 |
| 37 | 460386 | PTMA | prothymosin, alpha | 5757 | NM_001099285.1 | 764.6714 | 1.5063 | 0 | 0 |
| 38 | 4670048 | RPS26L | | 400156 | NR_002225.2 | 796.2257 | 0.6646 | 0 | 0 |
| 39 | 6420730 | RPL9 | ribosomal protein L9 | 6133 | NM_001024921.2 | 797.4422 | 1.481 | 0 | 0 |
| 40 | 6960022 | 5-Sep | septin 5 | 5413 | NM_002688.4 | 812.1586 | 0.6894 | 0 | 0 |
| 41 | 6180427 | G0S2 | G0/G1switch 2 | 50486 | NM_015714.2 | 8321428 | 1.1905 | 0 | 0 |
| 42 | 5290762 | UTS2 | urotensin 2 | 10911 | NM_006786.2 | 832.6698 | 1.2965 | 0 | 0 |
| 43 | 780403 | HLA-DQA1 | major histocompatibility complex, class II, DQ alpha 1 | 3117 | XM_936128.2 | 837.2042 | 1.3738 | 0 | 0 |
| 44 | 1010246 | .IFI6 | interferon, alpha-inducible protein 6 | 2537 | NM_022872 2 | 838.9897 | 0.6667 | 0 | 0 |

**[Table 4-5]**

| RankProd | | | | | | | | | |
|---|---|---|---|---|---|---|---|---|---|
| Table 4-5 | | | | | | | | | |
| No. | Probe ID | Name of gene | Description of gene | EntrezID | Acc | RP_Rsum | RP_FC | RP_pfp | RP_pval |
| 45 | 6270307 | LOC644934 | - | 644934 | XM_930344 2 | 846.7408 | 0.6838 | 0 | 0 |
| 46 | 4780075 | CEACAM8 | carcinoembryonic antigen-related cell adhesion molecule 8 | 1088 | NM_001816.2 | 851.5188 | 0.6171 | 0 | 0 |
| 47 | 1450139 | FCGR3B | Fc fragment of IgG, low affinity IIIb, receptor (CD16b) | 2215 | NM_000570.3 | 852.2208 | 0.7809 | 0 | 0 |
| 48 | 1400240 | LDHB | lactate dehydrogenase B | 3945 | NM_002300.4 | 869.5601 | 1.5215 | 0 | 0 |
| 49 | 2810601 | LEF1 | lymphoid enhancer-binding factor 1 | 51176 | NM_016269.2 | 885.7056 | 1.438 | 0 | 0 |
| 50 | 4230121 | RPS26L | - | 400156 | XR_017804.1 | 901.0353 | 0.6857 | 0 | 0 |
| 51 | 50689 | C7orf28B | chromosome 7 open reading frame | 221960 | NM_198097.1 | 901.4464 | 1.283 | 0 | 0 |
| 52 | 7160475 | SNHG5 | small nucleolar RNA host gene 5 (non-protein coding) | 387066 | XM_943699.1 | 915.2623 | 1 3754 | 0 | 0 |
| 53 | 1010546 | NULL | NULL | 649143 | XM_944822.1 | 940.4695 | 1.2482 | 0 | 0 |
| 54 | 4120053 | PFKFB3 | 6-phosphofructo-2-kinase/fructose-2,6-biphosphatase 3 | 5209 | NM_004566.2 | 949.932 | 0.7127 | 0 | 0 |
| 55 | 5870678 | NULL | NULL | 441763 | XM_930284.1 | 959.3277 | 0 7098 | 0 | 0 |

**[Table 4-6]**

| RankProd | | | | | | | | | |
|---|---|---|---|---|---|---|---|---|---|
| Table 4-6 | | | | | | | | | |
| No. | Probe ID | Name of gene | Description of gene | EntrezID | Acc | RP_Rsum | RP_FC | RP_pfp | RP_pval |
| 56 | 6250615 | PGLYRP1 | peptidoglycan recognition protein 1 | 8993 | NM_005091.1 | 978 6937 | 0.6314 | 0 | 0 |
| 57 | 5310044 | NBPF20 | neuroblastoma breakpoint family, member 20 | 400818 | NM_001037675.1 | 989.9365 | 0.6816 | 0 | 0 |
| 58 | 7160474 | HLA-DQB1 | major histocompatibility complex, class II, DQ beta 1 | 3119 | NM_002123.2 | 993.2267 | 1.3788 | 0 | 0 |
| 59 | 1510424 | S100P | S100 calcium binding protein P | 6286 | NM_005980.2 | 999.9308 | 0.776 | 0 | 0 |
| 60 | 7550358 | NELL2 | NEL-like 2 (chicken) | 4753 | NM_006159.1 | 1000.2823 | 1.4511 | 0 | 0 |
| 61 | 6370435 | ETS1 | v-ets erythroblastosis virus E26 oncogene homolog 1 (avian) | 2113 | NM_005238.2 | 1000.7099 | 1.383 | 0 | 0 |
| 62 | 4480730 | HBM | hemoglobin, mu | 3042 | NM_001003938.3 | 1002.8748 | 0.7114 | 0 | 0 |
| 63 | 6770673 | SOCS2 | suppressor of cytokine signaling 2 | 8835 | NM_003877.3 | 1008.5738 | 1.4385 | 0 | 0 |
| 64 | 3140242 | KIR2DL3 | killer cell immunoglobulin-like receptor, two domains, long cytoplasmic tail, 3 | 3804 | NM_014511.3 | 1012.7829 | 0.7052 | 0 | 0 |
| 65 | 5390246 | CCR7 | chemokine (C-C motif) receptor 7 | 1236 | NM_001838.2 | 1024.3788 | 1.3396 | 0 | 0 |
| 66 | 6280408 | TTN | titin | 7273 | NM_003319.3 | 1040 0032 | 1.4333 | 0 | 0 |

**[Table 4-7]**

| RankProd | | | | | | | | | |
|---|---|---|---|---|---|---|---|---|---|
| Table 4-7 | | | | | | | | | |
| No. | Probe ID | Name of gene | Description of gene | EntrezID | Acc | RP_Rsum | RP_FC | RP_pfp | RP_pval |
| 67 | 840685 | IL1B | interleukin 1, beta | 3553 | NM_000576.2 | 1043.9839 | 0.9118 | 0 | 0 |
| 68 | 1440564 | RUNX3 | runt-related transcription factor 3 | 864 | NM_004350.2 | 1045.6015 | 0.7504 | 0 | 0 |
| 69 | 2640301 | TNF | tumor necrosis factor (TNF superfamily, member | 7124 2) | NM_000594.2 | 1052.0345 | 0.9119 | 0 | 0 |
| 70 | 160047 | LOC389787 | - | 389787 | XM_497072.2 | 1058.0289 | 1.4065 | 0 | 0 |
| 71 | 1500280 | IFIT3 | interferon-induced protein with tetratricopeptide repeats 3 | 3437 | NM_001031683.1 | 1067.6439 | 0.7336 | 0 | 0 |
| 72 | 1780719 | PTGES3 | prostaglandin E synthase 3 (cytosolic) | 10728 | NM_006601.4 | 1094.4413 | 1.4426 | 0 | 0 |
| 73 | 7610440 | XAF1 | XIAP associated factor 1 | 54739 | NM_199139.1 | 1101.0267 | 0.6954 | 0 | 0 |
| 74 | 1260482 | GZMK | granzyme K (granzyme 3; tryptase | 3003 | NM_002104.2 | 1101.0382 | 1.379 | 0 | 0 |
| 75 | 2570112 | ABLIM1 | actin binding LIM protein 1 | 3983 | NM_006720.3 | 1109.2206 | 1.3947 | 0 | 0 |
| 76 | 5810685 | THBS1 | thrombospondin 1 | 7057 | NM_003246.2 | 1112.0822 | 0 7981 | 0 | 0 |
| 77 | 1050292 | GP9 | glycoprotein IX (platelet) | 2815 | NM_000174.2 | 1121.2912 | 0.7089 | 0 | 0 |

**[Table 4-8]**

| RankProd | | | | | | | | | |
|---|---|---|---|---|---|---|---|---|---|
| Table 4-8 | | | | | | | | | |
| No. | Probe ID | Name of gene | Description of gene | EntrezID | Acc | RP_Rsum | RP_FC | RP_pfp | RP_pval |
| 78 | 4010181 | FOLR3 | folate receptor 3 (gamma) | 2352 | NM_000804.2 | 1121.4294 | 0 7343 | 0 | 0 |
| 79 | 1580025 | GNG11 | guanine nucleotide binding protein (G protein), gamma 11 | 2791 | NM_004126.3 | 1123.8896 | 0.7606 | 0 | 0 |
| 80 | 4220307 | NULL | NULL | 647673 | XM_936731.1 | 1127.0089 | 1.4189 | 0 | 0 |
| 81 | 4220246 | CCL20 | chemokine (C-C motif) ligand 20 | 6364 | NM_004591.1 | 1130.1869 | 0.8647 | 0 | 0 |
| 82 | 6270605 | LOC649946 | - | 649946 | NR-003040.1 | 1141.8104 | 1.3897 | 0 | 0 |
| 83 | 3370202 | ANXA2P1 | annexin A2 pseudogene 1 | 303 | NR_001562.1 | 1148.68 | 1.3614 | 0 | 0 |
| 84 | 6020066 | NULL | NULL | 651202 | XM_940333.2 | 1156.9854 | 1.3818 | 0 | 0 |
| 85 | | 2970370 PCDH17 | protocadherin 17 | 27253 | NM_014459.2 | 1159.006 | 1.4416 | 0 | 0 |
| 86 | 2970575 | CCL4L2 | chemokine (C-C motif) ligand 4-like 2 | 388372 | NM_207007 2 | 1160.5275 | 0.8619 | 0 | 0 |
| 87 | 3360615 | FCER1A | Fc fragment of IgE, high affinity 1, receptor for; alpha polypeptide | 2205 | NM_002001.2 | 1161.9701 | 1.3166 | 0 | 0 |
| 88 | 6900634 | CD69 | CD69 molecule | 969 | NM_001781.1 | 1185.7514 | 1.3982 | 0 | 0 |

**[Table 4-9]**

| RankProd | | | | | | | | | |
|---|---|---|---|---|---|---|---|---|---|
| Table 4-9 | | | | | | | | | |
| No. | Probe ID | Name of gene | Description of gene | EntrezID | Acc | RP_Rsum | RP-FC | RP_pfp | RP_pval |
| 89 | 650349 | RPS28 | ribosomal protein S28 | 6234 | NM_001031.4 | 1190.4157 | 1.3501 | 0 | 0 |
| 90 | 6650242 | IFITM3 | interferon induced transmembrane protein 3 (1-8U) | 10410 | NM_021034.2 | 1198.6619 | 0.7139 | 0 | 0 |
| 91 | 2000148 | IFIT1 | interferon-induced protein with tetratricopeptide repeats 1 | 3434 | NM_001548 3 | 1201.038 | 0 728 | 0 | 0 |
| 92 | 2190139 | CA1 | carbonic anhydrase 1 | 759 | NM_001738.1 | 1216.0508 | 0.7338 | 0 | 0 |
| 93 | 2140753 | RPL14 | ribosomal protein L14 | 9045 | NM 001034996.1 | 12164627 | 1.3673 | 0 | 0 |
| 94 | 5050162 | CD83 | CD83 molecule | 9308 | NM_001040280.1 | 1221.184 | 1.1247 | 0 | 0 |
| 95 | 4200746 | BPI | bactericidal/permeability-increasing protein | 671 | NM_001725.1 | 1222 3488 | 0 676 | 0 | 0 |
| 96 | 2600747 | IFIT2 | interferon-induced protein with tetratricopeptide repeats 2 | 3433 | NM_001547.4 | 1226.2189 | 0.7229 | 0 | 0 |
| 97 | 3130543 | RNASE3 | ribonuclease, RNase A family, 3 (eosinophil cationic protein) | 6037 | NM_002935.2 | 1231.3433 | 0.6719 | 0 | 0 |
| 98 | 5670739 | AZU1 | azurocidin 1 | 566 | NM_001700.3 | 1238.4609 | 0 6567 | 0 | 0 |
| 99 | 7400377 | CEACAM6 | carcinoembryonic antigen-related cell adhesion molecule 6 (non-specific cross reacting antigen) | 4680 | NM_002483.3 | 1240.5422 | 0.6648 | 0 | 0 |

**[Table 4-10]**

| RankProd | | | | | | | | | |
|---|---|---|---|---|---|---|---|---|---|
| Table 4-10 | | | | | | | | | |
| No. | Probe ID | Name of gene | Description of gene | EntrezID; | Acc | RP_Rsum | RP_FC | RP_pfp | RP_pval |
| 100 | 4670390 | CXCL2 | chemokine (C-X-C motif) ligand 2 | 2920 | NM₋002089.3 | 1243,1968 | 1.0736 | 0 | 0 |
| 101 | 670010 | NULL | NULL | 650298 | XM_939387.1 | 1255.7097 | 0.7673 | 0 | 0 |
| 102 | 6590682 | CCL3 | chemokine (C-C motif) ligand 3 | 6348 | MM_002983.1 | 1258 8623 | 1.0301 | 0 | 0 |
| 103 | 4560088 | LOC439949 | | 439949 | XM 001129241.1 | 1259.3189 | 1.3845 | 0 | 0 |
| 104 | 6220671 | PLAUR | plasminogen activator, urokinase receptor | 5329 | NM_001005376.1 | 1269.8919 | 0.7674 | 0 | 0 |
| 105 | 3170152 | IL2RB | interleukin 2 receptor, beta | 3560 | NM_000878.2 | 1271.1735 | 0.8008 | 0 | 0 |
| 106 | 6620026 | CD83 | CD83 molecule | 9308 | NM_004233.3 | 1287.9941 | 1.0467 | 0 | 0 |
| 107 | 2360164 | KLRC2 | killer cell lectin-like receptor subfamily C, member 2 | 3822 | NM_002260.3 | 1288.636 | 0.8481 | 0 | 0 |
| 108 | 2650192 | C6orf105 | chromosome 6 open reading frame 105 | 84830 | MM_032744 .1 | 1289.3942 | 1.3412 | 0 | 0 |
| 109 | 2810010 | CCL3L3 | chemokine (C-C motif) ligand 3-like 3 | 414062 | NM_001001437.3 | 1296.3404 | 1.2483 | 0 | 0 |
| 110 | 3190148 | DDIT4 | DNA-damage-inducible transcript 4 | 54541 | NM_019058.2 | 1307.8573 | 0.7086 | 0 | 0 |

**[Table 4-11]**

| RankProd | | | | | | | | | |
|---|---|---|---|---|---|---|---|---|---|
| Table 4-11 | | | | | | | | | |
| No. | Probe ID | Name of gene | Description of gene | EntrezID | Acc | RP_Rsum | RP_FC | RP_µfp | RP_pval |
| 111 | 1770504 | HLA-DRB3 | major histocompatibility complex, class II DR beta 3 | 3125 | NM_ p22555.3 | 1317.7473. | 12121 | 0 | 0 |
| 112 | 6590594 | HIST1H2AC | histone cluster 1, H2ac | 8334 | NM_003512.3 | 1319.1937 | 0.7494 | 0 | 0 |
| 113 . | 2260129 | MS4A6A | membrane-spanning 4-domatns> subfamily A, member 6A | 64231 | MM022349.2 | 1320.1527 | 1,2241 | 0 | 0 |
| 114 | 2750367 | PMAIP1 | phorbol-12-myristate-13-acetate-induced protein 1 | 5366 | NM_021127.1 | 1322.9263 | 1.344 | 0 | 0 |
| 115 | 5890095 | LILRA5 | laukocyte immunoglobulin-like receptor, subfamily A (with TM domain), member 5 | 353514 | NM 021250.2 | 1328.6256 | 0.7741 | 0 | 0 |
| 116 | 2900360 | KIR2DL4 | killer cell immunoglobulin-like receptor, two domains, long cytoplasmic tail, 4 | 3805, | NM 002255.3 | 1329.4767 | 0.7459 | 0 | 0 |
| 117 | 6940176 | ACTR3 | 3 ARP3actin-related protein 3 homolog (yeast) | 10096 | NM_005721.3 | 1331.182 | 1.a'755 | 0 | 0 |
| 118 | 4010040 | HBG2 | hemoglobin, gamma G | 3048 | NM_000184.2 | 1331.5404 | 1.1022 : | 0 | 0 |
| 119 | 270338 | TRAT1 | T cell receptor assoriated transmembrane adaptor 1 | 50852 | NM 016388.2 | 1334.9472 | 1.2985 | 0 | 0 |
| 120 | 1070487 | KLRC3 | killer cell lectin-like receptor subfamify C, member 3 | 3823 | NM_002261.2 | 1341.8253 | 0.8231 | 0 | 0 |
| 121 | 6770326 | NLRPB | NLR family, pyrin domain containing 8 | 126205 | NM_176811.2 | 1341.9389 | 1.2932 | 0 | 0 |

| RankProd | | | | | | | | | |
|---|---|---|---|---|---|---|---|---|---|
| Table 4-12 | | | | | | | | | |
| No. | Probe ID | Name of gene | Description of gene | EntrezID | Acc | RP_Rsum | RP_FG | RP_pfp | RP_pval |
| 122 | 7160692 | NULL | NULL | 644250 | XM_929199.1 | 1343-5684 | 1.3113 | 0 | 0 |
| 123 | 5870136 | CLIC3 | chloride intracellular channel 3 | 9022 | NM_004869.2 | 1346.5828 | 0.7759 | 0 | 0 |
| 124 | 6260747 | LOC220433 | - | 220433 | XM_941684.2 | 1348.1888 | 1.3645 | 0 | 0 |
| 125 | 5260070 | HES4 | hairy and enhance of split 4 (Drosophila) | 57801 | NM_021170.2 | 1348.7064 | 0.7167 | 0 | 0 |
| 126 | 4250053 | CCL3L1 | chemokine(C-C motif) ligand 3-like 1 | 6349 | NM_021006.4 | 1356.1796 | 1.1002 | 0 | 0 |
| 127 | 6580041 | GNLY | granulysin | 10578 | NM_006433.2 | 1356.7575 | 0.7782. | 0 | 0 |
| 128 | 7040482 | L0C642934 | - | 642934 | XM_942991.2 | 1359.7468 | 1.3681 | 0 | 0 |
| 129 | 3890523 | IL7R | interleukin 7 receptor | 3575 | XM_937367.1 | 1362.7624 | 1.2775 | 0 | 0 |
| 130 | 1340600 | PPP1R15A | protein phosphatase 1, regulatory (inhibiter)subunt 15A regulatory | 23645 | NM_014330.2 | 1373.0141 | 0.7984 | 0 | 0 |
| 131 | 5900129 | CROP | - | 51747 | NM_006107.2 | 1375.3663 | 1.3602 | 0 | 0 |
| 132 | 1010592 | CD36 | molecule (thrombospondin receptor) | 948 | NM 001001548.1 | 1383.7856 | 11993 | 0 | 0 |

**[Table 4-13]**

| RankProd | | | | | | | | | |
|---|---|---|---|---|---|---|---|---|---|
| Table 4-13 | | | | | | | | | |
| No. | Probe ID | Name of gene | Description of gene | EntrezID | Acc | RP_Rsum | RP_FC | RP_pfp | RP _pval |
| 133 | 5720682 | TMEM176A | transmembrane protein 179A | 55365 | NM_018487.2 | 13849828 | 0.7916 | 1.00E 04 | 0 |
| 134 | 610437 | CD24 | CD24molecule | 100133941 | NM_013230.2 | 1388.7966 | 0.7457 | 1.00E-04 | 0 |
| 135 | 4180079 | CRISPLY2 | cysteine-rich secretory protein LCCL domain containing 2 | 83716 | NM_031476-2 | 4388.8351 | 0.72 | 1.00E-04 | 0 |
| 136 | 650112 | LST1 | leukocytespecific transcript 1 | 7940 | NM_0071612.2 | 1388.9553 | 07844 | 1.00E-04 | 0 |
| f37 | 520382 | CLK1 | CDC-liked kinase 1 | 1195 | NM_001024646.1 | 1395.6456 | 1.323 | 0 | 0 |
| 138 | 450537 | HBG1 | hemoglobin, gamma A | 3047 | NM_000558.2 | 1395.7174 | 1.0847 | 0 | 0 |
| 139 | 4490520 | GPR183 | G pratein-coupled receptor 183 | 1880 | MM_004951.3 | 1395.8256 | 1.273 | 0 | 0 |
| 140 | 4040398 | MAL | Mal,T-cell differentiation protein | 4118 | NM_022440.1 | 1396.4257 | 1.2775 | 0 | 0 |
| 141 | 5900482 | HMGB2 | high-mobility group box 2 | 3148 | NM_002129.2 | 1396_9288 | 1.3569 | 0 | 0 |
| 142 | 510468 | NFE2 | nuclear factor (erythroid-derived 2), 45kDa | 4778 | NM 006163.1 | 1400.397 | 0783 | 0 | 0 |
| 143 | 6330196 | MAL | mal, T-cell differentiation protein | 4118 | NM_002371 2 | 14032124 | 1-2786 | 0 | 0 |

**[Table 4-14]**

| RankProd | | | | | | | | | |
|---|---|---|---|---|---|---|---|---|---|
| Table 4-14 | | | | | | | | | |
| No. | Probe ID | Name of gene | Description of gene | EntrezlD | Acc | RP_Rsum | RP_FC | RP-pfp | RP_pval |
| 144 | 2100196 | ISG15 | ISG15 ubiquttin-like modifier | 9636 | NM_005101.1 | 1404.6098 | 0 7538 | 0 | 0 |
| 145 | 2260477 | PTGS2 | prostaglandin-endoperoxide synthase 2 (prostaglandin G/H and cyclooxygenase | 5743 | NM_000963 | 1417.97/9 | 1.1062 | 0 | 0 |
| 146 | 1780709 | DDX17 | DEAD(Asp-Glus-Ala-Asp)box polypeptide 17 | 10521 | NM_ 030881.2' | 1418.7298 | 1.3597 | 0 | 0 |
| 147 | 1400672 | AMFR | autocrine motility factor receptor | 267 | NM_001144.4 | 1425.012 | 1.2953 | 0 | 0 |
| 148 | 6110747 | GIMAP2 | GTPase, IMAP family member 2 | 26157 | NM_015660.2 | 1433.7316 | 1.3711 | 0 | 0 |
| 149 | 730379 | LOC388621 | - | 388621 | XM_941195.2 | 1434.8209 | 1.3642 | 0 | 0 |
| 150 | 870338 | EGR1 | early growth response 1 | 1958 | NM_001964.2 | 1437.3716 | 0.8285 | 0 | 0 |
| 151 | 730528 | PLAUR | Plasminogen activator, urokinase receptor | 5329 | NM_002659.2 | 1448.22 | 0.7926 | 0 | 0 |
| 152 | 2060440 | MAFB | fibrosarcoma oncogene homolog B (avian) | 9935 | NM_005461.3 | 1451.0344 | 0.7939 | 1.00E-04 | 0 |
| 153 | 3140750 | RBM38 | RNA binding motif protein 38 | 55544 | NM_183425.1 | 1459.9844 | 0.7692 | 1.00E-04 | 0 |
| 154 | 4490176 | RGS1 | regulator of G-protein signaling 1 | 5996 | NM_002922.3 | 1460 4038 | 1.0695 | 1.00E-04 | 0 |

**[Table 4-15]**

| RankProd | | | | | | | | | |
|---|---|---|---|---|---|---|---|---|---|
| Table 4-15 | | | | | | | | | |
| No. | Probe ID | Name of gene | Description of gene | EntrezID | Acc | RP_Rsum | RP_FC | RP_pfp | RP_pval |
| 155 | 2370010 | GZMH | granzyme H (cathepsin G-like 2, protein h-CCPX) | 2999 | NM_033423.3 | 1464.3431 | 0 8085 | 1.00E-04 | 0 |
| 156 | 7210035 | SNORD13 | small nucleolar RNA, C/D box 13 | 692084 | NR_003041.1 | 1465.3176 | 0.8786 | 1.00E-04 | 0 |
| 157 | 780465 | SLC11A1 | solute carrier family 11 (proton- coupled divalent metal ion transporters), member 1 | 6556 | NM_000578.3 | 1465.5854 | 0.7539 | 1.00E-04 | 0 |
| 158 | 1410278 | PF4V1 | platelet factor 4 vacant 1 | 5197 | NM_002620.2 | 1467.1678 | 0.7455 | 1.00E-04 | 0 |
| 159 | 10224 | METRNL | mateorin, glial cell differentiation regulator like | 284207 | XM_941466.2 | 1470.6115 | 0.7549 | 1.00E-04 | 0 |
| 160 | 6840324 | RPL13L | ribosomal protein L13-like | 283345 | NR_002803.1 | 1473.3493 | 1.3141 | 0 | 0 |
| 161 | 4760243 | NULL | NULL | 648210 | XR_018923.1 | 1473.563 | 1.3236 | 0 | 0 |
| 162 | 2690390 | HCG4 | HLA complex group 4 | 54435 | NR_002139.1 | 1483.3064 | 0.7057 | 1.00E-04 | 0 |
| 163 | 6130168 | GSTM2 | glutathione S-transferase mu 2 (muscle) | 2946 | NM_000848.2 | 1484.5662 | 1.1586 | 1.00E-04 | 0 |
| 164 | 6850035 | LOC338758 | - | 338758 | XM_931359.2 | 1485.0079 | 0.786 | 1.00E-04 | 0 |
| 165 | 2710575 | CD69 | CD69 molecule | 969 | NM_001781.1 | 1492.1903 | 1.1581 | 1.00E-04 | 0 |

**[Table 4-16]**

| RankProd | | | | | | | | | |
|---|---|---|---|---|---|---|---|---|---|
| Table 4-16 | | | | | | | | | |
| No. | Probe ID | Name of gene | Description of gene | EntrezID | Acc | RP_Rsum | RP FC | RP_pfp | RP_pval |
| 166 | 1030458 | C19orf10 | chromosome 19 open reading frame 10 | 56005 | NM_019107.3 | 1495.2283 | 0-7531 | 1.00E-04 | 0 |
| 167 | 3990703 | IL10 | interleukin 10 | 3586 | HM_000572.2 | 1496.2645 | 1.2881 | 1.00E-04 | 0 |
| 168 | 2230563 | PPBP | pro-platelet basic protein (chemokine (C-X-C motif) ligand 7) | 5473 | NM_002704.2 | 1498.9534 | 0.8688 | 1.00E-04 | 0 |
| 169 | 5490768 | GPR56 | G protein-coupled receptor 56 | 9289 | NM_201525.1 | 1502.85 | 0.9478 | 1.00E-04 | 0 |
| 170 | 40405/6 | IL6 | interleukin 6 (interferon, beta 2) | 3569 | NM_000600.1 | 1503.702 | 0.8304 | 1.00E-04 | 0 |
| 171 | 1850523 | GZMB | granzyme B (granzyme 2, cytotoxic T-lymphocyte-associated serine esterase 1) | 3002 | NM_004131.3 | 1505.0467 | 0.8201 | 1.00E-04 | 0 |
| 172 | 1570553 | IL8 | interleukin 8 | 3576 | NM_000584.2 | 1513.6436 | 1.115 | 1.00E-04 | 0 |
| 173 | 110332 | NULL | NULL | NULL | XM_378421 | 1518.0403 | 1.3148 | 1.00E-04 | 0 |
| 174 | 4670193 | PRF1 | perforin 1 (pore forming protein) | 5551, | NM_005041.4 | 1519.3926 | 0.8813 | 1.00E-04 | 0 |
| 175 | 5670605 | MATK | megakaryocyte-associated tyrosine kinase | 4145 | NM_139354.2 | 1520.0412 | 0.81 | 1.00E-04 | 0 |
| 176 | 6650735 | NULL | NULL | NULL | BC020840 | 1525.1509 | 1.2826 | 1.00E-04 | 0 |

**[Table 4-17]**

| RankProd | | | | | | | | | |
|---|---|---|---|---|---|---|---|---|---|
| Table 4-17 | | | | | | | | | |
| No. | Probe ID | Name of gene | Description of gene | EntrezID | Acc | RP_Rsum | RP_FC | RP_pfp | RP_pval |
| 177 | 6510246 | NULL | NULL | 648210 | XR_018923.1 | 1527.7265 | 1.3216 | 1.00E-04 | 0 |
| 178 | 1510681 | NULL | NULL | 440673 | NM_001039703.1 | 1530.4662 | 0.7386 | 2.00E-04 | 0 |
| 179 | 6650161 | GNA13 | guanine nucleotide binding protein (G protein), alpha 13 | 10672 | NM_006572.3 | 1532.1658 | 1.3018 | 1.00E-04 | 0 |
| 180 | 70162 | TDG | thymine-DNA glycosylase | 6996 | NM_003211.3 | 1532 3669 | 0.7618 | 2 00E-04 | 0 |
| 181 | 6290114 | SDPR | serum deprivation response (phosphatidylserine binding protein) | 8436 | NM_004657.4 | 1536.2237 | 0.7967 | 2.00E-04 | 0 |
| 182 | 3830242 | NULL | NULL | 642093' | XM_942754.1 | 1537 7061 | 1.2013 | 1.00E-04 | 0 |
| 183 | 150609 | LOC652493 | - | 652493 | XM_941953.1 | 1538.659 | 0.8658 | 2.00E-04 | 0 |
| 184 | 1410221 | S100A12 | S100 calcium binding protein A12 | 6283 | NM_005621.1 | 1538.9825 | 1.2033 | 1.00E-04 | 0 |
| 185 | 1030333 | CCL2 | chemokine (C-C motif) ligand 2 | 6347 | NM_002982.3 | 1539.4369 | 1.0091 | 2.00E-04 | 0 |
| 186 | 7000356 | RXRA | retinoid X receptor, alpha | 6256 | NM_002957.3 | 1541.9695 | 0.7849 | 2.00E-04 | 0 |
| 187 | 4610129 | RETN | resistin | 56729 | NM_020415.2 | 1542.2649 | 0.9579 | 1.00E-04 | 0 |

**[Table 4-18]**

| RankProd | | | | | | | | | |
|---|---|---|---|---|---|---|---|---|---|
| Table 4-18 | | | | | | | | | |
| No. | Probe ID | Name of gene | Description of gene | EntrezID | Acc | RP_Rsum | RP_FC | RP_pfp | RP_pval |
| 188 | 290687 | LOC653884 | - | 653884 | XM_936240.1 | 1544.2074 | 0.7447 | 2.00E-04 | 0 |
| 189 | 6280332 | CXCL16 | chemokine (C-X-C motif) ligand 16 | 58191 | NM_022059.1 | 1546.842 | 0.7614 | 2.00E-04 | 0 |
| 190 | 6860347 | FAM46C | family with sequence similarity 46, member C | 54855 | NM 017709.3 | 1546.9813 | 0.8189 | 2.00E-04 | 0 |
| 191 | 3890326 | SOD2 | superoxide dismutase 2, mitochondrial | 6648 | NM 001024465.1 | 1548.2034 | 0.8086 | 2.00E-04 | 0 |
| 192 | 940398 | 3M | eukaryotic translation initiation factor 3, subunit M | 10480 | NM_006360.3 | 1548.6574: | 1.3154 | 2.00E-04 | 0 |
| 193 | 5700070 | ALPL | alkaline phosphatase, liver/bone/kidney | 249 | NM_000478.3 | 1554.899 | 0.8918 | 2.00E-04 | 0 |
| 194 | 3940438 | NCF1 | neutrophil cytosolic factor 1 | 653361 | NM_000265.4 | 1557.7407 | 0.7547 | 2.00E-04 | 0 |
| 195 | 2900328 | PTPLAD2 | protein tyrosine phosphatase-like A domain containing 2 | 401494 | NM_001010915.1 | 1561.8011 | 1.2964 | 2.00E-04 | 0 |
| 196 | 3930537 | NULL | NULL | NULL | M97723 | 1563.1544 | 1.2251 | 2.00E-04 | 0 |
| 197 | 3060040 | PABPC1 | poly(A) binding protein, cytoplasmic | 26986 | NM_002568.3 | 1573.2119 | 13135 | 2.00E-04 | 0 |
| 198 | 3870338 | IFI44L | interferon-induced protein 44-like | 10964 | NM_006820.1 | 1574.4975 | 0.7976 | 2.00E-04 | 0 |

**[Table 4-19]**

| RankProd | | | | | | | | | |
|---|---|---|---|---|---|---|---|---|---|
| Table 4-19 | | | | | | | | | |
| No. | Probe ID | Name of gene | Description of gene | EntrezID | Acc | RP_Rsum | RP_FC | RP_pfp | RP_pval |
| 199 | 3840564 | MS4A7 | membrane-spanning 4-domains, subfamily A, member 7 | 58475 | NM_206938.1 | 1576.6213 | 0.7676 | 2.00E-04 | 0 |
| 200 | 3370201 | LOC648343 | - | 648343 | XR_018327.1 | 1584 6649 | 1.2593 | 2.00E-04 | 0 |
| 201 | 6280504 | LOC100008589 | - | 100008589 | NR_003287 1 | 1584,9019 | 0.8501 | 2.00E-04 | 0 |
| 202 | 7200435 | ASCL2 | achaete-scute complex homolog 2 (Drosophila) homolog 2 | 430 | NM 005170.2 | 1588.1072 | 0.7612 | 2.00E-04 | 0 |
| 203 | 7050326 | CDKN2D | cyclin-dependent kinase inhibitor 2D (p19, inhibits ODK4) | 1032 | NM_079421.2 | 1592.7956 | 0.771 | 2.00E-04 | 0 |
| 204 | 7320561 | OAS2 | 2'-5'-oligoadenylate synthetase 2, 69/71kDa | 4939 | NM_016817.2 | 1592.8459 | 0.782 | 2.00E-04 | 0 |
| 205 | 2640088 | ALDOA | aldolase A, fructose-bisphosphate | 226 | NM_184041 1 | 1601.3395 | 0.7836 | 2.00E-04 | 0 |
| 206 | 6380079 | FAM53B | family with sequence similarity 53, member B | 9679 | NM_014661.3 | 16018988 | 0.7927 | 2.00E-04 | 0 |
| 207 | 6590446 | NULL | NULL | 651149 | XM 940278.1 | 1603.0262 | 1.1639 | 2.00E-04 | 0 |
| 208 | 5050541 | RPL15P3 | ribosomal protein L15 pseudogene 3 | 653232 | XM_944104 2 | 1610.6217 | 1.2836 | 2.00E-04 | 0 |
| 209 | 6620136 | MYH9 | myosin, heavy chain 9, non-muscle | 4627 | NM 002473 3 | 1611.8118 | 0.7916 | 2.00E-04 | 0 |

**[Table 4-20]**

| RankProd | | | | | | | | | |
|---|---|---|---|---|---|---|---|---|---|
| Table 4-20 | | | | | | | | | |
| No. | Probe ID | Name of gene | Description of gene | EntrezID | Acc | RP_Rsum | RP_FC | RP_pfp | RP_pval |
| 210 | 2340743 | CPA3 | carboxypeptidase A3 (mast cell) | 1359 | NM_001870.1 | 1617.7401 | 1.2372 | 2.00E-04 | 0 |
| 211 | 6400392 | GSTM1 | glutathione S-transferase mu 1 | 2944 | NM_000561.2 | 1618.8346 | 1.1278 | 2.00E-04 | 0 |
| 212 | 1030348 | CDKN1B | cyclin-dependent kinase inhibitor 1B (p27, Kip1) | 1027 | NM_004064.2 | 1622.318 | 1.2788 | 2.00E-04 | 0 |
| 213 | 130717 | ARPC1B | actin related protein 2/3 complex, subunit 1B, 41kDa subunit 1B, 41kDa | 10095 | NM_005720.2 | 1622.3622 | 0.7728 | 2.00E-04 | 0 |
| 214 | 3420373 | SOD2 | superoxide dismutase 2, mitochondrial | 6648 | NM_001024466.1 | 1623.9115 | 0.8404 | 2.00E-04 | 0 |
| 215 | 7320424 | HNRPAIL-2 | - | 664709 | NR_002944.2 | 1627.4854 | 1.3497 | 2.00E-04 | 0 |
| 216 | 830356 | SLC4A5 | solute carrier family 4, sodium bicarbonate cotransporter, member 5 | 57835 | NM_133478.2 | 1629.4592 | 1.2662 | 3.00E-04 | 0 |
| 217 | 1240450 | CD27 | CD27 molecule | 939 | NM_001242.4 | 1630.1171 | 1.2929 | 3.00E-04 | 0 |
| 218 | 520408 | IFIT3 | interferon induced protein with tetratricopeptide repeats 3 | 3437 | NM_001549.2 | 1636.6346 | 0.7611 | 2.00E-04 | 0 |
| 219 | 5420095 | MYC | v-myc myelocytomatosis viral oncogene homolog (avian) | 4609 | NM_002467.3 | 1636.6524 | 1.3042 | 3.00E-04 | 0 |
| 220 | 7510253 | ACRBP | acrosin binding protein | 84519 | NM_032489.2 | 1637.0001 | 0.7754 | 2.00E-04 | 0 |

**[Table 4-21]**

| RankProd | | | | | | | | | |
|---|---|---|---|---|---|---|---|---|---|
| Table 4-21 | | | | | | | | | |
| No. | Probe ID | Name of gene | Description of gene | EntrezID | Acc | RP_Rsum | RP_FC | RP_pfp | RP_pval |
| 221 | 520392 | P2RY13 | purinergic receptor P2Y, G-protein coupled, 13 | 53829 | NM_023814.2 | 1637.1533 | 0.7731 | 2.00E-04 | 0 |
| 222 | 70630 | RNF144B | ring finger protein 144B | 255488 | NM_182757.2 | 1637.3458 | 0.7382 | 2.00E-04 | 0 |
| 223 | 5360273 | SNCA | synuclein, alpha (non A4 component of amyloid precursor) | 6622 | NM_007308.1 | 1637.8283 | 0.8495 | 2.00E-04 | 0 |
| 224 | 1690706 | HIST1H3H | histone cluster 1, H3h | 8357 | NM_003536.2 | 1645.7986 | 0.7667 | 3.00E-04 | 0 |
| 225 | 7560593 | OSM | oncostatin M | 5008 | NM_020530.3 | 1647.1147 | 0.8196 | 3.00E-04 | 0 |
| 226 | 770167 | CD79B | CD79b molecule, immunoglobulin-associated beta | 974 | NM_001039933.1 | 1648.4831 | 1.2138 | 3.00E-04 | 0 |
| 227 | 1010035 | NLRP3 | NLR family, pyrin domain containing | 114548 | NM_004895.3 | 1648.7639 | 0.8612 | 3.00E-04 | 0 |
| 228 | 2710451 | NULL | NULL | 651894 | XM_941155.2 | 1649.6418 | 1.2523 | 3.00E-04 | 0 |
| 229 | 2260551 | RAD21 | RAD21 homolog (S.pombe) | 5885 | NM_006265.1 | 1653.0804 | 1.2801 | 3.00E-04 | 0 |
| 230 | 5670465 | ADM | adrenomedullin | 133 | NM_001124.1 | 1655.0659 | 0.7829 | 3.00E-04 | 0 |
| 231 | 6420446 | CMPK1 | cytidine monophosphate (UMP-CMP) kinase 1, cytosolic | 51727 | NM_016308.1 | 1856.035 | 1.3183 | 3.00E-04 | 0 |

**[Table 4-22]**

| RankProd | | | | | | | | | |
|---|---|---|---|---|---|---|---|---|---|
| Table 4-22 | | | | | | | | | |
| No. | Probe ID | Name of gene | Description of gene | EntrezID | Acc | RP_Rsum | RP_FC | RP_pfp | RP_pval |
| 232 | 4260372 | GTSCR1 | Gilles de la Tourette syndrome chromosome region, candidate 1 | 220158 | XM_496277.2 | 1659.3612 | 1.26 | 3 00E-04 | 0 |
| 233 | 2490450 | hCG_1992539 | - | 91561 | XM_934176.1 | 1661.3741 | 1.2023 | 3.00E-04 | 0 |
| 234 | 4040170 | HBQ1 | hemoglobin, theta 1 | 3049 | NM 005331.3 | 1662.4015 | 0.8109 | 3.00E-04 | 0 |
| 235 | 870202 | TNFSF10 | tumor necrosis factor (ligand) superfamily, member 10 | 8743 | NM_003810.2 | 1663.4194 | 0.8562 | 3.00E-04 | 0 |
| 236 | 1980594 | FTHL8 | ferritin, heavy polypeptide-like 8 | 2501 | NR_002203.1 | 1668 0712 | 1.1674 | 3.00E-04 | 0 |
| 237 | 2370041 | LRRN3 | leucine rich repeat neuronal 3 | 54674 | NM_018334.3 | 1672.3412 | 1.3042 | 3.00E-04 | 0 |
| 238 | 4220110 | NULL | NULL | 647450 | XM_936518.1 | 1672.5458 | 0.8791 | 3.00E-04 | 0 |
| 239 | 1510612 | MYL4 | myosin, light chain 4, alkali; atrial, embryonic | 4635 | NM_002476.2 | 1681.4977 | 0.7979 | 4.00E-04 | 0 |
| 240 | 4010139 | BANK1 | B-cel scaffold protein with ankynn repeats 1 | 55024 | NM_001083907.1 | 1683.3115 | 1.2158 | 4.00E-04 | 0 |
| 241 | 5090215 | IFIG | interferon, alpha-inducible protein 6 | 2537 | NM_022873.2 | 1690.3545 | 0.818 | 4.00E-04 | 0 |
| 242 | 620717 | CCL5 | chemokine (C-C motif) ligand 5 | 6352 | NM_002985.2 | 1691.5746 | 0.812 | 4 00E-04 | 0 |

**[Table 4-23]**

| RankProd | | | | | | | | | |
|---|---|---|---|---|---|---|---|---|---|
| Table 4-23 | | | | | | | | | |
| No. | Probe ID | Name of gene | Description of gene | EntrezID | Acc | RP_Rsum | RP_FC | RP_pfp | RP pval |
| 243 | 4610681 | LOC653658 | - | 653658 | XM_939687.2 | 1693.2132 | 1.3052 | 4.00E-04 | 0 |
| 244 | 4290349 | LOC643949 | - | 643949 | XR_018645.1 | 1695.2143 | 1.2653 | 4.00E-04 | 0 |
| 245 | 450615 | MT2A | metallothionein 2A | 4502 | NM_005953.2 | 1696.9888 | 0.7903 | 4.00E-04 | 0 |
| 246 | 1110373 | C15orf48 | chromosome 15 open reading frame 48 | 84419 | NM_032413.2 | 1702.1009 | 0.946 | 4.00E-04 | 0 |
| 247 | 6400243 | ABLIM1 | actin binding LIM protein 1 | 3983 | NM_001003407.1 | 1703.3875 | 1.2737 | 4.00E-04 | 0 |
| 248 | 6560279 | VAV3 | vav 3 guanine nucleotide exchange factor | 10451 | NM_006113.4 | 1704.7097 | 1.0099 | 4.00E-04 | 0 |
| 249 | 3940504 | CD79A | CD79a molecule, immunoglobulin-associated alpha | 973 | NM_021601.3 | 1705.1684 | 1.136 | 4.00E-04 | 0 |
| 250 | 6510072 | ACTG1 | actin, gamma 1 | 71 | NM_001614.2 | 1705.2256 | 0.8476 | 4.00E-04 | 0 |
| 251 | 610524 | CCL3L1 | chemokine (G-C motif) ligand 3-like | 6349 | NM_021006.4 | 1713.0407 | 1.1746 | 4.00E-04 | 0 |
| 252 | 6590646 | FAM26F | family with sequence similarity 26, member F | 441168 | NM_001010919.1 | 1714.9816 | 1 2875 | 5.00E-04 | 0 |
| 253 | 3830136 | NACAP1 | nascent-polypeptide-associated complex alpha polypeptide pseudogene 1 | 83955 | NR_002182.1 | 1716.7585 | 1.3074 | 5.00E-04 | 0 |

**[Table 4-24]**

| RankProd | | | | | | | | | |
|---|---|---|---|---|---|---|---|---|---|
| Table 4-24 | | | | | | | | | |
| No. | Probe ID | Name of gene | Description of gene | EntrezID | Acc | RP_Rsum | RP_FC | RP_pfp | RP_pval |
| 254 | 7650220 | hCG_1787519 | - | 643007 | XM_938089.2 | 1718.0255 | 1.2797 | 5.00E-04 | 0 |
| 255 | 1070477 | ALDH1A1 | aldehyde dehydrogenase 1 family, member A1 | 216 | NM_000689.3 | 1718.8928 | 1.2672 | 5.00E-04 | 0 |
| 256 | 6510754 | ALDH1A1 | aldehyde dehydrogenase 1 family, member A1 | 216 | NM_000689.3 | 1720.5388 | 1.2203 | 5.00E-04 | 0 |
| 257 | 3930008 | RPL14 | ribosomal protein L14 | 9045 | NM_001034996.1 | 1720.6296 | 1.0405 | 5.00E-04 | 0 |
| 258 | 840551 | CEP27 | centrosomal protein 27kDa | 55142 | NM_018097.1 | 1724.7797 | 1.2511 | 5.00E-04 | 0 |
| 259 | 5050402 | H | histone cluster 1, H2bk | 85236 | NM_080593.1 | 1726_2525 | 0.7687 | 5.00E-04 | 0 |
| 260 | 4920228 | FFAR2 | free fatty acid receptor 2 | 2867 | NM_005306.1 | 1726.9269 | 0.8449 | 5.00E-04 | 0 |
| 261 | 3400521 | NULL | NULL | 653086 | XM_930995.1 | 1728.9741 | 1.249 | 5.00E-04 | 0 |
| 262 | 4890241 | GPR56 | G protein-coupled receptor 56 | 9289 | NM_201524.1 | 17307855 | 0.9285 | 5.00E-04 | 0 |
| 263 | 6280446 | NULL | NULL | 642989 | XM_926370.1 | 1731.2064 | 1.2277 | 5.00E-04 | 0 |
| 264 | 2030132 | LOC653506 | - | 653506 | XM_927769.1 | 1732_6509 | 0.8096 | 5.00E-04 | 0 |

**[Table 4-25]**

| RankProd | | | | | | | | | |
|---|---|---|---|---|---|---|---|---|---|
| Table 4-25 | | | | | | | | | |
| No. | Probe ID | Name of gene | Description of gene | EntrezID | Acc | RP_Rsum | RP_FC | RP_pfp | RP_pval |
| 265 | 3310538 | CD36 | CD36 molecule (thrombospondin receptor) | 948 | NM_000072.2 | 1732.7017 | 1.2213 | 5.00E-04 | 0 |
| 266 | 2640025 | HP | haptoglobin | 3240 | NM_005143.2 | 1733.6315 | 0.7543 | 5.00E-04 | 0 |
| 267 | 1940047 | AIF1 | allograft inflammatory factor 1 | 199 | NM_001623.3 | 1734.8998 | 0.7781 | 5-00E 04 | 0 |
| 268 | 5690671 | GOLGA8B | golgi autoantigen, golgin subfamily a, 8B | 440270 | NM_001023567.2 | 1735.7582 | 1.2537 | 5.00E-04 | 0 |
| 269 | 2470601 | IL1RN | interleukin 1 receptor antagonist | 3557 | NM_173842.1 | 1736.4413 | 0.8188 | 5.00E-04 | 0 |
| 270 | 4150201 | BCL2 | B-cell CLL/lymphoma 2 | 596' | NM_000633.2 | 1737.2395 1.2312 | | 5.00E-04 | 0 |
| 271 | 6580059 | UCP2 | uncoupling protein 2 (mitochondrial, proton carrier) | 7351 | NM_003355.2 | 1738_6131 | 1.2387 | 5.00E-04 | 0 |
| 272 | 3180131 | hCG_1984468 | - | 389672 | XM_933893.1 | 1749.2716 | 1.3078 | 5.00E-04 | 0 |
| 273 | 1770273 | ANKRD30B | ankyrin repeat domain 30B | 374860 | NM_001029862.1 | 1750.2072 | 1.2567 | 5.00E-04 | 0 |
| 274 | 64550102 | NULL | NULL | 642113 | XM_936253.1 | 1754.1531 | 0.9366 | 6.00E-04 | 0 |
| 275 | 1340743 | IL8 | interleukin 8 | 3576 | NM_000584.2 | 1754_2793 | 1.148 | 5_00E-04 | 0 |

**[Table 4-26]**

| RankProd | | | | | | | | | |
|---|---|---|---|---|---|---|---|---|---|
| Table 4-26 | | | | | | | | | |
| No, | Probe ID | Name of gene | Description of gene | EntrezID | Acc | RP_Rsum | RP_FC | RP_pfp | RP_pval |
| 276 | 6620292 | C10orf54 | chromosome 54 10 open reading frame | 64115 | NM_022153.1 | 1755.4366 | 0.7563 | 6.00E-04 | 0 |
| 277 | 2570358 | HNRPDL | heterogeneous nuclear ribonucleoproteinD-like | 9987 | NM_031372.1 | 1756.6323 | 1.304 | 6.00E-04 | 0 |
| 278 | 510209 | LOC643310 | - | 643310 | XM_926656.1 | 1756.8922 | 1.3155 | 6 00E-04 | 0 |
| 279 | 1770603 | TGN1 | transcobalamin I (vitamin B12 binding protein, R binder family) | 6947 | NM_001062.3 | 1757.6057 | 1.1103 | 6.00E-04 | 0 |
| 280 | 1440121 | tcag7 1261 | - | 645968 | XM_928934.1 | 1759.0714 | 1.2841 | 6.00E-04 | 0 |
| 281 | 160242 | C13orf15 | chromosome 13 open reading frame | 28984 | NM_014059.2 | 1764.5447 | 1.1056 | 6.00E-04 | 0 |
| 282 | 4200541 | FAM113B | family with sequence similarity 113, member B | 91523 | NM_138371 1 | 1768.2475 | 1.2217 | 6.00E-04 | 0 |
| 283 | 990500 | AVPI1 | arginine vasopressin-induced 1 | 60370 | NM_021732.1 | 1770.8805 | 0.8357 | 7.00E-04 | 0 |
| 284 | 1440605 | ZNF101 | zinc finger protein 101 | 94039 | NM_033204.2 | 1771.8967 | 1.2622 | 6.00E-04 | 0 |
| 285 | 2230167 | LOC653773 | - | 653773 | XM_938755.2 | 1772.9624 | 1 2915 | 6.00E-04 | 0 |
| 286 | 10358 | SPN | sialophorin | 6693 | NM_001030288.1 | 1772.9705 | 0.7869 | 7.00E-04 | 0 |

**[Table 4-27]**

| RankProd | | | | | | | | | |
|---|---|---|---|---|---|---|---|---|---|
| Table 4-27 | | | | | | | | | |
| No. | Probe ID | Name of gene | Description of gene | EntrezID | Acc | RP_Rsum | RP FC | RP_pfp | RP_pval |
| 287 | 7150711 | ZNF223 | zinc finger protein 223 | 7766 | NM_013361.3 | 1775.2142 | 1.2356 | 6.00E-04 | 0 |
| 288 | 3420154 | NLRP3 | NLR family, pyrin domain containing 3 | 114548 | NM_001079821.1 | 1778.0983 | 0.8935 | 7.00E-04 | 0 |
| 289 | 1710630 | ST6GAL1 | ST6 beta-galactosamide alpha 2,6-sialyltranferase 1 | 6480 | NM_003032.2 | 1778.5828 | 1.224 | 6.00E-04 | 0 |
| 290 | 5360064 | GNLY | granulysin | 10578 | NM_012483.1 | 1786.0213 | 0.8506 | 7.00E-04 | 0 |
| 291 | 1500201 | HNRNPC | heterogeneous nuclear ribonucleoprotein C (C1/C2) | 3183 | NM_031314.1 | 1788.8391 | 1.3177 | 7.00E-04 | 0 |
| 292 | 6020523 | PPP2R2B | protein phosphatase 2 (formerly 2A), regulatory subunit B, beta isoform | 5521 | NM_181676.1 | 1795.9286 | 0.8076 | 8.00E-04 | 0 |
| 293 | 6280008 | HSD17B11 | hydroxysteroid (17-beta) dehydrogenase | 51170 11 | NM_016245.3 | 1796.2197 | 1.2755 | 7.00E-04 | 0 |
| 294 | 3780193 | FCRLA | Fc receptor-like A | 84824 | NM_032738.3 | 1801.0361 | 1.1008 | 7.00E-04 | 0 |
| 295 | 580706 | CCR2 | chemokine (C-C motif) receptor 2 | 1231 | NM_000647.4 | 1810.4502 | 1.0875 | 8.00E-04 | 0 |
| 296 | 7510386 | IL1RN | interleukin 1 receptor antagonist | 3557 | NM_173843.1 | 1813.3956 | 0.9866 | 8.00E-04 | 0 |
| 297 | 1230767 | IFITM2 | interferon induced transmembrane protein 2 (1-8D) | 10581 | NM_006435.2 | 1814.7255 | 0.7643 | 8.00E-04 | 0 |

**[Table 4-28]**

| RankProd | | | | | | | | | |
|---|---|---|---|---|---|---|---|---|---|
| Table 4-28 | | | | | | | | | |
| No. | Probe ID | Name of gene | Description of gene | EntrezID | Acc | RP_Rsum | RP_FC | RP_pfp | RP_pval |
| 298 | 460164 | FTHL11 | ferritin, heavy polypeptide-like 11 | 2503 | NR_002204.1 | 1816.8226 | 1.1796 | 8.00E-04 | 0 |
| 299 | 4150100 | PASK | PAS domain containing serine/threonine kinase | 23178 | NM_015148.2 | 1817.7821 | 1.1603 | 8.00E-04 | 0 |
| 300 | 4050491 | TCEA3 | transcription elongation factor A (SII), 3 | 6920 | NM_003196.1 | 1817.9245 | 1.2597 | 8.00E-04 | 0 |

Table 5: List of top 300 genes selected by Spearman's rank correlation coefficient.

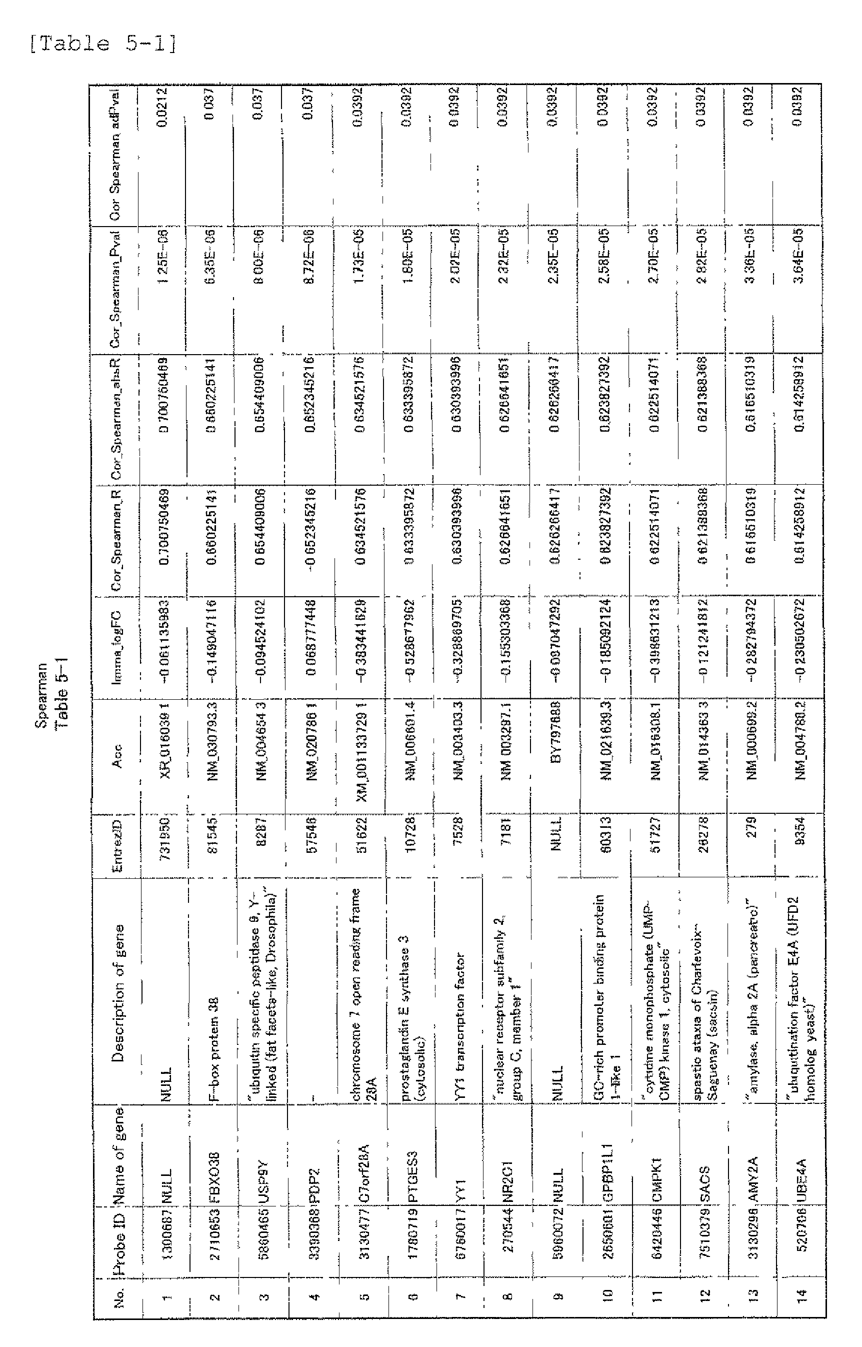

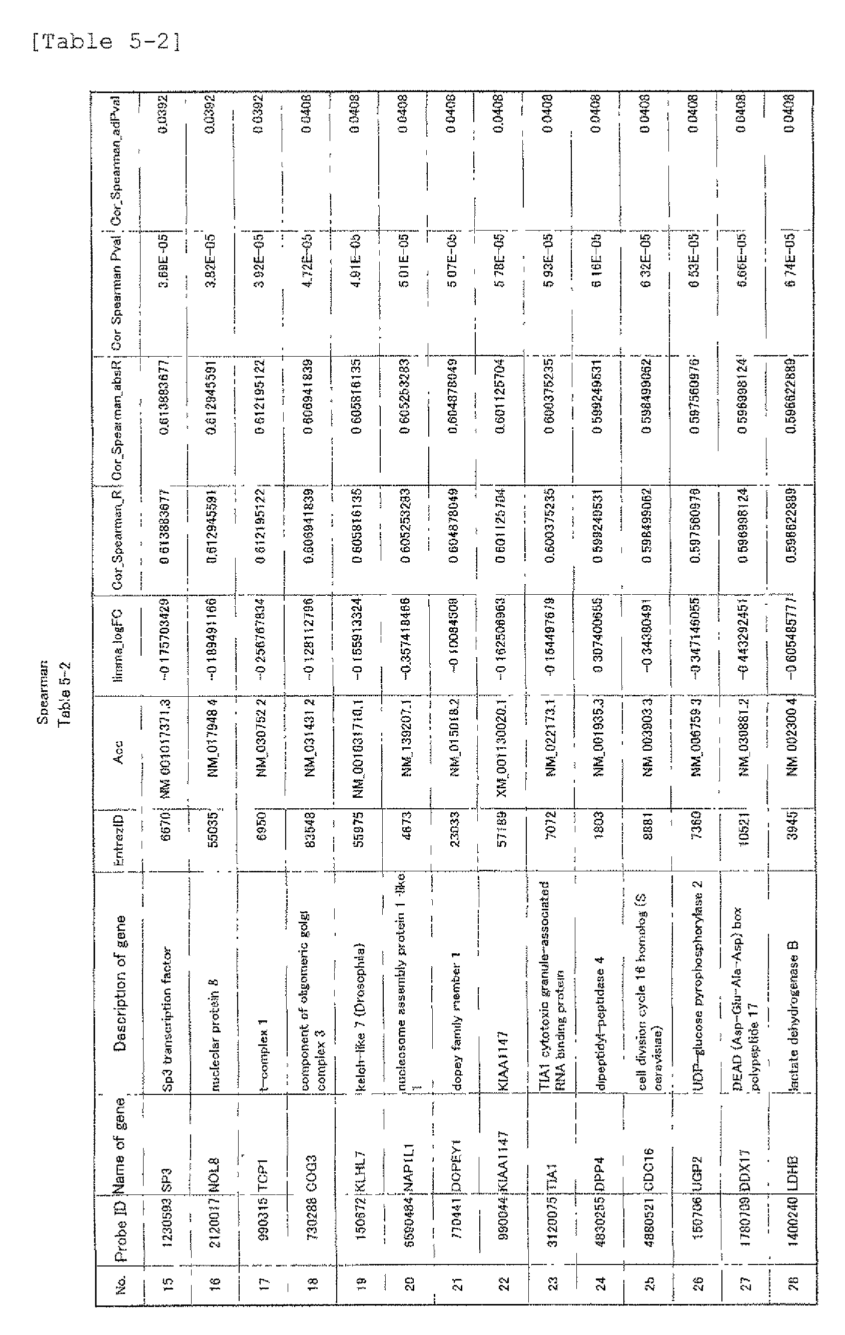

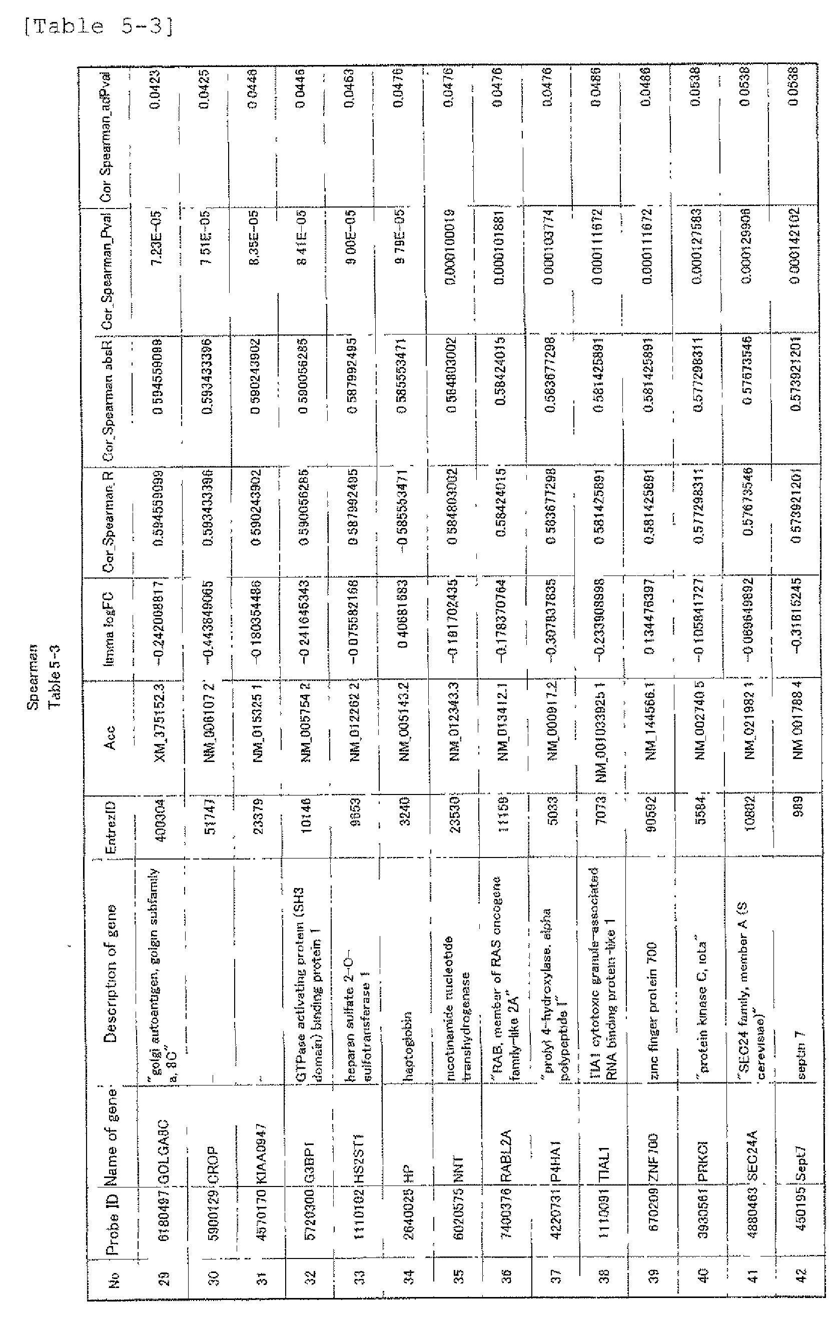

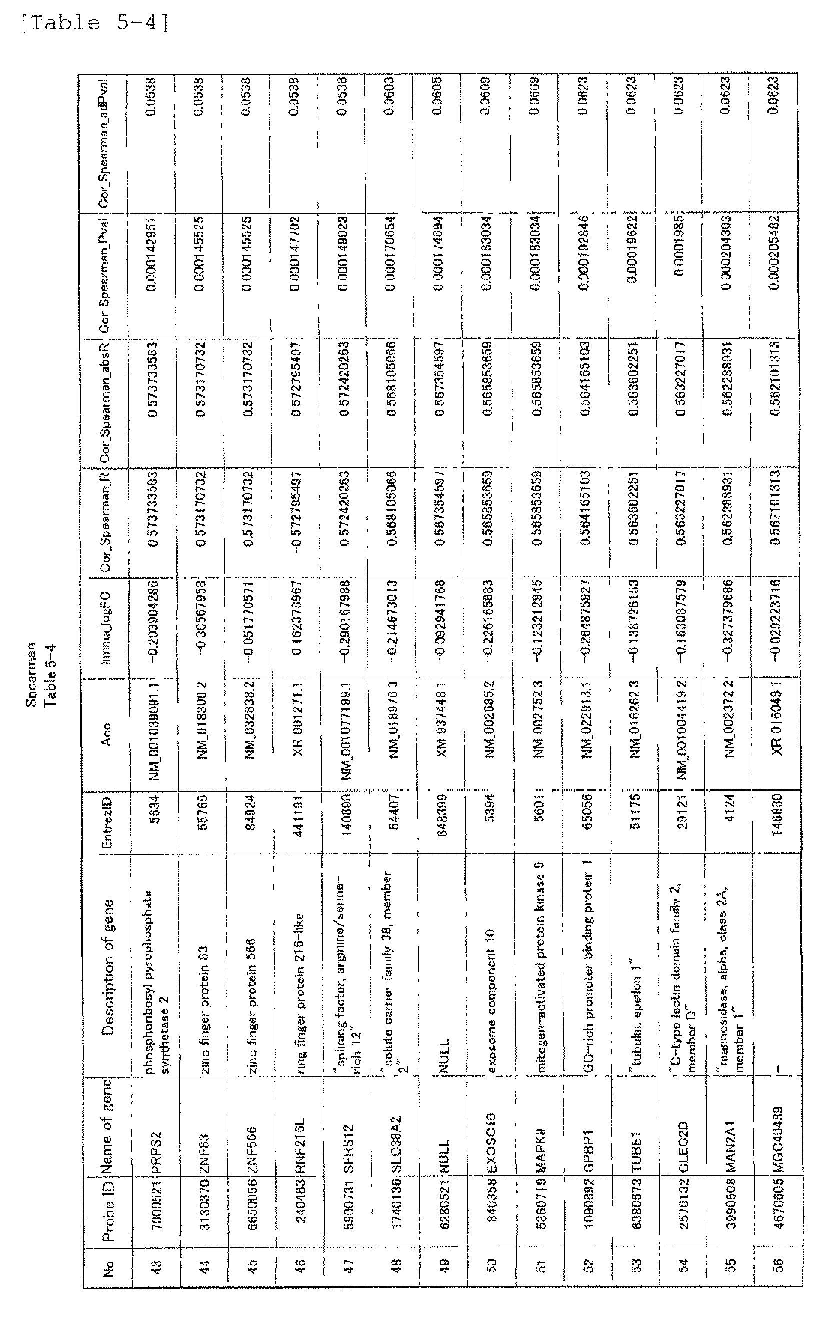

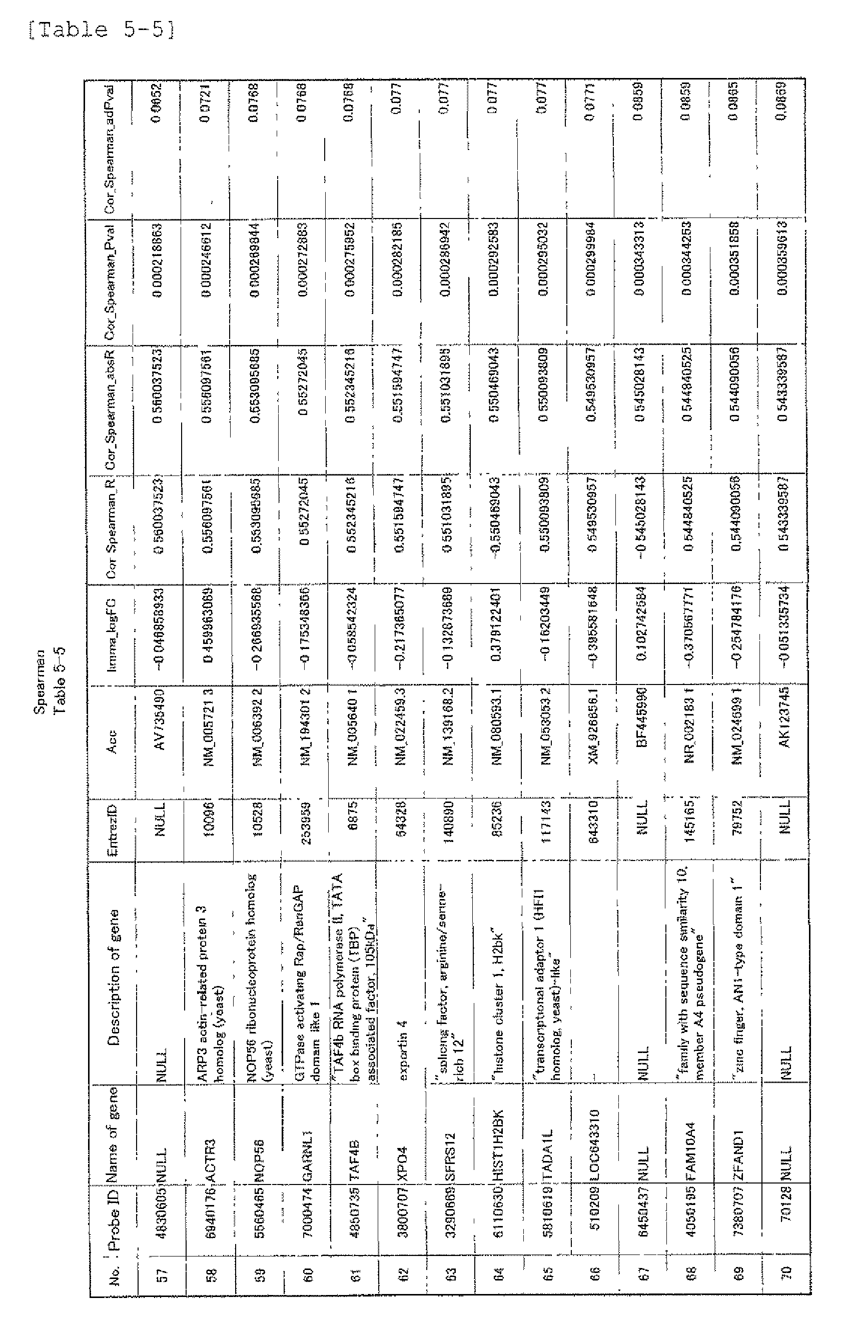

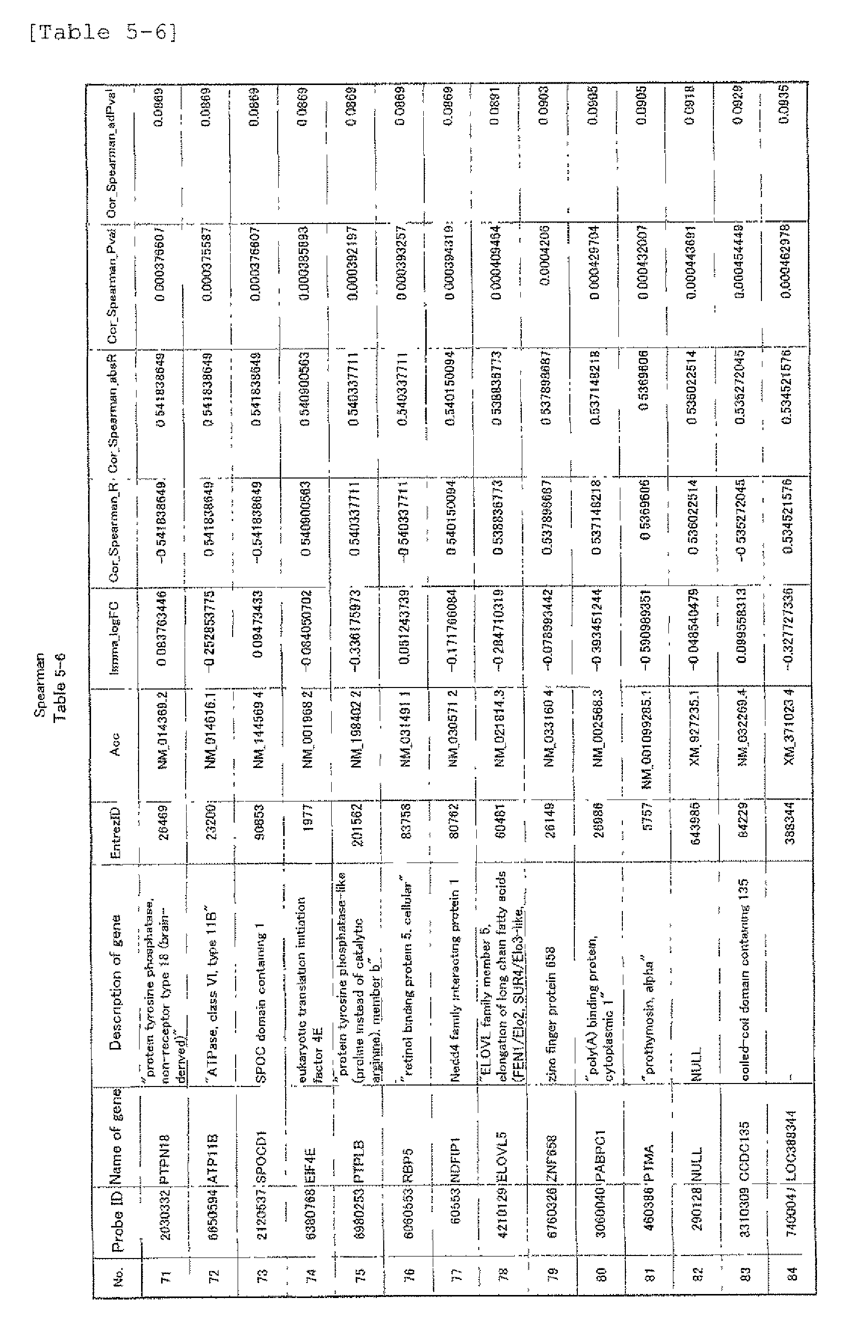

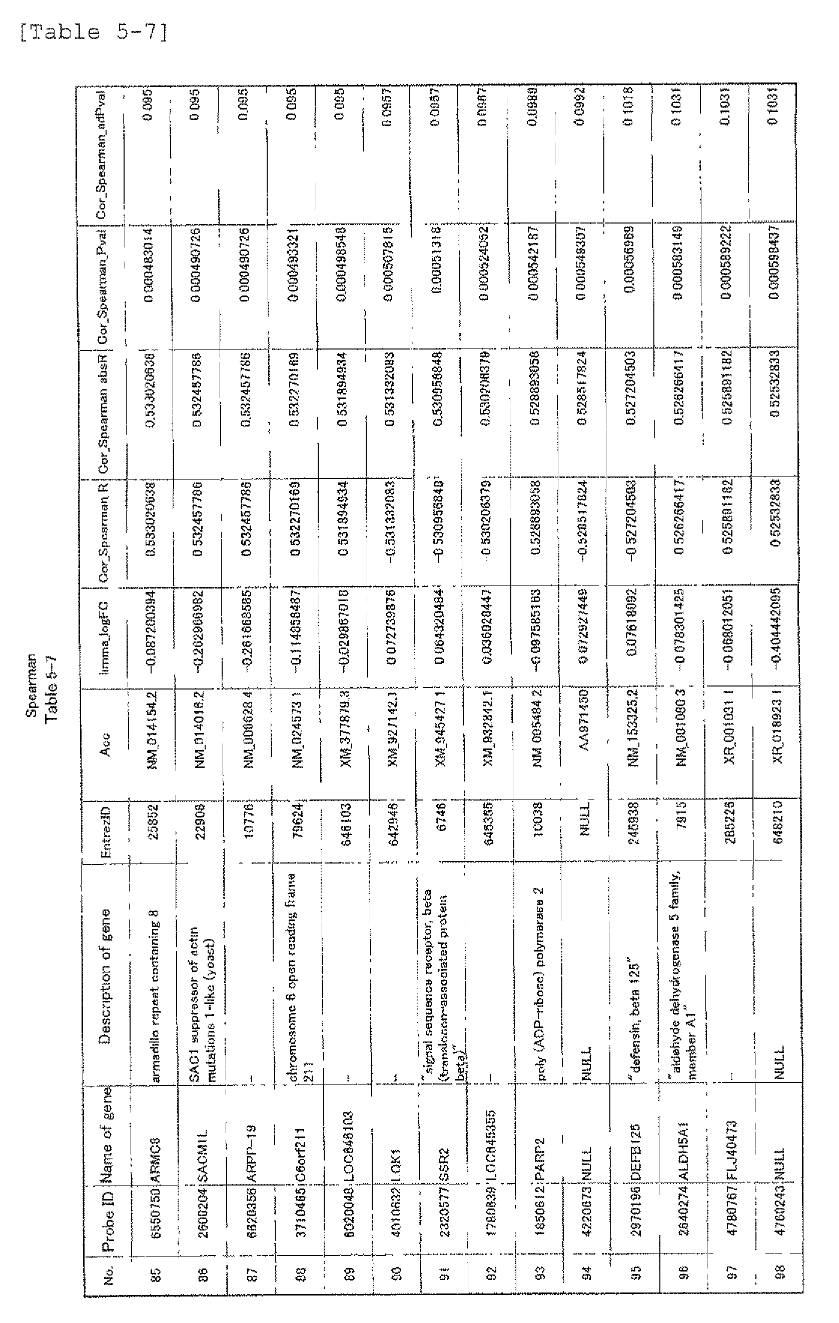

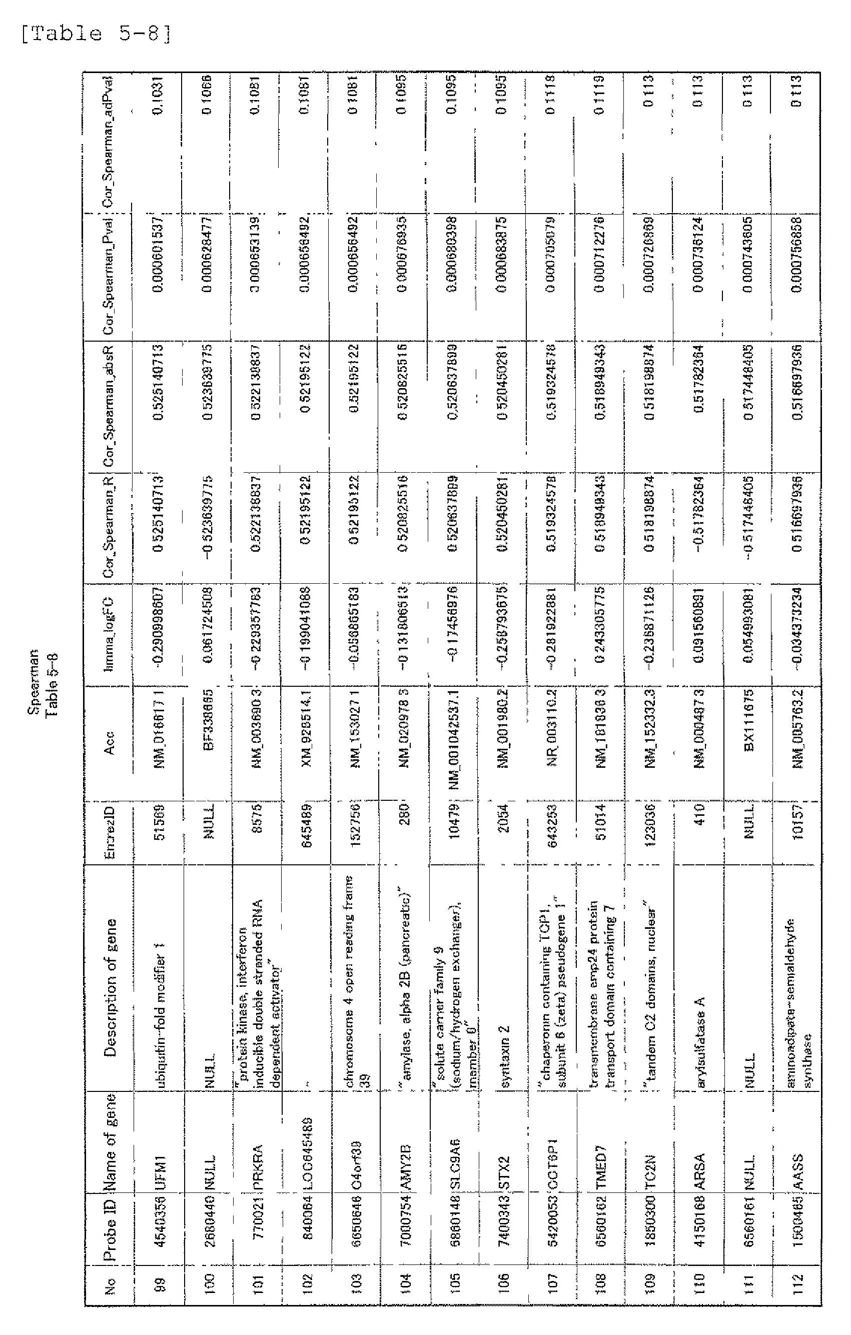

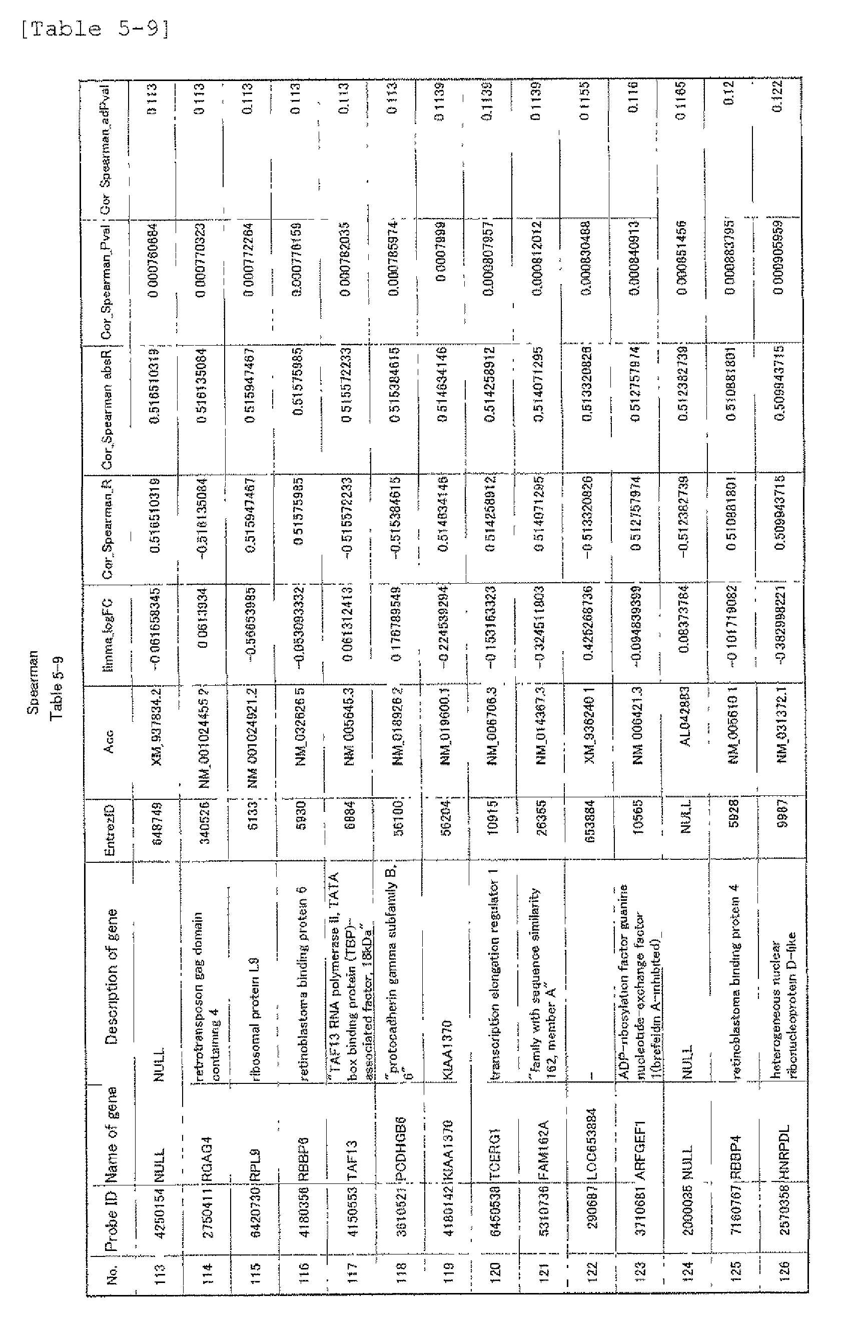

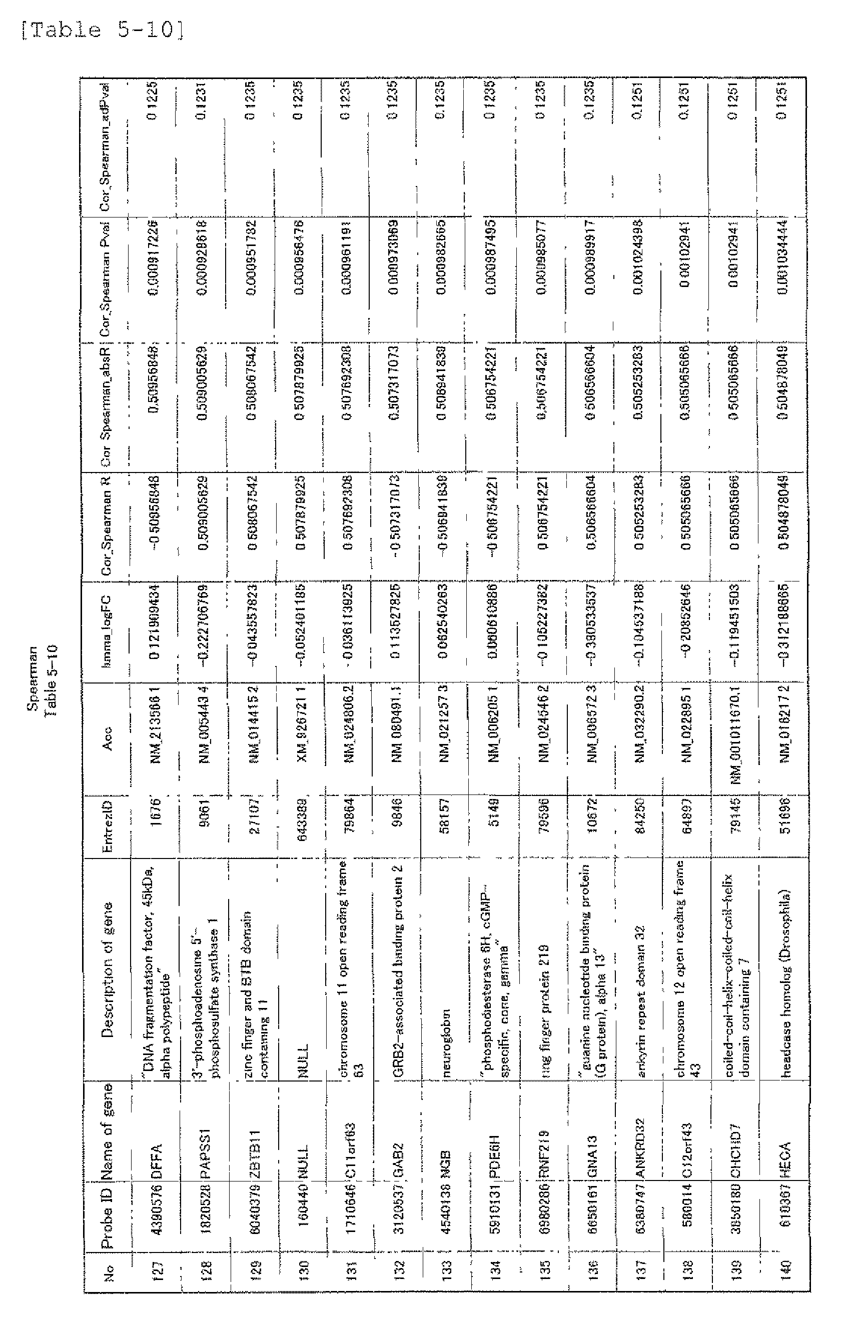

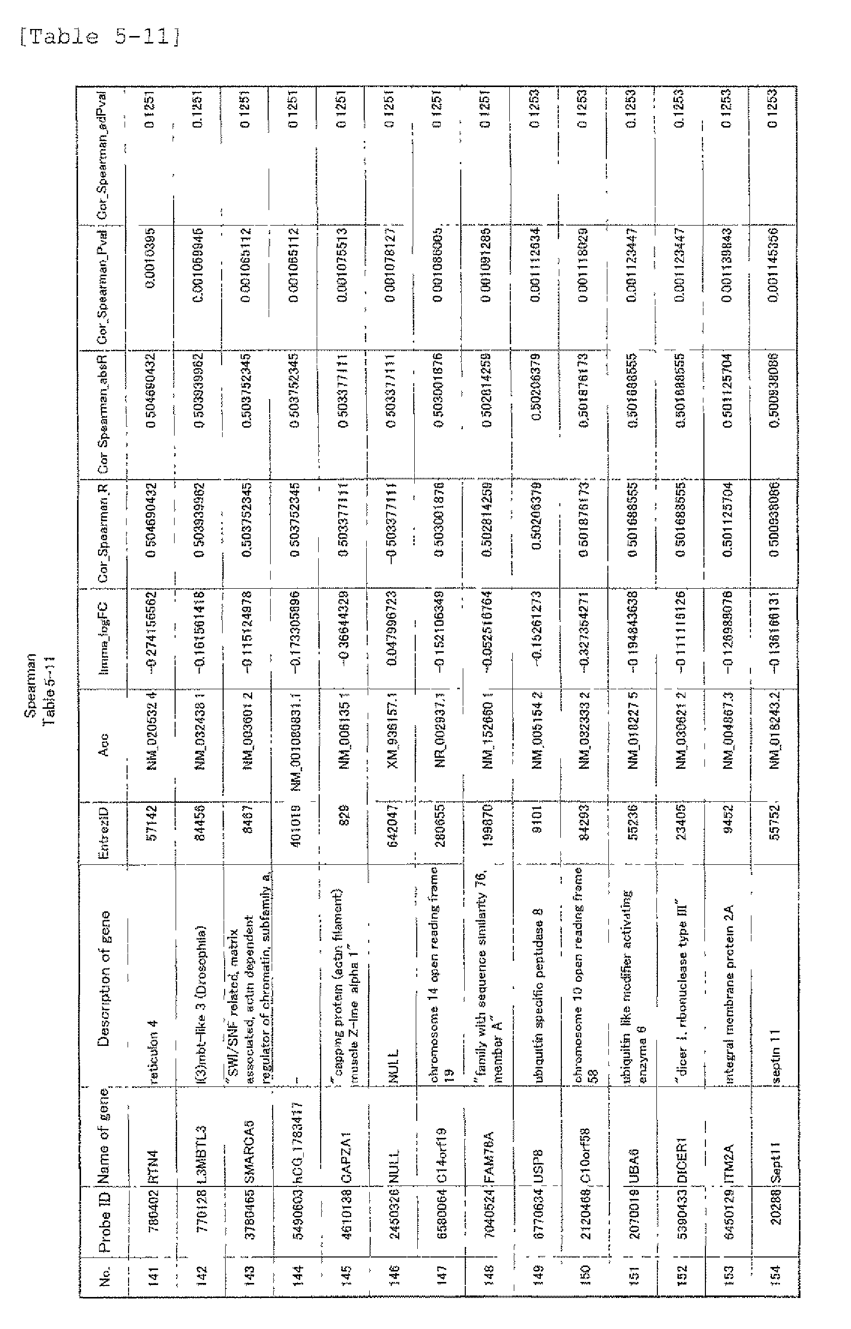

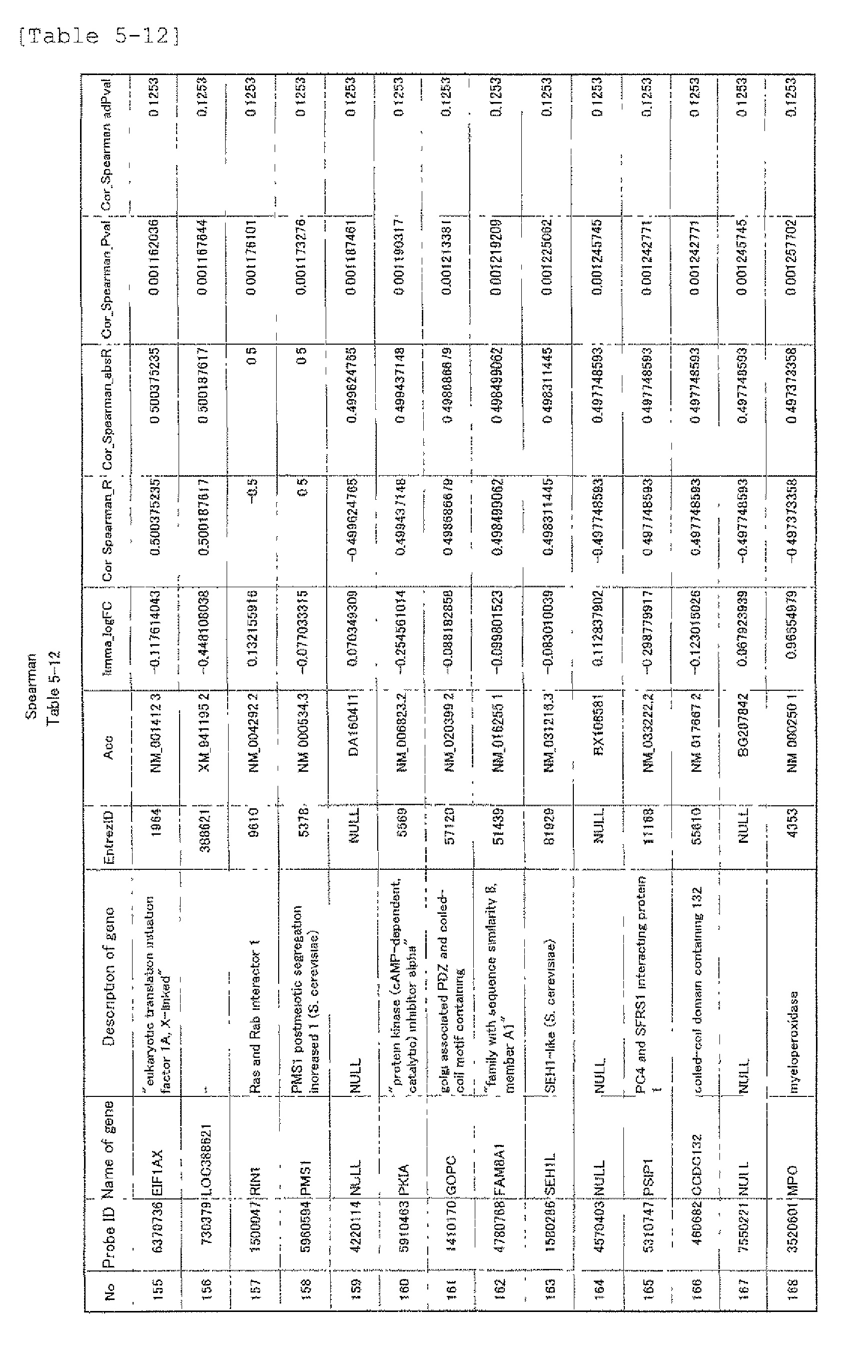

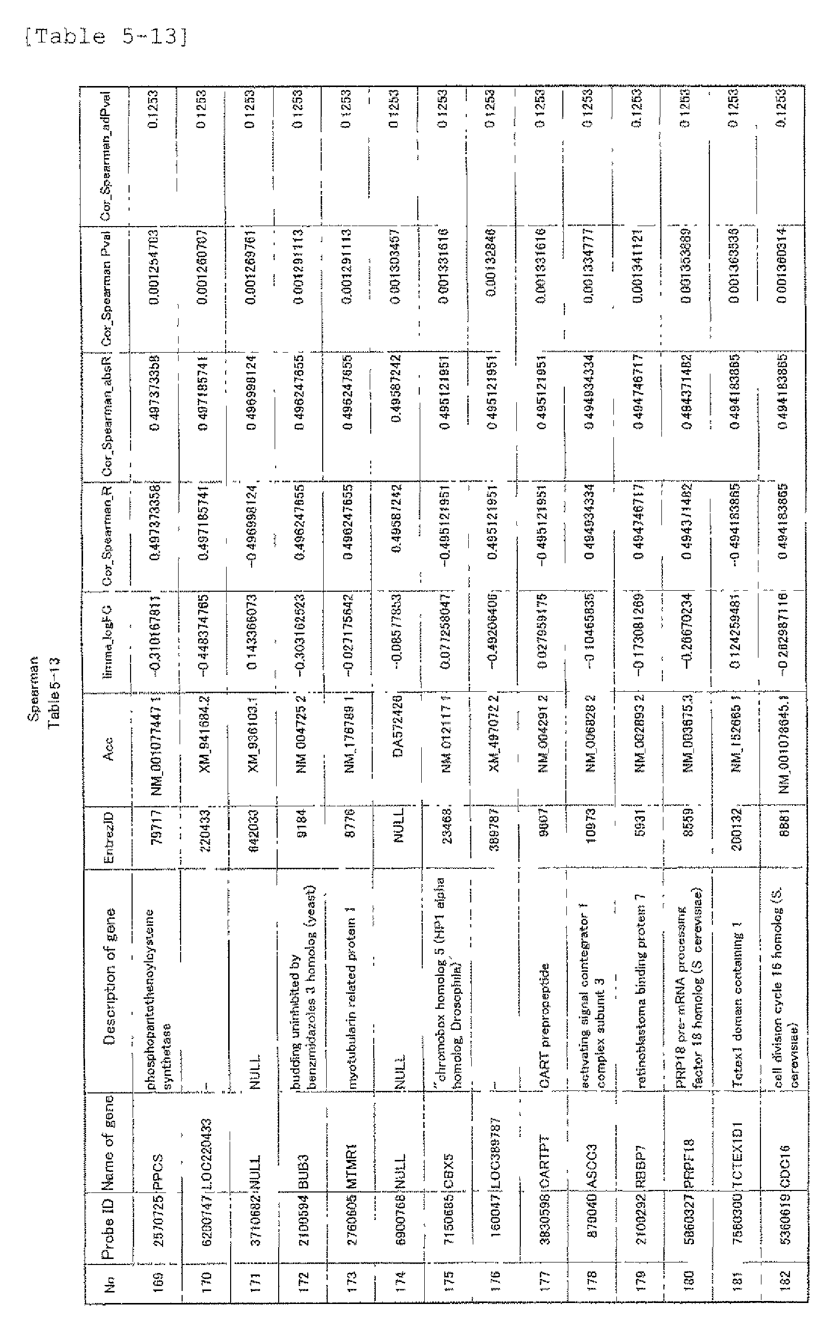

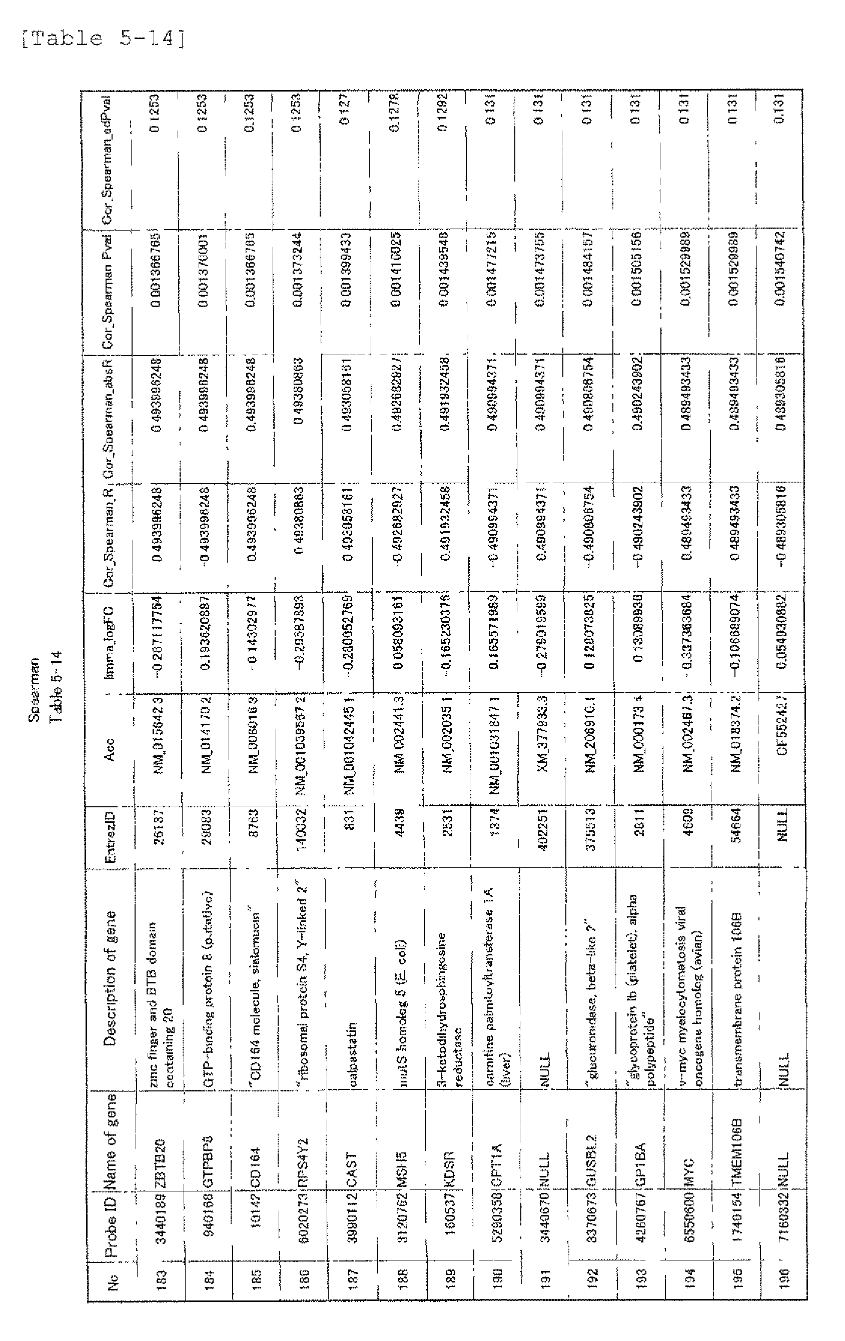

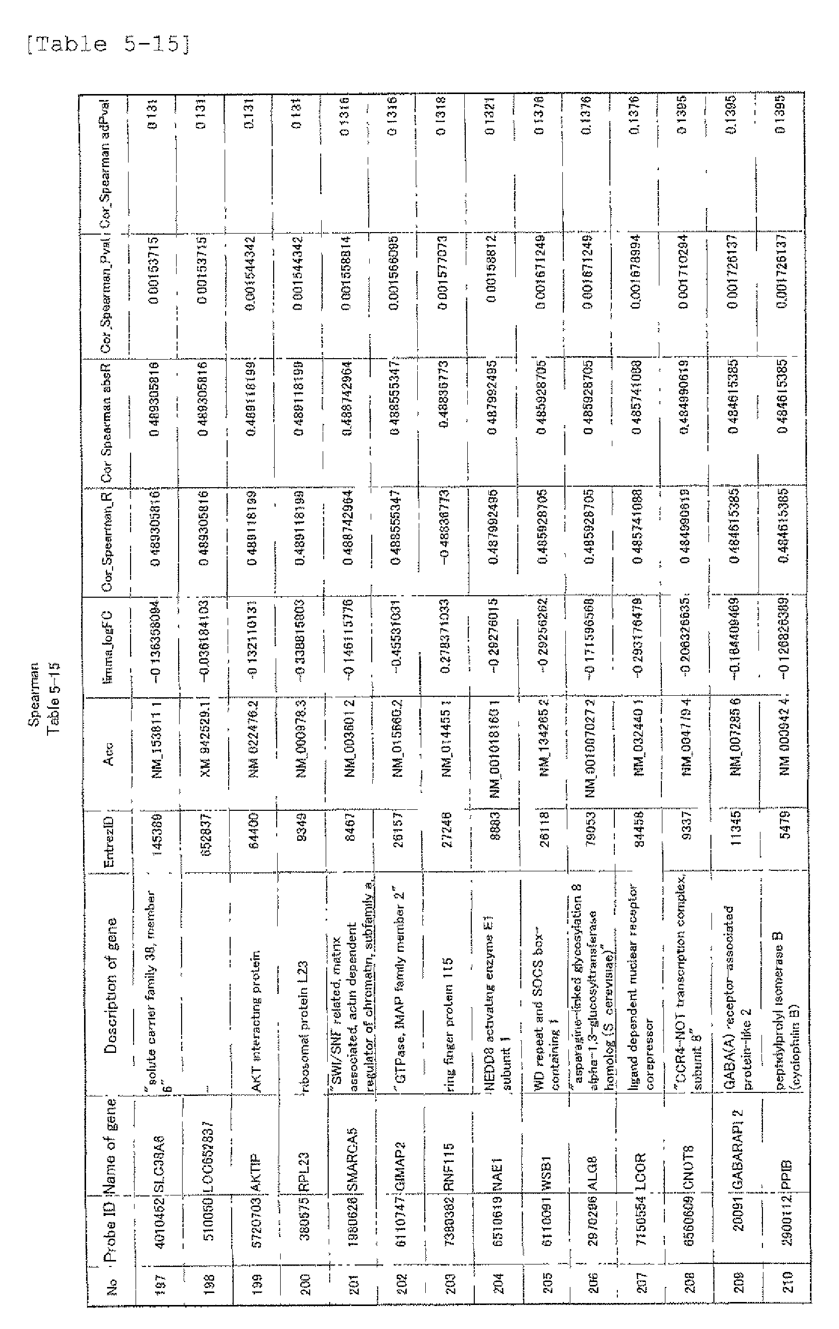

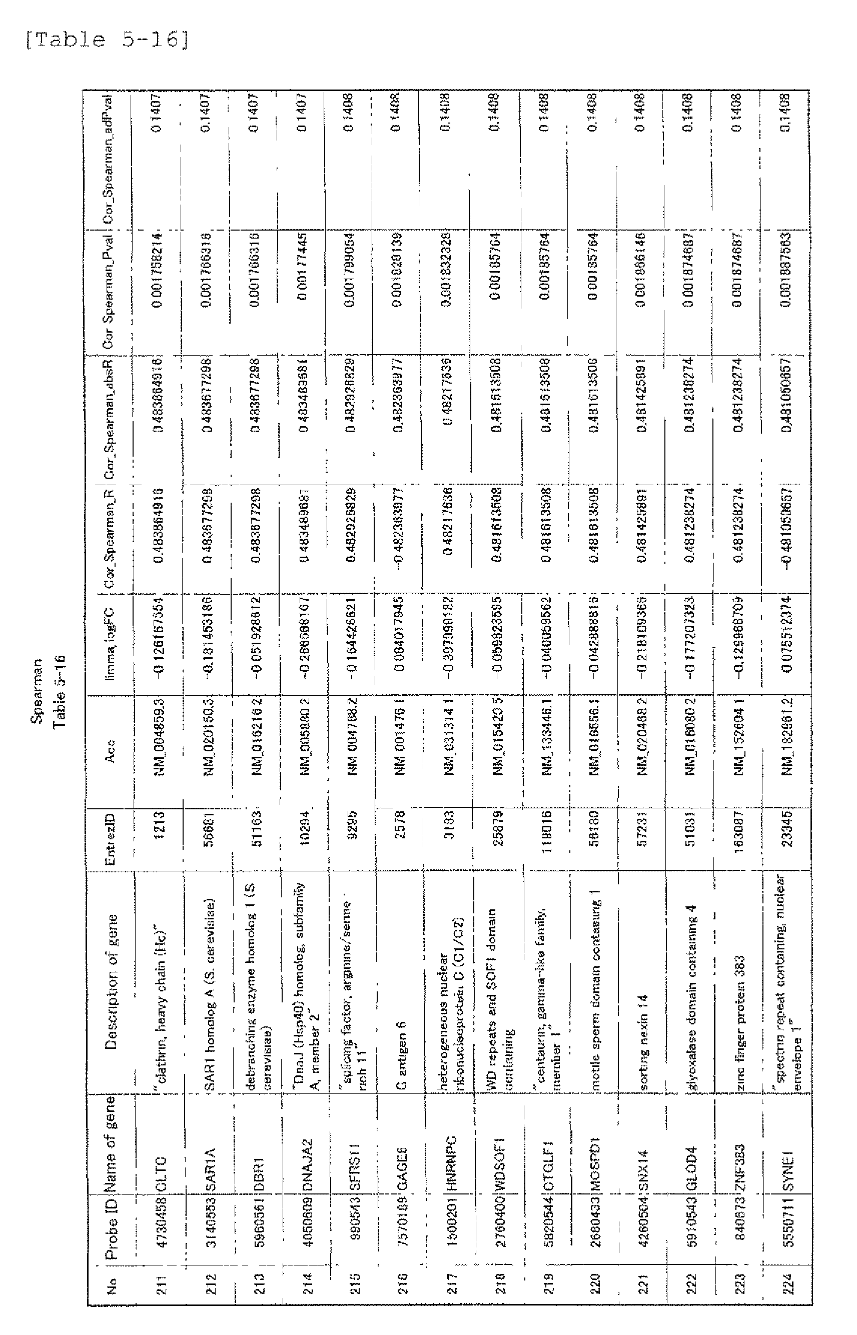

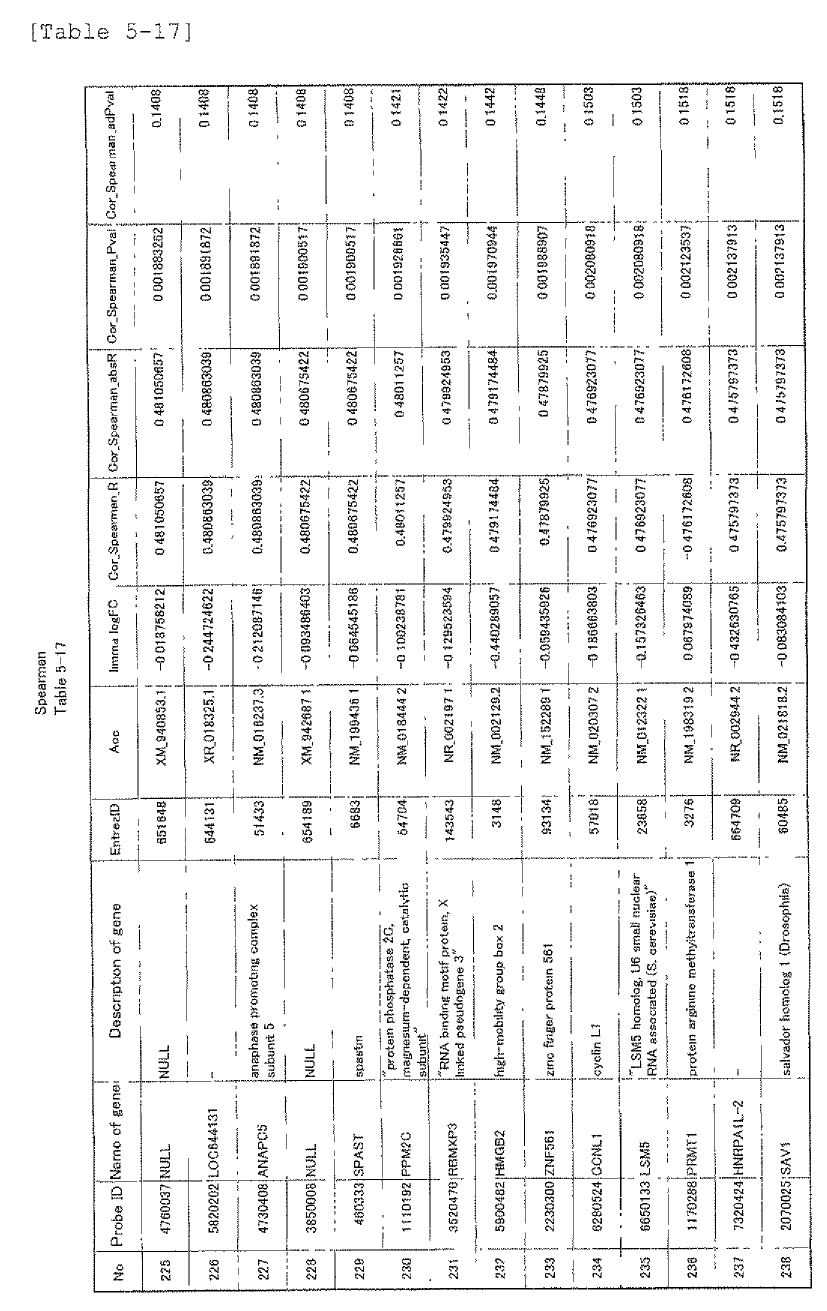

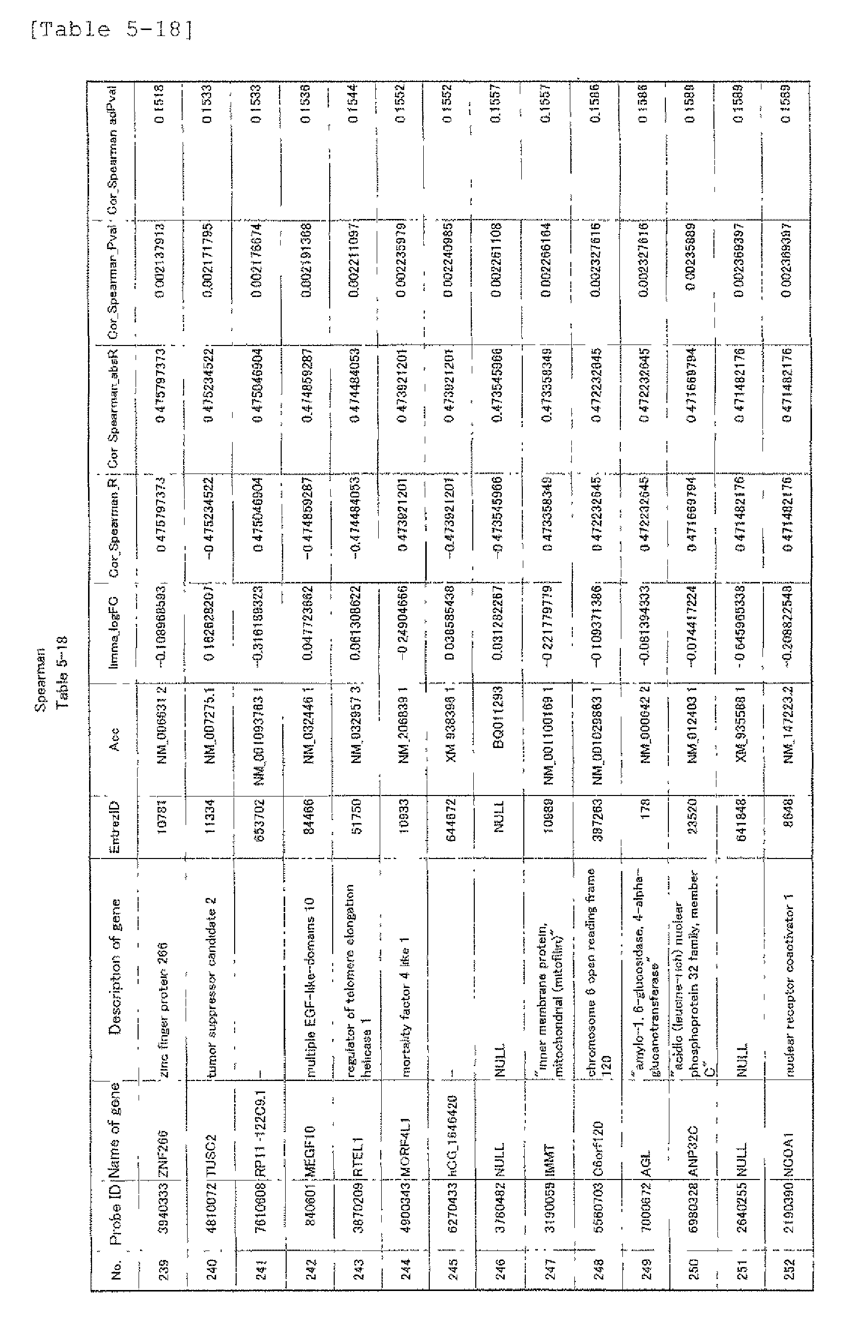

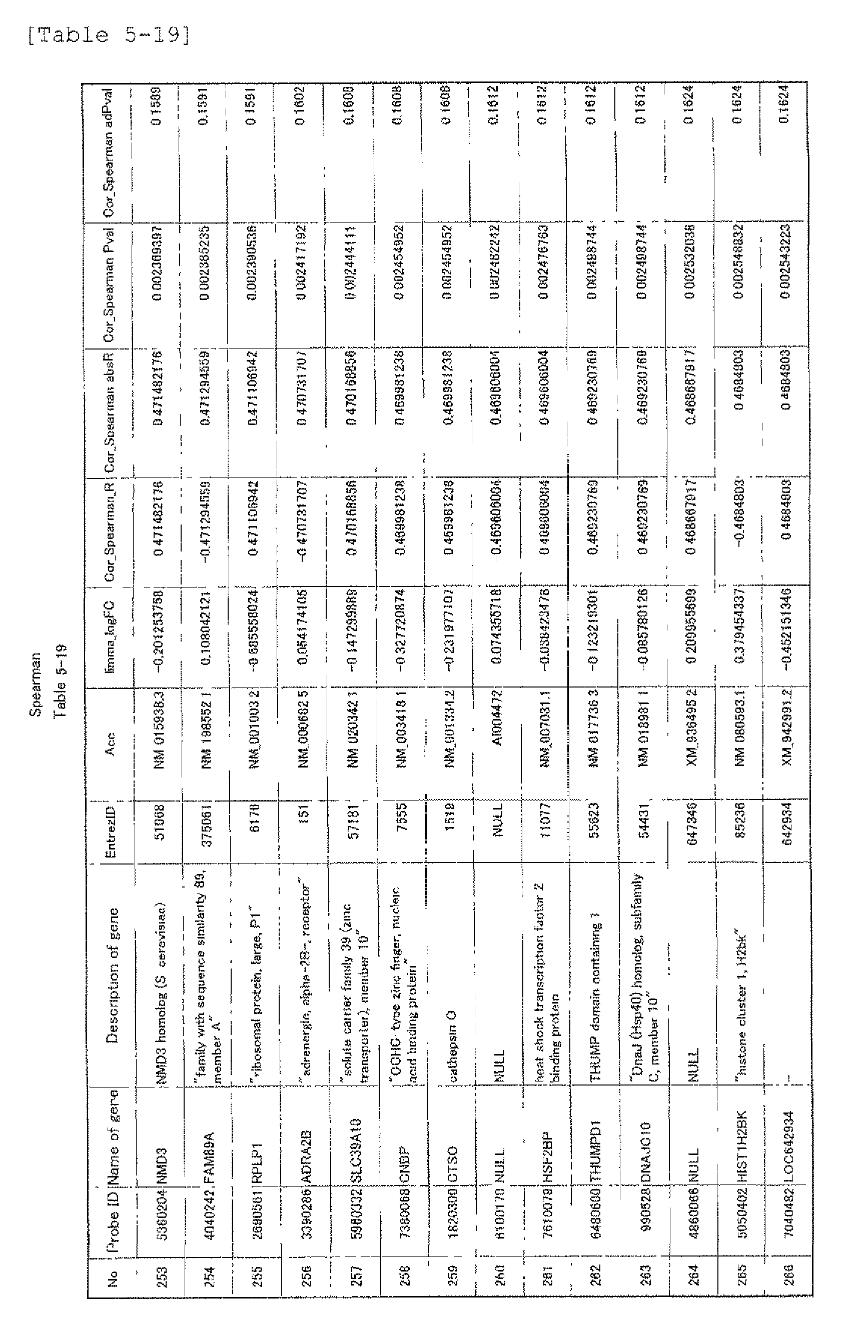

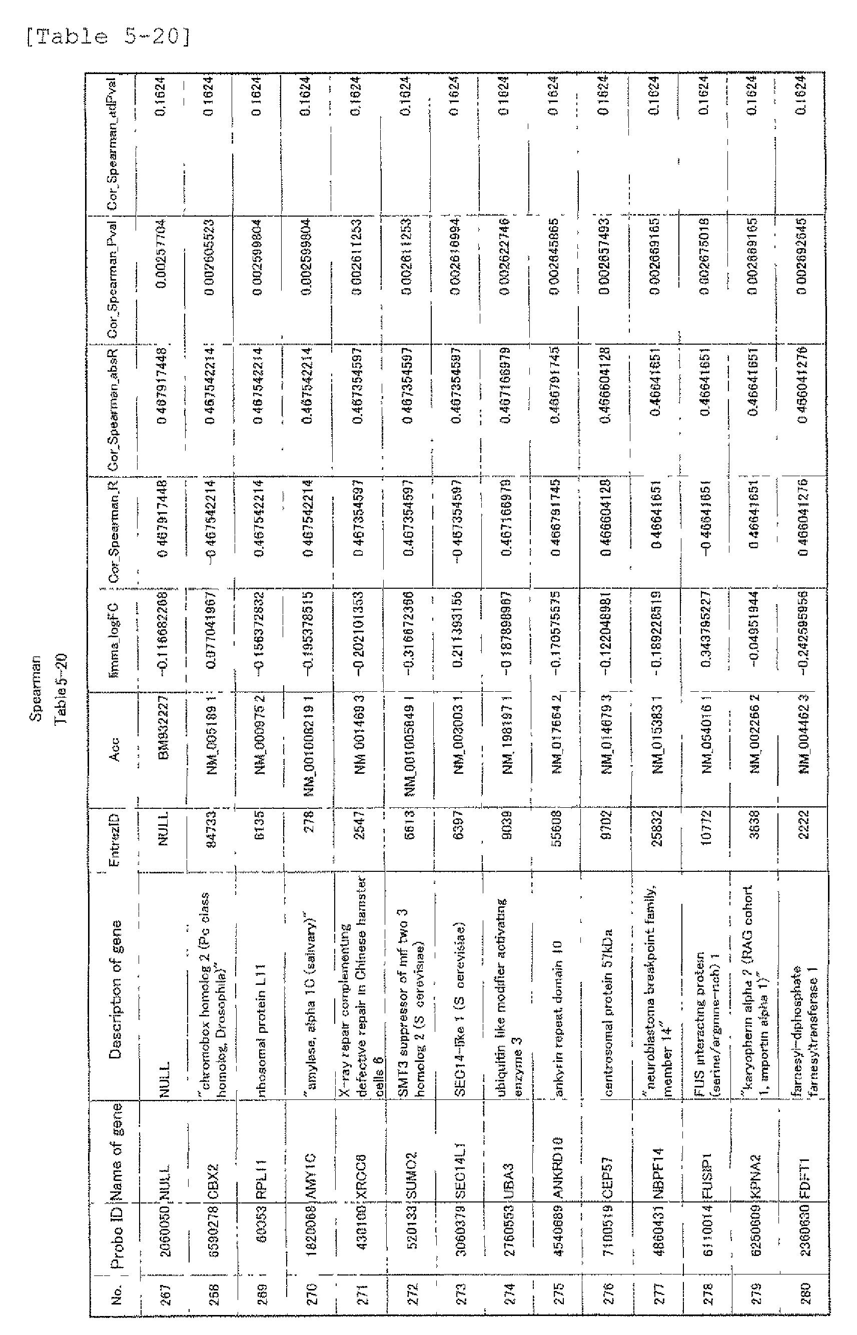

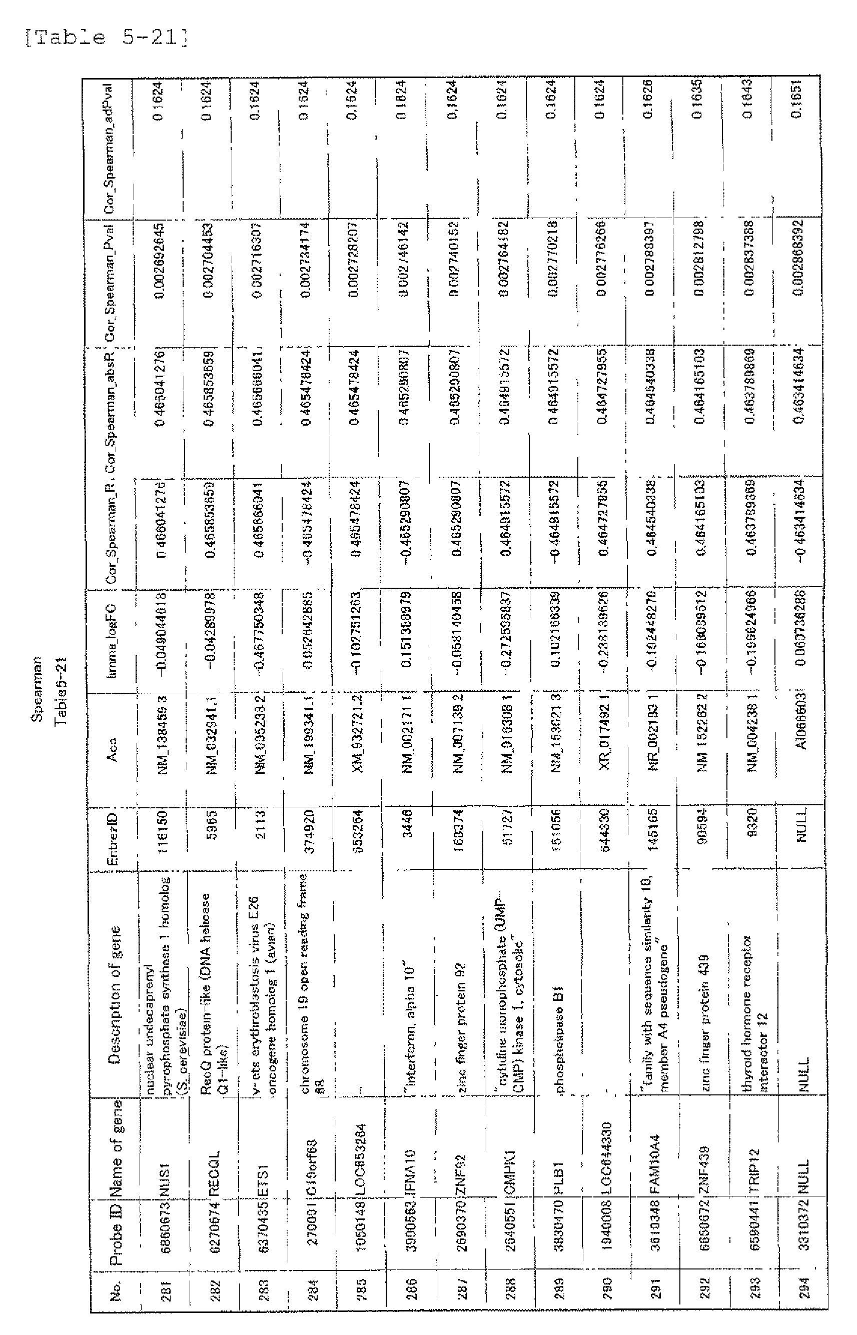

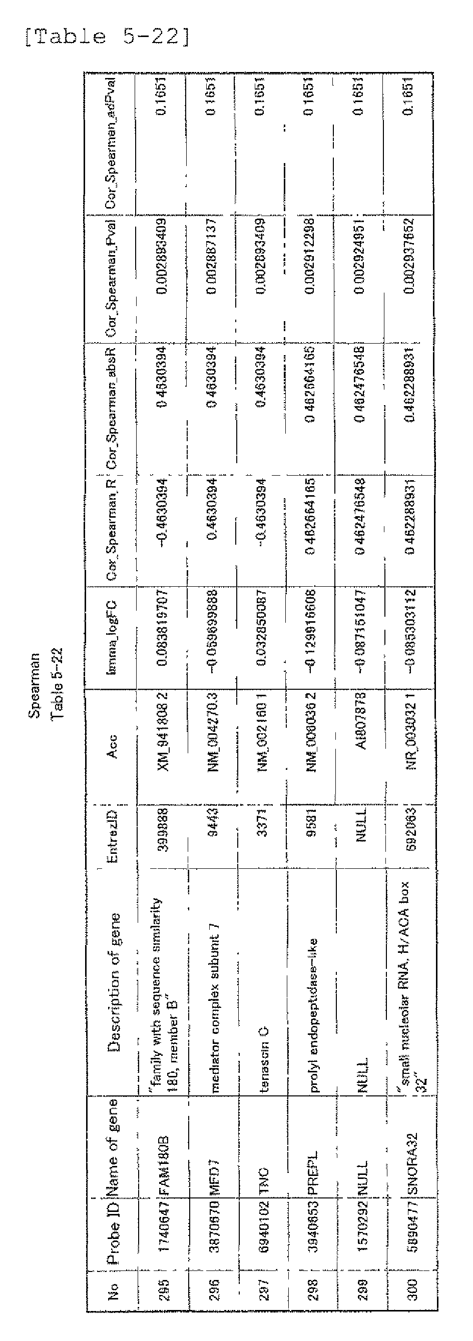

Table 6: Coefficients used for the prediction of survival time of each patient with the gene set consisting of top 50 genes selected by Pearson product-moment correlation coefficient, latent variable 3.

### [Table 6-1]

**[Table 6-1]**

| Table 6-1 | | | | | | | | | | |
|---|---|---|---|---|---|---|---|---|---|---|
| ProbeID | S2_pre | S3_pre | S4_pre | S6_pre | S7_pre | S8_pre | L3_pre | L4_pre | L1_pre | S10_pre |
| 3390368 | -185 | -188 | -199 | -198 | -173 | -185 | -184 | -184 | -190 | -187 |
| 1300687 | 127 | 134 | 118 | 138 | 138 | 130 | 130 | 131 | 135 | 128 |
| 5860465 | 38 | 35 | 45 | 33 | 32 | 37 | 34 | 31 | 35 | 32 |
| 4390576 | -348 | -362 | -360 | -383 | -349 | -346 | -354 | -350 | -366 | -362 |
| 4830255 | 185 | 201 | 184 | 208 | 174 | 206 | 186 | 177 | 186 | 202 |
| 4570403 | -406 | -422 | -427 | -449 | -416 | -423 | -415 | -410 | -422 | -433 |
| 1780719 | 33 | 69 | -21 | 74 | 32 | 27 | 59 | 64 | 70 | 33 |
| 3130477 | -34 | -41 | -45 | -49 | -84 | -62 | -53 | -31 | -51 | -79 |
| 6110630 | -478 | -476 | -513 | -525 | -493 | -476 | -469 | -473 | -446 | -508 |
| 3310309 | -344 | -350 | -340 | -366 | -352 | -335 | -342 | -337 | -363 | -351 |
| 4220731 | 137 | 146 | 224 | 170 | 140 | 157 | 153 | 167 | 149 | 151 |
| 520706 | 46 | 47 | 71 | 53 | 63 | 72 | 46 | 42 | 56 | 38 |
| 6420446 | -57 | -80 | -87 | -74 | -62 | -73 | -77 | -86 | -68 | -116 |
| 1400240 | 350 | 368 | 295 | 432 | 401 | 364 | 376 | 381 | 347 | 439 |
| 7510379 | 17 | 14 | 36 | 12 | 31 | 34 | 15 | 4 | 6 | 1 |
| 2640025 | -89 | -80 | -56 | -49 | -58 | -43 | -66 | -63 | -62 | -78 |
| 3130296 | -98 | -113 | -83 | -115 | -108 | -99 | -96 | -94 | -55 | -79 |
| 3520601 | -160 | -166 | -163 | -149 | -177 | -173 | -168 | -165 | -153 | -224 |
| 1110091 | 271 | 264 | 228 | 263 | 251 | 281 | 259 | 262 | 284 | 255 |
| 3130370 | 427 | 415 | 396 | 457 | 395 | 389 | 425 | 431 | 432 | 450 |
| 2030332 | -173 | -171 | -165 | -189 | -178 | -165 | -178 | -173 | -186 | -178 |
| 670209 | 246 | 244 | 261 | 263 | 256 | 256 | 247 | 263 | 252 | 264 |
| 6560161 | -235 | -240 | -235 | -260 | -241 | -245 | -240 | -238 | -244 | -250 |
| 1740647 | -300 | -310 | -305 | -321 | -296 | -303 | -300 | -297 | -305 | -322 |
| 4880463 | 68 | 68 | 86 | 70 | 70 | 83 | 69 | 66 | 79 | 61 |
| 990315 | -32 | -37 | -47 | -43 | -16 | -71 | -36 | -34 | -47 | -61 |
| 240463 | -72 | -69 | -65 | -61 | -63 | -63 | -56 | -67 | -32 | -22 |
| 2000035 | -298 | -304 | -289 | -326 | -309 | -297 | -298 | -293 | -308 | -309 |
| 1780709 | -58 | -53 | -115 | -60 | -65 | -27 | -61 | -70 | -50 | -95 |
| 2680440 | -184 | -187 | -191 | -203 | -184 | -183 | -188 | -189 | -186 | -198 |
| 150672 | 131 | 132 | 148 | 147 | 121 | 126 | 134 | 145 | 135 | 125 |
| 6940176 | -76 | -88 | 7 | -89 | -36 | -74 | -89 | -84 | -68 | -121 |
| 6180497 | 121 | 118 | 116 | 123 | 123 | 111 | 122 | 721 | 135 | 116 |
| 150706 | -20 | -22 | 17 | -8 | -33 | -45 | -23 | -26 | -29 | -22 |
| 5900129 | -181 | -167 | -165 | -166 | -144 | -156 | -151 | -169 | -119 | -187 |
| 6450437 | -363 | -368 | -359 | -396 | -368 | -369 | -365 | -360 | -379 | -369 |
| 3990608 | 293 | 286 | 317 | 336 | 239 | 291 | 287 | 288 | 280 | 298 |
| 3120075 | -57 | -65 | 8 | -67 | -61 | -74 | -67 | -68 | -68 | -84 |
| 1500047 | -193 | -196 | -222 | -209 | -206 | -186 | -198 | -190 | -208 | -204 |
| 840358 | -52 | -46 | -57 | -44 | -78 | -21 | -52 | -45 | -52 | -41 |
| 6590484 | -70 | -74 | -67 | -82 | -82 | -82 | -77 | -73 | -66 | -112 |
| 2100594 | -84 | -103 | -61 | -117 | -112 | -98 | -101 | -114 | -124 | -126 |
| 6650056 | 166 | 176 | 186 | 187 | 180 | 166 | 174 | 172 | 191 | 166 |
| 2120017 | 69 | 67 | 74 | 77 | 87 | 76 | 74 | 78 | 84 | 61 |
| 1780639 | -65 | -64 | -52 | -64 | -70 | -60 | -62 | -63 | -67 | -66 |
| 6650594 | -285 | -322 | -247 | -342 | -298 | -272 | -310 | -320 | -339 | -356 |
| 3420136 | -139 | -144 | -126 | -153 | -149 | -131 | -141 | -139 | -139 | -144 |
| 6760017 | -138 | -123 | -130 | -136 | -162 | -124 | -134 | -119 | -136 | -163 |
| 510209 | -66 | -60 | -126 | -63 | -44 | -70 | -56 | -66 | -80 | -14 |
| 4220673 | -191 | -199 | -199 | -210 | -175 | -195 | -188 | -185 | -191 | -198 |
| Intercept | 19978 | 20602 | 19656 | 20443 | 20673 | 19330 | 19853 | 19517 | 19384 | 23046 |

**[Table 6-2]**

| Table 6-2 | | | | | | | | | |
|---|---|---|---|---|---|---|---|---|---|
| ProbeID | S12_pre | S13_pre | S14_pre | S15_pre | S16_pre | S17_pre | S18_pre | S20_pre | S21_pre |
| 3390368 | -183 | -189 | -191 | -180 | -184 | -184 | -183 | -184 | -197 |
| 1300687 | 130 | 134 | 140 | 127 | 134 | 133 | 131 | 133 | 175 |
| 5860465 | 32 | 28 | 33 | 28 | 29 | 33 | 33 | 33 | 29 |
| 4390576 | -354 | -365 | -365 | -359 | -370 | -356 | -351 | -354 | -396 |
| 4830255 | 190 | 182 | 193 | 177 | 190 | 187 | 182 | 188 | 247 |
| 4570403 | -410 | -432 | -429 | -392 | -415 | -418 | -405 | -416 | -481 |
| 1780719 | 65 | 65 | 95 | 77 | 65 | 64 | 54 | 68 | 129 |
| 3130477 | -45 | -55 | -35 | -39 | -46 | -44 | -45 | -42 | -166 |
| 6110630 | -460 | -454 | -487 | -492 | -446 | -480 | -464 | -482 | -432 |
| 3310309 | -334 | -346 | -352 | -346 | -347 | -342 | -336 | -340 | -408 |
| 4220731 | 158 | 173 | 182 | 175 | 157 | 164 | 168 | 164 | 236 |
| 520706 | 45 | 55 | 33 | 47 | 51 | 49 | 42 | 47 | 57 |
| 6420446 | -78 | -71 | -78 | -89 | -66 | -78 | -89 | -75 | 17 |
| 1400240 | 383 | 347 | 373 | 367 | 370 | 368 | 366 | 360 | 296 |
| 7510379 | 10 | 3 | 10 | 8 | 7 | 12 | 13 | 10 | 67 |
| 2640025 | -38 | -54 | -64 | -12 | -48 | -63 | -68 | -66 | -94 |
| 3130296 | -104 | -110 | -124 | -116 | -107 | -100 | -84 | -103 | -185 |
| 3520601 | -176 | -182 | -165 | -202 | -176 | -176 | -165 | -169 | -231 |
| 1110091 | 260 | 263 | 252 | 245 | 243 | 261 | 251 | 258 | 172 |
| 3130370 | 444 | 436 | 415 | 411 | 418 | 427 | 408 | 421 | 352 |
| 2030332 | -177 | -178 | -186 | -185 | -182 | -180 | -168 | -181 | -200 |
| 670209 | 243 | 253 | 267 | 240 | 250 | 250 | 252 | 252 | 368 |
| 6560161 | -239 | -242 | -251 | -242 | -240 | -242 | -239 | -241 | -275 |
| 1740647 | -298 | -309 | -309 | -287 | -304 | -299 | -295 | -299 | -342 |
| 4880463 | 68 | 69 | 72 | 58 | 64 | 69 | 61 | 68 | 147 |
| 990315 | -29 | -32 | -25 | -39 | -32 | -34 | -43 | -29 | 4 |
| 240463 | -43 | -49 | -59 | -39 | -61 | -57 | -55 | -53 | -101 |
| 2000035 | -293 | -311 | -309 | -297 | -313 | -300 | -297 | -300 | -349 |
| 1780709 | -75 | -48 | -73 | -89 | -47 | -67 | -55 | -63 | -36 |
| 2680440 | -185 | -186 | -193 | -191 | -195 | -188 | -186 | -188 | -208 |
| 150672 | 136 | 141 | 150 | 142 | 144 | 137 | 134 | 140 | 213 |
| 6940176 | -81 | -104 | -81 | -80 | -92 | -86 | -97 | -90 | -141 |
| 6180497 | 118 | 111 | 120 | 78 | 124 | 122 | 129 | 118 | 93 |
| 150706 | -19 | -24 | -21 | 1 | -18 | -22 | -29 | -20 | -31 |
| 5900129 | -161 | -135 | -199 | -141 | -158 | -161 | -139 | -162 | -225 |
| 6450437 | -363 | -369 | -380 | -358 | -379 | -367 | -358 | -364 | -448 |
| 3990608 | 287 | 292 | 272 | 289 | 310 | 296 | 315 | 290 | 90 |
| 3120075 | -60 | -66 | -66 | -60 | -70 | -65 | -66 | -66 | -21 |
| 1500047 | -195 | -213 | -195 | -203 | -203 | -195 | -193 | -197 | -241 |
| 840358 | -51 | -36 | -52 | -61 | -30 | -45 | -37 | -47 | -50 |
| 6590484 | -81 | -92 | -84 | -91 | -70 | -79 | -93 | -80 | -218 |
| 2100594 | -112 | -114 | -114 | -137 | -118 | -109 | -130 | -101 | -141 |
| 6650056 | 173 | 181 | 184 | 171 | 177 | 175 | 176 | 174 | 234 |
| 21200177 | 79 | 79 | 73 | 102 | 78 | 72 | 80 | 76 | 117 |
| 1780639 | -62 | -65 | -67 | -60 | -61 | -62 | -63 | -62 | -78 |
| 6650594 | -312 | -331 | -332 | -311 | -330 | -310 | -300 | -319 | -270 |
| 3420136 | -140 | -146 | -146 | -145 | -147 | -142 | -139 | -140 | -185 |
| 6760017 | -128 | -108 | -113 | -114 | -137 | -122 | -113 | -126 | -20 |
| 510209 | -67 | -61 | -78 | -68 | -79 | -67 | -48 | -65 | -85 |
| 4220673 | -188 | -200 | -196 | -189 | -203 | -190 | -186 | -190 | -240 |
| Intercept | 19209 | 20247 | 20979 | 20488 | 20162 | 19945 | 19272 | 19998 | 24896 |

**[Table 6-3]**

| Table 6-3 | | | | | | | | | | |
|---|---|---|---|---|---|---|---|---|---|---|
| ProbeID | S22_pre | S23_pre | S25_pre | S26_pre | L5_pre | L6_pre | L7_pre | L8_pre | L9_pre | L10_pre |
| 3390368 | -184 | -172 | -180 | -185 | -180 | -198 | -100 | -179 | -160 | -170 |
| 1300687 | 134 | 128 | 144 | 137 | 128 | 149 | 84 | 109 | 126 | 124 |
| 5860465 | 33 | 21 | 31 | 21 | 32 | 15 | 34 | 19 | 26 | 41 |
| 4390576 | -352 | -337 | -359 | -374 | -362 | -374 | -211 | -318 | -314 | -308 |
| 4830255 | 187 | 170 | 162 | 144 | 215 | 209 | 97 | 258 | 187 | 212 |
| 4570403 | -418 | -412 | -427 | -443 | -421 | -436 | -237 | -359 | -373 | -377 |
| 1780719 | 98 | 102 | 66 | 98 | 61 | 83 | -7 | 163 | 66 | 11 |
| 3130477 | -21 | 14 | 0 | -51 | -48 | -60 | 57 | -55 | -43 | -53 |
| 6110630 | -481 | -446 | -453 | -429 | -493 | -435 | -515 | -399 | -472 | -444 |
| 3310309 | -337 | -322 | -356 | -362 | -341 | -372 | -193 | -292 | -285 | -297 |
| 4220731 | 147 | 142 | 202 | 159 | 161 | 230 | -48 | 186 | 147 | 180 |
| 520706 | 36 | 9 | 79 | 92 | 28 | 43 | 78 | 22 | 69 | 36 |
| 6420446 | -66 | -82 | -36 | -72 | -99 | -68 | -68 | -62 | -54 | -119 |
| 1400240 | 361 | 404 | 280 | 323 | 402 | 443 | 157 | 363 | 363 | 399 |
| 7510379 | 8 | 6 | 16 | 0 | 17 | 8 | -1 | -57 | 15 | 32 |
| 2640025 | -47 | -25 | -46 | -60 | -67 | -27 | -283 | 87 | -62 | -124 |
| 3130296 | -93 | -77 | -104 | -139 | -128 | -170 | 95 | -187 | -92 | -83 |
| 3520601 | -180 | -194 | -167 | -170 | -178 | -198 | -99 | -230 | -184 | -148 |
| 1110091 | 271 | 232 | 273 | 254 | 248 | 283 | 242 | 157 | 254 | 241 |
| 3130370 | 396 | 425 | 412 | 493 | 406 | 410 | 477 | 346 | 425 | 373 |
| 2030332 | -175 | -170 | -184 | -177 | -174 | -184 | -146 | -177 | -166 | -176 |
| 670209 | 244 | 232 | 243 | 261 | 273 | 256 | 109 | 198 | 239 | 213 |
| 6560161 | -235 | -228 | -253 | -251 | -246 | -260 | -133 | -227 | -220 | -214 |
| 1740647 | -287 | -279 | -314 | -325 | -312 | -325 | -157 | -273 | -255 | -266 |
| 4880463 | 66 | 75 | 75 | 76 | 63 | 83 | 36 | 79 | 67 | 50 |
| 990315 | -32 | -17 | -54 | -19 | -66 | -14 | -32 | -4 | -3 | -44 |
| 240463 | -59 | -33 | -75 | -78 | -77 | -52 | -59 | -30 | -75 | -73 |
| 2000035 | -287 | -293 | -306 | -324 | -299 | -326 | -175 | -252 | -266 | -264 |
| 1780709 | -87 | -94 | -63 | -40 | -32 | -126 | 25 | -84 | -63 | -83 |
| 2680440 | -190 | -166 | -178 | -208 | -194 | -173 | -125 | -177 | -180 | -164 |
| 150672 | 115 | 151 | 158 | 152 | 138 | 151 | 66 | 150 | 122 | 143 |
| 6940176 | -109 | -143 | -106 | -79 | -54 | -62 | -106 | -33 | -131 | -126 |
| 6180497 | 139 | 141 | 101 | 116 | 139 | 47 | 183 | 18 | 119 | 145 |
| 150706 | -20 | -14 | -31 | -26 | 11 | -24 | -73 | 51 | -13 | -77 |
| 5900129 | -159 | -154 | -168 | -158 | -173 | -190 | 47 | -266 | -164 | -123 |
| 6450437 | -367 | -333 | -376 | -399 | -373 | -404 | -231 | -313 | -333 | -314 |
| 3990608 | 286 | 249 | 360 | 292 | 275 | 286 | 214 | 334 | 316 | 334 |
| 3120075 | -65 | -66 | -42 | -74 | -61 | -52 | -73 | -87 | -62 | -82 |
| 1500047 | -173 | -196 | -189 | -177 | -196 | -195 | -39 | -222 | -153 | -194 |
| 840358 | -57 | -50 | -40 | -30 | -70 | -52 | 19 | -144 | -20 | -34 |
| 6590484 | -80 | -92 | -65 | -63 | -98 | -88 | -96 | 5 | -100 | -39 |
| 2100594 | -142 | -90 | -95 | -152 | -126 | -133 | -12 | -178 | -97 | -64 |
| 6650056 | 179 | 161 | 176 | 183 | 171 | 210 | 95 | 142 | 155 | 165 |
| 2120017 | 78 | 65 | 74 | 76 | 64 | 70 | 102 | 1 | 74 | 20 |
| 1780639 | -73 | -46 | -55 | -65 | -65 | -67 | -30 | -66 | -50 | -54 |
| 6650594 | -308 | -325 | -300 | -345 | -336 | -376 | -227 | -257 | -292 | -259 |
| 3420136 | -134 | -140 | -136 | -139 | -148 | -172 | -90 | -99 | -134 | -126 |
| 6760017 | -81 | -100 | -115 | -73 | -138 | -128 | -92 | -118 | -129 | -107 |
| 510209 | -81 | -61 | -84 | -99 | -86 | -27 | -121 | 49 | -63 | -37 |
| 4220673 | -188 | -175 | -180 | -202 | -194 | -213 | -97 | -165 | -171 | -169 |
| Intercept | 19939 | 18424 | 19115 | 20488 | 21351 | 20804 | 13062 | 17736 | 17185 | 17648 |

**[Table 6-4]**

| Table 6-4 | | | | | | | | | | |
|---|---|---|---|---|---|---|---|---|---|---|
| ProberID | L11_pre | L12_pre | L13_pre | L14_pre | L15_pre | L16_pre | L17_pre | L18_pre | L20_pre | L21_pre |
| 3390368 | -192 | -186 | -187 | -182 | -185 | -183 | -179 | -185 | -193 | -191 |
| 1300687 | 139 | 133 | 130 | 121 | 133 | 138 | 136 | 135 | 135 | 153 |
| 5860465 | 28 | 34 | 40 | 47 | 34 | 41 | 39 | 40 | 44 | 33 |
| 4390576 | -362 | -360 | -378 | -376 | -355 | -356 | -335 | -359 | -349 | -375 |
| 4830255 | 180 | 185 | 154 | 231 | 185 | 163 | 203 | 189 | 201 | 202 |
| 4570403 | -417 | -420 | -419 | -425 | -419 | -422 | -401 | -419 | -426 | -428 |
| 1780719 | 37 | 67 | 85 | 54 | 62 | 43 | 63 | 68 | 79 | 82 |
| 3130477 | -24 | -50 | -57 | -39 | -37 | -50 | -11 | -42 | -55 | -44 |
| 6110630 | -502 | -469 | -441 | -463 | -482 | -458 | -469 | -483 | -447 | -450 |
| 3310309 | -355 | -344 | -362 | -353 | -342 | -348 | -334 | -341 | -341 | -356 |
| 4220731 | 183 | 157 | 146 | 170 | 160 | 157 | 161 | 160 | 158 | 174 |
| 520706 | 18 | 55 | 74 | 41 | 52 | 28 | 43 | 51 | 32 | 56 |
| 6420446 | -62 | -78 | -89 | -47 | -78 | -75 | -83 | -82 | -100 | -96 |
| 1400240 | 336 | 368 | 368 | 332 | 366 | 369 | 383 | 375 | 385 | 367 |
| 7510379 | 20 | 15 | 31 | 17 | 14 | 20 | 21 | 9 | 11 | 20 |
| 2640025 | -10 | -70 | -109 | 25 | -58 | -51 | -55 | -70 | -25 | -54 |
| 3130296 | -108 | -100 | -102 | -102 | -100 | -85 | -97 | -111 | -99 | -134 |
| 3520601 | -169 | -169 | -153 | -165 | -176 | -170 | -148 | -170 | -177 | -133 |
| 1110091 | 275 | 260 | 299 | 273 | 260 | 264 | 254 | 254 | 271 | 268 |
| 3130370 | 454 | 421 | 398 | 377 | 425 | 430 | 433 | 426 | 441 | 411 |
| 2030332 | -172 | -178 | -175 | -184 | -180 | -170 | -181 | -183 | -180 | -178 |
| 670209 | 241 | 247 | 271 | 272 | 249 | 252 | 253 | 237 | 261 | 255 |
| 6560181 | -229 | -242 | -260 | -242 | -241 | -241 | -235 | -243 | -239 | -253 |
| 1740647 | -301 | -303 | -308 | -324 | -302 | -302 | -295 | -307 | -311 | -314 |
| 4880463 | 63 | 70 | 61 | 71 | 70 | 64 | 57 | 72 | 75 | 84 |
| 990315 | -37 | -31 | -26 | -31 | -31 | -24 | -28 | -40 | -45 | -8 |
| 240463 | -68 | -56 | -45 | -28 | -59 | -46 | -58 | -52 | -58 | -53 |
| 2000035 | -297 | -301 | -308 | -302 | -302 | -301 | -295 | -302 | -306 | -316 |
| 1780709 | -121 | -65 | -27 | -77 | -65 | -79 | -85 | -73 | -10 | -55 |
| 2680440 | -195 | -189 | -201 | -191 | -189 | -184 | -182 | -193 | -188 | -195 |
| 150672 | 123 | 137 | 135 | 107 | 137 | 140 | 144 | 151 | 150 | 168 |
| 6940176 | -62 | -91 | -94 | -80 | -85 | -96 | -71 | -77 | -63 | -54 |
| 6180497 | 92 | 121 | 119 | 145 | 121 | 138 | 138 | 77 | 170 | 134 |
| 150706 | -5 | -23 | -4 | -45 | -25 | -27 | -16 | -32 | -32 | -11 |
| 5900129 | -187 | -168 | -153 | -186 | -159 | -126 | -148 | -184 | -153 | -170 |
| 6450437 | -362 | -367 | -367 | -376 | -368 | -360 | -336 | -363 | -381 | -388 |
| 3990608 | 263 | 295 | 283 | 336 | 290 | 260 | 284 | 285 | 284 | 320 |
| 3120075 | -55 | -65 | -52 | -54 | -65 | -67 | -66 | -62 | -76 | -59 |
| 1500047 | -194 | -194 | -217 | -238 | -198 | -199 | -195 | -203 | -203 | -204 |
| 840358 | -51 | -48 | -22 | -74 | -44 | -39 | -57 | -37 | -53 | -65 |
| 6590484 | -38 | -79 | -136 | -38 | -82 | -75 | -53 | -67 | -100 | -42 |
| 2100594 | -68 | -106 | -120 | -76 | -109 | -119 | -113 | -114 | -121 | -103 |
| 6650056 | 173 | 176 | 170 | 170 | 174 | 174 | 177 | 185 | 180 | 179 |
| 2120017 | 97 | 78 | 85 | 77 | 74 | 84 | 66 | 77 | 54 | 92 |
| 1780639 | -62 | -63 | -65 | -66 | -63 | -62 | -64 | -64 | -70 | -63 |
| 6650594 | -277 | -309 | -307 | -266 | -309 | -291 | -302 | -320 | -317 | -321 |
| 3420136 | -141 | -141 | -148 | -129 | -142 | -142 | -139 | -143 | -151 | -155 |
| 6760017 | -146 | -117 | -118 | -123 | -122 | -120 | -125 | -111 | -112 | -134 |
| 510209 | -24 | -61 | -92 | -46 | -68 | -46 | -48 | -46 | -106 | -80 |
| 4220673 | -201 | -192 | -194 | -184 | -190 | -187 | -175 | -193 | -204 | -197 |
| Intercept | 19780 | 19946 | 20285 | 18703 | 20057 | 19357 | 17800 | 20390 | 19420 | 18749 |

**[Table 6-5]**

| | Table 6-5 |
|---|---|
| ProbelD | L19_pre |
| 3390368 | -195 |
| 1300687 | 125 |
| 5860465 | 21 |
| 4390576 | -385 |
| 4830255 | 161 |
| 4570403 | -435 |
| 1780719 | 83 |
| 3130477 | -14 |
| 6110630 | -519 |
| 3310309 | -348 |
| 4220731 | 206 |
| 520706 | 59 |
| 6420446 | -86 |
| 1400240 | 381 |
| 7510379 | -6 |
| 2640025 | -69 |
| 3130296 | -112 |
| 3520601 | -159 |
| 1110091 | 331 |
| 3130370 | 406 |
| 2030332 | -189 |
| 670209 | 255 |
| 6560161 | -250 |
| 1740647 | -311 |
| 4880463 | 57 |
| 990315 | -13 |
| 240463 | -97 |
| 2000035 | -291 |
| 1780709 | -68 |
| 2680440 | -193 |
| 150672 | 102 |
| 6940176 | -74 |
| 6180497 | 115 |
| 150706 | -24 |
| 5900129 | -195 |
| 6450437 | -390 |
| 3990608 | 312 |
| 3120075 | -80 |
| 1500047 | -193 |
| 840358 | -30 |
| 6590484 | -104 |
| 2100594 | -124 |
| 6650056 | 164 |
| 2120017 | 63 |
| 1780639 | -68 |
| 6650594 | -327 |
| 3420136 | -139 |
| 6760017 | -98 |
| 510209 | -81 |
| 4220673 | -195 |
| Intercept | 21296 |

Table 7: Thirthy gene sets by random selection.

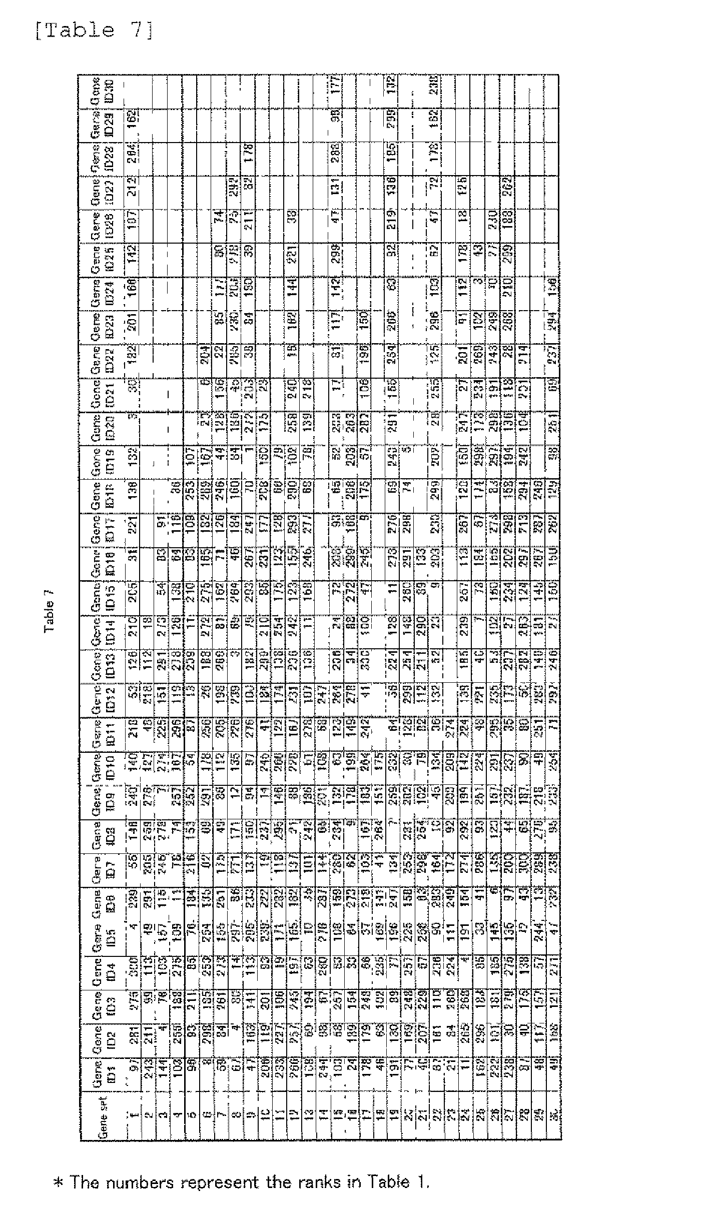

## Claims

1. A method for predicting effect of immunotherapy on a cancer patient and/or prognosis of the cancer patient after the immunotherapy, which comprises the steps of:
(1) determining expression level of each gene in a gene set which consists of at least 1 gene selected from the group of genes shown in any of Tables 1-5 and
(2) performing statistical processing of the expression level to calculate predicted survival time.

2. The method of Claim 1, wherein the statistical processing is performed by regression analysis.

3. The method of Claim 1 or 2, wherein the immunotherapy is peptide vaccine therapy.

4. The method of any one of Claims 1-3, wherein the cancer is prostate cancer.

5. A gene set for predicting effect of immunotherapy on a cancer patient and/or prognosis of the cancer patient after the immunotherapy, which consists of at least 1 gene selected from the group of genes shown in any of Tables 1-5.

6. The gene set of Claim 5, wherein said at least 1 gene is selected in descending order from the top of the group of genes shown in any of Tables 1-5.

7. The gene set of Claim 5 or 6, which consists of at least 10 genes.

8. The gene set of any one of Claims 5-7, wherein the immunotherapy is peptide vaccine therapy.

9. The gene set of any one of Claims 5-8, wherein the cancer is prostate cancer.

10. A kit for predicting effect of immunotherapy on a cancer patient and/or prognosis of the cancer patient after the immunotherapy, which comprises a probe or primer for each gene in the gene set of Claim 5.

11. The kit of Claim 10, which is used for DNA microarray analysts.

12. The kit of Claim 10 or 11, wherein the cancer is prostate cancer.
